# EUROPEAN PATENT SPECIFICATION

(11) **EP 1 054 886 B1**
(45) Date of publication and mention of the grant of the patent: **04.09.2002**
(21) Application number: 99906845.5
(22) Date of filing: 09.02.1999
(51) Int. Cl.: C07D 417/04, A61K 31/33, C07D 333/38, C07D 417/14, C07D 413/04, C07D 409/04, C07D 417/12

(54) **HETEROARYL AMIDINES, METHYLAMIDINES AND GUANIDINES AS PROTEASE INHIBITORS, IN PARTICULAR AS UROKINASE INHIBITORS**
HETEROARYL-AMIDINEN,-METHYLAMIDINEN UND -GUANIDINEN ALS PROTEASE INHIBITOREN, INSBESONDERE ALS UROKINASE INHIBITOREN
HETEROARYL AMIDINES, METHYLAMIDINES ET GUANIDINES UTILES EN TANT QU'INHIBITEURS DE PROTEASE ET PLUS PARTICULIEREMENT EN TANT QU'INHIBITEURS D'UROKINASE

(30) Priority: 09.02.1998 US 74110 P
(43) Date of publication of application: 29.11.2000
(73) Proprietor: 3-DIMENSIONAL PHARMACEUTICALS, INC., Exton, PA 19341 (US)
(72) Inventor: ILLIG, Carl, R., Phoenixville, PA 19460 (US); SUBASINGHE, Nalin, L., West Chester, PA 19380 (US); HOFFMAN, James, B., Ardmore, PA 19003 (US); WILSON, Kenneth, J., West Grove, Pennsylvania 19390 (US); RUDOLPH, M., Jonathan, Doylestown, PA 18901 (US)
(74) Representative: Schlich, George William
(86) International application number: US9902784
(87) International publication number: WO99040088

(56) References cited:
- EP-A- 0 568 289

## Description

The present invention relates to novel heteroaryl compounds that function as enzyme inhibitors, and particularly to a new class of non-peptidic inhibitors of proteolytic enzymes such as urokinase (uPa).

### Related Art

Proteases are enzymes that cleave proteins at single, specific peptide bonds. Proteases can be classified into four generic classes: serine, thiol or cysteinyl, acid or aspartyl, and metalloproteases (Cuypers *et al., J. Biol. Chem. 257*:7086 (1982)). Proteases are essential to a variety of biological activities, such as digestion, formation and dissolution of blood clots, reproduction and the immune reaction to foreign cells and organisms. Aberrant proteolysis is associated with a number of disease states in man and other mammals. The human neutrophil proteases, elastase and cathepsin G, have been implicated as contributing to disease states marked by tissue destruction. These disease states include emphysema, rheumatoid arthritis, corneal ulcers and glomerular nephritis. (Barret, in *Enzyme Inhibitors as Drugs*, Sandler, ed., University Park Press, Baltimore, (1980)). Additional proteases such as plasmin, C-1 esterase, C-3 convertase, urokinase and tissue-type plasminogen activators, acrosin, and kallikreins play key roles in normal biological functions of mammals. In many instances, it is beneficial to disrupt the function of one or more proteolytic enzymes in the course of therapeutically treating a mammal.

Serine proteases include such enzymes as elastase (human leukocyte), cathepsin G, plasmin, C-1 esterase, C-3 convertase, urokinase and tissue-type plasminogen activators, acrosin, chymotrypsin, trypsin, thrombin, factor Xa and kallikreins.

Human leukocyte elastase is released by polymorphonuclear leukocytes at sites of inflammation and thus is a contributing cause for a number of disease states. Cathepsin G is another human neutrophil serine protease. Compounds with the ability to inhibit the activity of these enzymes are expected to have an anti-inflammatory effect useful in the treatment of gout, rheumatoid arthritis and other inflammatory diseases, and in the treatment of emphysema. Chymotrypsin and trypsin are digestive enzymes. Inhibitors of these enzymes are useful in treating pancreatitis. Inhibitors of urokinase plasminogen activator are useful in treating excessive cell growth disease states, such as benign prostatic hypertrophy, prostatic carcinoma and psoriasis.

Urokinase (urinary-type plasminogen activator or uPA; International Union of Biochemistry Classification Number: EC3.4.21.31) is a proteolytic enzyme which is highly specific for a single peptide bond in plasminogen. It is a multidomain serine protease, having a catalytic "B" chain (amino acids (aa) 144-411), and an amino-terminal fragment ("ATF", aa 1-143) consisting of a growth factor-like domain (4-43) and a Kringle domain (aa 47-135). The uPA Kringle domain appears to bind heparin, but not fibrin, lysine, or aminohexanoic acid. The growth factor-like domain bears some similarity to the structure of epidermal growth factor (EGF) and is thus also referred to as "EGF-like" domain. The single chain pro-uPA is activated by plasmin, cleaving the chain into a two-chain active form that is stabilized by a disulfide bond.

Cleavage of the peptide bond in plasminogen by urokinase ("plasminogen activation") results in the formation of a potent general protease, plasmin. Many cell types use urokinase as a key initiator of plasmin-mediated proteolytic degradation or modification of extracellular support structures (e.g., the extracellular matrix (ECM) and the basement membrane (BM)). Cells exist, move, and interact with each other in tissues and organs within the physical framework provided by the ECM and BM. Movement of cells within the ECM or across the BM requires local proteolytic degradation or modification of these structures, allowing cells to "invade" into adjacent areas that were previously unavailable.

Central to the ability of urokinase to mediate cellular migration and invasiveness is the existence of specific high affinity urokinase receptors (uPARs) which concentrate urokinase on the cell surface, leading to the generation of locally high plasmin concentrations between cells and ECM or BM (Blasi, F., *et al., Cell Biol. 104*:801-804 (1987); Roldan, A.L., *et al., EMBO J. 9*:467-74 (1990)). The binding interaction is apparently mediated by the EGF-like domain (Rabbani, S.A., *et al., J. Biol. Chem. 267*:14151-56 (1992)). Cleavage of pro-uPA into active uPA is accelerated when pro-uPA and plasminogen are receptor-bound. Thus, plasmin activates pro-uPA, which in turn activates more plasmin by cleaving plasminogen. This positive feedback cycle is apparently limited to the receptor-based proteolysis on the cell surface, since a large excess of protease inhibitors is found in plasma, including α₂ antiplasmin, PAI-1 and PAI-2. High plasmin concentrations between invasive cells and ECM or BM are necessary in order to overcome inhibitory effect of these ubiquitous plasmin inhibitors. Thus, it is cell surface receptor-bound urokinase, and not simply free urokinase secreted by cells, which plays the predominant role in initiating cellular invasiveness.

Plasmin can activate or degrade extracellular proteins such as fibrinogen, fibronectin, and zymogens, including matrix metalloproteinases. Plasminogen activators thus can regulate extracellular proteolysis, fibrin clot lysis, tissue remodeling, developmental cell and smooth muscle cell migration, inflammation, and metastasis. Cellular invasiveness initiated by urokinase is central to a wide variety of normal and disease-state physiological processes (reviewed in Blasi, F., *et al., J. Cell Biol. 104*:801-804(1987);Danø, K., *et al., Adv. Cancer Res. 44*:139-266 (1985); Littlefield, B.A., *Ann. N.Y. Acad. Sci. 622*:167-175(1991); Saksela, O., *Biochim. Biophys. Acta 823*:35-65 (1985); Testa, J.E., and Quigley, J.P., *Cancer Metast. Rev. 9*:353-367 (1990)). Such processes include, but are not limited to, angiogenesis (neovascularization), bone restructuring, embryo implantation in the uterus, infiltration of immune cells into inflammatory sites, ovulation, spermatogenesis, tissue remodeling during wound repair, restenosis and organ differentiation, fibrosis, local invasion of tumors into adjacent areas, metastatic spread of tumor cells from primary to secondary sites, and tissue destruction in arthritis. Inhibitors of urokinase therefore have mechanism-based anti-angiogenic, anti-arthritic, anti-inflammatory, anti-restenotic, anti-invasive, anti-metastatic, anti-osteoporotic, anti-retinopathic (for angiogenesis-dependent retinopathies), contraceptive, and tumoristatic activities. Inhibitors of urokinase are useful agents in the treatment of a variety of disease states, including but not limited to, benign prostatic hypertrophy, prostatic carcinoma and psoriasis.

Beneficial effects of urokinase inhibitors have been reported using anti-urokinase monoclonal antibodies and certain other known urokinase inhibitors. For instance, anti-urokinase monoclonal antibodies have been reported to block tumor cell invasiveness *in vitro* (Hollas, W., *et al.*, *Cancer Res. 51*:3690-3695, (1991); Meissauer, A., *et al., Exp. Cell Res. 192*:453-459 (1991)), tumor metastasis and invasion *in vivo* (Ossowski, L., *J. Cell Biol. 107:2437-2445* (1988); Ossowski, L., *et al., J. Cancer Res. 51*:274-81 (1991)), and angiogenesis *in vivo* (Jerdan, J. A., *et al., J. Cell Biol. 115[3 Pt 2]*:402a(1991)). In addition, amiloride, a known urokinase inhibitor of only moderate potency, has been reported to inhibit tumor metastasis *in vivo* (Kellen, J.A., *et al*., *Anticancer Res. 8*:1373-1376 (1988)) and angiogenesis/capillary network information *in vitro* (Alliegro, M.A., *et al*., *J. Cell Biol. 115[3 Pt 2]*:402a (1991)).

Urokinase plays a significant role in vascular wound healing and arterial neointima formation after injury, most likely affecting cellular migration. Urokinase mediates plasmin proteolysis, which in turn promotes vascular wound-healing and associated neointima formation (Carmeliet *et al.*, *Circ. Res. 81*:829-839 (Nov. 1997), Lupu *et al.*, *Fibrinolysis 10 Supp 2*:33-35 (1996)). A viral serine proteinase inhibitor, SERP-1, has been employed to reduce plaque formation after primary balloon angioplasty in rabbits. This activity has been attributed to the inhibition by SERP-1 of cellular proteinases, such as plasmin or urokinase (Lucas *et al., Circulation 94*:2890-2900 (1996)).

A need continues for non-peptidic compounds that are potent and selective urokinase inhibitors, and which possess greater bioavailability and fewer side-effects than currently available urokinase inhibitors. Accordingly, new classes of potent urokinase inhibitors, characterized by potent inhibitory capacity and low toxicity, are potentially valuable therapeutic agents for a variety of conditions.

### Summary of the Invention

The present invention is broadly directed to the use of heteroaryl amidines, methylamidines and guanidines having Formula ***I*** (below) as protease inhibitors, preferably as urokinase inhibitors.

Compounds of the present invention exhibit anti-urokinase activity via direct, selective inhibition of urokinase, or are intermediates useful for forming compounds having such activity. Compounds of the present invention inhibit urokinase and are, therefore, useful anti-angiogenic, anti-arthritic, anti-inflammatory, anti-restenotic, anti-invasive, anti-metastatic, anti-osteoporotic, anti-retinopathic (for angiogenesis-dependent retinopathies), contraceptive, and tumoristatic treatment agents. For example, such treatment agents are useful in the treatment of a variety of disease states, including but not limited to, benign prostatic hypertrophy, prostatic carcinoma, tumor metastasis and psoriasis. Also provided are methods to inhibit extracellular proteolysis, methods to treat benign prostatic hypertrophy, prostatic carcinoma, tumor metastasis, psoriasis, arid other conditions by administering the compound of Formula ***I***.

A number of the heteroaryl amidines, methylamidines and guanidines described herein are novel compounds. Therefore, the present invention is also directed to novel compounds of Formula ***I***.

Further provided are pharmaceutical compositions comprising a compound of Formula ***I*** and one or more pharmaceutically acceptable carriers or diluents and said pharmaceutical compositions further comprising a thrombolytic agent such as tissue plasminogen activator and streptokinase.

Further provided are methods of synthesizing compounds of Formula ***I***.

### Detailed Description of the Preferred Embodiments

The present invention is directed to the use, in the manufacture of a medicament for treating a disease selected from beningn prostatic hypertrophy, prostatic carcinoma, tumor metastasis, restenosis and psoriasis, of a compound of Formula I: 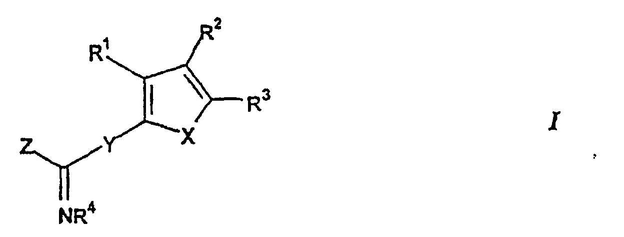 or a solvate, hydrate or pharmaceutically acceptable salt thereof; wherein:
X is sulphur;
Y is a direct covalent bond, CH₂ or NH;
Z is NR⁵R⁶, hydrogen or alkyl, provided that Y is NH whenever Z is hydrogen or alkyl;
R¹ is hydrogen, amino, hydroxy, halogen, cyano, C₁₋₄ alkyl or -CH₂R, where R is hydroxy, amino or C₁₋₃ alkoxy;
R² and R³ are independently:
   i. hydrogen,
   ii. halogen,
   iii. hydroxy,
   iv. nitro,
   v. cyano,
   vi. amino, monoalkylamino, dialkylamino, monoarylamino, diarylamino, monoalkylmonoarylamino, monoaralkylamino, diaralkylamino, alkylarylamino, alkoxycarbonylamino, aralkoxycarbonylamino, aryloxycarbonylamino, alkylsulfonylamino, aralkylsulfonylamino, arylsulfonylamino, formylamino, acylamino, H(S)CNH-, or thioacylamino,
   vii. aminocarbonyl, monoalkylaminocarbonyl, dialkylaminocarbonyl, acyl, aminoacyl, or arylaminocarbonyl,
   viii. aminothiocarbonyl, monoalkylaminothiocarbonyl, dialkylaminothiocarbonyl, thioacyl or aminothioacyl,
   ix. aminocarbonylamino, mono- and dialkylaminocarbonylamino, mono- and diarylaminocarbonylamino, or mono- and diaralkylaminocarbonylamino
   x. aminocarbonyloxy, mono- and dialkylaminocarbonyloxy, mono- and diarylaminocarbonyloxy, mono- and diaralkylaminocarbonyloxy,
   xi. aminosulfonyl, mono- and dialkylaminosulfonyl, mono- and diarylaminosulfonyl, or mono- and diaralkylaminosulfonyl,
   xii. alkoxy, or alkylthio, wherein the alkyl portion of each group may be optionally substituted,
   xiii. aralkoxy, aryloxy, aralkylthio, or arylthio, wherein the aryl portion of each group can be optionally substituted,
   xiv. alkylsulfonyl, wherein the alkyl portion can be optionally substituted,
   xv. aralkylsulfonyl, or arylsulfonyl, wherein the aryl portion of each group can be optionally substituted,
   xvi. alkenyl, or alkynyl,
   xvii. optionally substituted aryl,
   xviii. optionally substituted alkyl,
   xix. optionally substituted aralkyl,
   xx. optionally substituted heterocycle, or
   xxi. optionally substituted cycloalkyl; and
R⁴, R⁵ and R⁶ are independently hydrogen, C₁₋₄ alkyl, aryl, hydroxyalkyl, aminoalkyl, monoalkylamino(C₂₋₁₀)alkyl, dialkylamino(C₂₋₁₀)alkyl, carboxyalkyl, cyano, amino, alkoxy, or hydroxy, or -CO₂R^{w}, where
   R^{w} is alkyl, cycloalkyl, phenyl, benzyl, 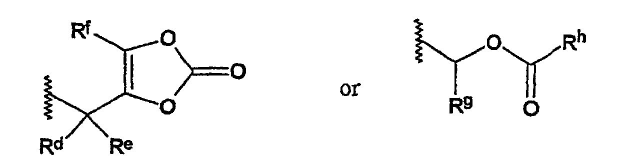 where R^{d} and R^{e} are independently hydrogen, C₁₋₆ alkyl, C₂₋₆ alkenyl or phenyl, R^{f} is hydrogen, C₁₋₆ alkyl, C₂₋₆ alkenyl or phenyl, R^{g} is hydrogen, C₁₋₆ alkyl, C₂₋₆ alkenyl or phenyl, and R^{h} is aralkyl or C₁₋₆ alkyl..

The present invention is also directed to novel compounds of Formula ***I,*** where X, Y and R¹-R⁶ are as defined above;
provided that at least one of R² or R³ is selected from the group consisting of:
(a) an optionally substituted alkyl group, preferably C₁-C₆ alkyl, more preferably C₁-C₃;
(b) alkoxy, aryloxy, alkylthio or arylthio, any of which is optionally substituted;
(c) optionally substituted C₆-C₁₄ aryl, or optionally substituted aralkyl, except that R³ is not nitrophenyl or aminophenyl, when R¹ and R² are both hydrogen or methyl;
(d) optionally substituted heterocycle; and
(e) optionally substituted cycloalkyl;
provided that the compound is not 5-(1,1-dimethylethyl)-N-hydroxy-2-thiophenethanimidamide or 5-carbamimidoyl-3,4-dimethyl-thiophene-2-carboxylic acid amide.

When an alkyl-containing group, heterocyclic-containing group or aryl-containing group of R² or R³ is optionally substituted, the optional substituents can be 1 to 4 non-hydrogen substituents, provided that the resulting compound is stable. Values of optional substituents on alkyl groups are halogen, hydroxy, thiol, amino, monoalkylamino, dialkylamino, formylamino, aminoiminomethyl, acylamino, aminoacyl, mono- or di- alkylaminocarbonyl, thiocarbonylamino, thioacylamino, aminothiocarbonyl, alkoxy, aryloxy, aminocarbonyloxy, mono- or di-alkylaminocarbonyloxy, mono- or diarylaminocarbonyloxy, mono- or diaralkylaminocarbonyloxy, alkylsulfonyl, arylsulfonyl, aralkylsulfonyl, alkylsulfonylamino, arylsulfonylamino, aralkylsulfonylamino, alkoxycarbonylamino, aralkoxycarbonylamino, aryloxycarbonylamino, mono- or di- alkylaminothiocarbonyl, aralkoxy, carboxy, carboxyalkyl, alkoxycarbonyl, alkoxycarbonylalkyl, nitro, cyano, trifluoromethyl, alkylthio and arylthio.

Preferred values of optional substituents on an alkyl group are chloro, hydroxy, amino, mono(C₁₋₄)alkylamino, di(C₁₋₄)alkylamino, formylamino, C₂₋₆ acylamino, aminocarbonyl, C₂₋₈ aminoacyl, C₁₋₆ alkoxy, C₆₋₁₄ aryloxy, carboxy, carboxy(C₁₋₆)alkyl, C₂₋₈ alkoxycarbonyl, nitro, cyano, trifluoromethyl, C₁₋₆ alkylthio, C₆₋₁₄ arylthio, C₁₋₆ alkylsulfonylamino, C₇₋₁₅ aralkylsulfonylamino, C₆₋₁₀ arylsulfonylamino, mono- or di(C₁₋₆)alkylaminocarbonyloxy, mono- or di- (C₆₋₁₀)arylaminocarbonyloxy, mono- or di(C₇₋₁₅)aralkylcarbonyloxy, C₁₋₆ alkoxycarbonylamino, C₇-C₁₅ aralkoxycarbonylamino, and C₆-C₁₀ aryloxycarbonylamino.

Preferred values of optional substituents on aryl-containing and heterocyclic-containing groups include chloro, hydroxy, amino, mono(C₁₋₄) alkylamino, di(C₁₋₄)alkylamino, formylamino, C₂₋₆ acylamino, aminocarbonyl, C₂₋₈ aminoacyl, C₃₋₇ cycloalkyl, C₁₋₆ alkyl, C₁₋₆ alkoxy, C₆₋₁₄ aryloxy, carboxy, carboxy(C₁₋₆)alkyl, C₂₋₈ alkoxycarbonyl, nitro, cyano, trifluoromethyl, C₁₋₆ alkylthio, C₆₋₁₄ arylthio, C₆₋₁₄ aryl, substituted phenyl, tetrazolyl, thienyl (further optionally substituted by one, two or three of chloro, hydroxy, C₁₋₄ alkyl, C₁₋₄ alkoxy, amino or carboxy), 3,4-methylenedioxy, 3,4-ethylenedioxy, 3,4-propylenedioxy, C₁₋₆ alkylsulfonylamino, C₇₋₁₅ aralkylsulfonylamino, C₁₋₆ arylsulfonylamino, mono- or di(C₁₋₆)alkylaminocarbonyloxy, mono- or di- C₆₋₁₀ arylaminocarbonyloxy, mono- or di-(C₇₋₁₅)aralkylcarbonyloxy, C₁₋₆ alkoxycarbonylamino, C₇-C₁₅ aralkoxycarbonylamino, C₆-C₁₀ aryloxycarbonylamino, C₂₋₆ thioacylamino, aminothiocarbonyl, and C₂₋₈ aminothioacyl.

A first preferred group of compounds falling within the scope of the present invention include compounds of Formula ***I*** wherein X is sulfur ; Y is a covalent bond or -NH-; R¹ is hydrogen, amino, hydroxy or halogen; R⁴, R⁵ and R⁶ are independently hydrogen, C₁₋₄ alkyl, amino, cyano, C₁₋₄ alkoxy or hydroxy, and are preferably all hydrogen; one of R² or R³ is hydrogen, C₁₋₆ alkyl (optionally substituted with hydroxy, amino, carboxy or aminocarbonyl), C₁₋₆ alkylthio or C₁₋₆ alkoxy; and the other of R² or R³ is aminoacyl, acylamino, aminosulfonyl, sulfonylamino, aminocarbonylamino, alkoxycarbonylamino, optionally substituted oxazolyl, optionally substituted isoxazolyl, optionally substituted benzothienyl, optionally substituted furanyl, optionally substituted pyrazolyl or optionally substituted pyridyl.

Preferred values of R¹ include hydrogen, amino, hydroxy and fluoro.

A preferred value of R² is Formula ***II*** (see below) where Ar is phenyl, thiazolyl, thiazolinyl, oxazolyl, isothiazolyl, isoxazolyl, imidazolyl, pyridyl, pyrimidinyl, pyrazinyl, thienyl (thiophenyl), pyrrolyl, oxazolinyl and benzothienyl.

Preferred values of R³ include C₁₋₄ alkyl (optionally substituted), halogen, amino, acylamino, C₁₋₆ alkylthio, (such as methylthio or ethylthio) C₁₋₆ alkoxy (such as methoxy and ethoxy) trifluoromethyl, methylsulfonyl, and benzylthio.

A preferred value of X is divalent sulfur (S).

Preferred values of Y are a covalent bond or ―NH―, most preferably a covalent bond.

Preferred values of R⁴, R⁵ and R⁶ in Formula ***I*** are hydrogen, hydroxy, cyano, C₁₋₆ alkyl, or C₁₋₆ alkoxy. Suitable values of R⁴, R⁵ and R⁶ include hydrogen, methyl, ethyl, propyl, *n*-butyl, hydroxy, methoxy, and ethoxy. In the most preferred embodiments, R⁴, R⁵ and R⁶ are each hydrogen.

Preferred values of R⁴, R⁵ and R⁶ in Formula ***I*** also include prodrugs such as -CO₂R^{w}, where R^{w}, in each instance, is preferably one of C₁₋₄alkyl, C₄₋₇cycloalkyl or benzyloxycarbonyl. Suitable values of R⁴, R⁵ and R⁶ include hydrogen, methyl, ethyl, propyl, *n*-butyl, hydroxy, methoxy, ethoxy, cyano, -CO₂CH₃, -CO₂CH₂CH₃ and -CO₂CH₂CH₂CH₃. In the most preferred embodiments, R⁴, R⁵ and R⁶ are each hydrogen.

Also preferred at R⁴, R⁵ and R⁶ is the group -CO₂R^{w}, where R^{w} is one of 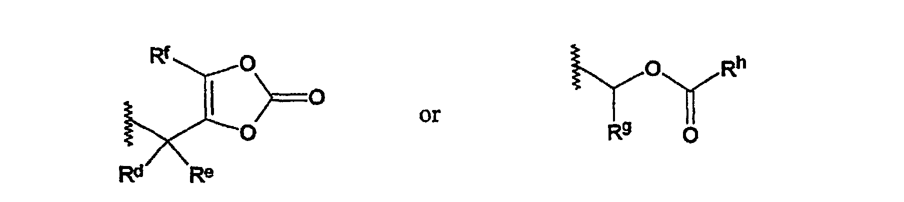 where R^{d}-R^{h} are defined as above. When R⁴, R⁵ and R⁶ are -CO₂R^{w}, where R^{w} is one of one of these moieties, the resulting compounds are prodrugs that possess desirable formulation and bioavailability characteristics. A preferred value for each of R^{d}, R^{e} and R^{g} is hydrogen, R^{f} is methyl, and preferred values for R^{h} include benzyl and *tert*-butyl.

Preferred values of R⁷ include hydrogen, C₁₋₆ alkyl, and C₆₋₁₀ ar(C₁₋₄) alkyl, C₂₋₆ hydroxyalkyl. Suitable values are hydrogen, methyl, ethyl, benzyl.

The term "alkyl" as employed herein by itself or as part of another group refers to both straight and branched chain radicals of up to 12 carbons, such as methyl, ethyl, propyl, isopropyl, butyl, *t*-butyl, isobutyl, pentyl, hexyl, isohexyl, heptyl, 4,4-dimethylpentyl, octyl, 2,2,4-trimethylpentyl, nonyl, decyl, undecyl, dodecyl.

The term "alkenyl" is used herein to mean a straight or branched chain radical of 2-20 carbon atoms, unless the chain length is limited thereto, including, but not limited to, ethenyl, 1-propenyl, 2-propenyl, 2-methyl-1-propenyl, 1-butenyl, 2-butenyl, and the like. Preferably, the alkenyl chain is 2 to 10 carbon atoms in length, more preferably, 2 to 8 carbon atoms in length most preferably from 2 to 4 carbon atoms in length.

The term "alkynyl" is used herein to mean a straight or branched chain radical of 2-20 carbon atoms, unless the chain length is limited thereto, wherein there is at least one triple bond between two of the carbon atoms in the chain, including, but not limited to, acetylene, 1-propylene, 2-propylene, and the like. Preferably, the alkynyl chain is 2 to 10 carbon atoms in length, more preferably, 2 to 8 carbon atoms in length, most preferably from 2 to 4 carbon atoms in length.

In all instances herein where there is an alkenyl or alkynyl moiety as a substituent group, the unsaturated linkage, i.e., the vinylene or acetylene linkage is preferably not directly attached to a nitrogen, oxygen or sulfur moiety.

The term "alkylthio" as employed herein by itself or as part of another group refers to a straight or branched chain radical of 1 to 20 carbon atoms, unless the chain length is limited thereto, bonded to a sulfur atom, including, but not limited to, methylthio, ethylthio, *n*-propylthio, isopropylthio, and the like. Preferably the alkylthio chain is 1 to 10 carbon atoms in length, more preferably 1 to 8 carbon atoms in length.

The term "alkoxy" as employed herein by itself or as part of another group refers to a straight or branched chain radical of 1 to 20 carbon atoms, unless the chain length is limited thereto, bonded to an oxygen atom, including, but not limited to, methoxy, ethoxy, *n*-propoxy, isopropoxy, and the like. Preferably the alkoxy chain is 1 to 10 carbon atoms in length, more preferably 1 to 8 carbon atoms in length.

The term "cycloalkyl" as employed herein by itself or as part of another group refers to cycloalkyl groups containing 3 to 9 carbon atoms. Typical examples are cyclopropyl, cyclobutyl, cyclopentyl, cyclohexyl, cycloheptyl, cyclooctyl and cyclononyl.

The term "halogen" or "halo"as employed herein by itself or as part of another group refers to chlorine, bromine, fluorine or iodine with chlorine being preferred.

The term "acyl" as employed herein by itself or as part of another group refers to the group -C(O)R^{g} where R^{g} is alkyl, alkenyl, alkynyl, aryl, or aralkyl. Preferred acyl groups are alkanoyl, aralkanoyl and aroyl groups (-C(O)R^{g} where R^{g} is C₁₋₈ alkyl, C₆₋₁₀ aryl(C₁₋₄)alkyl or C₆₋₁₀ aryl).

The term "thioacyl" as employed herein by itself or as part of another group refers to the group -C(S)R^{g} where R^{g} is alkyl, alkenyl, alkynyl, aryl or aralkyl, preferably C₁₋₈ alkyl.

The term "thiocarbonyl" as employed herein by itself or as part of another group refers to the group -C(S)-.

The term "monoalkylamine" as employed herein by itself or as part of another group refers to an amino group which is substituted with one alkyl group having from 1 to 6 carbon atoms.

The term "dialkylamine" as employed herein by itself or as part of another group refers to an amino group which is substituted with two alkyl groups, each having from 1 to 6 carbon atoms

The term "aryl" as employed herein by itself or as part of another group refers to monocyclic or bicyclic aromatic groups containing from 6 to 14 carbons in the ring portion, preferably 6-10 carbons in the ring portion, such as phenyl, naphthyl or tetrahydronaphthyl.

The term "aralkyl" or "arylalkyl" as employed herein by itself or as part of another group refers to C₁₋₆alkyl groups as discussed above having an aryl substituent, such as benzyl, phenylethyl or 2-naphthylmethyl.

The terms "heterocyclic," "heterocyclo" or "heterocycle" as employed herein by themselves or as part of larger groups refers to a saturated or wholly or partially unsaturated 3-7 membered monocyclic, or 7-10 membered bicyclic ring system, which consists of carbon atoms and from one to four heteroatoms independently selected from the group consisting of O, N, and S, wherein the nitrogen and sulfur heteroatoms can be optionally oxidized, the nitrogen can be optionally quaternized, and including any bicyclic group in which any of the above-defined heterocyclic rings is fused to a benzene ring, and wherein the heterocyclic ring can be substituted on carbon or on a nitrogen atom if the resulting compound is stable. Especially useful are rings containing one oxygen or sulfur, one to three nitrogen atoms, or one oxygen or sulfur combined with one or two nitrogen atoms. Examples of such heterocyclic groups include piperidinyl, piperazinyl, 2-oxopiperazinyl, 2-oxopiperidinyl, 2-oxopyrrolodinyl, 2-oxoazepinyl, azepinyl, pyrrolyl, 4-piperidonyl, pyrrolidinyl, pyrazolyl, pyrazolidinyl, imidazolyl, imidazolinyl, imidazolidinyl, pyridyl, pyrazinyl, pyrimidinyl, pyridazinyl, oxazolyl, oxazolidinyl, isoxazolyl, isoxazolidinyl, morpholinyl, thiazolyl, thiazolidinyl, isothiazolyl, quinuclidinyl, isothiazolidinyl, indolyl, quinolinyl, isoquinolinyl, benzimidazolyl, thiadiazoyl, benzopyranyl, benzothiazolyl, benzoxazolyl, furyl, tetrahydrofuryl, tetrahydropyranyl, thienyl, benzothienyl, thiamorpholinyl, thiamorpholinyl sulfoxide, thiamorpholinyl sulfone, and oxadiazolyl. Morpholino is the same as morpholinyl.

The term "heteroatom" is used herein to mean an oxygen atom ("O"), a sulfur atom ("S") or a nitrogen atom ("N"). It will be recognized that when the heteroatom is nitrogen, it may form an NR^{y}R^{z} moiety, wherein R^{y} and R^{z} are, independently from one another, hydrogen or C₁ to C₈ alkyl, or together with the nitrogen to which they are bound, form a saturated or unsaturated 5-, 6-, or 7-membered ring.

The term "heteroaryl" as employed herein refers to groups having 5 to 14 ring atoms; 6, 10 or 14 π electrons shared in a cyclic array; and containing carbon atoms and 1, 2 or 3 oxygen, nitrogen or sulfur heteroatoms (where examples of heteroaryl groups are: thienyl, benzo[b]thienyl, naphtho[2,3-b]thienyl, thianthrenyl, furyl, pyranyl, isobenzofuranyl, benzoxazolyl, chromenyl, xanthenyl, phenoxathiinyl, 2*H*-pyrrolyl, pyrrolyl, imidazolyl, pyrazolyl, pyridyl, pyrazinyl, pyrimidinyl, pyridazinyl, indolizinyl, isoindolyl, 3*H*-indolyl, indolyl, indazolyl, purinyl, 4*H*-quinolizinyl, isoquinolyl, quinolyl, phthalazinyl, naphthyridinyl, quinazolinyl, cinnolinyl, pteridinyl, 4α*H*-carbazolyl, carbazolyl, β-carbolinyl, phenanthridinyl, acridinyl, perimidinyl, phenanthrolinyl, phenazinyl, isothiazolyl, phenothiazinyl, isoxazolyl, furazanyl and phenoxazinyl groups).

The expression "prodrug" denotes a derivative of a known direct acting drug, which derivative has enhanced delivery characteristics and therapeutic value as compared to the drug, and is transformed into the active drug by an enzymatic or chemical process. Useful prodrugs are those where R⁴, R⁵ and/or R⁶ are -CO₂R^{w}, where R^{w} is defined above. *See,* U.S. Patent No. 5,466,811 and Saulnier *et al.*, *Bioorg. Med. Chem. Lett. 4*:1985-1990 (1994).

The term "substituted", as used herein, means that one or more hydrogens of the designated moiety are replaced with a selection from the indicated group, provided that no atom's normal valency is exceeded, and that the substitution results in a stable compound. When a substituent is keto (i.e., =O), then 2 hydrogens attached to an atom of the moiety are replaced.

By "stable compound" or "stable formula" is meant herein a compound that is sufficiently robust to survive isolation to a useful degree of purity from a reaction mixture and formulation into an efficacious therapeutic agent.

Specific compounds within the scope of the invention include the compounds described in the Examples, such as the following:
4-[4-(4-chlorophenyl)thiazol-2-yl]-5-methylthiothiophene-2-carboxamidine;
4-phenyl-5-methylthiothiophene-2-carboxamidine;
4-[4-(2,4-dichlorophenyl)thiazol-2-yl]-5-methylthiothiophene-2-carboxamidine;
4-(4-methylthiazol-2-yl)-5-methylthiothiophene-2-carboxamidine;
methyl 4-[4-(4-phenylphenyl)thiazol-2-yl]-5-methylthiothiophene-2-carboxylate;
4-[4-(3-methoxyphenyl)thiazol-2-yl]-5-methylthiothiophene-2-carboxamidine,
4-[4-(3-hydroxyphenyl)thiazol-2-yl]-5-methylthiothiophene-2-carboxamidine,
4-(4-phenylthiazol-2-yl)-5-methylthiothiophene-2-carboxamidine,
4-[4-(4-nitrophenyl)thiazol-2-yl]-5-methylthiothiophene-2-carboxamidine,
4-[4-(3,4-ethylenedioxyphenyl)thiazol-2-yl]-5-methylthiothiophene-2-carboxamidine,
4-[4-(3,4-propylenedioxyphenyl)thiazol-2-yl]-5-methylthiothiophene-2-carboxamidine,
4-[4-(4-(naphth-2-yl)thiazol-2-yl]-5-methylthiothiophene-2-carboxamidine,
4-isopropylsulfonyl-5-methylthiothiophene-2-carboxamidine;
4-phenyl-5-methylthiothiophene-2-carboxamidine;
4-[4-(4-chlorophenyl)thiazol-2-yl]-5-methylthiothiophene-2-carboxamidine;
4-[4-(4-phenylphenyl)thiazol-2-yl]-5-methylthiothiophene-2-carboxamidine;
4-[4-(4-methoxyphenyl)thiazol-2-yl]-5-methylthiothiophene-2-carboxamidie;
4-(2-naphthylthiazol-2-yl)-5-methylthiothiophene-2-carboxamidine;
4-[4-(4-chloro-3-methylphenyl)thiazol-2-yl]-5-methylthiothiophene-2-carboxamidine;
4-(5-methyl-4-phenylthiazol-2-yl)-5-methylthiothiophene-2-carboxamidine;
4-[4-(4-chloro-3-nitrophenyl)thiazol-2-yl]-5-methylthiothiophene-2-carboxamidine;
4-(5-phenyloxazol-2-yl)-5-methylthiothiophene-2-carboxamidine;
4-[4-(3-fluoro-5-trifluoromethylphenyl)-5-methylthiazol-2-yl]-5-methylthiothiophene-2-carboxamidine;
4-[4-(3,5-bis(trifluoromethyl)phenyl)-5-methyl-thiazol-2-yl]-5-methylthiothiophene-2-carboxamidine;
4-[4-(3-fluoro-5-trifluoromethylphenyl)thiazol-2-yl]-5-methylthiothiophene-2-carboxamidine;
4-[4-(3-bromophenyl)thiazol-2-yl]-5-methylthiothiophene-2-carboxamidine;
4-[4-(3,4-methylenedioxyphenyl)thiazol-2-yl]-5-methylthiothiophene-2-carboxamidine;
4-[4-(4-methylphenyl)thiazol-2-yl]-5-methylthiothiophene-2-carboxamidine;
4-[4-(3,5-bis(trifluoromethyl)phenyl)thiazol-2-yl]-5-methylthiothiophene-2-carboxamidine;
4-[4-(2-methoxyphenyl)thiazol-2-yl]-5-methylthiothiophene-2-carboxamidine;
4-(4-phenylimidazol-2-yl)-5-methylthiothiophene-2-carboxamidine;
4-[4-(2,4-dimethoxyphenyl)thiazol-2-yl]-5-methylthiothiophene-2-carboxamidine;
4-(4-benzylthiazol-2-yl)-5-methylthiothiophene-2-carboxamidine;
4-[4-(3,4-dichlorophenyl)thiazol-2-yl]-5-methylthiothiophene-2-carboxamidine;
4-[4-(3-methylphenyl)thiazol-2-yl]-5-methylthiothiophene-2-carboxamidine;
4-[4-(3,5-dimethoxyphenyl)thiazol-2-yl]-5-methylthiothiophene-2-carboxamidine;
4-[4-(2-methylphenyl)thiazol-2-yl]-5-methylthiothiophene-2-carboxamidine;
4-[4-(2,5-dimethoxyphenyl)thiazol-2-yl]-5-methylthiothiophene-2-carboxamidine;
4-(4,5-diphenylthiazol-2-yl)-5-methylthiothiophene-2-carboxamidine;
4-(2-phenyl)thiazol-4-yl-5-methylthiothiophene-2-carboxamidine;
4-[4-(2-chloro-3-pyridyl)thiazol-2-yl]-5-methylthiothiophene-2-carboxamidine;
4-[4-(phenoxymethyl)thiazol-2-yl]-5-methylthiothiophene-2-carboxamidine;
4-(4-cyclohexylthiazol-2-yl)-5-methylthiothiophene-2-carboxamidine;
4-[4-(4-chlorophenyl)thiazol-2-yl]-5-methylthiothiophene-2-carboxamidine;
4-[4-(2-hydroxyphenyl)thiazol-2-yl]-5-methylthiothiophene-2-carboxamidine;
4-[4-(3-trifluoromethoxyphenyl)thiazol-2-yl]-5-methylthiothiophene-2-carboxamidine;
4-[4-(2-chloro-4-pyridyl)thiazol-2-yl]-5-methylthiothiophene-2-carboxamidine;
4-(5-phenyl-2-pyridyl)-5-methylthiothiophene-2-carboxamidine;
4-[2-(2-chlorophenylamino)thiazol-4-yl]-5-methylthiothiophene-2-carboxamidine;
4-[2-(3-methoxyphenylamino)thiazol-4-yl]-5-methylthiothiophene-2-carboxamidine;
4-[2-(phenylamino)thiazol-4-yl]-5-methylthiothiophene-2-carboxamidine;
4-[2-(2,5-dimethoxyphenylamino)thiazol-4-yl]-5-methylthiothiophene-2-carboxamidine;
4-(2-aminothiazol-4-yl)-5-methylthiothiophene-2-carboxamidine;
4-[2-(4-chloro-2-methylphenylamino)thiazol-4-yl]-5-methylthiothiophene-2-carboxamidine;
4-[2-(4-dimethylaminophenylamino)thiazol-4-yl]-5-methylthiothiophene-2-carboxamidine;
4-[2-(4-methoxyphenylamino)thiazol-4-yl]-5-methylthiothiophene-2-carboxamidine;
4-[4-(4-hydroxy-3-methoxyphenyl)thiazol-2-yl]-5-methylthiothiophene-2-carboxamidine;
4-[4-(3-hydroxy-4-methoxyphenyl)thiazol-2-yl]-5-methylthiothiophene-2-carboxamidine;
4-[2-(2-fluorophenylamino)thiazol-4-yl]-5-methylthiothiophene-2-carboxamidine;
4-[2-(2,4,5-trimethylphenyl)aminothiazol-4-yl]-5-methylthiothiophene-2-carboxamidine;
4-[2-(3-chloro-2-methylphenyl)aminothiazol-4-yl]-5-methylthiothiophene-2-carboxamidine;
4-[2-(2-isopropylphenyl)aminothiazol-4-yl]-5-methylthiothiophene-2-carboxamidine;
4-[2-(4-benzyloxyphenyl)aminothiazol-4-yl]-5-methylthiothiophene-2-carboxamidine;
4-[2-(2-bromophenyl)aminothiazol-4-yl]-5-methylthiothiophene-2-carboxamidine;
4-[2-(2,5-dichlorophenyl)aminothiazol-4-yl]-5-methylthiothiophene-2-carboxamidine;
4-[2-(2-bromo-4-methylphenyl)aminothiazol-4-yl]-5-methylthiothiophene-2-carboxamidine;
4-[2-(2,3-dichlorophenyl)aminothiazol-4-yl]-5-methylthiothiophene-2-carboxamidine;
4-[2-(3,4,5-trimethoxyphenyl)aminothiazol-4-yl]-5-methylthiothiophene-2-carboxamidine;
4-[2-(2-piperidinylethyl)aminothiazol-4yl]-5-methylthiothiophene-2-carboxamidine;
4-[2-(4-methylphenyl)aminothiazol-4-yl]-5-methylthiothiophene-2-carboxamidine;
4-(4-phenyloxazol-2-yl)-5-methylthiothiophene-2-carboxaidine;
4-[2-(diphenylmethyl)aminothiazol-4-yl]-5-methylthiothiophene-2-carboxamidine; and
4-[2-(3-phenylpropyl)aminothiazol-4-yl]-5-methylthiothiophene-2-carboxamidine,
as well as pharmaceutically acceptable salts thereof, for example the hydrochloride, hydrobromide and acetate salts thereof.

A second preferred group of compounds falling within the scope of the present invention include compounds of Formula ***I*** wherein X is sulfur; Y is a covalent bond or -NH-; Z is NR⁵R⁶; R¹ is hydrogen, amino, hydroxy or halogen; R⁴, R⁵ and R⁶ are independently hydrogen, C₁₋₄ alkyl, amino, C₁₋₄ alkoxy or hydroxy, and are preferably all hydrogen; one of R² or R³ is hydrogen, C₁₋₆ alkylthio, C₁₋₆ alkyl optionally substituted with OH, NH_{2,} COOH or aminocarbonyl, or C₁₋₆ alkoxy; and the other of R² or R³ is: 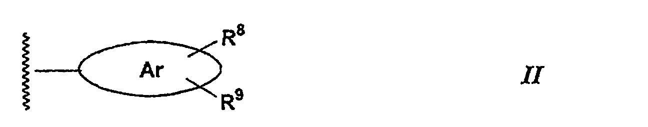 where;
Ar is a group selected from the group consisting of phenyl, thiazolyl, thiazolinyl, oxazolyl, isothiazolyl, isoxazolyl, furanyl, imidazolyl, pyridyl, pyrimidinyl, pyrazinyl, thienyl (thiophenyl), tetrazolyl, pyrrolyl, pyrazolyl, oxadiazolyl, oxazolinyl, isoxazolinyl, imidazolinyl, triazolyl, pyrrolinyl, benzothiazolyl, benzothienyl, benzimidazolyl, 1,3-oxazolidin-2-onyl, imidazolin-2-onyl, preferably phenyl, thiazolyl, thiazolinyl, oxazolinyl, isothiazolyl, isoxazolyl, imidazolyl, pyridyl, pyrimidinyl, thienyl, pyrrolyl, oxazolinyl and benzothienyl, any of which can optionally include an exocyclic = O or = NR^{V} group, where R^{V} is alkyl, aryl, aralkyl, alkylamino, arylimino or aralkylimino; and
R⁸ and R⁹ are independently selected from the group consisting of hydrogen, halogen, amino, mono(C₁₋₄)alkylamino, di(C₁₋₄)alkylamino, arylamino, mono- and di- (C₆₋₁₄)arylamino, mono- and di-(C₆₋₁₄)ar(C₁₋₆)alkylamino, formylamino, C₂₋₆ acylamino, aminocarbonyl, C₂₋₈ aminoacyl, C₂₋₆ thioacylamino, aminothiocarbonyl, C₂₋₈ aminothioacyl, C₁₋₆ alkyl, C₃₋₈ cycloalkyl, C₁₋₆ alkoxy, carboxy, carboxy(C₁₋₆)alkyl, C₂₋₈ alkoxycarbonyl, nitro, cyano, trifluoromethyl, thiazolyl, thiazolinyl, oxazolyl, isothiazolyl, isoxazolyl, furanyl, imidazolyl, pyridyl, pyrimidinyl, pyrazinyl, thienyl (thiophenyl), tetrazolyl, pyrrolyl, pyrazolyl, oxadiazolyl, oxazolinyl, isoxazolinyl, imidazolinyl, triazolyl, pyrrolinyl, benzothiazolyl, benzothienyl, benzimidazolyl, 1,3-oxazolidin-2-onyl, imidazolin-2-onyl, C₆₋₁₄ aryloxy, C₁₋₆ alkylthio, C₆₋₁₄ arylthio, C₆₋₁₄ aryl, C₆₋₁₄ ar(C₁₋₆)alkyl (wherein the aforementioned heteroaryl groups and the aryl portions of C₆₋₁₄ aryloxy, mono- and di C₆₋₁₄ aryl amino, mono- and di- C₆₋₁₄ ar(C₁₋₆)alkylamino, C₆₋₁₄ arylthio, C₆₋₁₄ ar(C₁₋₆)alkyl, and C₆₋₁₄ aryl can be further optionally substituted, preferably by one, two or three of halogen, hydroxy, amino, mono(C₁₋₄)alkylamino, di(C₁₋₄)alkylamino, formylamino, C₁₋₄acylamino, C₁₋₄aminoacyl, mono- or di-(C₁₋₄)alkylaminocarbonyl, thiocarbonylamino, C₁₋₄thioacylamino, aminothiocarbonyl, C₁₋₄alkoxy, C₆₋₁₀aryloxy, aminocarbonyloxy, mono- or di(C₁₋₄)alkylaminocarbonyloxy, mono- or di(C₆₋₁₀)arylaminocarbonyloxy, mono- or di(C₇₋₁₅)aralkylaminocarbonyloxy, C₁₋₄alkylsulfonyl, C₆₋₁₀arylsulfonyl, (C₇₋₁₅)aralkylsulfonyl, C₁₋₄alkylsulfonylamino, C₆₋₁₀arylsulfonylamino, (C₇₋₁₅)aralkylsulfonylamino, aminosulfonyl, mono- and di-alkylaminosulfonyl, mono- and di-arylaminosulfonyl, mono- and di-aralkylaminosulfonyl, C₁₋₄alkoxycarbonylamino, C₇₋₁₅aralkoxycarbonylamino, C₆₋₁₀aryloxycarbonylamino, mono- or di-(C₁₋₄)alkylaminothiocarbonyl, C₇₋₁₅aralkoxy, carboxy, carboxy(C₁₋₄)alkyl, C₁₋₄alkoxycarbonyl, C₁₋₄alkoxycarbonylalkyl, carboxy(C₁₋₄)alkoxy, alkoxycarbonylalkoxy, nitro, cyano, trifluoromethyl, C₁₋₄alkylthio and C₆₋₁₀arylthio, or by 3,4-methylenedioxy, 3,4-ethylenedioxy, and 3,4-propylenedioxy.

Preferred values of R⁸ and R⁹ are halogen, C₁₋₆ alkyl, C₁₋₆ alkoxy, hydroxy, nitro, trifluoromethyl, C₆₋₁₀ aryl (further optionally substituted by one or two of chloro, halogen, C₁₋₆ alkyl, C₁₋₆ alkoxy, hydroxy, nitro, trifluoromethyl, carboxy, C₁₋₆ alkyl, 3,4-methylenedioxy, 3,4-ethylenedioxy, 3,4-propylenedioxy, amino), 4-phenylphenyl (biphenyl), C₁₋₆ aminoalkyl, carboxy, C₁₋₆ alkyl, 3,4-methylenedioxy, 3,4-ethylenedioxy, 3,4-propylenedioxy, amino, C₁₋₆ alkanoylamino, C₆₋₁₄ aroylamino, C₁₋₆ hydroxyalkyl, thienyl (further optionally substituted by one or two of chloro, amino, methyl, methoxy, or hydroxy) and tetrazolyl. More preferably, R² is thienyl, oxazolyl, or thiazolyl, optionally substituted by any of the aforementioned groups.

Examples of preferred R⁸ and R⁹ groups include 4-chlorophenyl, 2,4-dichlorophenyl, methyl, 4-nitrophenyl, 3-nitrophenyl, 4-methoxyphenyl, 3-methoxyphenyl, 2-methoxyphenyl, 3-(2,4-dimethylthien-5-yl)phenyl, 3-hydroxyphenyl, 5-(carboxymethyl)thien-2-yl, phenyl, 3,4-ethylenedioxyphenyl, 3,4-propylenedioxyphenyl, naphth-2-yl, 3-phenyl-4-(tetrazol-5-yl)phenyl, 2,4-dichlorophenyl), 4-phenylphenyl, 3-methoxyphenyl, 3-hydroxyphenyl, 3-phenylphenyl, phenylthiomethyl, 2-chloro-4,5-dimethoxyphenyl, 4-chloro-3-methylphenyl, 5-methyl-4-phenyl, 4-chloro-3-nitrophenyl, 3-fluoro-5-trifluoromethylphenyl, 3,5-bis(trifluoromethyl), 3-fluoro-5-trifluoromethylphenyl, 3-bromophenol, 3,4-methylenedioxyphenyl, 4-methylphenyl, 3-methylphenyl, 3,5-bis(trifluoromethyl)phenyl, 2-methoxyphenyl, 6-phenyl-2-pyridyl, 2,4-dimethoxyphenyl, 3,4-dimethoxyphenyl, benzyl, 3,4-dichlorophenyl, 3-methylphenyl, 3,5-dimethoxyphenyl, 2-methylphenyl, 2,5-dimethoxyphenyl, 2-chloro-3-pyridyl, phenoxymethyl, cyclohexyl, 2-hydroxyphenyl, 3-trifluoromethoxyphenyl, 2-chloro-4-pyridyl, 3-chloro-4-pyridyl, 2-chlorophenylamino, 3-methoxyphenylamino, phenylamino, 2,5-dimethoxyphenylamino, amino, 4-chloro-2-methylphenylamino, 4-dimethylaminophenylamino, 4-methoxyphenylamino, 4-hydroxy-3-methoxyphenyl, 3-hydroxy-4-methoxyphenyl, 2-fluorophenylamino, 2,4,5-trimethylphenylamino, 3-chloro-2-methylphenylamino, 2-isopropylphenylamino, 4-benzyloxyphenylamino, 2-bromophenylamino, 2,5-dichlorophenylamino, 2-bromo-4-methylphenylamino, 2,3-dichlorophenylamino, 3,4,5-trimethoxyphenylamino, 2-piperidinylethylamino, 4-methylphenylamino, 2-thienyl, 2-5,6,7,8-tetrahydronaphthyl, 3-(2-phenoxyacetic acid)phenyl, 2-(2-phenoxyacetic acid)phenyl, diphenylmethylamino, 3-phenylpropylamino, 3-phenylphenyl, phenylthiomethyl, 2-chloro-4,5-dimethoxyphenyl, and isopropyl.

A third preferred group of compounds are those of Formula ***I*** wherein:
X is sulfur;
Y is a covalent bond;
Z is NR⁵R⁶;
R¹ is hydrogen;
R³ is methylthio or methyl;
R⁴, R⁵ and R⁶ are all hydrogen; and
R² is Formula ***II***, where Ar is phenyl, thiazolyl, oxazolyl, benzothienyl, pyridyl, or imidazolyl; and R⁸ and R⁹ are independently hydrogen, or C₆₋₁₀ aryl or heterocycle, optionally substituted by one, two or three of chloro, hydroxy, C₁₋₄ alkyl, C₃₋₆ cycloalkyl, C₁₋₄ alkoxy, amino, carboxy, phenyl, naphthyl, biphenyl, hydroxyphenyl, methoxyphenyl, dimethoxyphenyl, carboxyalkoxyphenyl, alkoxycarbonylalkoxy, carboxyethoxy, alkylsulfonylaminophenyl, arylsulfonylaminophenyl, acylsulfonylaminophenyl, aralkylsulfonylaminophenyl, heteroarylsulfonylaminophenyl where the heteroaryl portion is optionally halo or C₁₋₆alkyl substituted, chlorophenyl, dichlorophenyl, aminophenyl, carboxyphenyl, nitrophenyl, or by 3,4-methylenedioxy, 3,4-ethylenedioxy, and 3,4-propylenedioxy.

A fourth preferred group of compounds are those of Formula ***I*** wherein:
X is sulfur;
Y is a direct covalent bond;
Z is NR⁵R⁶;
R¹ is hydrogen;
R² is alkyl, ar(alkyl), alkylsulfonyl, -SO₂-alkyl, amido, amidino, or 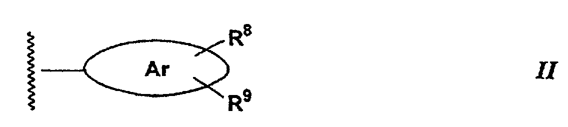
where
Ar is an aromatic or heteroaromatic group selected from the group consisting of phenyl, thiazolyl, oxazolyl, imidazolyl and pyridyl;
R⁸ and R⁹ are independently selected from the group consisting of hydrogen, carboxy, phenyl, naphthyl, alkyl, pyridyl, oxazolyl, furanyl, cycloalkyl and amino, any of which may be optionally substituted with 1 to 3 substituents independently selected from the group consisting of halogen, alkyl, haloalkyl, alkaryl, heteroaryl, phenyl, naphthyl, alkoxy, aryloxy, hydroxy, amino nitro, thiophenyl, benzothiophenyl, fluorenyl, 3,4-ethylenedioxy, 3,4-methylenedioxy, 3,4-propylenedioxy, arylsulfonamido, alkylsulfonamido and aryloxy, each of said 1 to 3 substituents may be further optionally substituted with one or more groups selected from alkoxy, haloalkyl, halogen, alkyl, amino, acetyl, hydroxy, dialkylamino, dialkylamino acyl, monoalkylaminoacyl, -SO₂-heteroaryl, -SO₂-aryl, or aryl;
R³ is -SO₂-alkyl, trifluoromethyl, S(O)-alkyl, hydrogen, alkoxy, alkylthio, alkyl, aralkylthio; and
R⁴, R⁵, R⁶ are hydrogen.

Preferred compounds of this embodiment are those where Ar is a thiazolyl, preferably thiazol-2-yl or thiazol-4-yl, and at least one of R¹⁷ and R¹⁸ is substituted phenyl, most preferably on the 4-position of the thiazol-2-yl group. Also preferred are compounds where R² is a 4-phenylthiazol-2-yl group wherein said phenyl is further optionally substituted. and R³ is methylthio.

A fifth preferred group of compounds are those of Formula ***III:*** 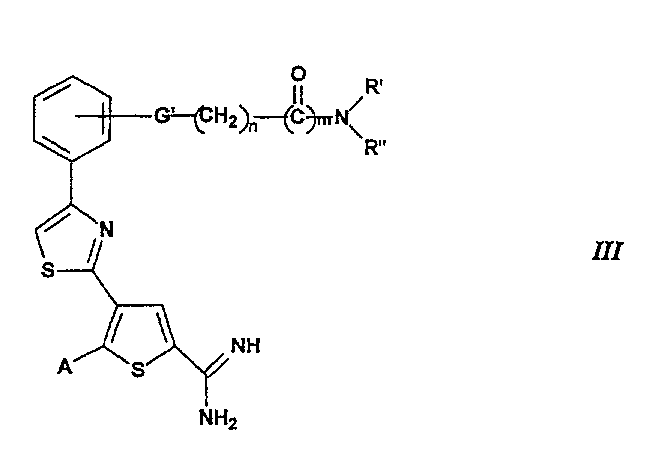 or a salt thereof, where
A is methylthio or methyl;
G' is ―O―, ―S―, ―NH―, or a covalent bond;
n is an integer from 1-10, preferably from 1-6;
m is an integer from 0-1; and
R' and R" are independently selected from hydrogen, alkyl, aryl or aralkyl, or R' and R" are taken together with the N atom to which they are attached form a 3-8 membered heterocyclic ring, optionally containing an additional O, N, or S atom, and when said 3-8 membered heterocyclic ring contains an additional N atom, said additional N atom is optionally substituted by hydrogen, C₁₋₄alkyl, C₆₋₁₀aryl, C₆₋₁₀ar(C₁₋₄)alkyl, acyl, alkoxycarbonyl or benzyloxycarbonyl.

Most preferred compounds of Formula ***III*** are those for which R' and R", taken together with the N atom to which they are attached, form a ring selected from piperazinyl, pyrrolidinyl, piperidinyl or morpholinyl, which are further optionally substituted with 1 to 4 non-hydrogen substituents selected from halogen, hydroxy, amino, monoalkylamino, dialkylamino, formylamino, acylamino, aminoacyl, mono- or di- alkylaminocarbonyl, thiocarbonylamino, thioacylamino, aminothiocarbonyl, alkoxy, aryloxy, aminocarbonyloxy, mono- or di-alkylaminocarbonyloxy, mono- or diarylaminocarbonyloxy, mono- or diarakylaminocarbonyloxy, alkylsulfonyl, arylsulfonyl, aralkylsulfonyl, alkylsulfonylamino, arylsulfonylamino, arakylsulfonylamino, alkoxycarbonylamino, aralkoxycarbonylamino, aryloxycarbonylamino, mono- or di- alkylaminothiocarbonyl, aralkoxy, carboxy, carboxyalkyl, alkoxycarbonyl, alkoxycarbonylalkyl, nitro, cyano, trifluoromethyl, alkylthio and arylthio, where each of these substituents has the preferred values set forth for Formulae ***I*** and ***II*** above.

Examples of preferred compounds of Formula ***III*** include 5-methylthio-4-[4-(3-{[N-(2-morpholin-4-ylethyl)carbamoyl]methoxy}phenyl)(1,3-thiazol-2-yl)]thiophene-2-carboxamidine, 5-methylthio-4-{4-[3-(2-morpholin-4-yl-2-oxoethoxy)phenyl](1,3-thiazol-2-yl)}thiophene-2-carboxamidine, 5-methylthio-4-{4-[3-(2-oxo-2-piperazinylethoxy)phenyl](1,3-thiazol-2-yl)}thiophene-2-carboxamidine, 4-[4-(3-{[N-(2-aminoethyl)carbamoyl]methoxy}phenyl)(1,3-thiazol-2-yl)]-5-methylthiothiophene-2-carboxamidine, 4-(4-{3-[2-(4-acetylpiperazinyl)-2-oxoethoxy]phenyl}(1,3-thiazol-2-yl))-5-methylthiothiophene-2-carboxamidine, 4-(4-{3-[2-(4-methylpiperazinyl)-2-oxoethoxy]phenyl}(1,3-thiazol-2-yl))-5-methylthiothiophene-2-carboxamidine, the compound described in Example 151, 5-methylthio-4-[4-(3-{2-oxo-2-[4-benzylpiperazinyl]ethoxy}phenyl)(1,3-thiazol-2-yl)]thiophene-2-carboxamidine, (D,L)-4-(4-{3-[2-(3-aminopyrrolidinyl)-2-oxoethoxy]phenyl}(1,3-thiazol-2-yl))-5-methylthiothiophene-2-carboxamidine, 5-methylthio-4-{4-[3-(2-oxo-2-piperidylethoxy)phenyl](1,3-thiazol-2-yl)}thiophene-2-carboxamidine, (D,L)-ethyl 1-(2-{3-[2-(5-amidino-2-methylthio-3-thienyl)-1,3-thiazol-4-yl]phenoxy}acetyl)piperidine-2-carboxylate, 5-methylthio-4-{4-[3-(2-oxo-2-pyrrolidinylethoxy)phenyl](1,3-thiazol-2-yl)}thiophene-2-carboxamidine, 5-methylthio-4-[4-(3-{2-oxo-2-[4-benzylpipendyl]ethoxy}phenyl)(1,3-thiazol-2-yl)]thiophene-2-carboxamidine, {D,L)-4-(4-{3-[2-(3-methylpiperidyl)2-oxoethoxy]phenyl}(1,3-thiazol-2-yl))-5-methylthiothiophene-2-carboxamidine, 4-(4-{3-[2-(4-methylpiperidyl)-2-oxoethoxy]phenyl}(1,3-thiazol-2-yl))-5-methylthiothiophene-2-carboxamidine, 4-(4-{3-[2-(2-azabicyclo[4.4.0]dec-2-yl)-2-oxoethoxy]phenyl}(1,3-thiazol-2-yl))-5-methylthiothiophene-2-carboxamidine, (D,L)-ethyl 1-(2-{3-[2-(5-amidino-2-methylthio-3-thienyl)-1,3-thiazol-4-yl]phenoxy}acetyl)piperidine-3-carboxylate, 5-methylthio-4-{4-[3-(2-oxo-2-(1,2,3,4-tetrahydroquinolyl)ethoxy)phenyl](1,3-thiazol-2-yl)}thiophene-2-carboxamidine, ethyl 1-(2-{3-[2-(5-amidino-2-methylthio-3-thienyl)-1,3-thiazol-4-yl]phenoxy}acetyl)piperidine-4-carboxylate, 4-(4-{3-[2-((3R)-3-hydroxypiperidyl)-2-oxoethoxy]phenyl}(1,3-thiazol-2-yl))-5-methylthiothiophene-2-carboxamidine, D,L-4-(4-{3-[2-(2-ethylpiperidyl)-2-oxoethoxy]phenyl}(1,3-thiazol-2-yl))-5-methylthiothiophene-2-carboxamidine, 4-(4-{3-[2-((3S)-3-hydroxypyrrolidinyl)-2-oxoethoxy]phenyl}(1,3-thiazol-2-yl))-5-methylthiothiophene-2-carboxamidine, D, L-4-[4-(3-{2-[3-(hydroxymethyl)piperidyl]-2-oxoethoxy}phenyl)(1,3-thiazol-2-yl)]-5-methylthiothiophene-2-carboxamidine, 4-{4-[3-(2-{(2R)-2-[(phenylamino)methyl]pyrrolidinyl}-2-oxoethoxy)phenyl](1,3-thiazol-2-yl)}-5-methylthiothiophene-2-carboxamidine, 4-[4-(3-{2-[(3R)-3-(methoxymethyl)pyrrolidinyl]-2-oxoethoxy}phenyl)(1,3-thiazol-2-yl)]-5-methylthiothiophene-2-carboxamidine, 1-(2-{3-[2-(5-amidino-2-methylthio-3-thienyl)-1,3-thiazol-4-yl]phenoxy}acetyl)piperidine-3-carboxamide, and 2-{3-[2-(5-{[(tert-butoxy)carbonylamino]iminomethyl}-2-methyl-3-thienyl)-1,3-thiazol-4-yl]phenoxy}acetic acid.

A sixth preferred group of compounds are those of Formula ***IV*** 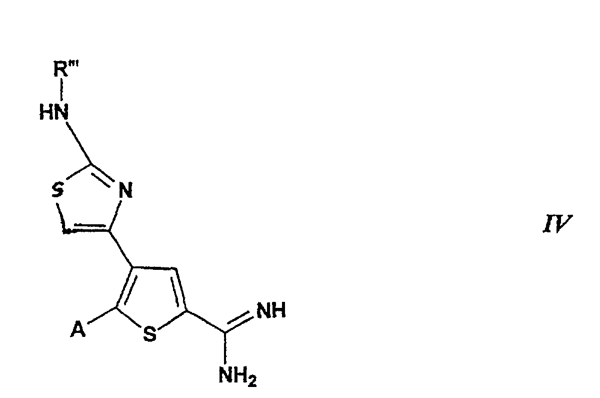 or a salt thereof, where
A is methylthio or methyl; and
R"' is hydrogen, C₆₋₁₄aryl, C₁₋₆alkyl, C₁₋₆alkoxy (C₆₋₁₄)aryl, amino(C₆₋₁₄)aryl, monoalkylamino(C₆₋₁₄)aryl, dialkylamino(C₆₋₁₄)aryl, C₆₋₁₀ar(C₁₋₆)alkyl, heterocycle(C2-6)alkyl such as morpholinoalkyl, piperazinylalkyl and the like, C₁₋₆alk(C₆₋₁₄)aryl, amino(C₁₋₆)alkyl, mono(C₁₋₆)alkylamino(C₁₋₆)alkyl, di(C₁₋₆)alkylamino(C₁₋₆)alkyl, hydroxy(C₆₋₁₄)aryl, or hydroxy(C₁₋₆)alkyl, any of which is further optionally substituted by 1-4 non-hydrogen substituents selected from halogen, hydroxy, amino, mono(C₁₋₆)alkylamino, di(C₁₋₆)alkylamino, formylamino, (C₁₋₆)acylamino, amino(C₁₋₆)acyl, mono- or di-(C₁₋₆)alkylaminocarbonyl, thiocarbonylamino, (C₁₋₆)thioacylamino, aminothiocarbonyl, (C₁₋₆)alkoxy, (C₆₋₁₀)aryloxy, aminocarbonyloxy, mono- or di-(C₁₋₆)alkylaminocarbonyloxy, mono- or di-(C₆₋₁₀)arylaminocarbonyloxy, mono- or di(C₆-₁₀)ar(C₁₋₆)alkylaminocarbonyloxy, (C₁₋₆)alkylsulfonyl, (C₆₋₁₀)arylsulfonyl, (C₆₋₁₀)ar(C₁₋₆)alkylsulfonyl, (C₁₋₆)alkylsulfonylamino, C₆₋₁₀ arylsulfonylamino, (C₆₋₁₀)ar(C₁₋₆)alkylsulfonylamino,(C₁₋₆)alkoxycarbonylamino, (C₆₋₁₀)ar(C₁₋₆)alkoxycarbonylamino, C₆₋₁₀aryloxycarbonylamino, mono- or di- (C₁₋₆)alkylaminothiocarbonyl, (C₆-₁₀)ar(C₁₋₆)alkoxy, carboxy, (C₁₋₆)carboxyalkyl, C₁₋₆alkoxycarbonyl, (C₁₋₆)alkoxycarbonyl(C₁₋₆)alkyl, nitro, cyano, trifluoromethyl, (C₁₋₆)alkylthio and C₆₋₁₀arylthio.

Examples of preferred compounds of Formula ***IV*** include 4-{2-[(3-methoxyphenyl)amino](1,3-thiazol-4-yl)}-5-methylthiothiophene-2-carboxamidine, 4-{2-[(4-methoxyphenyl)amino](1,3-thiazol-4-yl)}-5-methylthiothiophene-2-carboxamidine, 4-(2-{[4-(dimethylamino)phenyl]amino}(1,3-thiazol-4-yl))-5-methylthiothiophene-2-carboxamidine, 4-{2-[(4-chloro-2-methylphenyl)amino](1,3-thiazol-4-yl)}-5-methylthiothiophene-2-carboxamidine, 4-{2-[(diphenylmethyl)amino](1,3-thiazol-4-yl)}-5-methylthiothiophene-2-carboxamidine, 5-methylthio-4-{2-[(3-phenylpropyl)amino](1,3-thiazol-4-yl)}thiophene-2-carboxamidine, 5-methylthio-4-{2-[(2,4,5-trimethylphenyl)amino](1,3-thiazol-4-yl)}thiophene-2-carboxamidine, 4-{2-[(2-fluorophenyl)amino](1,3-thiazol-4-yl)}-5-methylthiothiophene-2-carboxamidine, 4-{2-[(3-chloro-2-methylphenyl)amino](1,3-thiazol-4-yl)}-5-methylthiothiophene-2-carboxamidine, 4-(2-{[2-(methylethyl)phenyl)amino}(1,3-thiazol-4-yl))-5-methylthiothiophene-2-carboxamidine, 5-methylthio-4-(2-{[4-(phenylmethoxy)phenyl]amino}(1,3-thiazol-4-yl))thiophene-2-carboxamidine, 4-{2-[(2-bromophenyl)amino](1,3-thiazol-4-yl)}-5-methylthiothiophene-2-carboxamidine, 4-{2-[(2,6-Dichlorophenyl)amino](1,3-thiazol-4-yl)}-5-methylthiothiophene-2-carboxamidine, 4-{2-[(2-bromo-4-methylphenyl)amino](1,3-thiazol-4-yl)}-5-methylthiothiophene-2-carboxamidine, 5-methylthio-4-{2-[(2-morpholin-4-ylethyl)amino](1,3-thiazol-4-yl)}thiophene-2-carboxamidine, 4-{2-[(2,3-dichlorophenyl)amino](1,3-thiazol-4-yl)}-5-methylthiothiophene-2-carboxamidine, 5-methylthio-4-{2-[(3,4,5-trimethoxyphenyl)amino](1,3-thiazol-4-yl)}thiophene-2-carboxamidine, 5-methylthio-4-{2-[(2-piperidylethyl)amino](1,3-thiazol-4-yl)}thiophene-2-carboxamidine, 4-(2-{[(4-methylphenyl)methyl]amino}(1,3-thiazol-4-yl))-5-methylthiothiophene-2-carboxamidine, 4-(2-{[4-(4-chlorophenoxy)phenyl]amino}(1,3-thiazol-4-yl))-5-methylthiothiophene-2-carboxamidine, 4-(2-{[4-phenoxyphenyl]amino}(1,3-thiazol-4-yl))-5-methylthiothiophene-2-carboxamidine, 5-methylthio-4-(2-{[4-(phenylamino)phenyl]amino}(1,3-thiazol-4-yl))thiophene-2-carboxamidine, 5-methylthio-4-(2-{[4-benzylphenyl]amino}(1,3-thiazol-4-yl))thiophene-2-carboxamidine, 5-methylthio-4-(2-{[4-(piperidylsulfonyl)phenyl]amino}(1,3-thiazol-4-yl))thiophene-2-carboxamidine 5-methylthio-4-[2-(3-quinolylamino)(1,3-thiazol-4-yl)]thiophene-2-carboxamidine, 5-methylthio-4-[2-(2-naphthylamino)(1,3-thiazol-4-yl)]thiophene-2-carboxamidine, 4-[2-(2H-benzo[3,4-d]1,3-dioxolan-5-ylamino)(1,3-thiazol-4-yl)]-5-methylthiothiophene-2-carboxamidine, 4-{2-[(7-bromofluoren-2-yl)amino](1,3-thiazol-4-yl)}-5-methylthiothiophene-2-carboxamidine, 4-{2-[(4-cyclohexylphenyl)amino](1,3-thiazol-4-yl)}-5-methylthiothiophene-2-carboxamidine, 5-methylthio-4-(2-{[4-(phenyldiazenyl)phenyl]amino}(1,3-thiazol-4-yl))thiophene-2-carboxamidine, 5-methylthio 4-(2-{[3-(hydroxymethyl)phenyl]amino}(1,3-thiazol-4-yl))-thiophene-2-carboxamidine, 4-[2-({3-[(3-methylpiperidyl)methyl]phenyl}amino)(1,3-thiazol-4-yl)]-5-methylthiothiophene-2-carboxamidine, 4-{2-[(3-hydroxyphenyl)amino](1,3-thiazol-4-yl)}-5-methylthiothiophene-2-carboxamidine, 4-(2-{[4-(carbamoylmethoxy)phenyl]amino}(1,3-thiazol-4-yl))-5-methylthiothiophene-2-carboxamidine, 5-methyl-4-{2-[(3,4,5-trimethoxyphenyl)amino](1,3-thiazol-4-yl)}thiophene-2-carboxamidine, 5-methyl-4-{2-[(4-phenoxyphenyl)amino](1,3-thiazol-4-yl)}thiophene-2-carboxamidine, 5-methyl-4-[2-(phenylamino)(1,3-thiazol-4-yl)]thiophene-2-carboxamidine, and 4-(4-isoxazol-5-yl(1,3-thiazol-2-yl))-5-methylthiothiophene-2-carboxamidine.

Many synthetic methods used to form compounds of the present invention generally involve the formation of an amidine from a carboxylic acid derivative, such as an ester. In the process a Lewis acid, such as trimethylaluminum, is added to a source of ammonia, such as ammonium chloride in an aprotic solvent, such as a toluene, under an inert atmosphere (e.g., under an atmosphere of nitrogen or argon gas) at a temperature between -15 °C and 5 °C, preferably at 0°C. An appropriate carboxylic acid derivative is added to the mixture and the mixture is heated at reflux for a predetermined period of time, preferably between 1 hr. and 24 hrs., and most preferably between 1 hr. and 4 hrs. The resulting solution is allowed to cool to room temperature and the amidine product isolated by known methods.

### Description of Syntheses

### Scheme 1a

Scheme **1a** illustrates a general approach to compounds of Formula ***I*** where X = S, R² = alkylthio, aralkylthio, arylthio, alkyloxy, aralkyloxy or aryloxy, Y = bond and Z = NR⁵R⁶. When R²² and R²³ of compounds **2** and **3** are retained in the final product, they correspond to R² and R³ of Formula ***I,*** respectively. Otherwise R²² and R²¹ represent groups which, after further transformations, will become R² and R³ of Formula ***I.***

Starting with the heterocycle where X = S appropriately substituted by two leaving groups, the leaving groups can be sequentially displaced by appropriate nucleophiles (preferably the anion of the group R²¹ or R²² to be substituted) to produce the mono- or disubstituted heterocycles. Examples of leaving groups include halogens (chlorine, bromine or iodine), sulfonates (methanesulfonate, toluenesulfonate or trifluoromethanesulfonate) or sulfones (methylsulfonyl). Preferable nucleophiles include anions of thiols or alcohols having as the counterion an alkali or alkali earth metal such as sodium, lithium, potassium, magnesium or cesium, or in some cases, a transition group metal such as zinc, copper or nickel. In certain cases where the nucleophile used contains an anion on carbon, catalysis of the displacement may be useful for this transformation. Examples of catalysts would include compounds containing palladium, silver or Ni salts.

### Scheme 1b

Scheme **1b** illustrates approaches to providing the functionality of Y(CNR⁴)Z in compounds of Formula ***I*** where X = S, R²² and R²¹ are defined as in Scheme **1a.** Depending on the nature of the group W in **3**, several methods may be employed in the transformation of W to Y(CNR⁴)Z.

When W in **3** is a cyano group (CN), primary amide (CONH₂) or ester (CO₂R²³), direct conversion to an unsubstituted amidine **5** (i.e. Formula ***I*** where Y = bond, Z = NR⁵R⁶ and R⁴, R⁵, R⁶ = H) can be effected by treatment with a reagent consisting of a Lewis acid complexed to ammonia. This complex is produced by treatment of ammonia or an ammonium salt, preferably an ammonium halide and most preferably ammonium chloride or bromide, with an appropriate Lewis acid, preferably a trialkylaluminum and most preferably trimethyl- or triethylaluminum in a solvent inert to the Lewis acid employed. For example, when a trialkylaluminum Lewis acid is employed with an ammonium halide, reaction occurs with loss of one equivalent of alkane to produce the dialkylhaloaluminum complex of ammonia (see for example Sidler, D.R., *et al, J. Org. Chem.*, *59*:1231 (1994)). Examples of suitable solvents include unsaturated hydrocarbons such as benzene, toluene, xylenes, or mesitylene, preferably toluene, or halogenated hydrocarbons such as dichloroethane, chlorobenzene or dichlorobenzene. The amidination reaction is generally carried out at elevated temperatures, preferably 40-200 °C, more preferably 80-140 °C, and most preferably at the reflux temperature of a solvent in the range of 80-120 °C.

When W is a cyano group (CN), direct conversion to a mono- or disubstituted amidine 5 (R⁴, R⁵, R⁶ = H) is also possible by treatment with a reagent consisting of a Lewis acid, preferably a trialkylaluminum, complexed to a mono- or disubstituted amine H₂NR⁵ or HNR⁵R⁶ (Garigipati, R., *Tetrahedron Lett. 31*: 1969 (1990)). Alternatively the same addition of a mono- or disubstituted amine may catalyzed by a copper salt such as Cu(I) chloride (Rousselet, G., *et al*, *Tetrahedron Lett. 34*: 6395 (1993)).

When W in **3** is a carboxyl group (CO₂H), indirect conversion to an unsubstituted amidine **5** can be carried out by initial esterification to **4** by any of a number of well-known dehydrating agents (for example, dicyclohexylcarbodiimide) with an alcohol (R²³OH). More preferably **4** can be made by initial formation of an acid chloride by treatment of **3** with any of a number of anhydrides of HCl and another acid, such as thionyl chloride, POCl₃, PCl₃, PCl₅, or more preferably oxalyl chloride, with or without an added catalyst such as *N,N*-dimethylformamide (DMF), followed by the alcohol R²³OH. Conversion to the unsubstituted amidine **5** (R⁴, R⁵, R⁶ = H) can be carried out by treatment with a Lewis acid complexed to ammonia.

Amidines **5** also can be produced indirectly by conversion of **3** (W = CN) to iminoethers **6** by exposure to a strong acid such as a hydrogen halide, HBF₄ or other non-nucleophilic acid, preferably gaseous HCl in the presence of an alcohol R²³OH (R²³= alkyl, branched alkyl or cycloalkyl, preferably Me or Et) and most preferably with the alcohol as solvent. Alternatively when W = CONH₂, conversion to an iminoether can be carried out by treatment with a trialkyloxonium salt (Meerwein's salts). In either case, treatment of the iminoether with ammonia (R⁵, R⁶ = H) or a mono- or disubstituted amine (HNR⁵R⁶) provides the corresponding unsubstituted or substituted amidines **5** (i.e. via classical Pinner synthesis: Pinner, A., Die Iminoaether und ihre Derivate, Verlag R. Oppenheim, Berlin (1892)).

When W = NH₂ in **3,** treatment with a reagent Z(CNR⁴)L where Z = alkyl and L is a leaving group such as O-alkyl and preferably OMe, provides the subclass of amidines **135** (Z = alkyl ) which are isomeric to **5** (Formula ***I***, where Y = NH, Z = H or alkyl). Examples of reagents for this reaction include methyl or ethyl acetimidate hydrochloride. Alternatively treatment of **3** (W = NH₂) with a trialkyl orthoformate ester, preferably trimethyl- or triethyl orthoformate, followed by an amine R⁴NH₂ affords the corresponding formidines **135** (Z = H) (Formula ***I***, where Y = NH, Z = H).

Also, when W = NH₂, **3** can be treated with a reagent Z(CNR⁴)L where R₄ = H and Z = NR⁵R⁶ and L is a leaving group such as pyrazole, methylpyrazole, SO₃H, S-alkyl, S-aryl, trifluoromethanesulfonate (OTf) or trifluoromethanesulfonamide (NHTf), preferably pyrazole, SO₃H or trifluoromethanesulfonamide (NHTf). Examples of these reagents include aminoiminosulfonic acid (Miller, A.E. and Bischoff, J.J., *Synthesis,* 777 (1986) and 1*H*-pyrazole-1-carboxamidine hydrochloride (Bernatowicz, M.S., *et al., J. Org. Chem. 57*:2497 (1992)). Such treatment provides guanidines **136** directly (Formula ***I*** where Y = NH, Z = NR⁵R⁶). Alternatively a reagent Z(CNP¹)L may be also used where Z = NHP² and L again a leaving group such as pyrazole, methylpyrazole, SO₃H, S-alkyl, S-aryl, trifluoromethanesulfonate (OTf) or trifluoromethanesulfonamide (NHTf), to provide protected guanidines (P¹, P² = alkoxylcarbonyl, aralkoxycarbonyl or polymer-bound alkoxylcarbonyl similar to those described below in Scheme **4a**) where the protecting groups P¹ and P² can then be removed to give unsubstituted **136** (R⁴, R⁵ and R⁶ = H). Protected guanidines are advantageous when further transformations are required after introduction of the guanidine functionality where an unprotected guanidine would not be stable. Examples of these protected reagents include reagents such as *N,N'*-bis(tert-butoxycarbonyl)-S-methylthiourea (Bergeron, R.J. and McManis, J.S, *J. Org. Chem. 52*:1700 (1987)), *N,N'*-bis(benzyloxycarbonyl)-1 *H*-pyrazole-1-carboxamidine or *N,N'*-bis(*tert*-butoxycarbonyl)-1 *H*-pyrazole*-*1-carboxamidine (Bernatowicz, M.S., *et al., Tetrahedron Letters, 34*: 3389 (1993)), *N,N'*-bis(benzyloxycarbonyl)-N"-trifluoromethanesulfonylguanidine, and *N,N'*-bis(bis(*tert*-butoxycarbonyl)-N"-trifluoromethanesulfonylguanidine (Feichtinger, K., *et al, J. Org. Chem. 63*:3804 (1998)). Detailed descriptions and examples of these protecting groups and their use as protection for amidines are further outlined in Schemes **4a**, **4b** and **5**.

When W in **3** is an ester (CO₂R²³) or carboxyl group (CO₂H), indirect conversion to an N-substituted or unsubstituted methylamidine (Formula ***I*** where Y = CH₂, Z = NR⁵R⁶) can be carried out by initial reduction of the ester or carboxyl by any of a number of well-known reducing agents. When W in **3** is an ester (CO₂R²³), examples of reducing agents include reducing agents such lithium aluminum hydride (LAH) and lithium borohydride. When W in **3** is a carboxyl group (CO₂H), examples of reducing agents include LAH and borane complexed to THF, dimethyl sulfide, dimethylamine or pyridine. The resulting hydroxymethyl derivative (W = CH₂OH) is converted to a cyanomethyl derivative (W = CH₂CN) by initial formation of a leaving group (W = CH₂L) where the leaving group L is a halogen (chlorine, bromine or iodine) or sulfonate ester (for example methanesulfonate, toluenesulfonate or trifluoromethanesulfonate). Displacement of L by cyanide can then be performed by treatment with a metal cyanide such as LiCN, NaCN, KCN or CuCN in a polar solvent such as DMF and with or without a catalyst such as a crown ether, to afford the cyanomethyl derivative (see for example Mizuno, Y., et al, *Synthesis,* 1008 (1980)). More preferably, the conversion of W = CH₂OH to W = CH₂CN may be effected by a Mitsunobu reaction (Mitsunobu, O., *Synthesis,* 1 (1981)) using an azodicarboxylate ester such as diethyl azodicarboxylate or diisopropyl azodicarboxylate, Ph₃P and a source of cyanide such as HCN or more preferably acetone cyanohydrin (Wilk, B. *Synthetic Commun.* 23:2481 (1993)). Treatment of the resulting cyanomethyl intermediate (W = CH₂CN) under the conditions described for the conversion of **3** (W = CN) to **5** (either directly or indirectly via **6**) provides the corresponding amidinomethyl products.

### Scheme 1c

When not commercially available, alkylthiothiophenes (**3**, X = S, R¹ = OH or NH₂, R²¹ = SR⁵⁴, W = CN, CO₂R²³, CONH₂) can be synthesized by the methods illustrated in Scheme **1c**. Condensation of carbon disulfide and a malonic acid derivative (R⁵²CH₂R²²) in the presence of two alkylating agents R⁵⁴L and WCH₂L and a base in a suitable medium provide **3** (Dolman, H., European Patent Application No. 0 234 622 A1 (1987)). When R²² = R⁵² = CN, the resulting R¹ will be NH₂; when R²² = R⁵² = CO₂R²³, the resulting R¹ will be OH; and when R²² and R⁵² = CN, CO₂R²³, the resulting R¹ can be selected to be OH or NH₂ (and R²² = CN or CO₂R²³) depending on the reaction conditions and order of reagent addition. Examples of malonic acid derivatives suitable for this transformation include but are not limited to malonate diesters such as dimethyl malonate or diethyl malonate (R⁵², R²²= CO₂R²³, R²³ = Me or Et), malononitrile (R⁵², R²² = CN), or methyl or ethyl cyanoacetate (R⁵² = CO₂R²³, R²² = CN, R²³ = Me or Et). Leaving groups L include halides such as chloride, bromide or iodide, preferably bromide or iodide, or sulfonates such as toluenesulfonate, benzenesulfonate, methanesulfonate or trifluoromethanesulfonate. Examples of alkylating agent R⁵⁴L include primary or secondary alkyl, allyl or aralkyl halides or sulfonates, such as methyl iodide, isopropyl bromide, allyl bromide, benzyl chloride or methyl trifluoromethanesulfonate, or a 2-haloacetate ester such as tert-butyl 2-bromoacetate. Examples of alkylating agents WCH₂L include 2-chloroacetonitrile, methyl 2-bromoacetate or 2-bromoacetamide. Suitable media are generally polar aprotic solvents, for example, *N,N*-dimethylformamide (DMF), *N,N*-dimethylacetamide (DMA), N-methylpyrrolidinone (NMP) or dimethylsulfoxide (DMSO), preferably DMF.

Alternatively compounds **3** (R²² = CN) can be synthesized from precursors 138 (derived from malononitrile, R⁵⁴L and carbon disulfide), a thioglycolate WCHSH and a base in a suitable polar solvent, preferably methanol (Tominaga, Y., *et al, J. Heterocyclic Chem. 31*:771 (1994)).

When **3** contains an amino group at R¹, it can be diazotized with subsequent loss of nitrogen to give **3**, R¹ = H by treatment with a nitrosating agent in suitable solvent. Nitrosating agents include nitrosonium tetrafluoroborate, nitrous acid or, more preferably and alkyl nitrite ester such as tert-butyl nitrite. Suitable solvents are those which are stable to the nitrosating agents, preferably DMF, benzene or toluene.

### Scheme 1d

When not commercially available, heterocyclic precursors 1 or 2 (X = S; W = CO₂R²³, COOH; L = halogen) used in Scheme **1a** can be synthesized by the methods illustrated in Scheme **1c**. Depending on the conditions used, treatment of compounds such as **139** with elemental halogen (Cl₂, Br₂ or I₂, preferably Br₂) or an N-halosuccinimide reagent, preferably N-bromosuccinimide (NBS), affords either **1** or **2** directly. Description of suitable solvents and conditions to selectively produce **1** or **2** are found in Karminski-Zamola, G. *et al, Heterocycles* 38:759 (1994); Divald, S., *et al, J. Org. Chem. 41*:2835 (1976); and Bury, P., *et al, Tetrahedron 50*:8793 (1994).

### Scheme 2a

Scheme **2a** illustrates the synthesis of compounds **12** representing the subclass of compounds for which R² is Formula ***II*,** where Ar = 2-thiazolyl, Y = bond and Z = NR⁵R⁶. Starting with compound **1** (L = Br) and using the sequential displacement methodology discussed for Scheme **1a,** R²¹ can be first introduced to give **7**. This is followed by a second displacement with a metal cyanide such as copper (I) cyanide, sodium cyanide or lithium cyanide and most preferably copper (I) cyanide at a temperature of 80-200 °C and preferably at 100-140 °C, in a polar aprotic solvent; preferably DMF or DMSO, to give **8**. After esterification by any of the means described for the conversion of **3** to **4**, conversion to the thioamide is carried out by treatment of the nitrile with any of the methods well known in the art (see for example Ren, W., *et al., J. Heterocyclic Chem. 23*:1757 (1986) and Paventi, M. and Edward, *J.T., Can. J. Chem. 65*:282 (1987)). A preferable method is treatment of the nitrile with hydrogen sulfide in the presence of a base such as a trialkyl or heterocyclic amine, preferably triethylamine or pyridine, in a polar solvent such as acetone, methanol or DMF and preferably methanol. Conversion to the thiazole can be executed by classical Hantzsch thiazole synthesis followed by amidine formation as discussed in Scheme **1b**.

### Scheme 2b

Scheme **2b** illustrates the synthesis of compounds representing the subclass of compounds for which R² is Formula ***II*** where, in addition to being an alternate route to Ar = 2-thiazolyl (**20**) (see **12,** Scheme **2a**) also provide compounds of Formula ***II*** where Ar = 2-oxazolyl (**16**) or 2-imidazolyl (**18**) (Y = bond and Z = NR⁵R⁶). Starting with compound **9**, a selective hydrolysis of the nitrile with a tetrahalophthalic acid, preferably tetrafluoro- or tetrachlorophthalic acid, can be used to give **7** according to the method of Gribble, G.W., *et al.*, *Tetrahedron Lett. 29*: 6557 (1988). Conversion to the acid chloride can be accomplished using the procedures discussed for conversion of **3** to **4**, preferably with oxalyl chloride in dichloromethane in the presence of a catalytic amount of DMF. Coupling of the acid chloride to an aminoketone (R²⁶COCH(R²⁷)NH₂) can be performed in the presence of an acid scavenger, preferably *N,N*-diisopropylethylamine (DIEA) or pyridine in a suitable solvent such as DMF, dichloromethane or tetrahydrofuran (THF) to afford the common intermediate **14**. Alternatively coupling of the acid chloride to a less-substituted aminoketone (R²⁶COCH₂NH₂) can be used followed by optional alkylation with alkylating agent R²⁷L in the presence of a base, preferably NaH or t-BuOK. Transformation of **14** to the corresponding 2-oxazolyl (**15**), 2-imidazolyl (**17**) or 2-thiazolyl (**19**) esters can carried out by the methodology of Suzuki, M., *et al., Chem. Pharm. Bull. 34*:3111 (1986) followed by amidination according to Scheme **1b.** In addition, direct conversion of ketoamide **14** to imidazolyl derivative **18** is possible under the same conditions for conversion of **17** to **18** when conducted for extended periods, preferably greater than 2 h.

### Scheme 2c

Scheme **2c** describes a general route to the synthesis of oxazoles, imidazoles and thiazoles of structure **27, 29** and **31** respectively. Acid **2** (see Scheme **1a**) is converted to the ester by methods that are well known in the art (Theodora W. Greene and Peter G. M. Wuts, John Wiley and Sons, Inc. 1991). For example methyl ester **21** is formed by treating the acid in an appropriate solvent such as methanol with trimethylsilyldiazomethane. Alternatively the acid is treated with oxalyl chloride and catalytic amounts of dimethylformamide (DMF) in an appropriate solvent such as dichloromethane to form the acid chloride, which is then treated with methanol to give the methyl ester. Ester **21** is treated with a palladium (0) catalyst such as palladium tetrakistriphenylphosphine, and an alkylstannane such as hexa-n-butyldistannane or tri-n-butyltin chloride in an appropriate solvent such as DMF at elevated temperatures (50 °C - 120 °C) to give the arylstannane of general structure **22** (Stille, J.K., *Angew. Chem. Int. Ed. Engl. 25*:508-524 (1986)). The stannane **22** is then treated with acid chlorides in the presence of a palladium(0) catalyst to give ketone **23**. The ketone is treated with ammonia/ammonium chloride to give amine **24**. Alternatively the ketone is reacted with an azide such as sodium azide in a suitable solvent such as DMF, and the resulting azidoketone is reduced to amine **23** with a suitable reducing agent such as catalytic hydrogenation in the presence of palladium on carbon and an acid such as HCl (*Chem. Pharm. Bull. 33*:509-514 (1985)). Ketoamides **25** are formed by coupling the ketoamine **24** with a variety of suitably functionalized acid chlorides. Alternatively amide coupling may be performed using any of a number of peptide coupling reagents such as 1,3-dicyclohexylcarbodiimide (Sheehan, J. C. *et al., J. Am*. *Chem. Soc., 77*:1067 (1955)) or Castro's reagent (BOP, Castro, B., *et al., Synthesis* 413 (1976)). In another approach, amides **25** are formed directly from ketones **23** by reacting with various amide salts in an appropriate solvent such as DMF. The amide salts are generated by treating the amides with a suitable base such as sodium hydride (NaH). For example acetamide is treated with NaH in DMF at 0 °C to give sodium acetamide. Keto amide **25** is cyclized to the oxazole **26**, imidazole **28** and thiazole **30** using procedures similar to that shown in scheme **2b**. Oxazole **26,** imidazole **28** and thiazole **30** are treated with trimethylaluminum and ammonium chloride in refluxing toluene to give the amidines **27**, **29** and **31** respectively.

### Scheme 2d

Scheme **2d** illustrates to the preparation of compounds of Examples 42-43, where R²¹ and R⁴³ correspond in Formula ***I*** to groups R³ and R², respectively. The acids **2** can be converted to the stannane by treatment with base, such as *n*-butyl lithium or *sec*-butyl lithium, followed by trimethyltin chloride. The resulting acid can be then converted to the ester **22** by methods that are well known in the art (Theodora W. Greene and Peter G. M. Wuts, John Wiley and Sons, Inc. 1991). For example the methyl ester can be made by treating the acid **2** in a suitable solvent such as methanol with trimethylsilyldiazomethane. The stannane **22** can be reacted with suitable halides in the presence of catalytic amounts of a palladium catalyst, such as palladium tetrakistriphenylphosphine, to give the esters **32** (Stille, J.K., *Angew. Chem. Int. Ed. Engl. 25*:508-524 (1986)). These esters are then treated with trimethylaluminum and ammonium chloride in refluxing toluene to give the amidines **33**. In the case where R⁴³Lₙ (n = 2), this can be cross-coupled to an aryl, heteroaryl or vinyl boronic acid or ester to give compounds **34** (Miyaura, N. and Suzuki, A., *Chem. Rev. 95*:2457-2483 (1995)). This can usually be done in the presence of catalytic amounts of a palladium (0) catalyst such as tetrakistriphenylphosphine palladium and a base such as potassium carbonate in DMF at 90°C. Similar cross-coupling reactions can also be achieved by using aryl, heteroaryl and vinyl stannanes instead of boronic acids or esters. These esters are converted to the amidines 35 in the manner previously described.

### Scheme 2e

Scheme **2e** represents a modification to the methodology outlined in Scheme **2b** which allows synthesis of compounds of Formula ***II*** where Ar = 2-thiazolyl, 2-oxazolyl or 2-imidazolyl (Y = bond and Z = NR⁵R⁶) but which are regioisomeric to **16, 18** or **20** in the relative positions of substituents R²⁶ and R²⁷. This is illustrated in Scheme **2b** by the synthesis of 2-oxazolyl derivative **39.** Thus, acid **13** can be coupled to an hydroxy-containing amine R²⁷CH(NH₂)CH(R²⁶)OH to give amide **36** by any of a number of amide coupling reagents well known in the art (see Bodanszky, M. and Bodanszky, *A., The Practice of Peptide Synthesis,* Springer-Verlag, New York (1984)). More preferably **13** can be converted to the corresponding acid chloride using any of the procedures mentioned for conversion of **3** to **4** followed by treatment with the R²⁷CH(NH₂)CH(R²⁶)OH in the presence of an acid scavenger, preferably *N,N*-diisopropylethylamine (DIEA) or pyridine in a suitable solvent such as DMF, dichloromethane or tetrahydrofuran (THF) to give **36**. Oxidation of the alcohol **36** to the aldehyde **37** (R²⁶ = H) or ketone **37** (R²⁶ = alkyl, aryl, aralkyl, heterocycle) can be effected by any of a number of common methods known in the art (see for example F. Carey, F.A., Sundberg, R.J. *Advanced Organic Chemistry, Part B: Reactions and Synthesis,* 3rd Edition, Plenum Press, New York (1990)), preferably by a mild Moffatt-type oxidation such as a Swern oxidation (Mancuso, A.J., Huang, S.L. and Swern, *D., J. Org. Chem.* 3329 (1976)) or more preferably using Dess-Martin reagent (Dess, D.B. and Martin, J.C., *J. Org. Chem. 48*:4155 (1983)). Conversion to the heterocycle (in this case the oxazole) is effected with any of a number of reagents including phosphorus oxychloride, P₂O₅ or thionyl chloride (see Moriya, T., et *al., J. Med. Chem. 31*:1197 (1988) and references therein). Alternatively closure of **37** with either Burgess reagent or under Mitsunobu conditions affords the corresponding oxazolinyl derivatives (Wipf, P.and Miller, C.P., *Tetrahedron Lett. 3*:907(1992)). Final amidination to **39** as in Scheme **1b** completes the synthesis.

### Scheme 2f

Scheme **2f** illustrates a general approach to the synthesis of thiazoles of structure **43** (Formula ***II,*** X = S, Ar = thiazolyl). Nitriles of structure **40** can be treated with hydrogen sulfide (H₂S) in a suitable solvent such as methanol, or pyridine in the presence of a base such as triethyamine to give thioamides **41** (Ren, W. *et al., J*. *Heterocyclic Chem. 23*:1757-1763 (1986)). Thioamides **41** can be then treated with various haloketones **42** preferably bromoketones under suitable reaction conditions such as refluxing acetone or DMF heated to 50° C - 80° C to form the thiazoles **43** (Hantzsch, A. *R. et al., Ber. 20*:3118 (1887)).

### Scheme 2g

Scheme **2g** illustrates one synthetic route to 2-haloketones of structure **42** which are employed in the synthesis of thiazolyl derivatives as in Schemes **2a** and **2f.** 2-Bromoketones **42** (L = Br) are prepared by treating the ketone **44** with a suitable brominating agent such as Br₂ or N-bromosuccinimide in a suitable solvent such as chloroform or acetic acid (EP 0393936 A1). Alternatively, the ketone **44** is treated with a polymer-supported brominating agent such as poly(4-vinyl)pyridinium bromide resin (Sket, B., *et al., Synthetic Communications 19*:2481-2487 (1989)) to give bromoketones **42.** In a similar fashion 2-chloroketones are obtained by treating **44** with copper (II) chloride in a suitable solvent such as chloroform (Kosower, E. M., *et al., J. Org. Chem. 28*:630 (1963)).

### Scheme 2h

Scheme **2h** illustrates another synthetic route to 2-haloketones of structure **42** which is particularly useful in that it employs acids **45** or activated carbonyl compounds such as **46** as precursors which are more readily available than the ketones **44.** The acid **45** is converted to the acid halide **46** (L = Cl, Br or OCOR³⁹) by treating with a suitable halogenating reagent For example, an acid chloride is formed by treating **45** with oxalyl chloride and catalytic amounts of DMF in dichloromethane. The acid chloride is converted to a diazoketone by treatment with trimethysilyldiazomethane (Aoyama, T. *et al., Tetrahedron Lett. 21*:4461-4462 (1980)). The resulting diazoketone is converted to a 2-haloketone of structure **42** by treatment with a suitable mineral acid. For example a bromoketone is formed by treating the diazoketone in a suitable solvent such as acetonitrile (CH₃CN) with a solution of 30% hydrogen bromide (HBr) in acetic acid (Organic Synthesis Collective Vol III, 119, John Wiley and Sons, New York, Ed. Horning E. C.). In an alternative approach the acid **45** is converted to the mixed-anhydride **46** by treatment with a suitable chloroformate such as isobutyl chloroformate or tert-butyl chloroformate in a suitable solvent, such as tetrahydrofuran or dichloromethane, in the presence of a base such as N-methylmorpholine. The mixed anhydride **46** is converted to a diazoketone by treatment with trimethylsilyldiazomethane and the resulting diazoketone is converted to a haloketone in the manner described above.

### Scheme 2i

When amide coupling as described in Scheme **2e** is followed directly by amidination, compounds of Formula I where R² or R³ is aminoacyl or aminoiminomethyl can be derived. Thus, coupling of acid **13** (or the corresponding acid chloride as previously described) with an amine R⁵¹R⁵²NH can afford **130** which can be carried on to the amidine **131**. Upon either longer or more vigorous additional treatment (for example, higher temperatures) with a Lewis acid-ammonia reagent as described in Scheme **1b,** the amide group can be converted to an aminoiminomethyl group to give a bisamidine compound **132**.

### Scheme 3a

Acid **13** can also be converted to an amine *47* from which sulfonamides, ureas and urethanes can be formed (Formula ***I*** where R² or R³ = NR³²SO₂R³¹, NHCONR⁵¹R⁵² or NHCOR³¹, respectively). Scheme **3a** illustrates this methodology for introduction of these three groups at R² of Formula ***I.*** Conversion of the acid **13** to an intermediate acyl azide can be followed by heating of such azide in the presence of an alcohol under Curtius rearrangement conditions to form the carbamate ester of the alcohol. Subsequent carbamate ester hydrolysis yields amine **47.** The intermediate acyl azide may be synthesized by coupling the acid **13** to hydrazine through the acid chloride or by any of the amide coupling procedures discussed for Scheme **2e** followed by nitrosation of the resulting hydrazide by any of the nitrosating agents discussed for conversion of **3** (R¹ = NH₂) to **3** (R¹ = H) in Scheme **1c.** More preferably conversion of **13** to **47** is carried out through treatment of acid **13** with diphenylphosphoryl azide in the presence of an alcohol, preferably tert-butanol, and a base, preferably triethylamine or DIEA, as shown in Scheme **3a,** to give a tert-butylcarbamate that is readily decomposed to the salt of amine **47** on exposure to an acid, preferably HCl or trifluoroacetic acid in a suitable solvent such as CH₂Cl₂. Further treatment with a base such as NaOH or preferably K₂CO₃ or NaHCO₃ provides the free base **47.** Treatment of amine **47** with a sulfonyl chloride R³¹SO₂Cl in the presence of an acid scavenger, such as pyridine or DIEA, followed by optional alkylation on nitrogen with an alkylating agent R³²L in the presence of a base such as K₂CO₃, DIEA or more preferably sodium hydride, in a solvent such as THF, MeCN or CH₂Cl₂ affords the sulfonylamine functionality at R² (**48**). When necessary, this transformation can be catalyzed by the presence of 4-dimethylaminopyridine for less reactive sulfonyl chlorides. Similar treatment of amine **47** with an isocyanate R⁵¹NCO or carbamyl chloride R⁵¹R⁵²COCl affords the aminocarbonylamine functionality at R²(**50**). Similar treatment of amine **47** with an acid chloride R³¹COCl affords the carbonylamine functionality at R² (**52**). Conversion of the esters in **48, 50** and **52** to amidines as previously mentioned gives the products **49, 51** and **53.** Further conversion of the acylamino group of **53** as discussed for synthesis of **132** also provides access to the iminomethylamino group at R² (**54**).

### Scheme 3b

Introduction of an aminosulfonyl group (including monoalkylaminosulfonyl and dialkylaminosulfonyl groups) for R² of Formula ***I*** can be carried out starting from amine such as **47** as well. Conversion to a sulfonyl chloride by the method of Gengnagel, *et al.* (U.S. Patent No. 3,947,512 (1976)) and treatment with an amine R³⁴NH₂ followed by optional alkylation on nitrogen with R³⁵L (under the sulfonylation and alkylation conditions described in Scheme **3a**) provides **56** which is further converted to amidines **57** as previously described.

### Scheme 4a

Scheme **4a** illustrates the preparation of the compounds of Formula ***III*** and Examples 48-59 and 61-77. The amidine moiety of compounds of structure **60** can be protected with a protecting group P¹ that can be readily removed from **62** and **64** using methods known to those skilled in the art (Theodora W. Greene and Peter G. M. Wuts, John Wiley and Sons, Inc. 1991). For example, a t-butoxycarbonyl (BOC) protecting group can be removed by exposure to strongly acidic medium such as hydrogen chloride in a suitable solvent such as dioxane, or by trifluoroacetic acid in a suitable solvent such as methylene chloride. Benzyloxycarbonyl (Cbz) protecting groups can be removed by catalytic hydrogenation using palladium on carbon as a catalyst in solvents such as ethanol or tetrahydrofuran.

In some cases, P¹ can be a solid support such as polystyrene or polyethyleneglycol-grafted polystyrene which can be attached to the amidine moiety via a cleavable linker such as 4-(benzyloxy)benzyloxy-carbonyl (using carbonate Wang resin). Attaching an amidine to a solid support can be achieved by treating a solid support having a linker containing an appropriately activated functional group with the amidine under suitable conditions. For example, an amidine can be attached to Wang resin by treating paranitrophenylcarbonate Wang resin with the amidine and a suitable base such as DBU in a suitable solvent such as DMF. When D is OH or SH the protected amidines **61** can be alkylated with carboxy-protected (protecting group is R³⁶) haloaliphatic acids, such as bromoacetic acid or bromopropionic acid in the presence of a suitable base such as cesium carbonate or DIEA, in a suitable solvent such as DMF with heating when necessary to give compounds of structure **62.** When D is NO₂, the nitro group can be reduced prior to alkylation using an appropriate reducing agent, such as tin (II) chloride, in a suitable solvent such as DMF, or by catalytic hydrogenation using palladium on carbon as a catalyst in solvents such as ethanol or tetrahydrofuran. Other useful carboxy protecting groups are well known in the art (Theodora W. Greene and Peter G. M. Wuts, John Wiley and Sons, Inc. 1991). For example, tert-butyl ester can be removed by exposure to strongly acidic medium such as hydrogen chloride in a suitable solvent such as dioxane or trifluoroacetic acid in a suitable solvent such as methylene chloride. Benzyl ester can be removed by catalytic hydrogenation using palladium on carbon as a catalyst in solvents such as ethanol or tetrahydrofuran or by base hydrolysis.

When protecting groups P¹ and R³⁶ in compounds **62** are orthogonal (as defined by the ability to remove one protecting group preferentially in the presence of the other), R³⁶ can be preferentially removed to give acids **63.** For example when P¹ is BOC and R³⁶ is OME, the methyl ester can be removed by treating with a base such as sodium hydroxide in a suitable solvent such as aqueous tetrahydrofuran leaving the BOC group intact. When protecting groups P¹ and R³⁶ in compounds **62** are not orthogonal, both protecting groups are removed, and the amidine can be protected with a suitable protecting group such as BOC or a suitably functionalized resin. The protected amidine **63** can be treated with various amines under suitable amide coupling conditions, such as in the presence 1-hydroxy-7-azabenzotriazole (HOAt), O-(7-azabenzotriazol-1-yl)-1,1,3,3-tetramethyluronium hexafluorophosphate (HATU) and DIEA to form amides of structure **64.** The amidine protecting group can be then removed, for example by treating with an acid, such as trifluoroacetic acid in a suitable solvent such as methylene chloride, when a BOC protecting group is employed, to give amidines **65**.

### Scheme 4b

Scheme **4b** illustrates a specific example which utilizes the method described in Scheme **4a**. The amidine moiety of **66** can be monoprotected with a tert-butyloxycarbonyl group. The monoprotected phenoxyamidine **67** can be alkylated on the phenolic hydroxy group with an ester of 2-bromoacetic acid to give 68. In the case where the ester can be removed by base, it can be hydrolyzed with aqueous base, such as NaOH, to give the acid **69**. This acid can be treated with various amines in the presence of 1-hydroxy-7-azabenzotriazole (HOAt), O-(7-azabenzotriazol-1-yl)-1,1,3,3-tetramethyluronium hexafluorophosphate (HATU) and DIEA to form amides of structure **70**. The amines are unsubstituted, di- or mono-substituted aliphatic or aromatic amines. In some cases the amines are cyclic-amines such as piperazine and piperidine. The amides **70** are then treated with trifluoroacetic acid to give the amidines **71**. In the case where the ester **68** is acid-labile, it can be treated with trifluoroacetic acid to give the amidino-acid 72. This amidine can be loaded on to an insoluble support, such as polystyrene or polyethyleneglycol-grafted polystyrene via a cleavable linker, such as Wang, which is functionalized as an activated carbonate such as *p*-nitrophenylcarbonate or succinimidyl carbonate. Generally this can be done by treating the activated carbonate resin with the amidine and a suitable base such as DBU in a suitable solvent such as DMF. The support-bound acid **73** can be treated with various amines in the presence of 1-hydroxy-7-azabenzotriazole (HOAt), O-(7-azabenzotriazol-1-yl)-1,1,3,3-tetramethyluronium hexafluorophosphate (HATU) and DIEA to form amides. These amides are then cleaved from the solid support by treating with trifluoroacetic acid to give compounds of structure **71.**

### Scheme 5

Scheme **5** illustrates a synthetic route to amidines containing disubstituted thiazoles represented by compounds for which R² is Formula ***II*** and both R⁸ and R⁹ are non-hydrogen substituents . The ketoamide **74** can be converted to the mono-bromoketoamide by treating with bromine in acetic acid. Thiazoles **76** are formed by reacting the bromoketoamide with **10** under suitable conditions, preferably by heating the mixture in DMF or acetone. Amidines **77** are formed by heating **76** in toluene with trimethylaluminum and ammonium chloride. The amidines **77** are treated with strong acid such as HCl to give the acids **78.** The amidines **78** are in one route protected with a suitable protecting group such as BOC to give **79**. The protected amidines **79** are treated with various amines under suitable coupling conditions, such as in the presence of HOAt, HATU, and DIEA to form various amides. The amidine protecting group can be then removed, for example by treating with trifluoroacetic acid in a suitable solvent such as methylene chloride, when a BOC protecting group is employed to give amidines **80**. In a second route, the amidines **78** can be loaded onto an insoluble support, such as polystyrene or polyethyleneglycol-grafted polystyrene via a cleaveable linker, such as Wang resin, which is functionalized as an activated carbonate ester, such as *p*-nitrophenylcarbonate or succinimidyl carbonate, to give a resin-bound scaffold **81**. The resin-bound acid **81** can be treated with various amines under suitable coupling conditions such as in the presence of HOAT, HATU and DIEA to form amides. These amides are then cleaved from the solid support by treating with trifluoroacetic acid to give amidines **80**.

### Scheme 6a

Scheme **6a** illustrates the preparation of compounds of Examples 34, 35, 36, 37, 38, 39, 40, and 41. Compounds of this invention correspond to those of Formula ***I*** where R² is Formula ***II*** and where Ar is thiazole and R³⁷ and R³⁸ (R⁸ and R⁹ of Formula ***II***) are phenyl, which can be additionally substituted. Starting from 2,5-dibromothiophene **90,** treatment with lithium diisopropylamide followed by R²¹L, where L is a leaving group, preferably a halogen, mesylate, tosylate, or methyl sulfate, and more preferably iodomethane or methyl sulfate, according to the procedure of Kano, *et al., Heterocycles 20(10)*:2035 (1983), gives **91.** Compound **91** can be treated with an appropriate base, preferably a lithium alkyl like *n*-butyllithium, *sec*-butyllithium, or *t*-butyllithium, and more preferably *n*-butyllithium, followed by carbon dioxide gas and conversion of the resulting carboxylate salt to the free acid with a mineral acid, preferably hydrochloric acid. Conversion to ester **21** can be carried out by preparation of the acid chloride using oxalyl chloride and treatment of this intermediate acid chloride with an alcohol R²³ in an appropriate solvent, preferably dichloromethane, with an appropriate base, preferably pyridine. Compound **21** can be treated with copper (I) cyanide in refluxing dimethylformamide to give compound **9.** Compound **9** can be treated with hydrogen sulfide gas in an appropriate solvent, preferably methanol, containing an appropriate base, preferably triethylamine to give compound **10**. Compound **10** can be treated with an appropriate ketone where L is a leaving group, preferably halogen, mesyl, or tosyl, and most preferably bromo, refluxing in a suitable solvent, preferably, acetone, dimethylformamide, dimethyl acetamide, methyl ethyl ketone, or other polar aprotic solvents, and most preferably acetone to give compound **92**. Compound **92** is treated with an appropriate reagent, preferably the aluminum amide reagent to give amidine **93**.

### Scheme 6b

Scheme **6b** illustrates the preparation of the compound of Example **34,** which corresponds to a compound for which R² is Formula ***II***, and where Ar is thiazole and R⁸ and R⁹ (R³⁷ and R³⁸ in Scheme **6b**) are phenyl, which can be optionally substituted. Starting from 2,5-dibromothiophene **90,** treatment with *n*-butyllithium produces an anion which undergoes a rearrangement (Kano, S., *et al, Heterocycles 20*:2035 (1983)). Quenching with carbon dioxide gas and conversion of the resulting carboxylate salt to the free acid with a mineral acid, preferably hydrochloric acid, gives acid **94**. Conversion to ester **95** can be carried out by preparation of the acid chloride using oxalyl chloride and treatment of this intermediate acid chloride with an alcohol R²³-OH in an appropriate solvent, preferably dichloromethane, with an appropriate base, preferably pyridine. Compound **95** can be treated with copper (I) cyanide in refluxing dimethylformamide to give compound **96**. Compound **96** can be treated with hydrogen sulfide gas in an appropriate solvent, preferably methanol, containing an appropriate base, preferably triethylamine to give compound **97**. Compound **97** can be treated with an appropriate ketone where L is a leaving group, preferably halogen, mesyl, or tosyl, and most preferably bromo, refluxing in a suitable solvent, preferably, acetone, dimethylformamide, dimethyl acetamide, methyl ethyl ketone, or other polar aprotic solvents, and most preferably acetone to give compound **98**. Compound **98** is treated with an appropriate reagent, preferably the aluminum amide reagent (Al(CH₃)₃/NH₄Cl) to give amidine **99.**

### Scheme 7a

Scheme 7a illustrates the preparation of compounds for which R² is Formula ***II*** and Ar is thiazol-4-yl. As illustrated, the acids **13** can be converted to their acid chlorides by treatment with oxalyl chloride with dimethylformamide catalysis in methylene chloride, or by using thionyl chloride, either neat or in an organic solvent, at ambient or elevated temperature. Compounds are then homologated to the desired a-haloketones **100** by sequential treatment with trimethylsilyldiazomethane and hydrogen bromide. An alternative would be to substitute diazomethane (generated from Diazald®, Aldrich Chemical Co., Milwaukee, WI) for the trimethylsilyldiazomethane. Also, the conversion of **13** to **100** can be effected using the procedure derived for the synthesis of compound **42** from compound **46**.

The alpha-haloketones **100** are then allowed to react with the appropriate thiourea (Scheme **7b**) or thioamide derivative in an organic solvent, preferably acetone or dimethylformamide at 70 °C to give 2-aminothiazoles or thiazoles **101**.

The thiazoles **101** can be treated with the aluminum amine reagent (Al(CH₃)₃/NH₄CL) formed at ambient temperature by the reaction of trimethylaluminum with ammonium chloride in an organic solvent, preferably toluene. The ester can then be converted to the amidines **102** at elevated temperatures, preferably higher than 80 °C.

### Scheme 7b

As shown in **Scheme 7b,** amines **110** (or their hydrochloride salts) can be converted to their respective mono-substituted thioureas (methan-1-thiones) **112** by treatment with thiophosgene to form the intermediate isothiocyanates **111.** Preferred conditions include treating the amine with thiophosgene in a biphasic solvent system composed of a halogenated solvent such as chloroform and an aqueous phase of saturated sodium bicarbonate. Alternatively, the reaction may be effected by treatment of **110** with a hindered amine and thiophosgene such as triethylamine or diisopropylethylamine in an organic solvent such as tetrahydrofuran or methylene chloride. Another alternative to forming isothiocyanates **111** is the direct treatment of primary amines and carbon disulfide in pyridine with dicyclohexylcarbodiimide (Jochims, *Chem. Ber. 101*:1746 (1968)).

Isothiocyanates **111** can be converted to thioureas **112** by treatment with an ammonia-alcohol solution, preferably a 2M ammonia in methanol or ethanol solution, at room temperature or elevated temperatures (>70°C). Alternatively, the thioureas **112** can be prepared directly form the appropriate urea (or thioamide from the appropriate amide when R⁸ =alkyl or aryl)) by treatment with Lawesson's reagent (Lawesson, S.-O., *et. al. Bull. Soc. Chim. Belg. 87*:223, 293(1978)).

### Scheme 8

Scheme **8** illustrates the preparation of compounds of this invention where R² is Formula ***II*** and Ar is thiazole and R³⁷ and R³⁸are phenyl which is further substituted by a sulfonylamino or carbonylamino group. Starting from thioamide **10,** treatment with a nitro substituted 2-halo-acetophenone, where the halogen is chloro, bromo, or iodo, preferably bromo, refluxing in a suitable solvent, preferably acetone, dimethylformamide, dimethyl acetamide, methyl ethyl ketone, or other polar aprotic solvents, and most preferably acetone. The reduction of nitroaryl compound **113** can be carried out with a suitable reducing agent, preferably tin (II) chloride, titanium (II) chloride, iron (III) chloride, lithium metal, sodium metal, catalytic hydrogenation over platinum or palladium catalyst, and most preferably 20% aqueous solution of titanium (III) chloride. The acylation of aniline **114** can be carried out with an appropriate acyl compound R⁴² where L is a halogen, preferably chloro, in an appropriate solvent, preferably dichloromethane, containing a base, preferably pyridine, N-methylmorpholine, or diisopropylethylamine. Alternatively, the acylation of aniline **114** is carried out with an activated carboxylic acid compound R⁴² where L is hydroxy activated with dicyclohexylcarbodiimide, ethyl-3-(diethylamino)propylcarbodiimide (EDAC), *O*-(7-azabenzotriazol-1-yl)-*N,N*,*N',N'*-tetramethyluronium hexafluorophosphate (HATU), or pentafluorophenyl. The sulfonylation of aniline **114** can be carried out with and appropriate sulfonyl chloride compound R⁴¹ in an appropriate solvent, preferably dichloromethane, containing a base, preferably N-methyl morpholine, diisopropylethylamine, or pyridine, most preferably N-methyl morpholine, with or without a condensation catalyst, preferable dimethylaminopyridine (DMAP). The amidinylation of compounds **115** and **117** can be carried out with an appropriate reagent, preferably the aluminum amide reagent (Al(CH₃)₃/NH₄Cl).

### Scheme 9

Scheme **9** illustrates the preparation of compounds of Formula ***I***, for which one of R⁵ and R⁶ is a non-hydrogen substituent. The amidines **5** are converted to the amidoximes **119** by heating with hydroxylamine in a suitable solvent such as ethanol. The cyanoamidines **120** are prepared by heating the amidines **5** with cyanamide in a suitable solvent such as ethanol. (Huffman, K.R. and Schaeffer, F., *J. Amer. Chem. Soc. 28*:1812 (1963). Alternatively **5** can be heated with an amine such as methylamine to give the N-alkylated amidines **121**.

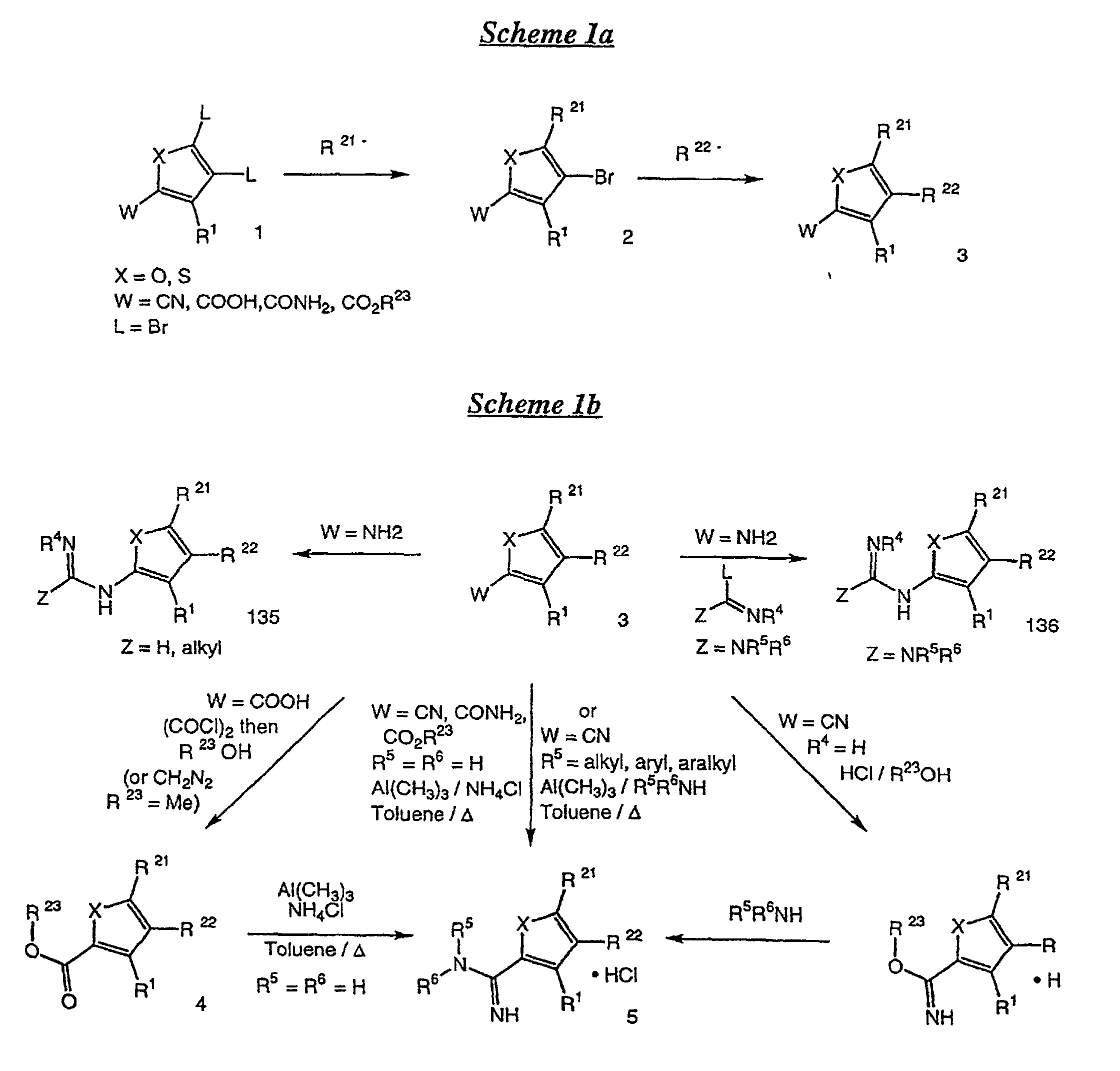

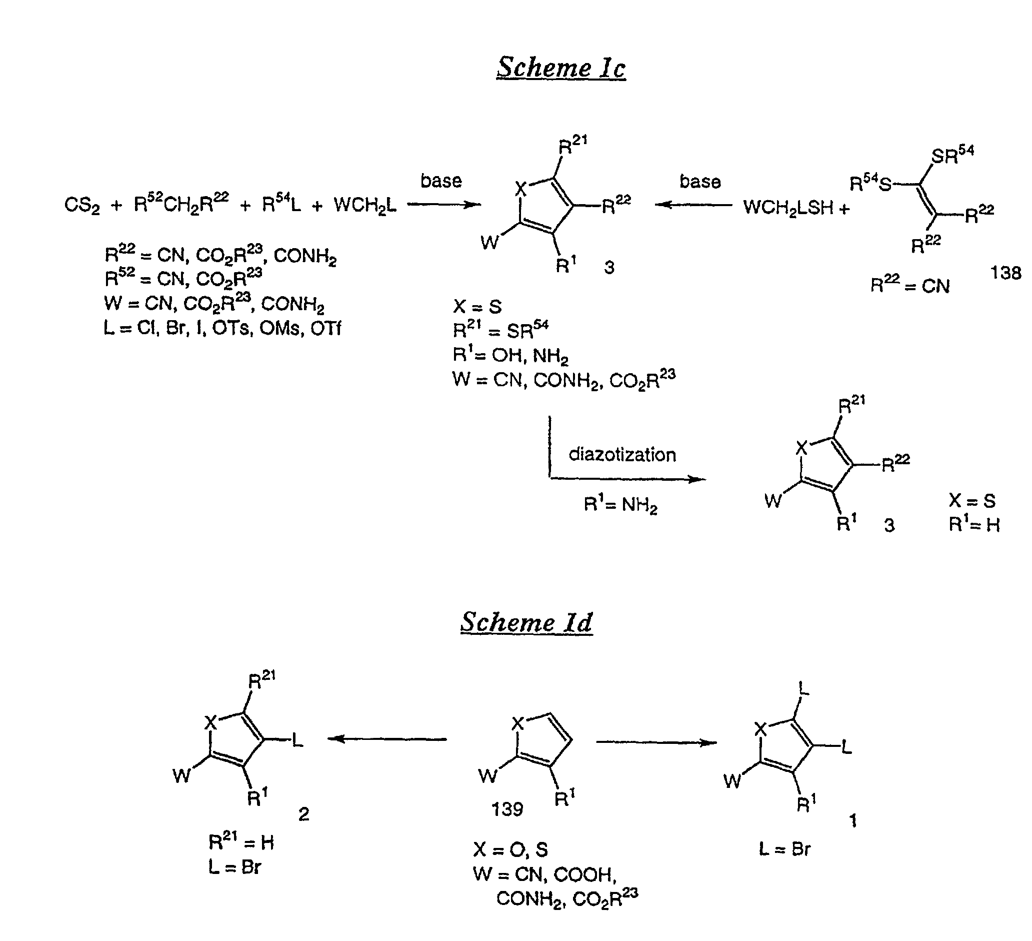

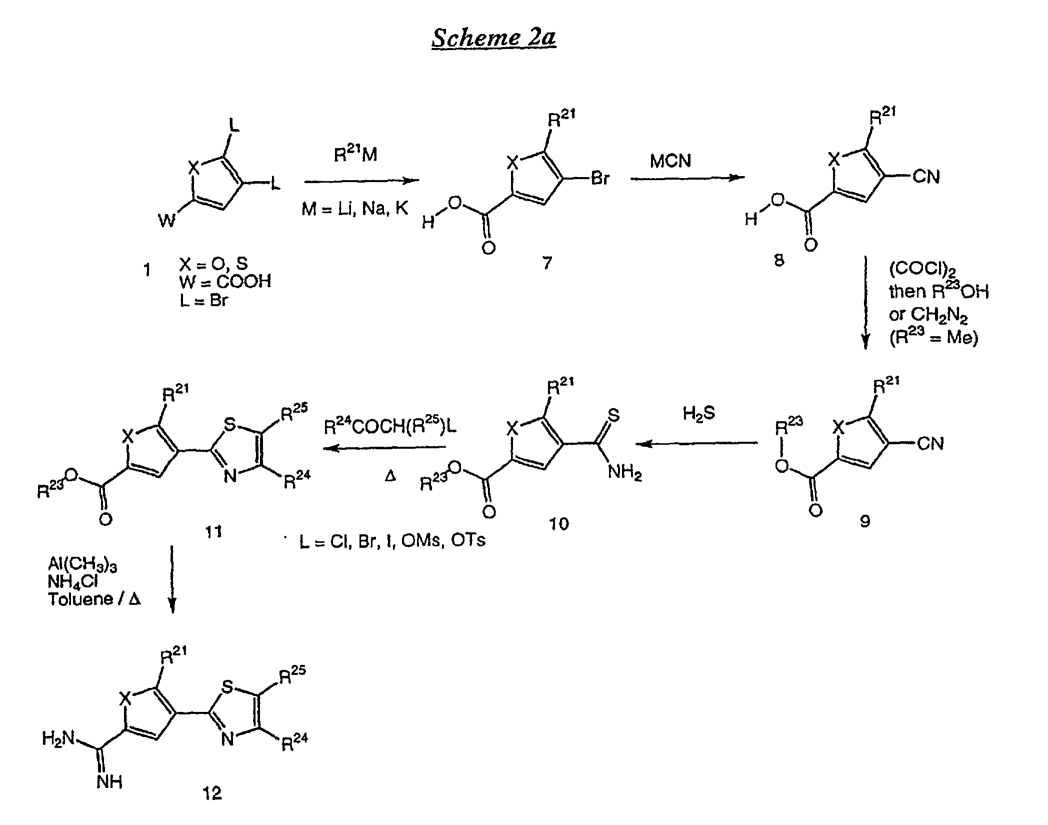

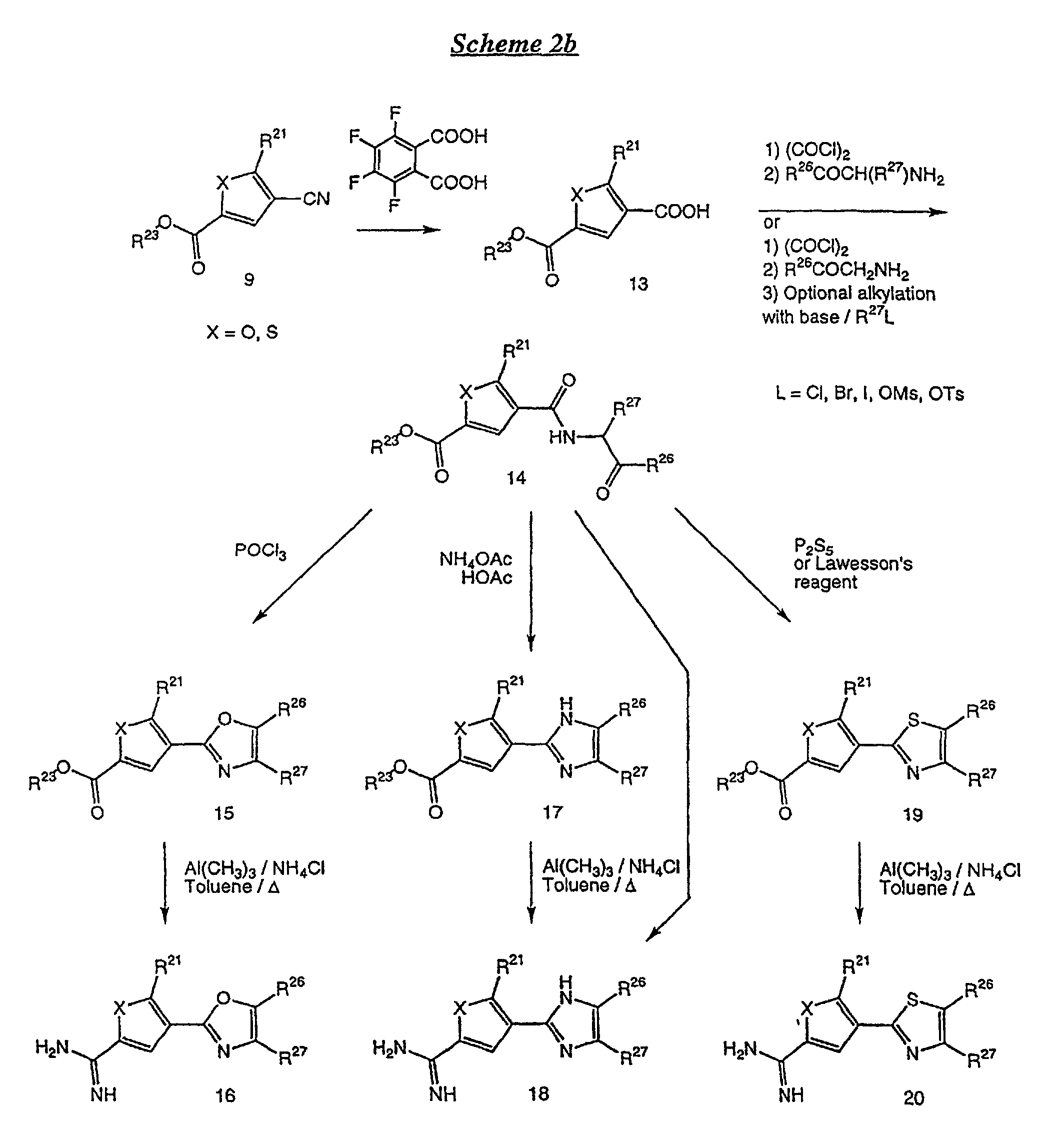

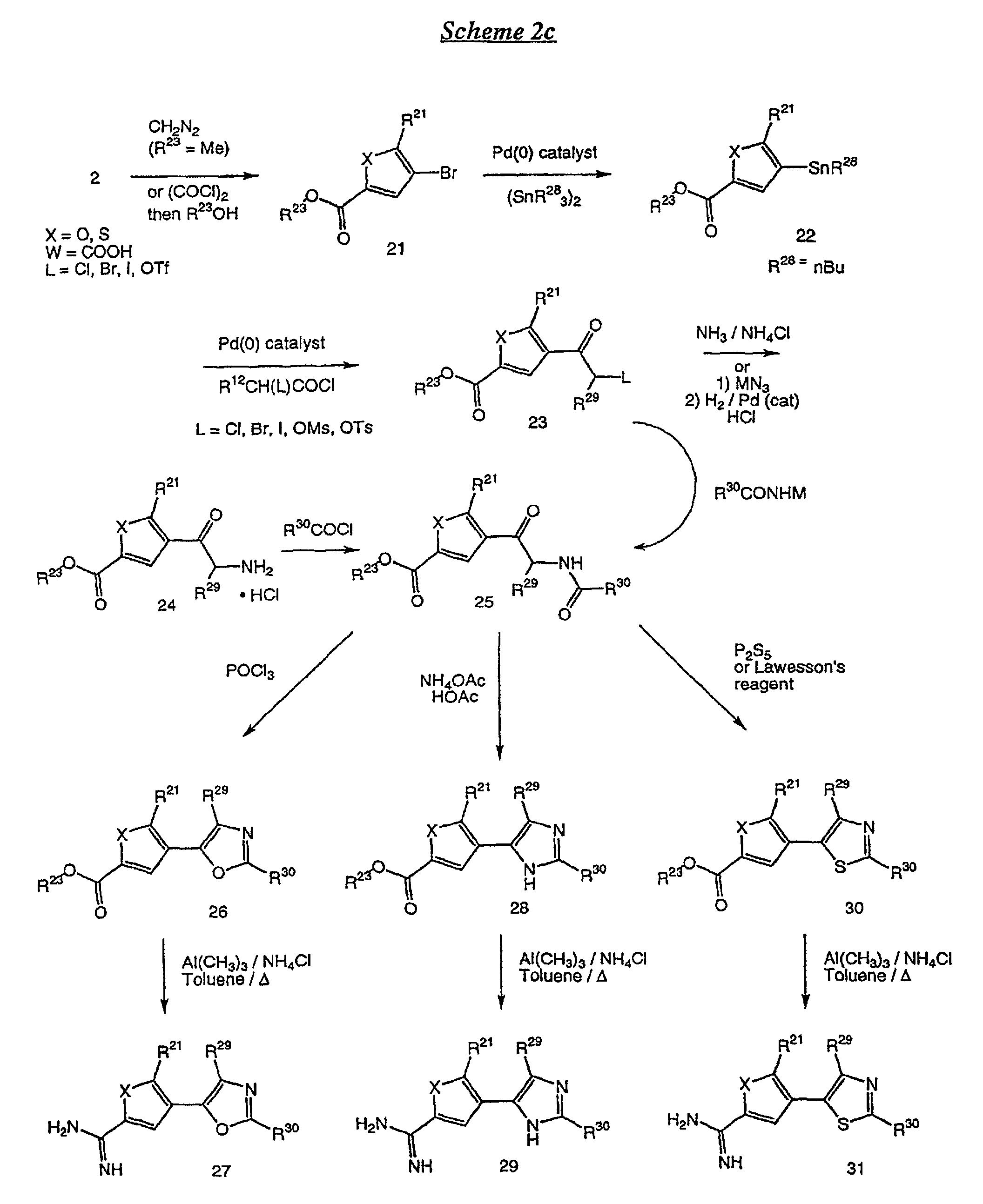

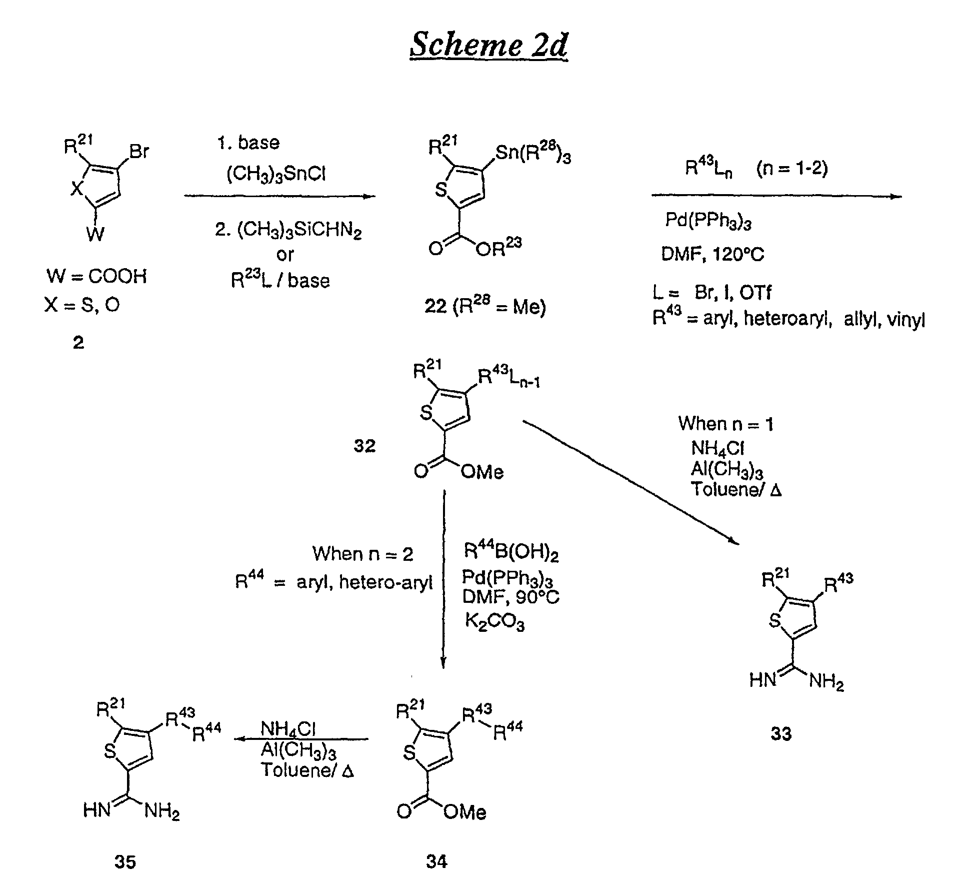

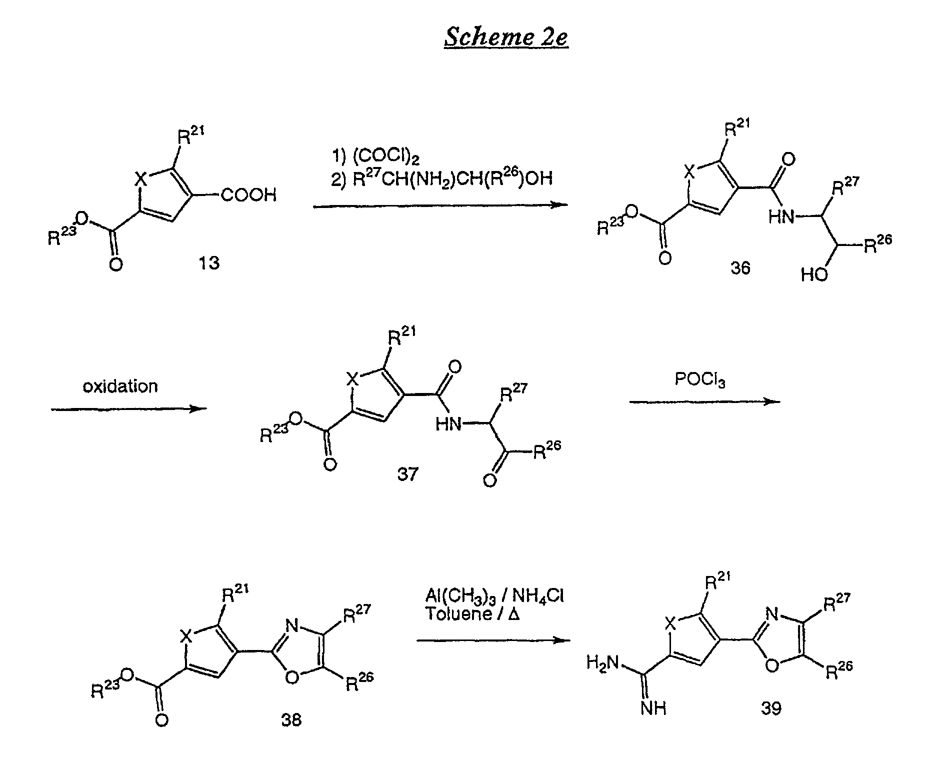

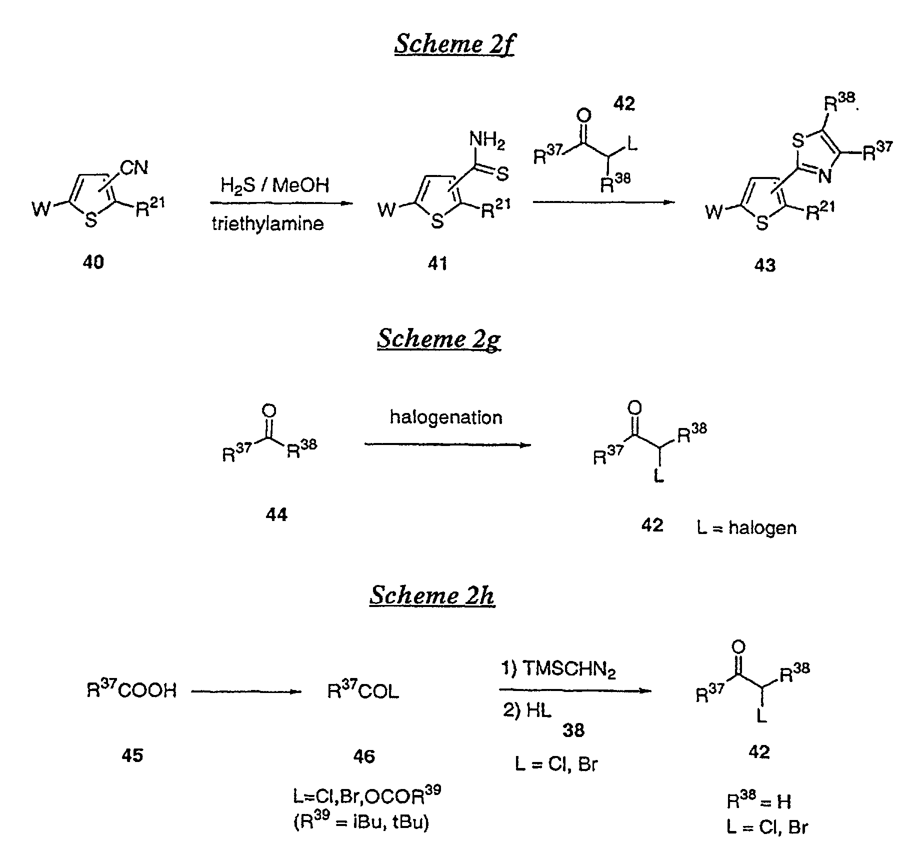

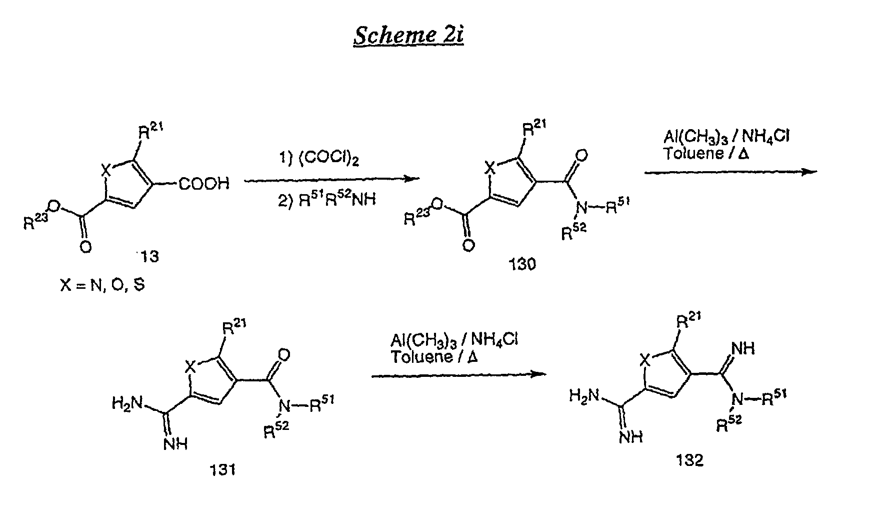

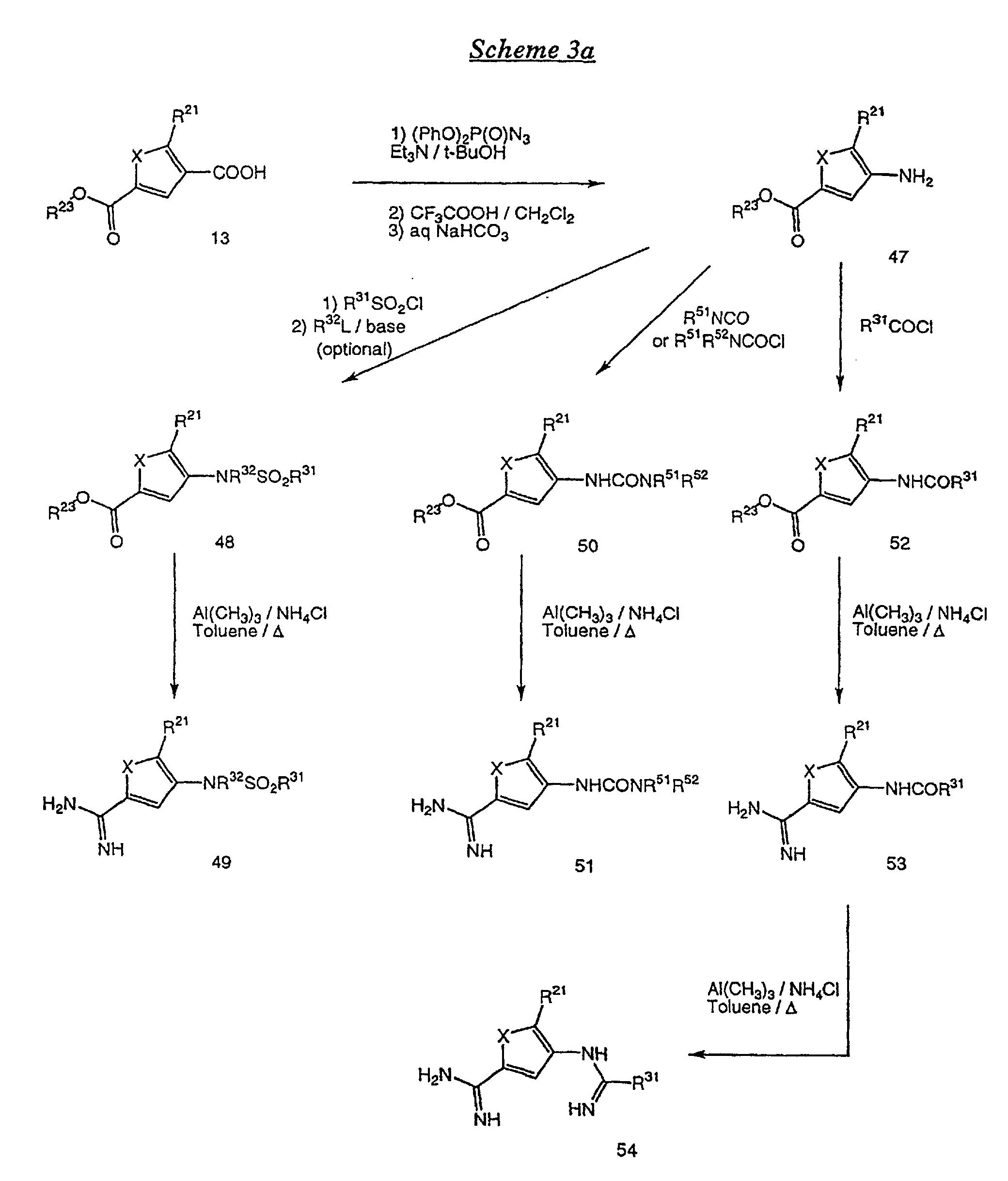

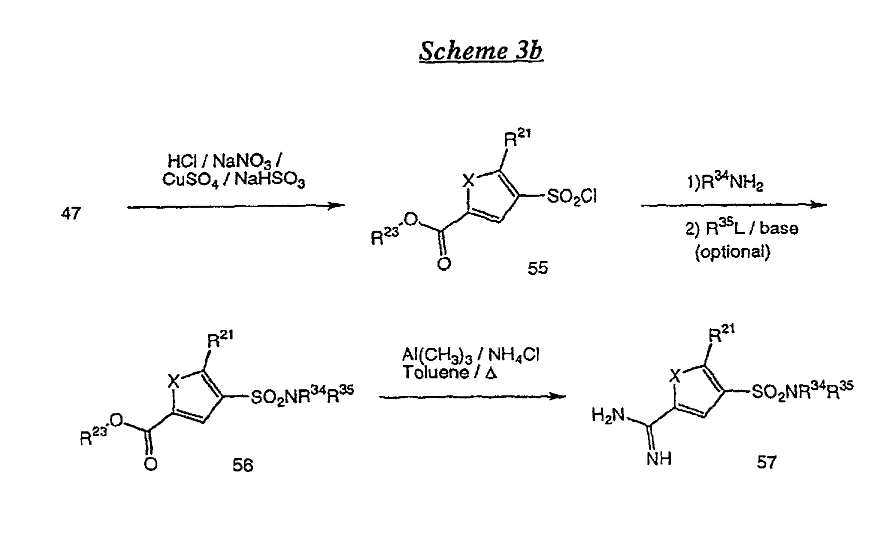

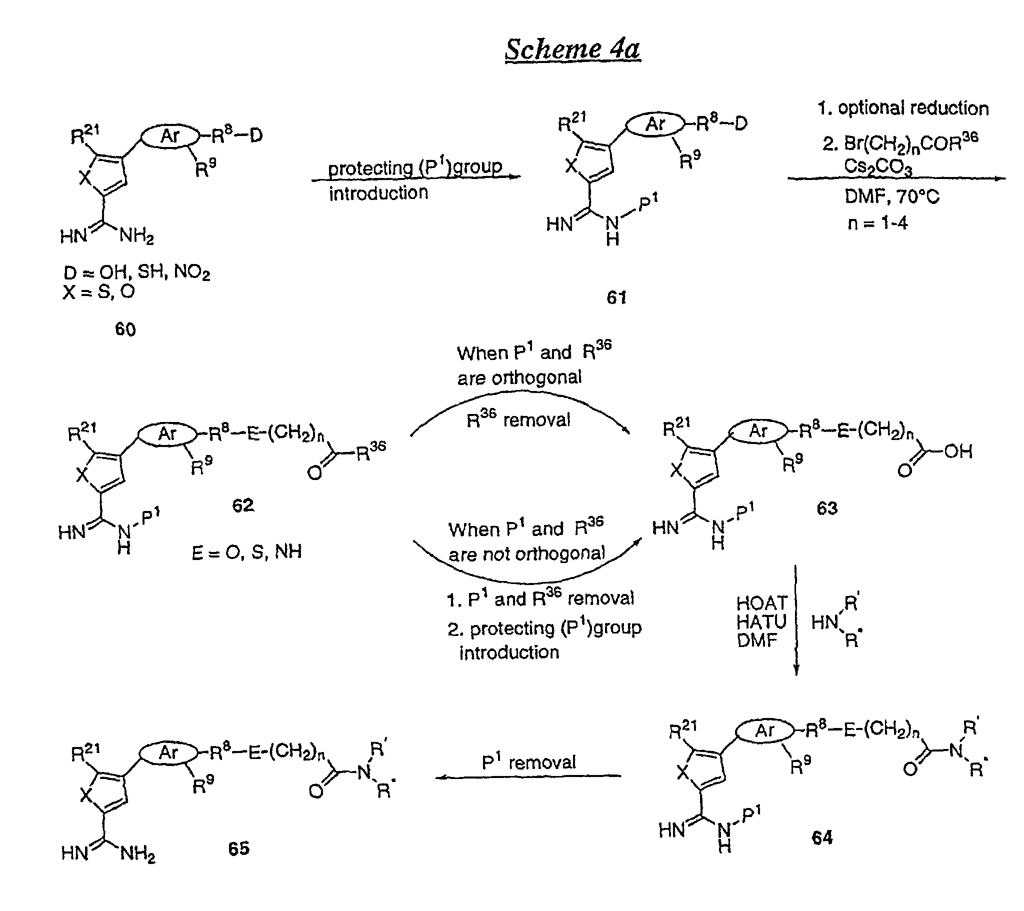

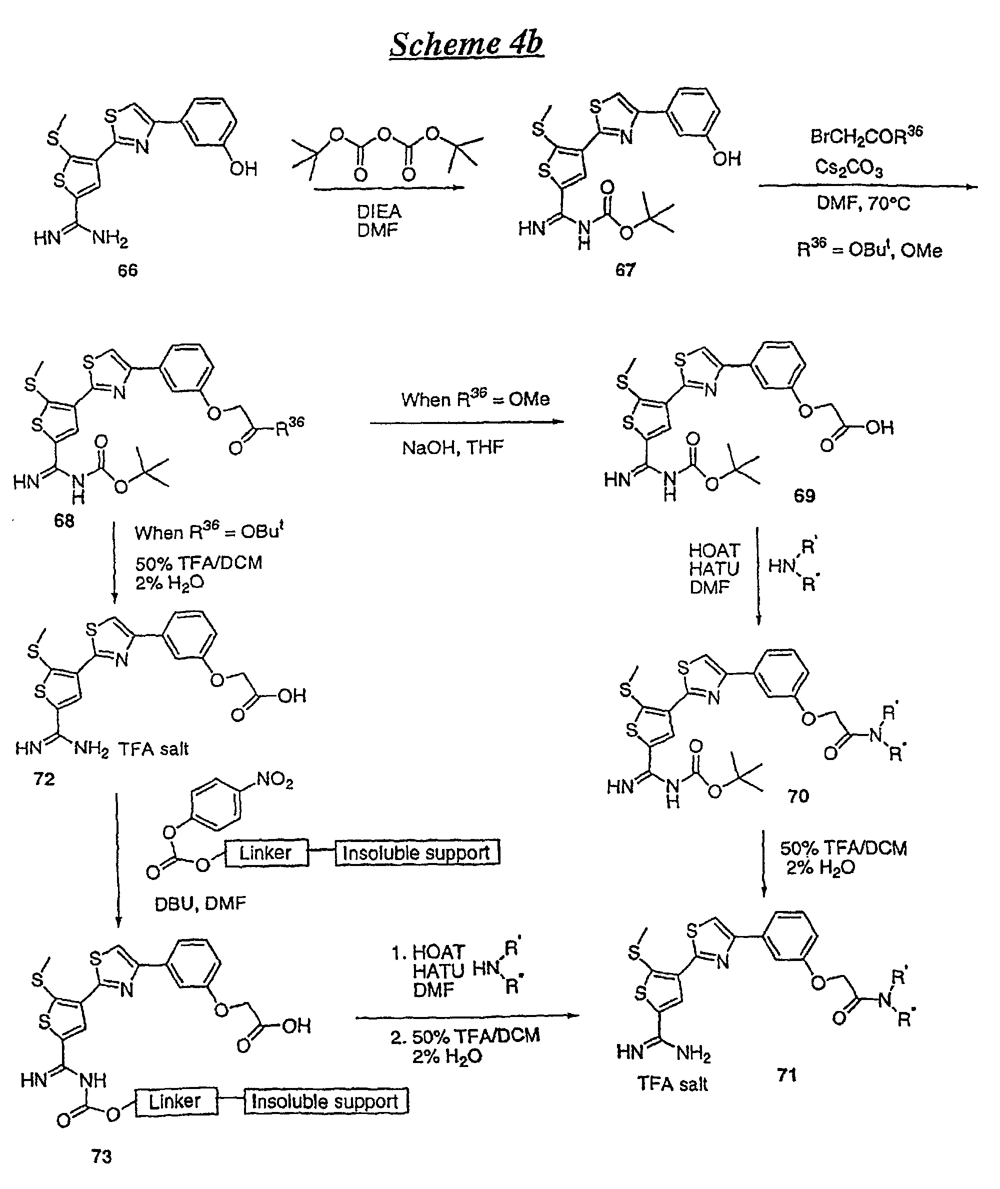

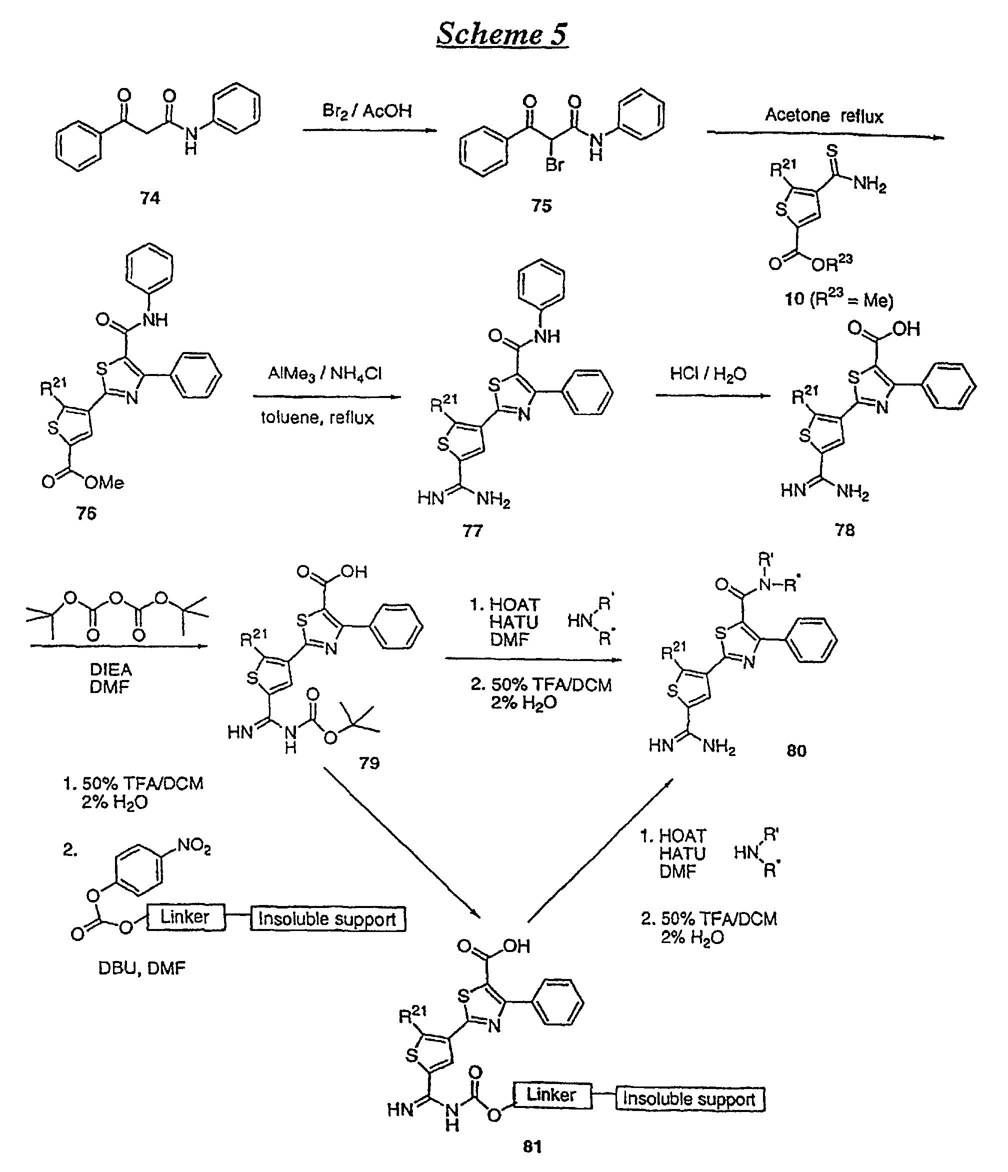

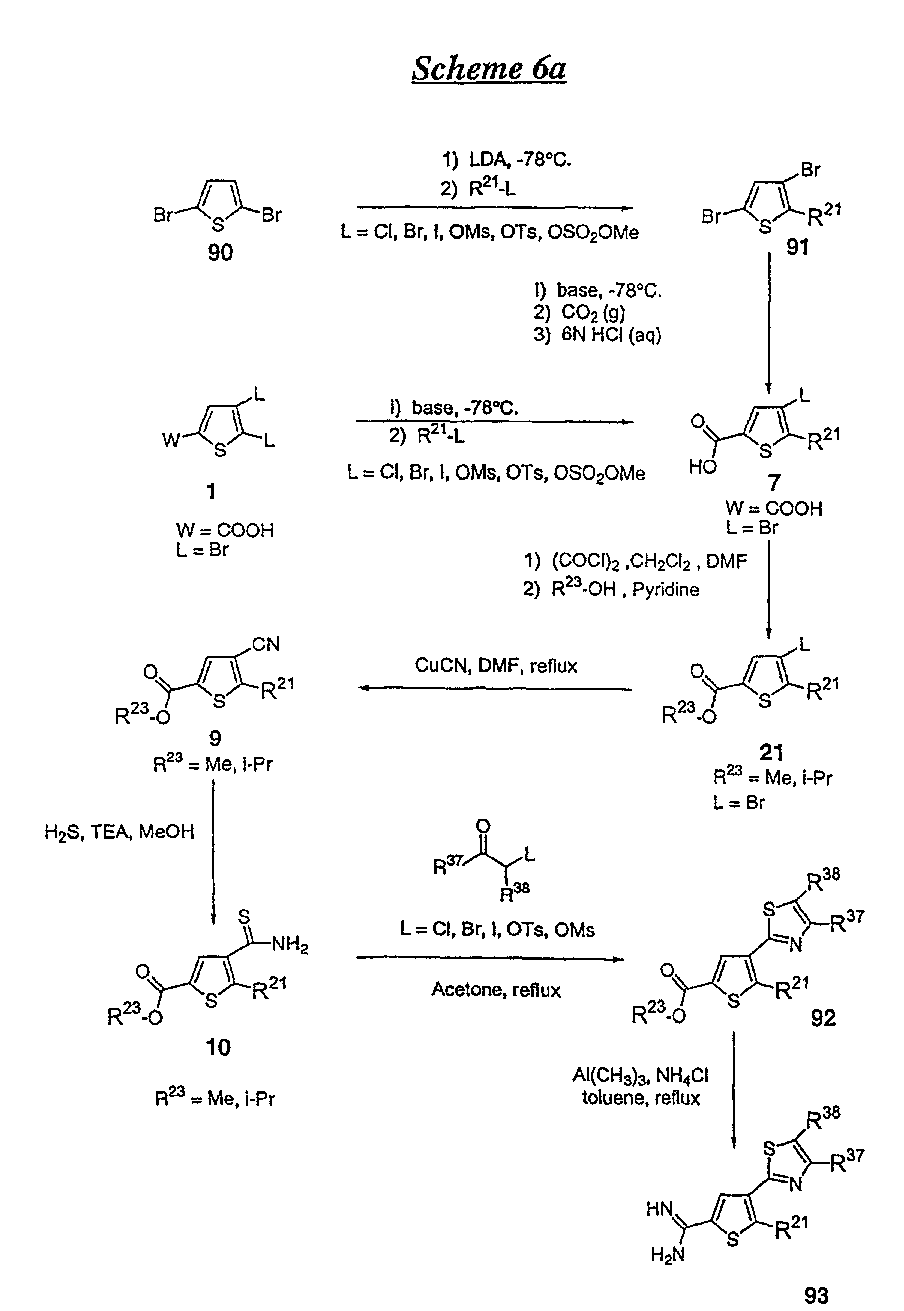

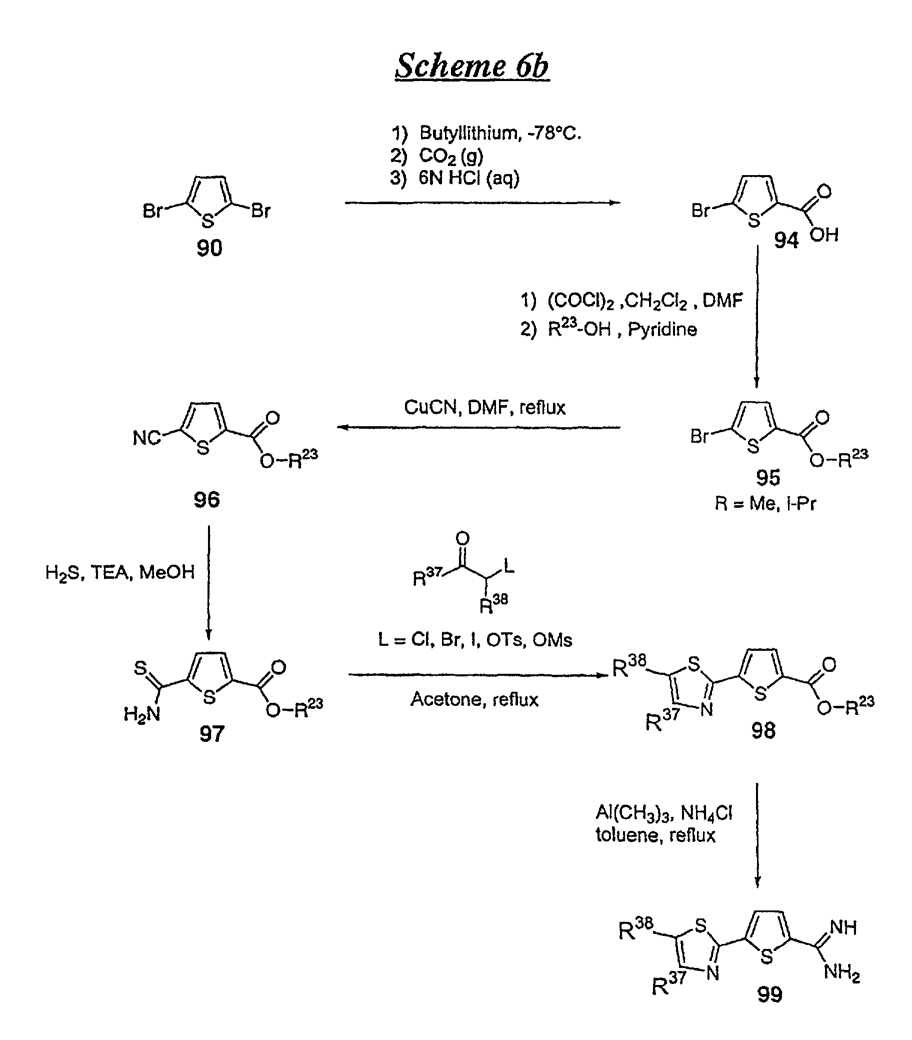

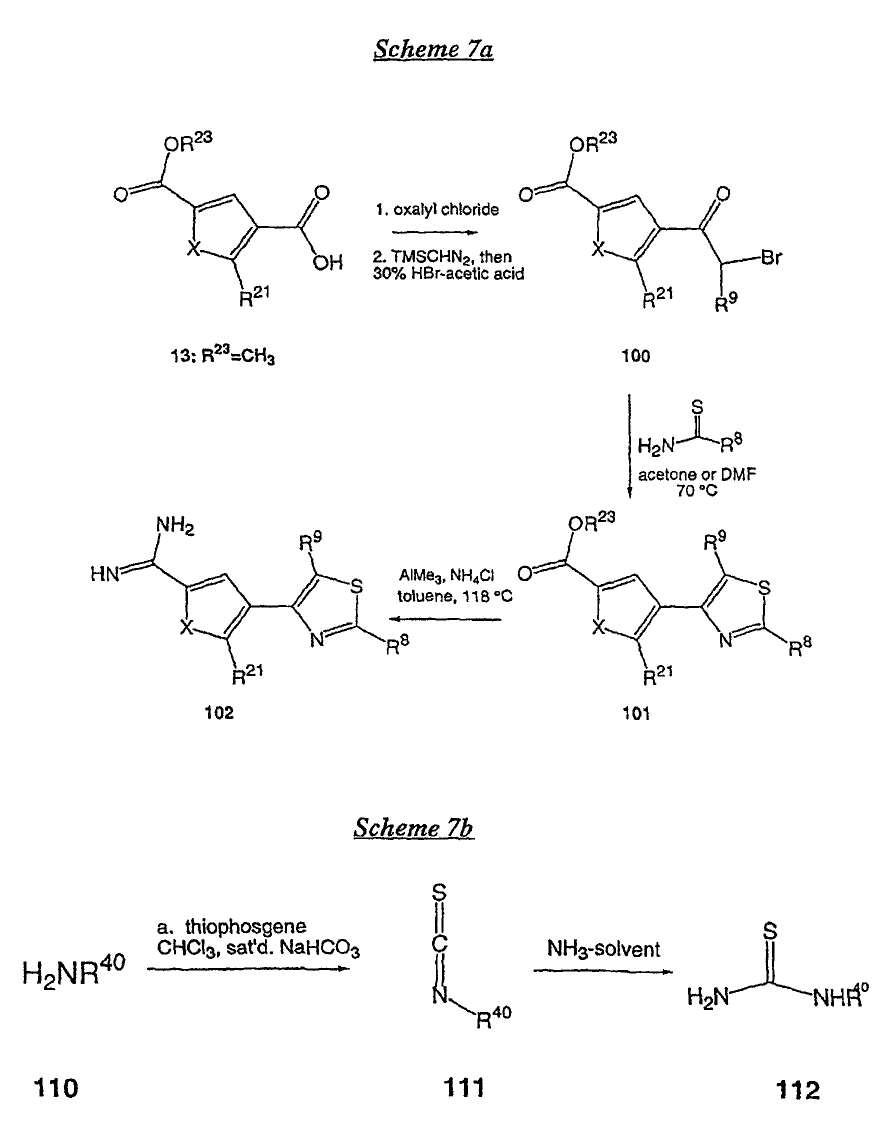

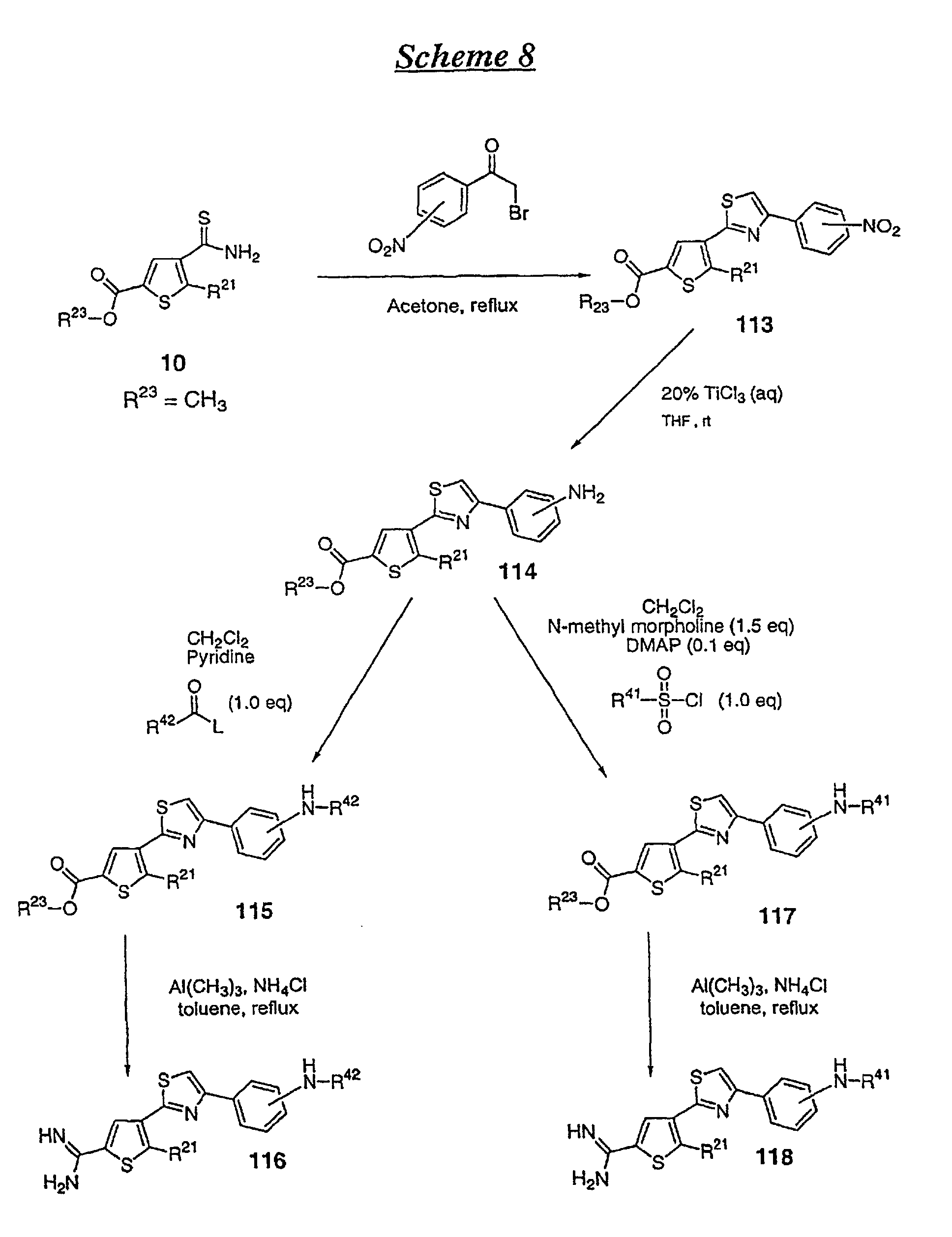

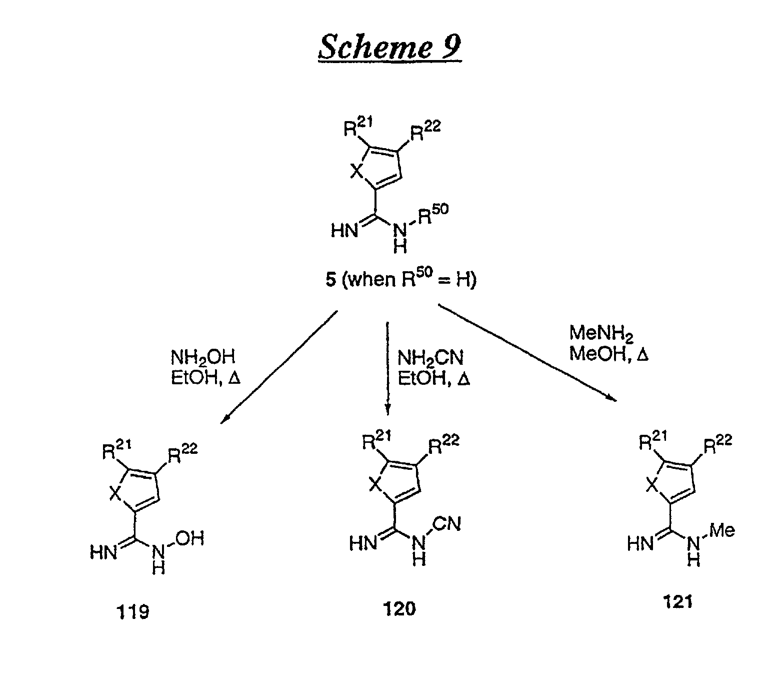

For medicinal use, the pharmaceutically acceptable acid addition salts, those salts in which the anion does not contribute significantly to toxicity or pharmacological activity of the organic cation, are preferred. The acid addition salts are obtained either by reaction of an organic base of Formula ***I*** with an organic or inorganic acid, preferably by contact in solution, or by any of the standard methods detailed in the literature available to any practitioner skilled in the art. Examples of useful organic acids are carboxylic acids such as maleic acid, acetic acid, tartaric acid, propionic acid, fumaric acid, isethionic acid, succinic acid, cyclamic acid, pivalic acid and the like; useful inorganic acids are hydrohalide acids such as HCl, HBr, HI; sulfuric acid; phosphoric acid and the like. Preferred acids for forming acid addition salts include HCl and acetic acid.

The compounds of the present invention represent a novel class of potent inhibitors of metallo, acid, thiol and serine proteases. Examples of the serine proteases inhibited by compounds within the scope of the invention include leukocyte neutrophil elastase, a proteolytic enzyme implicated in the pathogenesis of emphysema; chymotrypsin and trypsin, digestive enzymes; pancreatic elastase, and cathepsin G, a chymotrypsin-like protease also associated with leukocytes; thrombin and factor Xa, proteolytic enzymes in the blood coagulation pathway. Inhibition of thermolysin, a metalloprotease, and pepsin, an acid protease, are also contemplated uses of compounds of the present invention. The compounds of the present invention are preferably employed to inhibit trypsin-like proteases.

Compounds of the present invention that inhibit urokinase plasminogen activator are potentially useful in treating excessive cell growth disease state. Compounds of the present that inhibit urokinase are, therefore, useful as anti-angiogenic, anti-arthritic, anti-inflammatory, anti-invasive, anti-metastatic, anti-restenotic, anti-osteoporotic, anti-retinopathic (for angiogenesis-dependent retinopathies), contraceptive, and tumoristatic treatment agents. For example, such treatment agents are useful in the treatment of a variety of disease states, including but not limited to, benign prostatic hypertrophy, prostatic carcinoma, tumor metastasis, restenosis and psoriasis. Also provided are methods to inhibit extracellular proteolysis, methods to treat benign prostatic hypertrophy, prostatic carcinoma, tumor metastasis, restenosis and psoriasis by administering the compound of Formula ***I***. For their end-use application, the potency and other biochemical parameters of the enzyme inhibiting characteristics of compounds of the present invention are readily ascertained by standard biochemical techniques well known in the art. Actual dose ranges for this application will depend upon the nature and severity of the disease state of the patient or animal to be treated as determined by the attending diagnostician. It is to be expected that a general dose range will be about 0.01 to 50 mg, preferably 0.1 to about 20 mg per kg per day for an effective therapeutic effect.

An end use application of the compounds that inhibit chymotrypsin and trypsin is in the treatment of pancreatitis. For their end-use application, the potency and other biochemical parameters of the enzyme-inhibiting characteristics of the compounds of the present invention is readily ascertained by standard biochemical techniques well known in the art. Actual dose ranges for their specific end-use application will, of course, depend upon the nature and severity of the disease state of the patient or animal to be treated, as determined by the attending diagnostician. It is expected that a useful dose range will be about 0.01 to about 50 mg, preferably about 0.1 to about 20 mg per kg per day for an effective therapeutic effect.

Compounds of the present invention that are distinguished by their ability to inhibit either factor Xa or thrombin may be employed for a number of therapeutic purposes. As factor Xa or thrombin inhibitors, compounds of the present invention inhibit thrombin production. Therefore, these compounds are useful for the treatment or prophylaxis of states characterized by abnormal venous or arterial thrombosis involving either thrombin production or action. These states include, but are not limited to, deep vein thrombosis; disseminated intravascular coagulopathy which occurs during septic shock, viral infections and cancer; myocardial infarction; stroke; coronary artery bypass; fibrin formation in the eye; hip replacement; and thrombus formation resulting from either thrombolytic therapy or percutaneous transluminal coronary angioplasty (PCTA).

By virtue of the effects of both factor Xa and thrombin on a host of cell types, such as smooth muscle cells, endothelial cells and neutrophils, the compounds of the present invention find additional use in the treatment or prophylaxis of adult respiratory distress syndrome; inflammatory responses; wound healing; reperfusion damage; atherosclerosis; and restenosis following an injury such as balloon angioplasty, atherectomy, and arterial stent placement. The compounds of the present invention may be useful in treating neoplasia and metastasis as well as neurodegenerative diseases, such as Alzheimer's disease and Parkinson's disease.

When employed as thrombin or factor Xa inhibitors, the compounds of the present invention may be administered in an effective amount within the dosage range of about 0.1 to about 500 mg/kg, preferably between 0.1 to 30 mg/kg body weight, on a regimen in single or 2-4 divided daily doses.

Human leucocyte elastase is released by polymorphonuclear leukocytes at sites of inflammation and thus is a contributing cause for a number of disease states. Compounds of the present invention are expected to have an anti-inflammatory effect useful in the treatment of gout, rheumatoid arthritis and other inflammatory diseases, and in the treatment of emphysema. The leucocyte elastase inhibitory properties of compounds of the present invention are determined by the method described below. Cathepsin G has also been implicated in the disease states of arthritis, gout and emphysema, and in addition, glomerulonephritis and lung infestations caused by infections in the lung. In their end-use application the enzyme inhibitory properties of the compounds of Formula ***I*** is readily ascertained by standard biochemical techniques that are well-known in the art.

The Cathepsin G inhibitory properties of compounds within the scope of the present invention are determined by the following method. A preparation of partially purified human Cathepsin G is obtained by the procedure of Baugh *et al*., *Biochemistry 15*: 836 (1979). Leukocyte granules are a major source for the preparation of leukocyte elastase and cathepsin G (chymotrypsin-like activity). Leukocytes are lysed and granules are isolated. The leukocyte granules are extracted with 0.20 M sodium acetate, pH 4.0, and extracts are dialyzed against 0.05 M Tris buffer, pH 8.0 containing 0.05 M NaCl overnight at 4°C. A protein fraction precipitates during dialysis and is isolated by centrifugation. This fraction contains most of the chymotrypsinlike activity of leukocyte granules. Specific substrates are prepared for each enzyme, namely N-Suc-Ala-Ala-Pro-Val-*p*-nitroanilide and Suc-Ala-Ala-Pro-Phe-*p*-nitroanilide. The latter is not hydrolyzed by leukocyte elastase. Enzyme preparations are assayed in 2.00 mL of 0.10 M Hepes buffer, pH 7.5, containing 0.50 M NaCl, 10% dimethylsulfoxide and 0.0020 M Suc-Ala-Ala-Pro-Phe-*p*-nitroanilide as a substrate. Hydrolysis of the p-nitroanilide substrate is monitored at 405 nm and at 25°C.

Useful dose range for the application of compounds of the present invention as neutrophil elastase inhibitors and as Cathepsin G inhibitors depend upon the nature and severity of the disease state, as determined by the attending diagnostician, with a range of 0.01 to 10 mg/kg body weight, per day, being useful for the aforementioned disease states.

Additional uses for compounds of the present invention include analysis of commercial reagent enzymes for active site concentration. For example, chymotrypsin is supplied as a standard reagent for use in clinical quantitation of chymotrypsin activity in pancreatic juices and feces. Such assays are diagnostic for gastrointestinal and pancreatic disorders. Pancreatic elastase is also supplied commercially as a reagent for quantitation of α₁-antitrypsin in plasma. Plasma α₁-antitrypsin increases in concentration during the course of several inflammatory diseases, and α₁-antitrypsin deficiencies are associated with increased incidence of lung disease. Compounds of the present invention can be used to enhance the accuracy and reproducibility of these assays by titrametric standardization of the commercial elastase supplied as a reagent. See, U.S. Patent No. 4,499,082.

Protease activity in certain protein extracts during purification of particular proteins is a recurring problem which can complicate and compromise the results of protein isolation procedures. Certain proteases present in such extracts can be inhibited during purification steps by compounds of the present invention, which bind tightly to various proteolytic enzymes.

The pharmaceutical compositions of the invention can be administered to any animal that can experience the beneficial effects of the compounds of the invention. Foremost among such animals are humans, although the invention is not intended to be so limited.

The pharmaceutical compositions of the present invention can be administered by any means that achieve their intended purpose. For example, administration can be by parenteral, subcutaneous, intravenous, intramuscular, intraperitoneal, transdermal, buccal, or ocular routes. Alternatively, or concurrently, administration can be by the oral route. The dosage administered will be dependent upon the age, health, and weight of the recipient, kind of concurrent treatment, if any, frequency of treatment, and the nature of the effect desired.

In addition to the pharmacologically active compounds, the new pharmaceutical preparations can contain suitable pharmaceutically acceptable carriers comprising excipients and auxiliaries that facilitate processing of the active compounds into preparations that can be used pharmaceutically.

The pharmaceutical preparations of the present invention are manufactured in a manner that is, itself, known, for example, by means of conventional mixing, granulating, dragee-making, dissolving, or lyophilizing processes. Thus, pharmaceutical preparations for oral use can be obtained by combining the active compounds with solid excipients, optionally grinding the resulting mixture and processing the mixture of granules, after adding suitable auxiliaries, if desired or necessary, to obtain tablets or dragee cores.

Suitable excipients are, in particular, fillers such as saccharides, for example, lactose or sucrose, mannitol or sorbitol, cellulose preparations and/or calcium phosphates, for example, tricalcium phosphate or calcium hydrogen phosphate, as well as binders, such as, starch paste, using, for example, maize starch, wheat starch, rice starch, potato starch, gelatin, tragacanth, methyl cellulose, hydroxypropylmethylcellulose, sodium carboxymethylcellulose, and/or polyvinyl pyrrolidone. If desired, disintegrating agents can be added, such as, the above-mentioned starches and also carboxymethyl-starch, crosslinked polyvinyl pyrrolidone, agar, or alginic acid or a salt thereof, such as, sodium alginate. Auxiliaries are, above all, flow-regulating agents and lubricants, for example, silica, talc, stearic acid or salts thereof, such as, magnesium stearate or calcium stearate, and/or polyethylene glycol. Dragee cores are provided with suitable coatings that, if desired, are resistant to gastric juices. For this purpose, concentrated saccharide solutions can be used, which may optionally contain gum arabic, talc, polyvinyl pyrrolidone, polyethylene glycol, and/or titanium dioxide, lacquer solutions and suitable organic solvents or solvent mixtures. In order to produce coatings resistant to gastric juices, solutions of suitable cellulose preparations, such as, acetylcellulose phthalate or hydroxypropylmethyl-cellulose phthalate, are used. Dye stuffs or pigments can be added to the tablets or dragee coatings, for example, for identification or in order to characterize combinations of active compound doses.

Other pharmaceutical preparations which can be used orally include push-fit capsules made of gelatin, as well as soft, sealed capsules made of gelatin and a plasticizer, such as, glycerol or sorbitol. The push-fit capsules can contain the active compounds in the form of granules that may be mixed with fillers such as lactose, binders such as starches, and/or lubricants such as talc or magnesium stearate and, optionally, stabilizers. In soft capsules, the active compounds are preferably dissolved or suspended in suitable liquids, such as, fatty oils or liquid paraffin. In addition, stabilizers may be added.

Suitable formulations for parenteral administration include aqueous solutions of the active compounds in water-soluble form, for example, water-soluble salts, alkaline solutions and cyclodextrin inclusion complexes. Especially preferred salts are hydrochloride and acetate salts. One or more modified or unmodified cyclodextrins can be employed to stabilize and increase the water solubility of compounds of the present invention. Useful cyclodextrins for this purpose are disclosed in U.S. Patent Nos. 4,727,064, 4,764,604, and 5,024,998.

In addition, suspensions of the active compounds as appropriate oily injection suspensions can be administered. Suitable lipophilic solvents or vehicles include fatty oils, for example, sesame oil, or synthetic fatty acid esters, for example, ethyl oleate or triglycerides or polyethylene glycol-400 (the compounds are soluble in PEG-400). Aqueous injection suspensions can contain substances that increase the viscosity of the suspension, for example, sodium carboxymethyl cellulose, sorbitol, and/or dextran. Optionally, the suspension may also contain stabilizers.

The following examples are illustrative, but not limiting, of the method and compositions of the present invention. Other suitable modifications and adaptations of the variety of conditions and parameters normally encountered and obvious to those skilled in the art are within the spirit and scope of the invention.

### Example 1

### 4-[4-(4-chlorophenyl)(1,3-thiazol-2-yl)]-5-methylthiothiophene-2-carboxamidine

Trimethylaluminum (2.0 M in toluene, 2 mL) was added dropwise over 10 min to a suspension of ammonium chloride (216 mg) in toluene (2 mL), stirred under N₂ at 0°C. When gas evolution moderated, the mixture was stirred at 25°C for 30 min, when most of the solid had dissolved, methyl 4-[4-(4-chlorophenyl)(1,3-thiazol-2-yl)]-5-methylthiothiophene-2-carboxylate (100 mg, Maybridge Chemical Co., Cornwall, U.K.) was added in one portion. This solution was heated to reflux in stages over 1 h. After 2.5 h of reflux, the reaction mixture was allowed to cool to 25°C, and was poured on to a vigorously stirred slurry of silica gel (2 g) in CHCl₃ (20 mL). After 20 min the solids were collected by suction filtration, and washed with MeOH (3x10 mL). The combined filtrates were evaporated to dryness, and the residual yellow solid was subjected to preparative thin-layer chromatography to obtain 77 mg of 4-[(4-chlorophenyl)thiazol-2-yl]-5-methylthiothiophene-2-carboxamidine as a yellow solid. ¹H-NMR (DMSO-d₆; 300 MHz) δ 2.80 (s, 3H), 7.55-7.59 (m, 1H), 8.04-8.13 (m, 1H), 8.31 (s, 1H), 8.69 (s, 1H), ), 9.2 (broad s, 4H). Mass spectrum (MALDI-TOF, m/z): Calcd. for C₁₅H₁₂ClN₃S₃, 365.9 (M+H), found 366.9.

### Example 2

### 5-Methylthiothiophene-2-carboxamidine

5-(Methylthio)thiophene-2-carbonitrile (100 mg, Maybridge Chemical Company, Cornwall, UK) was taken in a dry 2 dram vial. To this a solution of saturated HCl in anhydrous MeOH (4 mL) was added. The vial was tightly capped and the mixture was stirred for 24 h. The vial was cooled in an ice bath, uncapped and N₂ was bubbled through the solution to remove dissolved HCl. The solvent was removed under reduced pressure and the resulting residue was dried under high vacuum for 24 h. A solution of methanolic ammonia (2M NH₃ in MeOH) was added to the vial, and the mixture was stirred for 3 days. Methanol was removed under vacuum and the resulting residue was subjected to preparative thin-layer chromatography to obtain 5-(methylthio)thiophene-2-carboxamidine as a yellow solid. ¹H-NMR (DMSO-d₆; 300 MHz) δ 2.64 (s, 3H), 7.22 (d, J = 3.75 Hz, 1H), 7.95 (broad d, J = 3.33 Hz, 1H), 9.4 (broad s, 4H). Mass spectrum (MALDI-TOF, m/z): Calcd. for C₆H₈N₂S₂, 172.3 (M+H), found 173.0.

### Example 3

### 5-Methylthio-4-phenylthiophene-2-carboxamidine

Methyl 5-methylthio-4-phenylthiophene-2-carboxylate (100 mg, Maybridge Chemical Company, Cornwall, UK) was treated in a manner similar to that for Example 1, to give 50 mg of 4-phenyl-5-methylthiothiophene-2-carboxamidine as an off-white solid. ¹H-NMR (DMSO-d₆; 300 MHz) δ 2.65 (s, 3H), 7.39-7.60 (m, 5H), 8.27 (s, 1H), 9.2 (broad s, 4H). Mass spectrum (MALDI-TOF, m/z): Calcd. for C₁₂H₁₂N₂S₂, 248.4 (M+H), found 249.0.

### Example 4

### 4-[4-(2,4-Dichlorophenyl)(1,3-thiazol-2-yl)]-5-methylthiothiophene-2-carboxamidine

Methyl 4-[4-(2,4-dichlorophenyl)(1,3-thiazol-2-yl)]-5-methylthiothiophene-2-carboxylate (100 mg, Maybridge Chemical Company, Cornwall, UK) was treated in a manner similar to that for Example 1, to give 60 mg of 4-[4-(2,4-dichlorophenyl)thiazol-2-yl]-5-methylthiothiophene-2-carboxamidine as a yellow solid. ¹H-NMR (DMSO-d₆; 300 MHz) δ 2.77 (s, 3H), 7.6 (dd, J = 2.2 and 8.5 Hz, 1H), 7.79 (d, J = 2.2 Hz, 1H), 8.09 (d, J = 8.5 Hz, 1H), 8.3 (s, 1H), 8.6 (s, 1H). Mass spectrum (MALDI-TOF, m/z): Calcd. for C₁₅H₁₁N₃S₃Cl₂, 400.0 (M+H), found 400.1.

### Examples 5

### 4-(4-Methyl(1,3-thiazol-2-yl))-5-methylthiothiophene-2-carboxamidine

Methyl 4-(4-methyl(1,3-thiazol-2-yl))-5-methylthiothiophene-2-carboxylate (100 mg, Maybridge Chemical Company, Cornwall, UK) was treated in a manner similar to that for Example 1, to give 40 mg of 4-(4-methylthiazol-2-yl)-5-methylthiothiophene-2-carboxamidine as a yellow solid. ¹H-NMR (DMSO-d₆; 300 MHz) δ 2.43 (s, 3H), 2.7 (s, 3H), 7.38 (s, 1H), 8.28 (s, 1H). Mass spectrum (MALDI-TOF, m/z): Calcd. for C₁₀H₁₁N₃S₃, 270.0 (M+H), found 270.1.

### Example 6

***a) Methyl 5-methylthio-4-(4-(2-naphthyl)(1,3-thiazol-2-yl))thiophene-2-carboxylate:*** Methyl 4-(aminothioxomethyl)-5-methylthiothiophene-2-carboxylate (40 mg, Maybridge Chemical Company, Cornwall, UK) was reacted with 2-bromo-2'-acetonaphthone (1.1 eq) in a manner similar to Example 13 step (a) to give 40 mg of methyl 5-methylthio-4-(4-(2-naphthyl)(1,3-thiazol-2-yl))thiophene-2-carboxylate. ¹H-NMR (CDCl₃/CD₃OD; 300 MHz) δ 3.71 (s, 3H), 3.94 (s, 3H), 7.47-7.55 (m, 2H), 7.67 (s, 1H), 7.84-7.99 (m, 3H), 8.08 (dd, J = 1.75 Hz and 8.6 Hz, 1H), 8.3 (s, 1H), 8.5 (s, 1H).

***b) 5-Methylthio-4-(4-(2-naphthyl)(1,3-thiazol-2-yl))thiophene-2-carboxamidine:*** Methyl 5-methylthio-4-(4-(2-naphthyl)(1,3-thiazol-2-yl))thiophene-2-carboxylate, (40 mg) as prepared in the previous step was treated in a manner similar to that for Example 1, to give 30 mg of4-[4-(naphth-2-yl)thiazol-2-yl]-5-methylthiothiophene-2-carboxamidine. ¹H-NMR (DMSO-d₆; 300 MHz) δ 2.83 (s, 3H), 7.52-7.69 (m, 2H), 7.95-8.01 (m, 2H), 8.05 (d, J = 8.6 Hz, 1H), 8.24 (dd, J = 1.69 Hz and 8.6 Hz, 1H), 8.4 (s, 1H), 8.65 (s, 1H), 8.74 (s, 1H). Mass spectrum (MALDI-TOF, CHCA matrix, m/z): Calcd. for C₁₉H₁₅N₃S₃, 382.1 (M+H), found 382.0.

### Example 7

### Synthesis of 5-methylthio-4-[4-(4-phenylphenyl)(1,3-thiazol-2-yl)]thiophene-2-carboxamidine hydrochloride

***a) Synthesis of methyl 5-methylthio-4-[4-(4-phenylphenyl)(1,3-thiazol-2-yl)]thiophene-2-carboxylate:*** 27 mg (0.109 mmol) of methyl 4-(aminothioxomethyl)-5-methylthiothiophene-2-carboxylate (Maybridge Chemical Co. LTD., Cornwall, U.K.) was dissolved in 2 mL of reagent grade acetone. 4'-Phenyl-2-bromoacetophenone (33 mg; 0.120 mmol; Aldrich Chemical Co., Milwaukee, WI) was added and the solution was allowed to reflux for 2.5 h. The solution was allowed to cool and solid was filtered and washed with methanol and dried *in vacuo* to afford 30 mg (65% yield) of methyl 5-methylthio-4-[4-(4-phenylphenyl)(1,3-thiazol-2-yl)]thiophene-2-carboxylate. ¹H-NMR (DMSO-d₆, 300 MHz) δ 8.28 (s, 1H), 8.24 (s, 1H), 8.17 (d, J=8.5 Hz, 2H), 7.8 (d, J=8.5Hz, 2H), 7.74-7.77 (m, 2H), 7.48-7.53 (m, 2H), 7.37-7.42(m, 1H), 2.78 (s, 3H). Mass Spectrum (MALDI-TOF, CHCA matrix, m/z) Calcd. for C₂₂H₁₆NO₂S₃: 423.0 (M+H), found 424.4.

***b) Synthesis of 5-methylthio-4-[4-(4-phenylphenyl)(1,3-thiazol-2-yl)]thiophene-2-carboxamidine hydrochloride:*** To a stirred suspension of 0.473 mmol (25 mg) of ammonium chloride (Fisher Scientific Pittsburgh, PA) in 2 mL of anhydrous toluene (Aldrich Chemical Co.) placed under nitrogen atmosphere at 0°C, 237 µL (0.473 mmol) of 2M trimethylaluminum in toluene (Aldrich Chemical Co.) was added via syringe over 10 min and then let stir at 0°C for 30 min after which 20 mg (0.0473 mmol) of methyl 5-methylthio-4-[4-(4-phenylphenyl)(1,3-thiazol-2-yl)]thiophene-2-carboxylate was added to solution and allowed to reflux for 2.5 h. The reaction mixture was quenched by pouring over a slurry of 500 mg of silica in 10 mL of chloroform. The silica was poured onto a sintered glass funnel and washed with a 10% methanol/CH₂Cl₂ solution and concentrated. The crude product was purified on a 1 mm silica prep plate eluting with 10% methanol/CH₂Cl₂ to afford 10 mg (53% yield) of 5-methylthio-4-[4-(4-phenylphenyl)(1,3-thiazol-2-yl)]thiophene-2-carboxamidine hydrochloride. Mass Spectrum (MALDI-TOF, CHCA matrix, m/z) Calcd. for C₂₁H₁₇N₃S₃: 408.1(M+H), found 408.0.

### Examples 8 & 9

### Synthesis of 4-[4-(3-methoxyphenyl)(1,3-thiazol-2-yl)]-5-methylthiothiophene-2-carboxamidine hydrochloride and 4-[4-(3-hydroxyphenyl)(1,3-thiazol-2-yl)]-5-methylthiothiophene-2-carboxamidine hydrochloride

***a) Synthesis of methyl 4-[4-(3-methoxyphenyl)(1,3-thiazol-2-yl)]-5-methylthiothiophene-2-carboxylate:*** 32 mg (0.133 mmol) of methyl 4-(aminothioxomethyl)-5-methylthiothiophene-2-carboxylate (Maybridge Chemical Co. LTD., Cornwall, U.K.) was dissolved in 2 mL of reagent grade acetone. 3'-Methoxy-2-bromo acetophenone (0.155 mmol; 36 mg; Aldrich Chemical Co.) was added and the solution was allowed to reflux for 2.5 h The solution was allowed to cool and a solid was filtered and washed with methanol and dried *in vacuo*. The solid was purified on 1 mm silica plate eluting with 25% ethyl acetate/hexane to afford 31 mg (63% yield) of methyl 4-[4-(3-methoxyphenyl)(1,3-thiazol-2-yl)]-5-methylthiothiophene-2-carboxylate.

***b) Synthesis of 4-[4-(3-methoxyphenyl)(1,3-thiazol-2-yl)]-5-methylthiothiophene-2-carboxamidine hydrochloride and 4-[4-(3-hydroxyphenyl)(1,3-thiazol-2-yl)]-5-*** ***methylthiothiophene-2-carboxamidine hydrochloride:*** To a stirred suspension of 0.821 mmol ( 44 mg) of ammonium chloride (Fisher Scientific) in 2 mL of anhydrous toluene (Aldrich Chemical Co.) placed under nitrogen atmosphere at 0°C, was added 411 µL (0.821 mmol) of 2M trimethylaluminum in toluene (Aldrich Chemical Co.) via syringe over 10 min and then let stir at 0°C for 30 min after which 31 mg (0.0821 mmol) of methyl 4-[4-(3-methoxyphenyl)(1,3-thiazol-2-yl)]-5-methylthiothiophene-2-carboxylate was added to solution and allowed to reflux for 2.5 h. The reaction mixture was quenched by pouring over a slurry of 500 mg of silica in 10 mL of chloroform. The silica was poured onto a sintered glass funnel and washed with a 10% methanol/CH₂Cl₂ solution and concentrated. The crude product was purified on a 1 mm silica prep plate eluting with 10% methanol/CH₂Cl₂ to afford 4.4 mg (15% yield) of 4-[4-(3-methoxyphenyl)(1,3-thiazol-2-yl)]-5-methylthiothiophene-2-carboxamidine hydrochloride and 4.2 mg (15% yield) of 4-[4-(3-hydroxyphenyl)(1,3-thiazol-2-yl)]-5-methylthiothiophene-2-carboxamidine hydrochloride. 4-[4-(3-methoxyphenyl)(1,3-thiazol-2-yl)]-5-methylthlothiophene-2-carboxamidine hydrochloride: ¹H-NMR (CD₃OD; 300 MHz) δ 8.5 (s, 1H), 7.9 (s, 1H), 7. 59-7.65 (m, 2H), 7.33-7.38 (m, 1H), 6.91-6.95 (m, 1H), 3.87 (s, 1H), 2.8 (s, 3H) Mass Spectrum (MALDI-TOF, CHCA matrix, m/z) Calcd. for C₁₆H₁₅N₃OS₃: 361.5(M+H), found 362.2. 4-[4-(3-hydroxyphenyl)(1,3-thiazol-2-yl)]-5-methylthiothiophene-2-carboxamidine hydrochloride: ¹H-NMR (CD₃OD; 300 MHz) δ 8.5 (s, 1H), 7.81 (s, 1H), 7.26-7.51 (m, 2H), 7.22-7.25 (m, 1H), 6.77-6.81 (m, 1H), 2.8 (s, 3H) Mass Spectrum (MALDI-TOF, CHCA matrix, m/z) Calcd. for C₁₅H₁₃N₃OS₃: 347.5(M+H), found 348.0.

### Example 10

### Synthesis of 5-methylthio-4-(4-phenyl(1,3-thiazol-2-yl))thiophene-2-carboxamidine hydrochloride

***a) Synthesis of methyl 5-methylthio-4-(4-phenyl(1,3-thiazol-2-yl))thiophene-2-carboxylate:*** 33 mg (0.133 mmol) methyl 4-(aminothioxomethyl)-5-methylthiothiophene-2-carboxylate (Maybridge Chemical Co. LTD., Cornwall, U.K.) was dissolved in 2 mL of reagent grade acetone. 2-Bromoacetophenone (0.133 mmol; 27 mg; Aldrich Chemical Co.) was added and the solution was allowed to reflux for 2.5 h. The solution was allowed to cool and the solid was filtered and washed with methanol and dried *in vacuo.* The solid was purified on I mm silica plate eluting with 25% ethyl acetate/hexane mixture to afford 46 mg (90% yield) of methyl 5-methylthio-4-(4-phenyl(1,3-thiazol-2-yl))thiophene-2-carboxylate.

***b) Synthesis of 5-methylthio-4-(4-phenyl(1,3-thiazol-2-yl))thiophene-2-carboxamidine hydrochloride:*** To a stirred suspension of 1.32 mmol (71 mg) of ammonium chloride (Fisher Scientific) in 2 mL of anhydrous toluene (Aldrich Chemical Co.) placed under nitrogen atmosphere at 0°C, 662 µL (1.32 mmol) of 2M trimethylaluminum in toluene (Aldrich Chemical Co.) was added via syringe over 10 min and then let stir at 0°C for 30 min after which 46 mg (0.133 mmol) of methyl 5-methylthio-4-(4-phenyl(1,3-thiazol-2-yl))thiophene-2-carboxylate was added to solution and allowed to reflux for 2.5 h. The reaction mixture was quenched by pouring over a slurry of 500 mg of silica in 10 mL of chloroform. The silica was poured onto a sintered glass funnel and washed with a 10% methanol/CH₂Cl₂ solution and concentrated. The crude product was purified on a 2 g silica silica SPE column eluting with 10% methanol/CH₂Cl₂ to afford 32.5 mg (75% yield) of 5-methylthio-4-(4-phenyl(1,3-thiazol-2-yl))thiophene-2-carboxamidine hydrochloride. ¹H-NMR (DMSO-d₆; 300 MHz) δ 8.7 (s, 1H), 8.25 (s, 1H), 8.07-811 (m, 2H), 7.37-7.53 (m, 3H), 2.8 (s, 3H). Mass Spectrum (MALDI-TOF, CHCA matrix, m/z) Calcd. for C₁₅H₁₃N₃S₃: 331.5(M+H), found 332.1.

### Example 11

### Synthesis of 5-methylthio-4-[4-(4-nitrophenyl)(1,3-thiazol-2-yl)]thiophene-2-carboxamidine hydrochloride

***a) Synthesis of methyl 5-methylthio-4-[4-(4-nitrophenyl)(1,3-thiazol-2-yl)]thiophene-2-carboxylate:*** 38 mg (0.141 mmol) of methyl 4-(aminothioxomethyl)-5-methylthiothiophene-2-carboxylate (Maybridge Chemical Co. LTD., Cornwall, U.K.) was dissolved in 2 mL of reagent grade acetone. 2-Bromo-4'-nitroacetophenone (0.155 mmol; 38 mg; Aldrich Chemical Co.) was added and the solution was allowed to reflux for 2.5 h. The solution was allowed to cool and a solid was filtered and washed with methanol and dried *in vacuo.* The crude product was dissolved in CH₂Cl₂ and 0.141 mmol of N-(2-mercapto)aminoethyl polystyrene resin (Calbiochem, San Diego, CA; 1.28mmol/g; 110 mg) was added and allowed to stir overnight. The solution was filtered, concentrated and dried to afford 60 mg (90% yield) of crude methyl 5-methylthio-4-[4-(4-nitrophenyl)(1,3-thiazol-2-yl)]thiophene-2-carboxylate.

***b) Synthesis of 5-methylthio-4-[4-(4-nitrophenyl)(1,3-thiazol-2-yl)]thiophene-2-carboxamidine hydrochloride:*** To a stirred suspension of 1.66 mmol (90 mg) of ammonium chloride (Fisher Scientific) in 2 mL of anhydrous toluene (Aldrich Chemical Co.) placed under nitrogen atmosphere at 0°C, 830 µL (1.66 mmol) of 2M trimethylaluminum in toluene (Aldrich Chemical Co.) was added via syringe over 10 min and then let stir at 0°C for 30 min after which 60 mg (0.166 mmol) of 5-methylthio-4-[4-(4-nitrophenyl)(1,3-thiazol-2-yl)]thiophene-2-carboxylate was added to solution and allowed to reflux for 2.5 h. The reaction mixture was quenched by pouring over a slurry of 500 mg of silica in 10 mL of chloroform. The silica was poured onto a sintered glass funnel and washed with a 10% methanol/CH₂Cl₂ solution and concentrated. The crude product was purified on a 1 mm silica prep plate eluting with 10% methanol/CH₂Cl₂ to afford 12 mg (19% yield) of 5-methylthio-4-[4-(4-nitrophenyl)(1,3-thiazol-2-yl)]thiophene-2-carboxamidine hydrochloride. ¹H-NMR (CD₃OD, 300 MHz) δ 8.58 (s, 1H), 8.32-8.33 (m, 4H), 8.24 (s, 1H), 2.83 (s, 3H). Mass Spectrum (MALDI-TOF, CHCA matrix, m/z) Calcd. for C₁₅H₁₂N₄O₂S₃: 376.5(M+H), found 377.3.

### Example 12

### Synthesis of 4-[4-(3,4-ethylenedioxyphenyl)thiazol-2-yl]-5-methylthiothiophene-2-carboxamidine hydrochloride

***a) Synthesis of methyl 4-(4-(2H,3H-benzo[3,4-e]1,4-dioxin-6-yl)(1,3-thiazol-2-yl))-5-methylthiothiophene-2-carboxylate:*** 40 mg (0.162 mmol) of methyl 4-(aminothioxomethyl)-5-methylthiothiophene-2-carboxylate (Maybridge Chemical Co. LTD., Cornwall, U.K.) was dissolved in 2 mL of reagent grade acetone. 1-(2*H*,3*H*-benzo[e]1,4-dioxin-6-yl)-2-bromoethan-1-one (0.162 mmol; 42 mg; Maybridge Chemical Co. LTD., Cornwall, U.K.) was added and the solution was allowed to reflux for 3 h. The solution was allowed to cool and allowed to stir for 2 days after which the reaction solution was concentrated *in vacuo*. The crude product was dissolved in 50 mL of CH₂Cl₂ and partitioned between 50 mL of 1 N NaOH (aq.). The organic layer was obtained and dried over sodium sulfate and concentrated to afford 60 mg (90% yield) of methyl 4-[4-(3,4-ethylenedioxyphenyl)thiazol-2-yl]-5-methylthiothiophene-2-carboxylate.

***b) Synthesis of 4-[4-(3,4-ethylenedioxyphenyl)thiazol-2-yl]-5-methylthiothiophene-2-carboxamidine hydrochloride:*** To a stirred suspension of 1.62 mmol ( 86 mg) of ammonium chloride (Fisher Scientific) in 2 mL of anhydrous toluene (Aldrich Chemical Co.) placed under nitrogen atmosphere at 0°C, 810 µL (1.62 mmol) of 2M trimethylaluminum in toluene (Aldrich Chemical Co.) was added via syringe over 10 min and then let stir at 0°C for 30 min after which 60 mg (0.162 mmol) of methyl 4-[4-(3,4-ethylenedioxyphenyl)thiazol-2-yl]-5-methylthiothiophene-2-carboxylate was added to solution and allowed to reflux for 2.5 h. The reaction mixture was quenched by pouring over a slurry of 500 mg of silica in 10 mL of chloroform. The silica was poured onto a sintered glass funnel and washed with a 10% methanol/CH₂Cl₂ solution and concentrated. The crude product was purified on a 1 mm silica prep plate eluting with 10% methanol/CH₂Cl₂ to afford 47 mg (75% yield) of 4-[4-(3,4-ethylenedioxyphenyl)thiazol-2-yl]-5-methylthiothiophene-2-carboxamidine hydrochloride. ¹H-NMR (CD₃OD; 300 MHz) δ 8.53 (s, 1H), 7.73 (s, 1H), 7.56 (d, J= 2Hz, 1H), 7.5 (dd, J = 2.1 Hz and 8.4 Hz, 1H), 6.89 (d, J=8.4 Hz, 1H), 4.28 (s, 4H), 2.8 (s, 3H). Mass Spectrum (MALDI-TOF, CHCA matrix, m/z) Calcd. for C₁₇H₁₅N₃O₂S₃: 389.5(M+H), found 390.1.

### Example 13

### Synthesis of 4-[4-(4-methoxyphenyl)(1,3-thiazol-2-yl)]-5-methylthiothiophene-2-carboxamidine hydrochloride

***a) Synthesis of methyl 4-[4-(4-methoxyphenyl)(1,3-thiazol-2-yl)]-5-methylthiothiophene-2-carboxylate:*** 30 mg (0.122 mmol) of methyl 4-(aminothioxomethyl)-5-methylthiothiophene-2-carboxylate (Maybridge Chemical Co. LTD., Cornwall, U.K.) was dissolved in 1.2 mL of reagent grade acetone. 2-bromo-4'-methoxy acetophenone (0.146 mmol; 28 mg; Aldrich Chemical Co.) was added and the solution was allowed to reflux for 3 h. The solution was allowed to cool and a solid was filtered and washed with methanol and dried *in vacuo* to afford 46 mg (90% yield) of methyl 4-[4-(4-methoxyphenyl)(1,3-thiazol-2-yl)]-5-methylthiothiophene-2-carboxylate.

***b) Synthesis of 4-[4-(4-methoxyphenyl)(1,3-thiazol-2-yl)]-5-methylthiothiophene-2-carboxamidine hydrochloride:*** To a stirred suspension of 1.22 mmol (66 mg) of ammonium chloride (Fisher Scientific) in 2 mL of anhydrous toluene (Aldrich Chemical Co.) placed under nitrogen atmosphere at 0°C, 612 µL (1.22 mmol) of 2M trimethylaluminum in toluene (Aldrich Chemical Co.) was added via syringe over 10 min and then let stir at 0°C for 30 min after which 46 mg (0.122 mmol) of 4-[4-(4-methoxyphenyl)(1,3-thiazol-2-yl)]-5-methylthiothiophene-2-carboxylate was added to solution and allowed to reflux for 2.5 h. The reaction mixture was quenched by pouring over a slurry of 500 mg of silica in 10 mL of chloroform. The silica was poured onto a sintered glass funnel and washed with a 10% methanol/CH₂Cl₂ solution and concentrated. The crude product was purified on a 1 mm silica prep plate eluting with 10% methanol/CH₂Cl₂ to afford 32 mg (73% yield) of 4-[4-(4-methoxyphenyl)(1,3-thiazol-2-yl)]-5-methylthiothiophene-2-carboxamidine hydrochloride. ¹H-NMR (CD₃OD; 300 MHz) δ 8.53 (s, 1H), 7.99-7.96 (d, J = 7 Hz, 2H), 7.75 (s, 1H), 7.00-7.02 (d, J = 5 Hz, 2H), 3.9 (s, 3H), 2.8 (s, 3H). Mass Spectrum (MALDI-TOF, CHCA matrix, m/z) Calcd. for C₁₆H₁₅N₃OS₃: 362.0(M+H), found 362.2.

### Example 14

### Synthesis of 4-[4-(3,4-propylenedioxyphenyl)thiazol-2-yl]-5-methylthiothiophene-2-carboxamidine hydrochloride

***a) Synthesis of methyl 4-[4-(3,4-propylenedioxyphenyl)thiazol-2-yl]-5-methylthiothiophene-2-carboxylate:*** 42 mg (0.170 mmol) of methyl 4-(aminothioxomethyl)-5-methylthiothiophene-2-carboxylate (Maybridge Chemical Co. LTD., Cornwall, U.K.) was dissolved in 5 mL of reagent grade acetone. 3',4'-Propylenedioxy-2-bromoacetophenone (0.170 mmol; 28 mg; Maybridge Chemical Co. LTD., Cornwall, U.K.) was added and the solution was allowed to reflux for 3 h. The solution was allowed to cool and a solid was filtered and purified on a 1 mm silica prep plate eluting with 20% ethyl acetate/hexane and dried *in vacuo* to afford 42 mg (59% yield) of methyl 4-[4-(3,4-propylenedioxyphenyl)thiazol-2-yl]-5-methylthiothiophene-2-carboxylate.

***b) Synthesis of 4-[4-(3,4-propylenedioxyphenyl)thiazol-2-yl]-5-methylthiothtophene-2-carboxanudine hydrochloride:*** To a stirred suspension of 1.01 mmol (54 mg) of ammonium chloride (Fisher Scientific) in 2 mL of anhydrous toluene (Aldrich Chemical Co.) placed under nitrogen atmosphere at 0°C, 510 µL (1.01 mmol) of 2M trimethylaluminum in toluene (Aldrich Chemical Co.) was added via syringe over 10 min and then let stir at 0°C for 30 min after which 42 mg (0.101 mmol) of methyl 4-[4-(3,4-propylenedioxyphenyl)thiazol-2-yl]-5-methylthiothiophene-2-carboxylate was added to solution and allowed to reflux for 3 h. The reaction mixture was quenched by pouring over a slurry of 500 mg of silica in 20 mL of chloroform. The silica was poured onto a sintered glass funnel and washed with a 10% methanol/CH₂Cl₂ solution and concentrated to afford 20 mg (50% yield) of 4-[4-(3,4-propylenedioxyphenyl)thiazol-2-yl]-5-methylthiothiophene-2-carboxamidine hydrochloride. ¹H-NMR (CD₃OD; 300 MHz) δ 8.53 (s, 1H), 7.78 (s, 1H), 7.68 (d, J = 2.2 Hz, 1H), 7.6 (dd, J = 2.2 Hz and 8.4 Hz, 1H), 7.0 (d, J = 8.3 Hz; 1H), 4.19-4.28 (m, 4H), 2.77 (s, 3H), 2.18-2.23 (m, 2H). Mass Spectrum (MALDI-TOF, CHCA matrix, m/z) Calcd. for C₁₈H₁₇N₃O₂S₃: 404.1(M+H), found 404.1.

### Example 15

### Synthesis of 5-methylthio-4-(4-(2-thienyl)(1,3-thiazol-2-yl))thiophene-2-carboxamidine acetate

***a) Synthesis of 2-bromo-1-(2-thienyl)ethan-1-one:*** To a solution of 500 mg (3.96 mmol) of 2-acetyl thiophene (Aldrich Chemical Co.) dissolved in 20 mL of CHCl₃, was added 1 drop of 30% HBr/CH₃COOH (Aldrich Chemical Co.) followed by 3.96 mmol (633 mg; 204 µL) of bromine (Aldrich Chemical Co.) added dropwise over 30 min. The reaction was allowed to stir for 1 h. The solution was concentrated to an oil and dried *in vacuo*. The crude product was purified on 1 mm silica prep plates eluting with neat CH₂Cl₂ to obtain 300 mg (37% yield) of 2-bromo-1-(2-thienyl)ethan-1-one. ¹H-NMR (CDCl₃; 300 MHz) δ 7.8 (m, 2H), 7.18 (m, 1H), 4.37 (s, 2H).

***b) Synthesis of methyl 5-methylthio-4-(4-(2-thienyl)(1,3-thiazol-2-yl))thiophene-2-carboxylate:*** 44 mg (0.176 mmol) of methyl 4-(aminothioxomethyl)-5-methylthiothiophene-2-carboxylate (Maybridge Chemical Co. LTD., Cornwall, U.K.) was dissolved in 3 mL of reagent grade acetone. 2-Bromo-1-(2-thienyl)ethan-1-one (0.176 mmol; 36 mg) was added and the solution was allowed to reflux for 3 h. The solution was allowed to cool and was concentrated. The crude product was dissolved in 20 mL of CH₂Cl₂ and washed with 20 mL of 1N HCl (aq.). The organic layer was obtained and dried over sodium sulfate to afford 115 mg (80% yield) of crude methyl 5-methylthio-4-(4-(2-thienyl)(1,3-thiazol-2-yl))thiophene-2-carboxylate.

***c) Synthesis of 5-methylthio-4-(4-(2-thienyl)(1,3-thiazol-2-yl))thiophene-2-carboxamidine acetate:*** To a stirred suspension of 2.80 mmol (150 mg) of ammonium chloride (Fisher Scientific) in 5 mL of anhydrous toluene (Aldrich Chemical Co.) placed under nitrogen atmosphere at 0°C, 1.5 mL (2.8 mmol) was added 2M trimethylaluminum in toluene (Aldrich Chemical Co.) via syringe over 15 min and then let stir at 0°C for 25 min. after which 115 mg (0.280 mmol) of methyl 5-methylthio-4-(4-(2-thienyl)(1,3-thiazol-2-yl))thiophene-2-carboxylate in 5 mL of anhydrous toluene was added to solution and allowed to reflux for 1.5 h. The reaction mixture was quenched by pouring over a slurry of silica in CH₂Cl₂. The silica was poured onto a sintered glass funnel and washed with a 10% methanol/CH₂Cl₂ solution and concentrated. The crude product was purified on a 1 mm silica prep plate eluting with 10% methanol/CH₂Cl₂ with 1% CH₃COOH to afford 40 mg (43% yield) of 5-methylthlo-4-(4-(2-thienyl)(1,3-thiazol-2-yl))thiophene-2-carboxamidine acetate. ¹H-NMR (CD₃OD; 300 MHz) δ 8.52 (s, 1H), 7.74 (s, 1H), 7.58-7.6 (dd, J = 2 Hz and 5 HZ, 1H), 7.43-7.41(dd, J = 2 Hz and 5 Hz, 1H), 7.12-7.09 (m, 1H), 2.79 (s, 3H). Mass Spectrum (MALDI-TOF, CHCA matrix, m/z) Calcd. for C₁₃H₁₁N₃S₄: 338.0 (M+H), found 337.9.

### Example 16

### Synthesis of 4-[4-(3-bromophenyl)(1,3-thiazol-2-yl)]-5-methylthiothiophene-2-carboxamidine hydrochloride

***a) Synthesis of methyl 4-[4-(3-bromophenyl)(1,3-thiazol-2-yl)]-5-methylthiothiophene-2-carboxylate:*** 99 mg (0.400 mmol) of methyl 4-(aminothioxomethyl)-5-methylthiothiophene-2-carboxylate (Maybridge Chemical Co. LTD., Cornwall, U.K.) was dissolved in 25 mL of reagent grade acetone. 2-bromo-3'-Bromo acetophenone (0.4 mmol; 111 mg) was added and the solution was allowed to reflux for 3 h. The solution was allowed to cool and a solid was filtered and dissolved in 5 mL of hot tetrahydrofuran (THF), (Aldrich Chemical Co.) and purified on a 1 mm silica prep plate eluting with 20% ethyl acetate/hexane and dried *in vacuo* to afford 66 mg (40% yield) of methyl 4-[4-(3-bromophenyl)(1,3-thiazol-2-yl)]-5 -methylthiothiophene-2-carboxylate.

***b) Synthesis of 4-[4-(3-bromophenyl)(1,3-thiazol-2-yl)]-5-methylthiothiophene-2-carboxamidine hydrochloride:*** To a stirred suspension of 1.55 mmol (83 mg) of ammonium chloride (Fisher Scientific) in 10 mL of anhydrous toluene (Aldrich Chemical Co.) placed under nitrogen atmosphere at 0°C, 774 µL (1.55 mmol) of 2M trimethylaluminum in toluene (Aldrich Chemical Co.) was added via syringe over 10 min and then let stir at 25°C for 20 min after which 66 mg (0.155 mmol) of 4-[4-(3-bromophenyl)(1,3-thiazol-2-yl)]-5-methylthiothiophene-2-carboxylate was added to solution and allowed to reflux for 3 h. The reaction mixture was quenched by pouring over a slurry of 5 g of silica in 25 mL of chloroform. The silica was poured onto a sintered glass funnel and washed with a 10% methanol/CH₂Cl₂ solution and concentrated. The crude product was purified on 1 mm silica plates eluting with 10% methanol/CH₂Cl₂ to afford 63 mg (90% yield) of 4-[4-(3-bromophenyl)(1,3-thiazol-2-yl)]-5-methylthiothiophene-2-carboxamidine hydrochloride. ¹H-NMR (CD₃OD; 300 MHz) δ 8.49 (s, 1H), 8.21 (m, 1H), 7.94-7.98 (m, 2H), 7.50 (m, 1H), 7.5 (m, 1H), 7.31-7.37 (m, 1H), 2.8 (s, 3H). Mass Spectrum (MALDI-TOF, CHCA matrix, m/z) Calcd. for C₁₅H₁₂BrN₃S₃: 411.9 (M+H), found 411.9.

### Example 17

### Synthesis of 4-[4-(4-chloro-3-nitrophenyl)(1,3-thiazol-2-yl)]-5-methylthiothiophene-2-carboxamidine hydrochloride

***a) Synthesis of methyl 4-[4-(4-chloro-3-nitrophenyl)(1,3-thiazol-2-yl)]-5-methylthiothiophene-2-carboxylate:*** 50 mg (0.202 mmol) of methyl 4-(aminothioxomethyl)-5-methylthiothiophene-2-carboxylate (Maybridge Chemical Co. LTD., Cornwall, U.K.) was dissolved in 10 mL of reagent grade acetone. 2-Bromo-4'-chloro-3'-nitroacetophenone (0.212 mmol; 59 mg) was added and the solution was allowed to reflux for 3 h. The solution was allowed to cool and a solid was filtered and dissolved in hot tetrahydrofuran (THF) (Aldrich Chemical Co.) and purified on a 1 mm silica prep plate eluting with 20% ethyl acetate/hexane and dried *in vacuo* to afford 60 mg (70% yield) of methyl 4-[4-(4-chloro-3-nitrophenyl)(1,3-thiazol-2-yl)]-5-methylthiothiophene-2-carboxylate.

***b) Synthesis of 4-[4-(4-chloro-3-nitrophenyl)(1,3-thiazol-2-yl)]-5-methylthiothiophene-2-carboxamidine hydrochloride:*** To a stirred suspension of 1.40 mmol (75 mg) of ammonium chloride (Fisher Scientific) in 10 mL of anhydrous toluene (Aldrich Chemical Co.) placed under nitrogen atmosphere at 0°C, 700 µL (1.40 mmol) of 2M trimethylaluminum in toluene (Aldrich Chemical Co.) was added via syringe over 10 min and then let stir for 20 min after which 60 mg (0.140 mmol) of 4-[4-(4-chloro-3-nitrophenyl)(1,3-thiazol-2-yl)]-5-methylthiothiophene-2-carboxylate was added to solution and allowed to reflux for 3 h. The reaction mixture was quenched by pouring over a slurry of 5 g of silica in 50 mL of chloroform. The silica was poured onto a sintered glass funnel and washed with a 10% methanol/CH₂Cl₂ solution and concentrated. The crude product was purified on 1 mm silica plates eluting with 10% methanol/CH₂Cl₂ to afford 17 mg (32% yield) of 4-[4-(4-chloro-3-nitrophenyl)(1,3-thiazol-2-yl)]-5-methylthiothiophene-2-carboxamidine hydrochloride. ¹H-NMR (CD₃OD; 300 MHz) δ 8.53-8.58 (m, 2H), 8.24-8.28 (dd, J = 2.2 Hz and 8.5 Hz, 1H), 8.16 (s, 1H), 7.70-7.73 (d, J= 8.5 Hz, 1H), 2.8 (s, 3H).

### Example 18

### Synthesis of 4-[4-(4-chloro-3-methylphenyl)(1,3-thiazol-2-yl)]-5-methylthiothiophene-2-carboxamidine hydrochloride

***a) Synthesis of methyl 4-[4-(4-chloro-3-methylphenyl)(1,3-thiazol-2-yl)]-5-methylthiothiophene-2-carboxylate:*** 155 mg (0.627 mmol) of methyl 4-(aminothioxomethyl)-5-methylthiothiophene-2-carboxylate (Maybridge Chemical Co. LTD., Cornwall, U.K.) was dissolved in 10 mL of reagent grade acetone. 2-Bromo-1-(4-chloro-3-methylphenyl)ethan-1-one (0.658 mmol; 163 mg) was added and the solution was allowed to reflux for 3 h. The solution was allowed to cool and the reaction mixture was concentrated and dissolved in 50 mL of CH₂Cl₂. The organic layer was washed with 50 mL of 1N HCl (aq.), dried over sodium sulfate and concentrated. The crude product was purified on a 1 mm silica plate eluting with 20% ethyl acetate/ hexane to afford 168 mg (68% yield) of methyl 4-[4-(4-chloro-3-methylphenyl)(1,3-thiazol-2-yl))-5-methylthiothiophene-2-carboxylate.

***b) Synthesis of 4-[4-(4-chloro-3-methylphenyl)(1,3-thiazol-2-yl)]-5-methylthiothiophene-2-carboxamidine hydrochloride:*** To a stirred suspension of 4.24 mmol (227 mg) of ammonium chloride (Fisher Scientific) in 15 mL of anhydrous toluene (Aldrich Chemical Co.) placed under nitrogen atmosphere at 0°C, 2.2 mL (4.24 mmol) of 2M trimethylaluminum in toluene (Aldrich Chemical Co.) was added via syringe over 10 min and then let stir for 20 min at 25°C after which 168 mg (0.424 mmol) of methyl 4-[4-(4-chloro-3-methylphenyl)(1,3-thiazol-2-yl)]-5-methylthiothiophene-2-carboxylate was added to solution and allowed to reflux for 2.5 h. The reaction mixture was quenched by pouring over a slurry of 5g silica in chloroform. The silica was poured onto a sintered glass funnel and washed with a 10% methanol/CH₂Cl₂ solution and concentrated to afford 117 mg (73% yield) of 4-[4-(4-chloro-3-methylphenyl)(1,3-thiazol-2-yl)]-5-methylthiothiophene-2-carboxamidine hydrochloride. ¹H-NMR (CD₃OD; 300 MHz) δ 8.53 (s, 1H), 7.97-8.07 (dd, J= 1.2 Hz and 27 Hz, 1H), 7.9 (s, 1H), 7.83-7.87 (dd, J= 2 Hz and 8.5 Hz 1H), 7.34-7.42 (dd, J= 8. 3 Hz and 17.4 Hz, 1H), 2.8 (s, 3H) 2.45 (s, 3H). Mass Spectrum (MALDI-TOF, CHCA matrix, m/z) Calcd. for C₁₆H₁₄ClN₃S₃: 380.0 (M+H), found 380.3.

### Example 19

### Synthesis of 4-(5-methyl-4-phenyl(1,3-thiazol-2-yl))-5-methylthiothiophene-2-carboxamidine hydrochloride

***a) Synthesis of methyl 4-(5-methyl-4-phenyl(1,3-thiazol-2-yl))-5-methylthiothiophene-2-carboxylate:*** 48 mg (0.194 mmol) of methyl 4-(aminothioxomethyl)-5-methylthiothiophene-2-carboxylate (Maybridge Chemical Co. LTD., Cornwall, U.K.) was dissolved in 5 mL of reagent grade acetone. 2-Bromo-1-phenylpropan-1-one (0.223 mmol; 48 mg) was added and the solution was allowed to reflux for 5 h. The solution was allowed to cool and the reaction mixture was concentrated and dissolved in 50 mL of CH₂Cl₂. The organic layer was washed with 50 mL of 1N HCl (aq.), dried over sodium sulfate and concentrated. The crude product was purified on a 1 mm silica plate eluting with 20% ethyl acetate/ hexane to afford 53 mg (76% yield) of methyl 4-(5-methyl-4-phenyl(1,3-thiazol-2-yl))-5-methylthiothiophene-2-carboxylate.

***b) Synthesis of 4-(5-methyl-4-phenyl(1,3-thiazol-2-yl))-5-methylthiothiophene-2-carboxamidine hydrochloride:*** To a stirred suspension of 1.47 mmol (78 mg) of ammonium chloride (Fisher Scientific) in 5 mL of anhydrous toluene (Aldrich Chemical Co.) placed under nitrogen atmosphere at 0°C, 735µL (1.47 mmol) of 2M trimethylaluminum in toluene (Aldrich Chemical Co.) was added via syringe over 10 min and then let stir for 20 min at 25°C then, 53 mg (0.147 mmol) of methyl 4-(5-methyl-4-phenyl(1,3-thiazol-2-yl))-5-methylthiothiophene-2-carboxylate were added to solution and allowed to reflux for 2.5 h. The reaction mixture was quenched by pouring over a slurry of 5g silica in chloroform. The silica was poured onto a sintered glass funnel and washed with a 10% methanol/CH₂Cl₂ solution and concentrated to afford 26 mg (51% yield) of 4-(5-methyl-4-phenyl(1,3-thiazol-2-yl))-5-methylthiothiophene-2-carboxamidine hydrochloride. ¹H-NMR (CD₃OD; 300 MHz) δ 8.45 (s, 1H), 7.74-7.77 (m, 2H), 7.44-7.50 (m, 2H), 7.38-7.41 (m, 1H), 2.8 (s, 3H) 2.6 (s, 3H). Mass Spectrum (MALDI-TOF, CHCA matrix, m/z) Calcd. for C₁₆H₁₅N₃S₃: 346.0 (M+H), found 345.6.

### Example 20

### Synthesis of 4-[4-(4-methylphenyl)(1,3-thiazol-2-yl)]-5-methylthiothiophene-2-carboxamidine trifluoroacetate

***a) Synthesis of methyl 4-[4-(4-methylphenyl)(1,3-thiazol-2-yl)]-5-methylthiothiophene-2-carboxylate:*** 103 mg (0.416 mmol) of methyl 4-(aminothioxomethyl)-5-methylthiothiophene-2-carboxylate (Maybridge Chemical Co. LTD., Cornwall, U.K.) was dissolved in 5 mL of reagent grade acetone. 2-Bromo-4'-methyl acetophenone (0.416 mmol; 89 mg) was added and the solution was allowed to reflux for 3 h. The solution was allowed to cool and crude product was filtered and washed two times with acetone and purified on a 1 mm silica plate eluting with 20% ethyl acetate/ hexane to afford 104 mg (69% yield) of methyl 4-[4-(4-methylphenyl)(1,3-thiazol-2-yl)]-5-methylthiothiophene-2-carboxylate.

***b) Synthesis of 4-[4-(4-methylphenyl)(1,3-thiazol-2-yl)]-5-methylthiothiophene-2-carboxamidine trifluoroacetate:*** To a stirred suspension of 2.87 mmol (154 mg) of ammonium chloride (Fisher Scientific) in 10 mL of anhydrous toluene (Aldrich Chemical Co.) placed under nitrogen atmosphere at 0°C, 144µL (2.87 mmol) of 2M trimethylaluminum in toluene (Aldrich Chemical Co.) was added via syringe over 10 min and then let stirred for 20 min at 25°C after which 104 mg (0.287 mmol) of 4-[4-(4-methylphenyl)(1,3-thiazol-2-yl)]-5-methylthiothiophene-2-carboxylate was added to solution and allowed to reflux for 3 h. The reaction mixture was quenched by pouring over a slurry of 5 g of silica in 50 mL of chloroform. The silica was poured onto a sintered glass funnel and washed with a 10% methanol/CH₂Cl₂ solution and concentrated. The crude product was then purified on a 1 mm silica prep plate eluting with 10% methanol/CH₂Cl₂ with 1% CH₃COOH. The product was then basified with aq. NaOH and extracted with CHCl₃ and concentrated. TFA was added and the product was crystallized from methanol as 4-[4-(4-methylphenyl)(1,3-thiazol-2-yl)]-5-methylthiothiophene-2-carboxamidine trifluoroacetate (20 mg; 30% yield). ¹H-NMR (DMSO-d₆; 300 MHz) δ 8.62 (s, 1H), 8.12 (s, 1H), 7.96-7.99 (d, 1H, J= 8.1 Hz) 7.29-7.32 (d, 1H, J= 8.1 Hz), 2.8 (s, 3H) 2.5 (s, 3H). Mass Spectrum (MALDI-TOF, CHCA Matrix, m/z) Calcd. for C₁₆H₁₅N₃S₃: 346.0 (M+H), found 346.1.

### Example 21

### Synthesis of 4-[4-(2-methoxyphenyl)(1,3-thiazol-2-yl)]-5-methylthiothiophene-2-carboxamidine hydrochloride

***a) Synthesis of methyl 4-[4-(2-methoxyphenyl)(1,3-thiazol-2-yl)]-5-methylthiothiophene-2-carboxylate:*** 105 mg (0.424 mmol) of methyl 4-(aminothioxomethyl)-5-methylthiothiophene-2-carboxylate (Maybridge Chemical Co. LTD., Cornwall, U.K.) was dissolved in 5 mL of reagent grade acetone. 2-Bromo-2'-methoxy acetophenone (0.467 mmol; 110 mg) was added and the solution was allowed to reflux for 3 h. The solution was allowed to cool and the solution concentrated. The crude product was dissolved in 100 mL of CH₂Cl₂ and washed one time with 50 mL of 1N NaOH. The organic layer was obtained, dried over sodium sulfate, concentrated and purified on a 1 mm silica plate eluting with 20% ethyl acetate/ hexane to afford 160 mg (95% yield) of methyl 4-[4-(2-methoxyphenyl)(1,3-thiazol-2-yl)]-5-methylthiothiophene-2-carboxylate.

***b) Synthesis of 4-[4-(2-methoxyphenyl)(1,3-thiazol-2-yl)]-5-methylthiothiophene-2-carboxamidine hydrochloride:*** To a stirred suspension of 4.23 mmol ( 227 mg) of ammonium chloride (Fisher Scientific) in 10 mL of anhydrous toluene (Aldrich Chemical Co.) placed under nitrogen atmosphere at 0°C, 2.12 mL (4.23 mmol) of 2M trimethylaluminum in toluene (Aldrich Chemical Co.) was added via syringe over 10 min and then let stir for 20 min at 25°C after which 160 mg (0.287 mmol) of methyl 4-[4-(2-methoxyphenyl)(1,3-thiazol-2-yl)]-5-methylthiothiophene-2-carboxylate in a solution of 5 mL of anhydrous toluene was added to solution and allowed to reflux for 3 h. The reaction mixture was quenched by pouring over a slurry of 5 g of silica in 30 mL of chloroform. The silica was poured onto a sintered glass funnel and washed with a 10% methanol/CH₂Cl₂ solution and concentrated. The crude product was then purified on a 2 mm silica prep plate eluting with 10% methanol/CH₂Cl₂ with 1% NH₄OH. The product was then dissolved in 2 mL of 4N HCl/dioxane and concentrated to afford 45 mg (29% yield) of 4-[4-(2-methoxyphenyl)(1,3-thiazol-2-yl)]-5-methylthiothiophene-2-carboxamidine hydrochloride. ¹H-NMR (DMSO-d₆; 300 MHz) δ 8.68 ( s, 1H), 8.34-8.38 (dd, J=1.6 Hz and 7.74 Hz, 1H), 8.21 (s, 1H), 7.36-7.42 (m, 1H), 7.05-7.22 (m, 3 H), 3.97 (s, 3H), 2.8 (s, 3H).
Mass Spectrum (MALDI-TOF, CHCA Matrix, m/z) Calcd. for C₁₆H₁₅N₃OS₃: 362.0(M+H), found 361.7.

### Example 22

### Synthesis of 4-[4-(2,4-dimethoxyphenyl)(1,3-thiazol-2-yl)]-5-methylthiothiophene-2-carboxamidine hydrochloride

***a) Synthesis of methyl 4-[4-(2,4-dimethoxyphenyl)(1,3-thiazol-2-yl)]-5-methylthiothiophene-2-carboxylate:*** 99 mg (0.424 mmol) of methyl 4-(aminothioxomethyl)-5-methylthiothiophene-2-carboxylate (Maybridge Chemical Co. LTD., Cornwall, U.K.) was dissolved in 5 mL of reagent grade acetone. 2-Bromo-2',4'-dimethoxyacetophenone (0.440 mmol; 114 mg) was added and the solution was allowed to reflux for 2.5 h. The solution was allowed to cool and the crude product was collected as a solid and washed with methanol and dried yielding 91 mg (56% yield) of methyl 4-[4-(2,4-dimethoxyphenyl)(1,3-thiazol-2-yl)]-5-methylthiothiophene-2-carboxylate.

***b) Synthesis of 4-[4-(2,4-dimethoxyphenyl)(1,3-thiazol-2-yl)]-5-methylthiothiophene-2-carboxamidine hydrochloride:*** To a stirred suspension of 2.23 mmol (119 mg) of ammonium chloride (Fisher Scientific) in 10 mL of anhydrous toluene (Aldrich Chemical Co.) placed under nitrogen atmosphere at 0°C, 1.1 mL (2.23 mmol) of 2M trimethylaluminum in toluene (Aldrich Chemical Co.) was added via syringe over 10 min and then let stirred for 20 min at 25°C after which 81 mg (0.223 mmol) of methyl 4-[4-(2,4-dimethoxyphenyl)(1,3-thiazol-2-yl)]-5-methylthiothiophene-2-carboxylate was added to solution and allowed to reflux for 2.5 h. The reaction mixture was quenched by pouring over a slurry of silica in chloroform. The silica was poured onto a sintered glass funnel and washed with a 10% methanol/CH₂Cl₂ solution and concentrated. The crude product was then purified on a 0.5 mm silica prep plate eluting with 10% methanol/CH₂Cl₂ to afford 32 mg (37% yield) of 4-[4-(2,4-dimethoxyphenyl)(1,3-thiazol-2-yl)]-5-methylthiothiophene-2-carboxamidine hydrochloride. ¹H-NMR (CD₃OD; 300 MHz) δ 8.49 ( s, 1H), 8.29-8.32 (d, J= 8.5 Hz, 1H), 7.93 (s, 1H), 6.61-6.67 (m, 2H), 3.97 (s, 3 H), 3.85 (s, 3H), 2.79 (s, 3H). Mass Spectrum (MALDI-TOF, CHCA Matrix, m/z) Calcd. for C₁₇H₁₇N₃O₂S₃: 392.1(M+H), found 392.4.

### Example 23

### Synthesis of 4-[4-(3,4-dichlorophenyl)(1,3-thiazol-2-yl)]-5-methylthiothiophene-2-carboxamidine hydrochloride

***a) Synthesis of methyl 4-[4-(3,4-dichlorophenyl)(1,3-thiazol-2-yl)]-5-methylthiothiophene-2-carboxylate:*** 176 mg (0.712 mmol) of methyl 4-(aminothioxomethyl)-5-methylthiothiophene-2-carboxylate (Maybridge Chemical Co. LTD., Cornwall, U.K.) was reacted with 2-bromo-3',4'-dichloroacetophenone (0.854 mmol; 330 mg) in a manner similar to Example 22, step (a) to afford 270 mg (91% yield) of methyl 4-[4-(3,4-dichlorophenyl)(1,3-thiazol-2-yl)]-5-methylthiothiophene-2-carboxylate.

***b) Synthesis of 4-[4-(3,4-dichlorophenyl)(1,3-thiazol-2-yl)]-5-methylthiothiophene-2-carboxamidine hydrochloride:*** 270 mg (0.648 mmol) of methyl 4-[4-(3,4-dichlorophenyl)(1,3-thiazol-2-yl)]-5-methylthiothiophene-2-carboxylate was treated in a manner similar to Example 22, step (b) to afford 135 mg (52% yield) of 4-[4-(3,4-dichlorophenyl)(1,3-thiazol-2-yl)]-5-methylthiothiophene-2-carboxamidine hydrochloride. ¹H-NMR (CD₃OD; 300 MHz) δ 8.54 (s, 1H), 8.21-8.22 (d, J= 2 Hz, 1H), 8.02 (s, 1H), 7.92-7.96 (dd, J= 2 Hz and 8.4 Hz, 1H), 7.56-7.59 (d, J= 8.5 Hz, 1 H), 2.79 (s, 3H). Mass Spectrum (MALDI-TOF, CHCA Matrix, m/z) Calcd. for C₁₅H₁₁Cl₂N₃S₃: 400.0 (M+H), found 400.6.

### Example 24

### Synthesis of 4-[4-(3-methylphenyl)(1,3-thiazol-2-yl)]-5-methylthiothiophene-2-carboxamidine hydrochloride

***a) Synthesis of methyl 4-[4-(3-methylphenyl)(1,3-thiazol-2-yl)]-5-methylthiothiophene-2-carboxylate:*** Methyl 4-(aminothioxomethyl)-5-methylthiothiophene-2-carboxylate, 106 mg (0.428 mmol) (Maybridge Chemical Co. LTD., Cornwall, U.K.) was reacted with 2-bromo-3' methylacetophenone (0.428 mmol, 91 mg) in a manner similar to Example 22, step (a) to afford 98 mg (63% yield) of methyl 4-[4-(3-methylphenyl)(1,3-thiazol-2-yl)]-5-methylthiothiophene-2-carboxylate.

***b) Synthesis of 4-[4-(3-methylphenyl)(1,3-thiazol-2-yl)]-5-methylthiothiophene-2-carboxamidine hydrochloride:*** 4-[4-(3-methylphenyl)(1,3-thiazol-2-yl)]-5-methylthiothiophene-2-carboxylate, (98 mg, 0.271 mmol) was treated in a similar manner to Example 22, step (b) to afford 75 mg (80% yield) of 4-[4-(3-methylphenyl)(1,3-thiazol-2-yl)]-5-methylthiothiophene-2-carboxamidine hydrochloride. ¹H-NMR (CD₃OD; 300 MHz) δ 8.56 (s, 1H), 7.88 (s, 1H), 7.83-7.88 (d, J= 14 Hz, 2H), 7.30-7.35 (m, 1H), 7.18-7.20 (m, 1 H), 2.79 (s, 3H), 2.42 (s, 3H). Mass Spectrum (MALDI-TOF, CHCA Matrix, m/z) Calcd. for C₁₆H₁₅N₃S₃: 346.0 (M+H), found 346.7

### Example 25

### Synthesis of 5-methylthio-4-(4-(2-5,6,7,8-tetrahydronaphthyl)(1,3-thiazol-2-yl))thiophene-2-carboxamidine hydrochloride

***a) Synthesis of methyl 5-methylthio-4-(4-(2-5,6,7,8-tetrahydronaphthyl)(1,3-thiazol-2-yl))thiophene-2-carboxylate:*** Methyl 4-(aminothioxomethyl)-5-methylthiothiophene-2-carboxylate, (160 mg, 0.647 mmol) (Maybridge Chemical Co. LTD., Cornwall, U.K.) was reacted with 2-bromo-1-(2-5,6,7,8-tetrahydronaphthyl)ethan-1-one (0.712 mmol; 180 mg) in a manner similar to Example 22, step (a) to afford 106 mg (41% yield) of methyl 5-methylthio-4-(4-(2-5,6,7,8-tetrahydronaphthyl)(1,3-thiazol-2-yl))thiophene-2-carboxylate.

***b) 5-methylthio-4-(4-(2-5,6,7,8-tetrahydronaphthyl)(1,3-thiazol-2-yl))thiophene-2-carboxamidine hydrochloride:*** 106 mg (0.264 mmol) of methyl 5-methylthio-4-(4-(2-5,6,7,8-tetrahydronaphthyl)(1,3-thiazol-2-yl))thiophene-2-carboxylate was treated in a similar manner to Example 22, step (b) to afford 88 mg (80% yield) of 5-methylthio-4-(4-(2-5,6,7,8-tetrahydronaphthyl)(1,3-thiazol-2-yl))thiophene-2-carboxamidine hydrochloride. ¹H-NMR (CD₃OD; 300 MHz) δ 8.49 (s, 1H), 7.78 (s, 1H), 7.72-7.74 (m, 2H), 7.09-7.12 (m, 1 H), 2.79 (m, 7H), 1.82-1.86 (m, 4H). Mass Spectrum (MALDI-TOF, CHCA Matrix, m/z) Calcd. for C₁₉H₁₉N₃S₃: 386.1 (M+H), found 386.2

### Example 26

### Synthesis of 4-[4-(3,5-dimethoxyphenyl)(1,3-thiazol-2-yl)]-5-methylthiothiophene-2-carboxamidine hydrochloride

***a) Synthesis of methyl 4-[4-(3,5-dimethoxyphenyl)(1,3-thiazol-2-yl)]-5-methylthiothiophene-2-carboxylate:*** 100 mg (0.404 mmol) of methyl 4-(aminothioxomethyl)-5-methylthiothiophene-2-carboxylate (Maybridge Chemical Co. LTD., Cornwall, U.K.) was reacted with 2-bromo-3',5'-dimethoxy acetophenone (0.444 mmol) in a manner similar to Example 22, step (a) to afford 44 mg (27% yield) of methyl 4-[4-(3,5-dimethoxyphenyl)(1,3-thiazol-2-yl)]-5-methylthiothiophene-2-carboxylate.

***b) Synthesis of 4-[4-(3,5-dimethoxyphenyl)(1,3-thiazol-2-yl)]-5-methylthiothiophene-2-carboxamidine hydrochloride:*** 44 mg (0.108 mmol) of methyl 4-[4-(3,5-dimethoxyphenyl)(1,3-thiazol-2-yl)]-5-methylthiothiophene-2-carboxylate was treated in a manner similar to Example 22, step (b) to afford 25 mg (60% yield) of 4-[4-(3,5-dimethoxyphenyl)(1,3-thiazol-2-yl)]-5-methylthiothiophene-2-carboxamidine hydrochloride. ¹H-NMR (CD₃OD; 300 MHz) δ 8.52 (s, 1H), 7.91 (s, 1H), 7.22-7.23 (d, J= 2.2 Hz, 1H), 6.49-6.51 (t, 1H), 3.85 (s, 6 H), 2.89 (s, 3H). Mass Spectrum (MALDI-TOF, CHCA Matrix, m/z) Calcd. for C₁₇H₁₇N₃O₂S₃: 392.11 (M+H), found 392.4.

### Example 27

### Synthesis of 4-[4-(2-methylphenyl)(1,3-thiazol-2-yl)]-5-methylthiothiophene-2-carboxamidine hydrochloride

***a) Synthesis of methyl 4-[4-(2-methylphenyl)(1,3-thiazol-2-yl)]-5-methylthiothiophene-2-carboxylate:*** 160 mg (0.647 mmol) of methyl 4-(aminothioxomethyl)-5-methylthiothiophene-2-carboxylate (Maybridge Chemical Co. LTD., Cornwall, U.K.) was reacted with 2-bromo-2'-methyl acetophenone (0.711 mmol, 152 mg) in a manner similar to Example 22, step (a) to afford 124 mg (53% yield) of methyl 4-[4-(2-methylphenyl)(1,3-thiazol-2-yl)]-5-methylthiothiophene-2-carboxylate.

***b) Synthesis of 4-[4-(2-methylphenyl)(1,3-thiazol-2-yl)]-5-methylthiothiophene-2-carboxamidine hydrochloride:*** 124 mg (0.343 mmol) of methyl 4-[4-(2-methylphenyl)(1,3-thiazol-2-yl)]-5-methylthiothiophene-2-carboxylate was treated in a manner similar to Example 22, step (b) to afford 60 mg (50% yield) of 4-[4-(2-methylphenyl)(1,3-thiazol-2-yl)]-5-methylthiothiophene-2-carboxamidine hydrochloride. ¹H-NMR (CD₃OD; 300 MHz) δ 8.50 (s, 1H), 7.63-7.66 (m, 2H), 7.22-7.32 (m, 3H), 2.79 (s, 3H), 2.51 (s, 3H). Mass Spectrum (MALDI-TOF, CHCA Matrix, m/z) Calcd. for C₁₆H₁₅N₃S₃: 346.0 (M+H), found 346.2.

### Example 28

### Synthesis of 4-[4-(2,5-dimethoxyphenyl)(1,3-thiazol-2-yl)]-5-methylthiothiophene-2-carboxamidine hydrochloride

***a) Synthesis of methyl 4-[4-(2,5-dimethoxyphenyl)(1,3-thiazol-2-yl)]-5-methylthiothiophene-2-carboxylate:*** 132 mg (0.534 mmol) of methyl 4-(aminothioxomethyl)-5-methylthiothiophene-2-carboxylate (Maybridge Chemical Co. LTD., Cornwall, U.K.) was reacted with 2-bromo-2',5'-dimethoxy acetophenone (0.587 mmol; 152 mg) in a manner similar to Example 22, step (a) to afford 97 mg (45% yield) of methyl 4-[4-(2,5-dimethoxyphenyl)(1,3-thiazol-2-yl)]-5-methylthiothiophene-2-carboxylate.

***b) Synthesis of 4-[4-(2,5-dimethoxypheny)(1,3-thiazol-2-yl)]-5-methylthiothiophene-2-carboxamidine hydrochloride:*** 97 mg (0.238 mmol) of methyl 4-[4-(2,5-dimethoxyphenyl)(1,3-thiazol-2-yl)]-5-methylthiothiophene-2-carboxylate was treated in a manner similar to Example 22, step (b) to afford 30 mg (32% yield) of 4-[4-(2,5-dimethoxyphenyl)(1,3-thiazol-2-yl)]-5-methylthiothiophene-2-carboxamidine hydrochloride. ¹H-NMR (CD₃OD; 300 MHz) δ 8.46 (s, 1H), 8.10 (s, 1H), 7.98-7.99 (d, J= 3.2 Hz, 1H), 7.03-7.06 (d, J= 9 Hz, 1H), 6.92-6.93 (d, J= 3.2 Hz, 1H), 6.89-6.90 (d, J= 3.2 Hz, 1H), 3.94 (s, 3H), 3.83 (s, 3H), 2.51 (s, 3H). Mass Spectrum (MALDI-TOF, CHCA Matrix, m/z) Calcd. for C₁₇H₁₇N₃O₂S₃: 392.1 (M+H), found 392.1.

### Example 29

### Synthesis of 4-[4-(4-chloro(3-pyridyl))(1,3-thiazol-2-yl)]-5-methylthiothiophene-2-carboxamidine hydrochloride

***a) Synthesis of methyl 4-[4-(4-chloro(3-pyridyl))(1,3-thiazol-2-yl)]-5-methylthiothiophene-2-carboxylate:*** 240 mg (0.970 mmol) of methyl 4-(aminothioxomethyl)-5-methylthiothiophene-2-carboxylate (Maybridge Chemical Co. LTD., Cornwall, U.K.) was reacted with 2-bromo-1-(4-chloro(3-pyridyl))ethan-1-one (1.06 mmol; 250 mg) in a manner similar to Example 22, step (a) to afford 286 mg (77% yield) of methyl 4-[4-(4-chloro(3-pyridyl))(1,3-thiazol-2-yl)]-5-methylthiothiophene-2-carboxylate.

***b) Synthesis of 4-[4-(4-chloro(3-pyridyl))(1,3-thiazol-2-yl)]-5-methylthiothiophene-2-carboxamidine hydrochloride:*** 286 mg (0.747 mmol) of methyl 4-[4-(4-chloro(3-pyridyl))(1,3-thiazol-2-yl)]-5-methylthiothiophene-2-carboxylate was treated in a manner similar to Example 22, step (b) to afford 134 mg (49% yield) of 4-[4-(4-chloro(3-pyridyl))(1,3-thiazol-2-yl)]-5-methylthiothiophene-2-carboxamidine hydrochloride. Mass Spectrum (MALDI-TOF, CHCA Matrix, m/z) Calcd. for C₁₄H₁₁N₄ClS₃: 366.9 (M+H), found 366.6

### Example 30

### Synthesis of 4-(4-(2H-benzo[d]1,3-dioxolen-5-yl)(1,3-thiazol-2-yl))-5-methylthiothiophene-2-carboxamidine hydrochloride

***a) Synthesis of 1-(2H-benzo[3,4-d]1,3-dioxolen-5-yl)-2-bromoethan-1-one:*** To a solution of 2.5 g (15.23 mmol) of 3,4-methylenedioxy acetophenone in 200 mL of anhydrous methanol was added 61 mmol (20 g) of poly (4-vinylpyridinium tribromide), Aldrich Chemical Co., and allowed to reflux for 2.5 h. The solution was filtered and concentrated. 1-(2H-benzo[3,4-d]1,3-dioxolen-5-yl)-2-bromoethan-1-one (1.4 g, 38% yield) was obtained methylene chloride/hexanes as off-white crystals. ¹H-NMR (DMSO-d₆; 300 MHz) δ 8.2 ( s, 1H), 8.07 (s, 1H), 7.61-7.64 (m,2H), 7.01-7.04 (dd, J= 1.2 Hz and 7.1 Hz, 1H), 6.09 (s, 2H), 3.86 (s, 3H), 2.75 (s, 3H).

***b) Synthesis of methyl 4-(4-(2H-benzo[d]1,3-dioxolen-5-yl)(1,3-thiazol-2-yl))-5-methylthiothiophene-2-carboxylate:*** 1.4 g (5.66 mmol) of methyl 4-(aminothioxomethyl)-5-methylthiothiophene-2-carboxylate (Maybridge Chemical Co. LTD., Cornwall, U.K.) was reacted 1-(2H-benzo[3,4-d]1,3-dioxolen-5-yl)-2-bromoethan-1-one (5.66 mmol, 1.37 g) in a manner similar to Example 22, step (a) to afford 1.55 g (70% yield) of methyl 4-(4-(2H-benzo[d]1,3-dioxolen-5-yl)(1,3-thiazol-2-yl))-5-methylthiothiophene-2-carboxylate.

***c) Synthesis of 4-(4-(2H-benzo[d]1,3-dioxolen-5-yl)(1,3-thiazol-2-yl))-5-methylthiothiophene-2-carboxamidine hydrochloride:*** 1.55 g (3.95 mmol) of methyl 4-(4-(2H-benzo[d]1,3-dioxolen-5-yl)(1,3-thiazol-2-yl))-5-methylthiothiophene-2-carboxylate was treated in a manner similar to Example 22, step (b) to afford 130 mg (9% yield) of 4-(4-(2H-benzo[d] 1,3-dioxolen-5-yl)(1,3-thiazol-2-yl))-5-methylthiothiophene-2-carboxamidine hydrochloride. ¹H-NMR (CD₃OD; 300 MHz) δ 8.51 (s, 1H), 7.73 (s, 1H), 7.53-7.59 (m, 2H), 6.88-6.90 (d, J= 8 Hz, 1H), 6.00 (s, 2H), 2.79 (s, 3H). Mass Spectrum (MALDI-TOF, CHCA Matrix, m/z) Calcd. for C₁₆H₁₃N₃O₂S₃: 376.0 (M+H), found 376.1.

### Example 31

### Synthesis of 4-[4-(3,4-dimethoxyphenyl)(1,3-thiazol-2-yl)]-5-methylthiothiophene-2-carboxamidine hydrochloride

***a) Synthesis of 1-(3,4-dimethoxyphenyl)-2-bromoethan-1-one:*** 2 g of 1-(3,4-dimethoxyphenyl)ethan-1-one (11.1 mmol) was reacted in a manner similar to Example 15, step (a), to yield 1.2 g (42% yield) of 1-(3,4-dimethoxyphenyl)-2-bromoethan-1-one.

***b) Synthesis of methyl 4-[4-(3,4-dimethoxyphenyl)(1,3-thiazol-2-yl)]-5-methylthiothiophene-2-carboxylate:*** 105 mg (0.424 mmol) of methyl 4-(aminothioxomethyl)-5-methylthiothiophene-2-carboxylate (Maybridge Chemical Co. LTD., Cornwall, U.K.) was reacted with 1-(3,4-dimethoxyphenyl)-2-bromoethan-1-one (0.467 mmol; 120 mg) in a manner similar to Example 22, step (a) to afford 148 mg (85% yield) of methyl 4-[4-(3,4-dimethoxyphenyl)(1,3-thiazol-2-yl)]-5-methylthiothiophene-2-carboxylate.

***c) Synthesis of 4-[4-(3,4-dimethoxyphenyl)(1,3-thiazol-2-yl)]-5-methylthiothiophene-2-carboxamidine hydrochloride:*** 148 mg (0.363 mmol) of methyl 4-[4-(3,4-dimethoxyphenyl)(1,3-thiazol-2-yl)]-5-methylthiothiophene-2-carboxylate was reacted in a manner similar to Example 22, step (b) to afford 70 mg (50% yield) of 4-[4-(3,4-dimethoxyphenyl)(1,3-thiazol-2-yl)]-5-methylthiothiophene-2-carboxamidine hydrochloride. ¹H-NMR (CD₃OD; 300 MHz) δ 8.50 (s, 1H), 7.76 (s, 1H), 7.58-7.64 (m, 2H), 7.22-7.39 (d, J= 51 Hz, 1H), 6.99-7.02 (d, J= 8 Hz, 1H), 3.9 (s, 3H) 3.86 (s, 3H), 2.78 (s, 3H). Mass Spectrum (MALDI-TOF, CHCA Matrix, m/z) Calcd. for C₁₇H₁₇N₃O₂S₃: 392.1 (M+H), found 392.4.

### Example 32

### 4-[4-(2-Chloro(3-pyridyl))(1,3-thiazol-2-yl)]-5-methylthiothiophene-2-carboxamidine

***a) Methyl 4-[4-(2-chloro(3-pyridyl))(1,3-thiazol-2-yl)]-5-methylthiothiophene-2-carboxylate:*** 2-Chloropyridine-3-carbonyl chloride (300 mg, 1.7 mmol) was dissolved in anhydrous CH₃CN (4 mL). While stirring well with a magnetic stirrer, trimethylsilyldiazomethane (4 mL, 2M solution in hexane, 8 mmol) was dripped into the reaction mixture. The resulting yellow solution was stirred for 2h at room temperature, at which time the mixture was cooled in an ice bath. To the cold solution, 30% HBr in acetic acid (2 mL) was added dropwise with vigorous evolution of gas. This solution was stirred for 1h during which time 2-bromo-1-(2-chloro(3-pyridyl))ethan-1-one precipitated. This solid was collected by filtration and dried under vacuum. The dry solid (142 mg, 0.6 mmol) was dissolved in acetone (10 ml). To this solution 5-(methoxycarbonyl)-2-(methylthio)-thiophene-3-thiocarboxamide (100 mg, 0.4 mmol, Maybridge Chemical Company, Cornwall, UK) was added and heated at reflux for 5 h. At this point the solid that precipitated was filtered off and washed with methanol and dried under vacuum to give 110 mg (71%) of methyl 4-[4-(2-chloro(3-pyridyl))(1,3-thiazol-2-yl)]-5-methylthiothiophene-2-carboxylate. ¹H-NMR (CDCl₃; 300 MHz) δ 2.70 (s, 3H), 3.92 (s, 3H), 7.39 (dd, J = 4.7 and 7.7 Hz, 1H), 8.11 (s, 1H), 8.22 (s, 1H), 8.38 (dd, J = 1.9 and 4.7 Hz, 1H), 8.62 (dd, J = 1.9 and 7.7 Hz, 1H).

***b) 4-[4-(2-Chloro(3-pyridyl))(1,3-thiazol-2-yl)]-5-methylthiothiophene-2-carboxamidine:*** Methyl 4-[4-(2-chloro(3-pyridyl))(1,3-thiazol-2-yl)]-5-methylthiothiophene-2-carboxylate (100 mg, 0.26 mmol) as prepared in previous step was treated in a manner similar to that for Example 1, to give 50 mg (52%) of 4-[4-(2-chloro(3-pyridyl))(1,3-thiazol-2-yl)]-5-methylthiothiophene-2-carboxamidine as a solid. ¹H-NMR (DMSO-d₆; 300 MHz) δ 2.79 (s, 3H), 7.62 (dd, J = 4.89 and 7.43 Hz, 1H), 8.41 (s, 1H), 8.47-8.51 (m, 2H), 8.69 (s, 1H), 9.1 (broad s, 2H), 9.4 (broad s, 2H). Mass spectrum (ESI, m/z): Calcd. for C₁₄H₁₁N₄S₃Cl: 367.0 (M+H), found 369.0.

### Example 33

### 4-(4-Cyclohexyl(1,3-thiazol-2-yl))-5-methylthiothiophene-2-carboxamidine

***a) Methyl 4-(4-cyclohexyl(1,3-thiazol-2-yl))-5-methylthiothiophene-2*-carboxylate**: Cyclohexanecarbonyl chloride (300 mg, 2.0 mmol) was treated in a manner similar to that for Example 32 to give 2-bromo-1-cyclohexylethan-1-one. The dry solid (125 mg) was dissolved in acetone (10 ml). To this solution 5-(methoxycarbonyl)-2-(methylthio)-thiophene-3-thiocarboxamide (100 mg, 0.4 mmol, Maybridge Chemical Company, Cornwall, UK) was added and heated at reflux for 5 h. At this point the solid that precipitated was filtered off and washed with methanol and dried under vacuum to give 100 mg (70%) of methyl 4-(4-cyclohexyl(1,3-thiazol-2-yl))-5-methylthiothiophene-2-carboxylate which was used without further purification in the following step.

***b) 4-(4-Cyclohexyl(1,3-thiazol-2-yl))-5-methylthiothiophene-2-carboxamidine:*** Methyl 4-(4-cyclohexyl(1,3-thiazol-2-yl))-5-methylthiothiophene-2-carboxylate (100 mg, 0.28 mmol) as prepared in previous step was treated in a manner similar to that for Example 1, to give 60 mg (63%) of 4-(4-cyclohexyl(1,3-thiazol-2-yl))-5-methylthiothiophene-2-carboxamidine as a solid. ¹H-NMR (DMSO-d₆; 300 MHz) δ 1.21-1.53 (m, 5H), 1.61-1.78 (m, 3H), 2.03-2.07 (m, 2H), 2.7 (s, 3H), 2.73-2.75 (m, 1H), 7.33 (s, 1H), 8.32 (s, 1H). Mass spectrum (MALDI-TOF, m/z): Calcd. for C₁₅H₁₉N₃S₃, 338.1 (M+H), found 338.1.

### Example 34

### 4-Phenyl-5-(trifluoromethyl)thiophene-2-carboxamidine

Methyl 4-phenyl-5-(trifluoromethyl)thiophene-2-carboxylate (100 mg, 0.37 mmol, Maybridge Chemical Company, Cornwall, UK) was treated in a manner similar to that for Example 1 to give 80 mg (85%) of 4-phenyl-5-(trifluoromethyl)thiophene-2-carboxamidine as a solid. ¹H-NMR (DMSO-d₆; 300 MHz) δ 7.45-7.52 (m, 5H), 7.79 (d, J = 1.4 Hz, 1H). Mass spectrum (MALDI-TOF, m/z): Calcd. for C₁₂H₉F₃N₂S, 271.1 (M+H), found 271.2.

### Example 35

### 5-Methylthio-4-(2-phenyl(1,3-thiazol-4-yl))thiophene-2-carboxamidine

***a) Methyl 4-(2-bromoacetyl)-5-methylthiothiophene-2-carboxylate:*** 5-(Methoxycarbonyl)-2-methylthiothiophene-3-carboxylic acid (200 mg, 0.86 mmol) as prepared in Example 95 was taken in a round bottomed flask and anhydrous CH₂Cl₂ (10 mL) was introduced to the flask. This solution was cooled in an ice bath under an argon atmosphere. To this mixture oxalyl chloride (328 mg, 2.6 mmol) was added followed by anhydrous DMF (500 µL). The resulting solution was stirred at 4°C for 30 min and then allowed to warm up to room temperature, while monitoring for the disappearance of the acid by TLC. After 2 h solvents were removed under vacuum and the residual oxalyl chloride was removed azeotropically with toluene. The resulting residue was dried under high-vacuum to give the acid chloride as a gray solid. This solid was dissolved in anhydrous CH₃CN (8 mL). While stirring well with a magnetic stirrer trimethylsilyldiazomethane (4 mL, 8 mmol, 2M solution in hexane) was dripped into the reaction mixture. The resulting yellow solution was stirred for 2h at room temperature, at which time the mixture was cooled in an ice bath. To the cold solution 30% HBr in acetic acid ( 2 mL) was added dropwise, with vigorous evolution of gas. This solution was stirred for 1h, during which methyl 4-(2-bromoacetyl)-5-methylthiothiophene-2-carboxylate precipitates. This solid was collected by filtration and dried under vacuum to give 120 mg (45%). ¹H-NMR (CDCl₃; 300 MHz) δ 2.64 (s, 3H), 3.91 (s, 3H), 4.27 (s, 2H), 8.10 (s, 1H).

***b) Methyl 5-methylthio-4-(2-phenyl(1,3-thiazol-4-yl))thiophene-2-carboxylate:*** 5-(Methoxycarbonyl)-2-(methylthio)-thiophene-3-thiocarboxamide (100 mg, 0.4 mmol, Maybridge Chemical Company, Cornwall, UK) was dissolved in acetone (20 ml). To this solution, methyl 4-(2-bromoacetyl)-5-methylthiothiophene-2-carboxylate (112 mg) as prepared in previous step was added and heated at reflux for 3 h. At this point the solid that precipitated was filtered off and washed with acetone and dried under vacuum to give 82 mg (65%) of methyl 5-methylthio-4-(2-phenyl(1,3-thiazol-4-yl))thiophene-2-carboxylate. ¹H-NMR (CDCl₃; 300 MHz) δ 2.67 (s, 3H), 3.91 (s, 3H), 7.44-7.49 (m, 3H), 7.61 (s, 1H), 8.03-8.06 (m, 2H), 8.28 (s, 1H).

***c) 5-Methylthio-4-(2-phenyl(1,3-thiazol-4-yl))thiophene-2-carboxamidine:*** Methyl 5-methylthio-4-(2-phenyl(1,3-thiazol-4-yl))thiophene-2-carboxylate (80 mg) as prepared in previous step was treated in a manner similar to that for Example 1, to give 50 mg of 5-methylthio-4-(2-phenyl(1,3-thiazol-4-yl))thiophene-2-carboxamidine as a solid. ¹H-NMR (DMSO-d₆; 300 MHz) δ 2.75 (s, 3H), 7.51-7.60 (m, 3H), 8.02 (s, 1H), 8.03-8.09 (m, 2H), 8.70 (s, 1H), 9.06 (broad s, 2H), 9.38 (broad s, 2H). Mass spectrum (MALDI-TOF, m/z): Calcd. for C₁₅H₁₃N₃S₃, 332.0 (M+H), found 332.1.

### Example 36

### 4-[4-(2-Chloro(4-pyridyl))(1,3-thiazol-2-yl)]-5-methylthiothiophene-2-carboxamidine

***a) Methyl 4-[4-(2-chloro(4-pyridyl))(1,3-thiazol-2-yl)]-5-methylthiothiophene-2-carboxylate:*** 2-Chloropyridine-4-carbonyl chloride (300 mg, 1.7 mmol) was dissolved in anhydrous CH₃CN (4 mL). While stirring well with a magnetic stirrer trimethylsilyldiazomethane (4 mL, 8 mmol, 2M solution in hexane) was dripped into the reaction mixture. The resulting yellow solution was stirred for 2 h at room temperature, at which time the mixture was cooled in an ice bath. To the cold solution 30% HBr in acetic acid (2 mL) was added dropwise, with vigorous evolution of gas. This solution was stirred for 1h, during which time 2-bromo-1-(2-chloro(4-pyridyl))ethan-1-one precipitates. This solid was collected by filtration and dried under vacuum. The dry solid (142 mg, 0.6 mmol) was dissolved in acetone (10 ml). To this solution 5-(methoxycarbonyl)-2-(methylthio)-thiophene-3-thiocarboxamide (100 mg, 0.4 mmol, Maybridge Chemical Company, Cornwall, UK) was added and heated at reflux for 5 h. At this point the solid that precipitated was filtered off and washed with methanol and dried under vacuum to give 100 mg of methyl 4-[4-(2-chloro(4-pyridyl))(1,3-thiazol-2-yl)]-5-methylthiothiophene-2-carboxylate. ¹H-NMR (CD₃OD; 300 MHz) δ 2.73 (s, 3H), 3.94 (s, 3H, overlapping H₂O peak), 7.92-7.99 (m, 2H), 8.05 (s, 1H), 8.24 (s, 2H), 8.47-8.49 (m, 1H).

***b) 4-[4-(2-Chloro(4-pyridyl))(1,3-thiazol-2-yl)]-5-methylthiothiophene-2-carboxamidine:*** Methyl 4-[4-(2-chloro(4-pyridyl))(1,3-thiazol-2-yl)]-5-methylthiothiophene-2-carboxylate (100 mg, 0.26 mmol) as prepared in previous step was treated in a manner similar to that for Example 1, to give 50 mg of 4-[4-(2-chloro(4-pyridyl))(1,3-thiazol-2-yl)]-5-methylthiothiophene-2-carboxamidine as a solid. ¹H-NMR (CDCl₃/CD₃OD; 300 MHz) δ 2.82 (s, 3H), 7.95 (dd, J = 1.42 and 5.25 Hz, 1H), 8.08 (d, J = 1.03 Hz, 1H), 8.23 (s, 1H), 8.42 (d, J = 5.34 Hz, 1H), 8.56 (s, 1H). Mass spectrum (MALDI-TOF, m/z): Calcd. for C₁₄H₁₁N₄S₃Cl, 367.0 (M+H), found 367.1.

### Example 37

### 4-[4-(4-Chlorophenyl)(1,3-thiazol-2-yl)]-5-(methylsulfonyl)thiophene-2-carboxamidine

4-[4-(4-Chlorophenyl)( 1,3-thiazol-2-yl)]-5-methylthiothiophene-2-carboxamidine (35 mg, 0.1 mmol) prepared according to Example 1 was dissolved in a mixture of MeOH and CH₂Cl₂ (1:1,6 mL). While stirring well, *m*-chloroperoxybenzoic acid (100 mg) was added in portions to this solution over a 3h period. The mixture was stirred for a further 2 h and the solvents were removed under vacuum. The resulting residue was dissolved in MeOH (8 mL). Strong anion exchange resin (AG 1-X8, 5 ml, 1.4 meq/mL) was packed into a disposable chromatography column and washed with H₂O (5x5 mL) and MeOH (3x5 mL). The methanolic solution from the reaction was slowly introduced into this column, and the column effluent was collected. The column was washed with MeOH (2x5 mL) and these washings were also collected. The combined effluents were evaporated under vacuum and the residue was subjected to preparative thin layer chromatography (silica gel, 10% MeOH in CH₂Cl₂ with 2% acetic acid). The major band was isolated and suspended in CH₂Cl₂ and filtered. The filtrate was collected and the residue was washed with 10% MeOH in CH₂Cl₂ saturated with NH₃. The washings were combined with the original filtrate and the solvents were removed under vacuum. The resulting solid was dissolved in 10% MeOH in CHCl₃ and filtered through a 0.45 micron filter. The filtrate was collected and evaporated under vacuum to give 20 mg (53%) of an off-white solid. ¹H-NMR (CDCl₃/CD₃OD; 300 MHz) δ 3.78 (s, 3H), 7.47 (d, J = 8.7 Hz, 2H), 7.96 (d, J = 8.7 Hz, 1H), 8.00 (s, 1H), 8.35 (s, 1H). Mass spectrum (MALDI-TOF, m/z): Calcd. for C₁₅H₁₂O₂N₃S₃Cl, 398.0 (M+H), found 398.0.

### Example 38

### Hydrazino[5-methylthio-4-(4-phenyl(1,3-thiazol-2-yl))(2-thienyl)]methanimine

***a) 5-Methylthio-4-(4-phenyl(1,3-thiazol-2-yl))thiophene-2-carboxamide:*** Liquid ammonia (5 mL) was condensed into a cold (-78°C) Teflon-lined steel bomb. Methyl 5-methylthio-4-(4-phenyl(1,3-thiazol-2-yl))thiophene-2-carboxylate (0.6 g, 1.7 mmol) as prepared in Example 10 step (a) was introduced in one portion and the bomb was sealed and heated in an oil bath at 80°C for 48h. The bomb was cooled to -78°C, opened and the ammonia was allowed to evaporate at room temperature. The residual solid was collected and dried under vacuum to give 0.5 g (88%) of 5-methylthio-4-(4-phenyl(1,3-thiazol-2-yl))thiophene-2-carboxamide. ¹H-NMR (DMSO-d₆; 300 MHz) δ 2.75 (s, 3H), 7.35-7.40 (m, 1H), 7.40-7.51 (m, 2H), 8.04-8.18 (m, 2H), 8.19 (s, 1H), 8.20 (s, 1H).

***b) 5-Methylthio-4-(4-phenyl(1,3-thiazol-2-yl))thiophene-2-carbonitrile:*** A slurry of P₂O₅ (2.7 g, 19 mmol) and hexamethyldisiloxane (6.7 mL) in dichloroethane (13 mL) was heated to 90°C, while stirring under a N₂ atmosphere. After stirring for 2 h, the resulting clear solution was allowed to cool to 40°C. 5-methylthio-4-(4-phenyl(1,3-thiazol-2-yl))thiophene-2-carboxamide (0.9 g, 2.7 mmol) as prepared in previous step was added to this solution and the mixture was heated at 75°C for 5h. This solution was cooled to room temperature and stirred with aqueous NaCl (6 M, 100 mL) for 10 min. As the aqueous solution is added a yellow solid precipitated. After 10 min this solid was separated by filtration, and dried under vacuum to give (0.5 g, 59%) of 5-methylthio-4-(4-phenyl(1,3-thiazol-2-yl))thiophene-2-carbonitrile as a yellow solid. ¹H-NMR(DMSO-d₆; 300 MHz) δ 2.76 (s, 3H), 7.35-7.40 (m, 1H), 7.45-7.50 (m, 2H), 8.05-8.08 (m, 2H), 8.22 (s, 1H), 8.51 (s, 1H).

***c) Hydrazino[5-methylthio-4-(4-phenyl(1,3-thiazol-2-yl))(2-thienyl)]methanimine:*** 5-Methylthio-4-(4-phenyl(1,3-thiazol-2-yl))thiophene-2-carbonitrile (100 mg, 0.32 mmol) as prepared in previous step was dissolved in EtOH (10 mL). To this solution hydrazine monohydrate (10 eq) was added and the mixture was heated at reflux for 3h. The EtOH solution was concentrated down to 1 mL and water (2 mL) was added to this solution. This resulted in the formation of a white solid. The solid was collected by filtration washed with a small amount of water and dried under vacuum to give 50 mg (45%) of hydrazino[5-methylthio-4-(4-phenyl(1,3-thiazol-2-yl))(2-thienyl)]methanimine. ¹H-NMR (CD₃OD/CDCl₃; 300 MHz) δ 2.69 (s, 3H), 7.35-7.43 (m, 1H), 7.44-7.49 (m, 2H), 7.52 (s, 1H), 7.96-7.99 (m, 2H), 8.10 (s, 1H). Mass spectrum (ESI, m/z): Calcd. for C₁₅H₁₄N₄S₃, 347.04 (M+H), found 347.1.

### Example 39

### {Imino[5-methylthio-4-(4-phenyl(1,3-thiazol-2-yl)(2-thienyl)]methyl}methylamine

5-Methylthio-4-(4-phenyl(1,3-thiazol-2-yl))thiophene-2-carboxamidine (20 mg, 0.06 mmol) as prepared in Example 10 step (b) was dissolved in MeOH, and to this solution methylamine (0.6 mL, 2M solution in tetrahydrofuran) was added. This solution was refluxed for 6h, at which time the solvents were removed under vacuum to give a solid. This solid was dissolved in a small amount of MeOH. H₂O was added dropwise to the methanolic solution until a precipitate was formed. This solid was isolated, washed with a small amount of water and dried under vacuum to give 15 mg (72%) of {imino[5-methylthio-4-(4-phenyl(1,3-thiazol-2-yl))(2-thienyl)]methyl}methylamine. ¹H-NMR (DMSO-d₆; 300 MHz) δ 2.77 (s, 3H), 3.00 (s, 3H), 7.36-7.42 (m, 1H), 7.47-7.52 (m, 2H), 8.07-8.10 (m, 2H), 8.23 (s, 1H), 8.55 (s, 1H). Mass spectrum (ESI, m/z): Calcd. for C₁₆H₁₅N₃S₃, 346.5 (M+H), found 346.2.

### Example 40

### 2-{3-[2-(5-Amidino-2-methylthio-3-thienyl)-1,3-thiazol-4-yl]phenoxy}acetic acid

***a) 2-Bromo-1-(3-hydroxyphenyl)ethan-1-one:*** 2-Bromo-1-(3-methoxyphenyl)ethan-1-one (2 g, 8.7 mmol) was taken in a round bottomed flask equipped with magnetic stir bar. The flask was put under a N₂ atmosphere and CH₂Cl₂ was introduced into the flask. The resulting solution was cooled in a dry ice acetone bath and BBr₃ (27 mL, 1M in CH₂Cl₂) was introduced dropwise. The resulting solution was allowed to warm up to room temperature over-night. The solvents were removed under vacuum and the residue was purified by passing through a short pad of silica gel (50 g) to give 1.3 g (69%) of 2-bromo-1-(3-hydroxyphenyl)ethan-1-one as an oil. ¹H-NMR (CDCl₃; 300 MHz) δ 4.47 (s, 2H), 6.21 (s, 1H), 7.08-7.19 (m, 1H), 7.23-7.48 (m, 1H), 7.52-7.82 (m, 2H).

***b) Methyl 4-[4-(3-hydroxyphenyl)(1,3-thiazol-2-yl)]-5-methylthiothiophene-2-carboxylate:*** 2-Bromo-1-(3-hydroxyphenyl)ethan-1-one (229 mg, 1.1 mmol) as prepared in previous step was treated in a manner similar to that of Example 13, step (a) to give 225 mg (61%) of methyl 4-[4-(3-hydroxyphenyl)(1,3-thiazol-2-yl)]-5-methylthiothiophene-2-carboxylate as a solid. ¹H-NMR (DMSO-d₆; 300 MHz) δ 2.76 (s, 3H), 3.86 (s, 3H), 6.77-6.97 (m, 1H), 7.27 (t, J = 7.8 Hz, 1H), 7.47-7.51 (m, 2H), 8.12 (s, 1H), 8.20 (s, 1H).

***c) (tert-Butoxy)-N-({4-[4-(3-hydroxyphenyl)(1,3-thiazol-2-yl)]-5-methylthio(2-thienyl)}iminomethyl)carboxamide:*** 4-[4-(3-Hydroxyphenyl)(1,3-thiazol-2-yl)]-5-methylthiothiophene-2-carboxamidine (2 g, 5.8 mmol), prepared by treating methyl 4-[4-(3-hydroxyphenyl)(1,3-thiazol-2-yl)]-5-methylthiothiophene-2-carboxylate in a manner similar to that for Example 1, was dissolved in anhydrous DMF (10 mL). To this solution di-tert-butyl dicarbonate (1.38 g, 6.3 mmol) and DIEA (2 mL, 11.5 mmol) was added, and the mixture was stirred at room temperature for 18 h. DMF was removed under vacuum and the residue was purified by silica gel column chromatography to give 1.8 g (70%) of (tert-butoxy)-N-({4-[4-(3-hydroxyphenyl)(1,3-thiazol-2-yl)]-5-methylthio(2-thienyl)}iminomethyl)carboxamide as an oil. ¹H-NMR (DMSO-d₆; 300 MHz) δ 1.58 (s, 9H), 2.81 (s, 3H), 6.79-6.83 (m, 1H), 7.28 (t, J = 8.0 Hz, 1H), 7.49-7.52 (m, 2H), 8.09 (s, 1H), 8.71 (s, 1H).

***d) tert-Butyl 2-{3-[2-(5-{[(tert-butoxy)carbonylamino]iminomethyl}-2-methylthio-3-thienyl)-1,3-thiazol-4-yl]phenoxy}acetate:*** (tert-Butoxy)-N-({4-[4-(3-hydroxyphenyl)(1,3-thiazol-2-yl)]-5-methylthio(2-thienyl)}iminomethyl)carboxamide (23 mg, 0.05 mmol) as prepared in previous step was dissolved in anhydrous DMF (1 mL). To this solution tert-butyl 2-bromoacetate (20 mg, 0.1 mmol), Cs₂CO₃ (33.5 mg, 0.1 mmol) and KI (5 mg) was added and the mixture was heated at 70°C for 18 h. The solvents were removed under vacuum and the residue was purified by preparative silica gel thin-layer chromatography to give 12 mg (42%) of tert-butyl 2-{3-[2-(5-{[(tert-butoxy)carbonylamino]iminomethyl}-2-methylthio-3-thienyl)-1,3-thiazol-4-yl]phenoxy}acetate which was used in the following step.

***e) 2-{3-[2-(5-Amidino-2-methylthio-3-thienyl)-1,3-thiazol-4-yl]phenoxy}acetic acid:*** tert-Butyl 2-{3-[2-(5-{[(tert-butoxy)carbonylamino]iminomethyl}-2-methylthio-3-thienyl)-1,3-thiazol-4-yl]phenoxy}acetate (12 mg, 0.02 mmol) as prepared in previous step was dissolved in 1 ml 50% TFA in CH₂Cl₂ containing 2% H₂O and stirred for 4h. The solvents were removed under vacuum. The residual TFA was removed by azeotroping with toluene to give 8.7 mg (100%) of 2-{3-[2-(5-amidino-2-methylthio-3-thienyl)-1,3-thiazol-4-yl]phenoxy}acetic acid as a buff colored solid. ¹H-NMR (CD₃OD/CDCl₃; 300 MHz) δ 2.77 (s, 3H), 4.74 (S, 2H), 6.91-6.95 (m, 1H), 7.35 (t, J = 7.91 Hz, 1H), 7.60-7.63 (m, 1H), 7.67-7.68 (M, 1H), 7.84 (s, 1H), 8.46 (s, 1H). Mass spectrum (ESI, m/z): Calcd. for C₁₇H₁₅N₃O₃S₃, 406.5 (M+H), found 406.3.

### Example 41

### 2-{2-[2-(5-Amidino-2-methylthio-3-thienyl)-1,3-thiazol-4-yl]phenoxy}acetic acid

***a) tert-Butyl 2-{2-[2-(5-{[(tert-butoxy)carbonylamino]iminomethyl}-2-methylthio-3-thienyl)-1,3-thiazol-4-yl]phenoxy}acetate:*** 4-[4-(2-Hydroxyphenyl)(1,3-thiazol-2-yl))-5-methylthiothiophene-2-carboxamidine (100 mg, 0.29 mmol) as prepared in Example 196 step (b) was treated in a manner similar to that shown in Example 40 step (c) to give 100 mg (0.22 mmol, 77%) of (tert-butoxy)-N-({4-[4-(2-hydroxyphenyl)(1,3-thiazol-2-yl)]-5-methylthio(2-thienyl)}iminomethyl)carboxamide. This compound was treated in a manner similar to that shown in Example 40, step (d) to give 63 mg (50 %) of tert-butyl 2-{2-[2-(5-{[(tert-butoxy)carbonylamino]iminomethyl}-2-methylthio-3-thienyl)-1,3-thiazol-4-yl]phenoxy}acetate. ¹H-NMR (CDCl₃; 300 MHz) δ 1.55 (s, 9H), 1.56 (s, 9H), 2.69 (s, 3H), 4.66 (s, 2H), 6.88 (dd, J = 0.81 and 8.31 Hz, 1H), 7.14 (dt, J = 1.0 and 7.63 Hz, 1H), 7.27-7.32 (m, 1H), 8.08 (s, 1H), 8.48 (dd, J = 1.8 and 7.77 Hz, 1H), 8.51 (s, 1H).

***b) 2-{2-[2-(5-Amidino-2-methylthio-3-thienyl)-1,3-thiazol-4-yl]phenoxy}acetic acid:*** tert-Butyl 2-{2-[2-(5-{[(tert-butoxy)carbonylamino]iminomethyl}-2-methylthio-3-thienyl)-1,3-thiazol-4-yl]phenoxy}acetate (60 mg, 0.12 mmol) as prepared in previous step was treated in a manner similar to that shown in Example 40, step (e) to give 22 mg (50 %) of 2-{2-[2-(5-amidino-2-methylthio-3-thienyl)-1,3-thiazol-4-yl]phenoxy}acetic acid. ¹H-NMR (DMSO-d₆; 300 MHz) δ 2.80 (s, 3H), 4.90 (S, 2H), 7.09-7.25 (m, 2H), 7.34-7.38 (m, 1H), 8.41 (d, J = 6.32 Hz, 1H), 8.60 (s, 1H), 8.62 (s, 1H), 9.00 (broad s, 2H), 9.37 (broad s, 2H). Mass spectrum (ESI, m/z): Calcd. for C₁₇H₁₅N₃O₃S₃, 406.5 (M+H), Found 406.1.

### Example 42

### 5-Methylthio-4-(6-phenyl(2-pyridyl))thiophene-2-carboxamidine

***a) Methyl 4-(1,1-dimethyl-1-stannaethyl)-5-methylthiothiophene-2-carboxylate:*** 4-Bromo-5-methylthiothiophene-2-carboxylic acid (EP 0676395 A2) (4.67 g, 18.4 mmol) was dissolved in anhydrous THF (30 mL), taken in a round bottomed flask and cooled to -78°C under a N₂ atmosphere. To this solution *n*-butyllithium (20.3 mL, 40.6 mmol, 2M in cyclohexane) was introduced in a dropwise manner. The resulting solution was stirred at -78°C for 45 min and then allowed to warm up to -60°C. To this solution trimethyltin chloride (40.6 mL, 40.6 mmol, 1M in THE) was added dropwise. This solution was stirred at -60°C for 30 min and then allowed to warm up to room temperature. The THF was removed under vacuum and the residue was treated with H₂O and extracted with hexane. The hexane layer was evaporated and the residue was dissolved in Et₂O. The Et₂O solution was washed with 10% HCl, saturated NaCl and dried over anhydrous MgSO₄. Et₂O was removed under vacuum and the residue was taken in MeOH. The MeOH solution was treated with trimethylsilyldiazomethane (18.5 mL, 2M in hexane) and stirred at room temperature for 1h. The solvents were removed under vacuum to give 2 g (31%) of methyl 4-(1,1-dimethyl-1-stannaethyl)-5-methylthiothiophene-2-carboxylate as an oil. ¹H-NMR (CDCl₃; 300 MHz) δ 0.31 (S, 9H), 2.57 (s, 3H), 3.86 (S, 3H), 6.98 (S, 1H).

***b) Methyl 4-(6-bromo(2-pyridyl))-5-methylthiothiophene-2-carboxylate:*** Methyl 4-(1,1-dimethyl-1-stannaethyl)-5-methylthiothiophene-2-carboxylate (195 mg, 0.56 mmol) as prepared in previous step, and 2,6-dibromopyridine (398 mg, 1.7 mmol) were taken in anhydrous DMF (2 mL). To this mixture tetrakistriphenylphosphine-palladium (20 mg) was added and heated at 120°C for 24 h. DMF was removed under vacuum and the residue was purified by preparative silica gel thin -layer chromatography to give 78 mg (41%) of methyl 4-(6-bromo(2-pyridyl))-5-methylthiothiophene-2-carboxylate as a solid. ¹H-NMR (CDCl₃; 300 MHz) δ 2.60 (S, 3H), 3.78 (s, 3H), 7.19 (S, 1H), 7.47 (dd, J = 1.09 and 7.67 Hz, 1H)), 7.58 (t, J = 7.70, 1H), 7.65 (dd, J = 1.12 and 7.43 Hz, 1H).

***c) Methyl 5-methylthio-4-(6-phenyl(2-pyridyl))thiophene-2-carboxylate:*** Methyl 4-(6-bromo(2-pyridyl))-5-methylthiothiophene-2-carboxylate (78 mg, 0.23 mmol) as prepared in previous step, phenylboronic acid (33 mg, 0.27 mmol) and tetrakistriphenylphosphine-palladium (10 mg) were taken in DMF (1 mL). To this solution K₂CO₃ (75 mg, 0.54 mmol) and H₂O (0.3 mL) were added and the mixture was stirred and heated at 90°C for 18h. Solvents were removed under vacuum and the residue was dissolved in EtOAc and extracted with H₂O, washed with saturated NaCl and dried over anhydrous Na₂SO₄. Thin-layer chromatography of the aqueous layer indicated the presence of some hydrolyzed product. Therefore the aqueous layer was separated acidified with 10% HCl and extracted with EtOAc. The EtOAc layer was washed with saturated NaCl and dried over anhydrous Na₂SO₄. This second EtOAc fraction was evaporated and the residue was dissolved in MeOH and treated with trimethylsilyldiazomethane (1.2 eq). This methanolic solution and the first EtOAc fraction were combined and evaporated. The residue was subjected to preparative thin-layer chromatography (10% EtOAc in Hexane) to give 40 mg (51%) of methyl 5-methylthio-4-(6-phenyl(2-pyridyl))thiophene-2-carboxylate which was used directly in the next step.

***d) 5-Methylthio-4-(6-phenyl(2-pyridyl))thiophene-2-carboxamidine:*** Methyl 5-methylthio-4-(6-phenyl(2-pyridyl))thiophene-2-carboxylate (40 mg, 0.12 mmol) as prepared in previous step was treated in a manner similar to that for Example 1, to give 10 mg of 5-methylthio-4-(6-phenyl(2-pyridyl))thiophene-2-carboxamidine as a solid. ¹H-NMR (CD₃OD; 300 MHz) δ 2.69 (s, 3H), 7.45-7.60 (m, 3H), 7.62 (s, 1H), 7.79 (dd, J = 0.92 and 7.79 Hz, 1H), 7.96 (dd, J = 0.85 and 7.98 Hz, 1H), 8.03-8.12 (m, 3H). Mass spectrum (ESI, m/z): Calcd. for C₁₇H₁₅N₃S₂, 326.1 (M+H), found 326.1.

### Example 43

### 5-Methylthio-4-(3-phenylphenyl)thiophene-2-carboxamidine

***a) Methyl 5-methylthio-4-(3-phenylphenyl)thiophene-2-carboxylate:*** Methyl 4-(1,1-dimethyl-1-stannaethyl)-5-methylthiothiophene-2-carboxylate (200 mg, 0.57 mmol, as prepared in Example 42, step a) and 1-bromo-3-phenylbenzene (266 mg, 1.14 mmol) were taken in anhydrous DMF (2 mL). To this mixture tetrakistriphenylphosphine-palladium (20 mg) was added and heated at 120°C for 24 h. DMF was removed under vacuum and the residue was purified by preparative silica gel thin -layer chromatography to give 39 mg (20 %) methyl 5-methylthio-4-(3-phenylphenyl)thiophene-2-carboxylate as a solid. ¹H-NMR (CD₃OD; 300 MHz) δ 2.60 (s, 3H), 3.75 (s, 3H), 7.3-7.5 (m, 6H), 7.6-7.66 (m, 4H).

***b) 5-Methylthio-4-(3-phenylphenyl)thiophene-2-carboxamidine:*** Methyl 5-methylthio-4-(3-phenylphenyl)thiophene-2-carboxylate (35 mg, 0.1 mmol) as prepared in previous step was treated in a manner similar to that for Example 1, to give 17 mg of 5-methylthio-4-(3-phenylphenyl)thiophene-2-carboxamidine as a solid. ¹H-NMR (CD₃OD; 300 MHz) δ 2.60 (s, 3H), 7.3-7.6-7.66 (m, 10H). Mass spectrum (ESI, m/z): Calcd. for C₁₈H₁₆N₂S₂, 325.4 (M+H), found 325.2.

### Example 44

### 5-Methylthio-4-[4-(phenylthiomethyl)(1,3-thiazol-2-yl)]thiophene-2-carboxamidine

***a) Methyl 5-methylthio-4-[4-(phenylthiomethyl)(1,3-thiazol-2-yl)]thiophene-2-carboxylate:*** 2-Phenylthioacetyl chloride (1 g, 5.4 mmol) was treated in a manner similar to that for Example 32 step (a) to give 2-bromo-1-phenylthiomethylethan-1-one. The dry solid (1.3 g, 5.3 mmol) was dissolved in acetone (25 ml). To this solution 5-(methoxycarbonyl)-2-(methylthio)-thiophene-3-thiocarboxamide (1.32 g, 5.3 mmol, Maybridge Chemical Co.) was added and heated at reflux for 5 h, At this point the solid that precipitated was filtered off and washed with acetone and dried under vacuum to give 1.5 g (71%) of methyl 5-methylthio-4-[4-(phenylthiomethyl)(1,3-thiazol-2-yl)]thiophene-2-carboxylate which was used without further purification in the following step.

***b) 5-Methylthio-4-[4-(phenylthiomethyl)(1,3-thiazol-2-yl)]thiophene-2-carboxamidine:*** Methyl 5-methylthio-4-[4-(phenylthiomethyl)(1,3-thiazol-2-yl)]thiophene-2-carboxylate (1.5 g, 3.8 mmol) as prepared in previous step was treated in a manner similar to that for Example 1, however the product was purified by crystallizing from methanol to give 0.86 g (60%) 5-methylthio-4-[4-(phenylthiomethyl)(1,3-thiazol-2-yl)]thiophene-2-carboxamidine as a solid. ¹H-NMR (DMSO-d₆; 300 MHz) δ 2.72 (s, 3H), 4.38 (s, 2H), 7.18-7.39 (m, 5H), 7.57 (s, 1H), 8.46 (s, 1H). Mass spectrum (MALDI-TOF, m/z): Calcd. for C₁₆H₁₅N₃S₄, 378.0 (M+H), found 378.1.

### Example 45

### 4-[4-(2-Chloro-4,5-dimethoxyphenyl)(1,3-thiazol-2-yl)]-5-methylthiothiophene-2-carboxamidine

***a) Methyl 4-[4-(2-chloro-4,5-dimethoxyphenyl)(1,3-thiazol-2-yl)]-5-methylthiothiophene-2-carboxylate:*** 2-Chloro-4,5-dimethoxybenzoic acid (0.5 g, 2.3 mmol) and PCl₅ (0.54 g, 2.6 mmol) were placed in a round bottomed flask fitted with a reflux condenser. The mixture was heated in an oil bath at 120 °C for 70 min. The mixture was allowed to cool and the formed phosphorus oxychloride was removed under vacuum to give 0.52 g (96%) of 2-chloro-4,5-dimethoxybenzoyl chloride as a solid. 2-Chloro-4,5-dimethoxybenzoyl chloride (0.52 g, 2.2 mmol) was treated in a manner similar to that for Example 32 step (a) to give 2-bromo-1-(2-chloro-4,5-dimethoxyphenyl) ethan-1-one. The dry solid (0.65 g, 2.2 mmol) was dissolved in acetone (25 ml). To this solution 5-(methoxycarbonyl)-2-(methylthio)-thiophene-3-thiocarboxamide (0.55 g, 2.2 mmol) was added and heated at reflux for 5 h. At this point the solid that precipitated was filtered off and washed with acetone and dried under vacuum to give 0.53 g (54%) of methyl 4-[4-(2-chloro-4,5-dimethoxyphenyl)(1,3-thiazol-2-yl)]-5-methylthiothiophene-2-carboxylate. ¹H-NMR (DMSO-d₆; 300 MHz) δ 2.73 (s, 3H), 3.83 (s, 3H), 3.84 (s, 3H), 3.85 (s, 3H), 7.13 (s, 1H), 7.69 (s, 1H), 8.13 (s, 1H), 8.17 (s, 1H).

***b) 4-[4-(2-Chloro-4,5-dimethoxyphenyl)(1,3-thiazol-2-yl)]-5-methylthiothiophene-2-carboxamidine:*** Methyl 4-[4-(2-chloro-4,5-dimethoxyphenyl)(1,3-thiazol-2-yl)]-5-methylthiothiophene-2-carboxylate (0.53 g, 1.2 mmol) as prepared in previous step was treated in a manner similar to that for Example 1, however the product was purified by crystallizing from methanol to give to give 0.3 g (60%) 4-[4-(2-chloro-4,5-dimethoxyphenyl)(1,3-thiazol-2-yl]-5-methylthiothiophene-2-carboxamidine as a solid. ¹H-NMR (DMSO-d₆; 300 MHz) δ 2.77 (s, 3H), 3.84 (s, 6H), 7.13 (s, 1H), 7.71 (s, 1H), 8.17 (s, 1H), 8.69 (s, 1H), 9.16 (broad s, 2H), 9.48 (broad s, 2H). Mass spectrum (MALDI-TOF, m/z): Calcd. for C₁₇H₁₆N₃O₂S₃Cl, 426.0 (M+H), found 426.6.

### Example 46

### 4-[(Methylethyl)sulfonyl]-5-methylthiothiophene-2-carboxamidine

Methyl 4-[(methylethyl)sulfonyl]-5-methylthiothiophene-2-carboxylate (100 mg, Maybridge Chemical Company, Cornwall, UK) was treated in a manner similar to that for Example 1, to give 50 mg of 4-[(methylethyl)sulfonyl]-5-methylthiothiophene-2-carboxamidine. ¹H-NMR (DMSO-d₆; 300 MHz) δ 1.21 (d, J = 6.77 Hz, 6H), 2.66 (s, 3H), 3.25-3.84 (m, 1H), 7.85 (s, 1H). Mass spectrum (MALDI-TOF, CHCA matrix, m/z): Calcd. for C₉H₁₄N₂O₂S₃, 279.0 (M+H), found 279.3.

### Example 47

### Synthesis of methyl 2-{3-[2-(5-amidino-2-methylthio-3-thienyl)-1,3-thiazol-4-yl]phenoxy}acetate trifluoroacetate

To a solution of 42 mg (0.094 mmol) of(tert-butoxy)-*N*-({4-[4-(3-hydroxyphenyl)(1,3-thiazol-2-yl)]-5-methylthio(2-thienyl)}iminomethyl)carboxamide, prepared in a manner similar to Example 40, step (c), in 2 mL of anhydrous *N'N'*-dimethylformamide (DMF) was added potassium iodide (0.006 mmol, 1 mg, Aldrich Chemical Co.), cesium carbonate (0.187 mmol, 61 mg, Aldrich Chemical Co.), and methyl bromoacetate (0.187 mmol, 18 µL, Aldrich Chemical Co.) and heated to 60°C overnight. The reaction solution was concentrated and purified on a 1 mm silica prep plate eluting with 3% methanol/CH₂Cl₂ to afford 11 mg (23% yield) of methyl 2-{3-[2-(5-{[(tert-butoxy)carbonylamino]iminomethyl}-2-methylthio-3-thienyl)-1,3-thiazol-4-yl]phenoxy}acetate which was then subjected to a solution of 50% trifluoracetic acid/CH₂Cl₂ for 1 h then concentrated and triturated with diethyl ether and dried to afford 7 mg (77% yield) of methyl 2-{3-[2-(5-amidino-2-methylthio-3-thienyl)-1,3-thiazol-4-yl]phenoxy}acetate trifluoroacetate. ¹H-NMR (CD₃OD; 300 MHz) δ 8.51 (s, 1H), 7.92 (s, 1H), 7.64-7.68 (m, 2H), 7.34-7.39 (t, 1H), 6.91-6.95 (m, 1H), 4.8 (s, 2H) 3.80 (s, 3H), 2.78 (s, 3H). Mass Spectrum (LC-Q ESI, m/z) Calcd. for C₁₈H₁₇N₃O₃S₃: 419.5 (M+H), found 420.3.

### Example 48

### Synthesis of 5-methylthio-4-[4-(3-{[N-benzylcarbamoyl]methoxy}phenyl)(1,3-thiazol-2-yl)]thiophene-2-carboxamidine trifluoroacetate

100 mg (0.197 mmol) of 2-{3-[2-(5-{[(tert-butoxy)carbonylamino]iminomethyl}-2-methylthio-3-thienyl)-1,3-thiazol-4-yl]phenoxy}acetic acid, as prepared in the previous step, were dissolved in 1 mL of anhydrous DMF and PyBOP (0.396 mmol, 206 mg), benzylamine (0.396 mmol, 42 mg), and diisopropylethylamine (0.494 mmol; 86 µL) were added to the solution and stirred for 18 hrs after which the solution was concentrated and purified on a 2 g silica SPE column and deprotected with 50% trifluoroacetic acid/ methylene chloride to afford 60 mg (67% yield) of 5-methylthio-4-[4-(3-{[N-benzylcarbamoyl]methoxy}phenyl)(1,3-thiazol-2-yl)]thiophene-2-carboxamidine trifluoroacetate. ¹H-NMR (CD₃Cl₃/TFA-d; 300 MHz) δ 8.97 (s, 1H), 7.86 (s, 1H), 7.50-7.56 (t, 1H), 7.26-7.39 (m, 7H), 7.16-7.18 (d, 1H), 4.79 (s, 2H) 4.59 (s, 2H), 2.95 (s, 3H). Mass Spectrum (ESI, m/z) Calcd. for C₂₄H₂₂N₄O₂S₃: 494.6 (M+H), found 495.2.

### Example 49

### Synthesis of 4-{4-[3-({N-[(3,4-dimethoxyphenyl)methyl]carbamoyl}methoxy)phenyl](1,3-thiazol-2-yl)}-5-methylthiothiophene-2-carboxamidine trifluoroacetate

Dissolved 100 mg (0.197 mmol) of2-{3-[2-(5-{[(tert-butoxy)carbonylamino]iminomethyl}-2-methylthio-3-thienyl)-1,3-thiazol-4-yl]phenoxy}acetic acid, prepared in a manner similar to Example 48, step (c), in 1 mL of anhydrous DMF and added PyBOP (0.396 mmol, 206 mg), 3,4-dimethoxybenzylamine (0.396 mmol,66 mg), and diisopropylethylamine (0.494 mmol; 86 µL) and let stir for 18 hrs after which solution was concentrated and purified on a 2 g silica SPE column and deprotected with 50% trifluoroacetic acid/ methylene chloride to afford 45 mg (41% yield) 4-{4-[3-({N-[(3,4-dimethoxyphenyl)methyl]carbamoyl}methoxy)phenyl](1,3-thiazol-2-yl)}-5-methylthiothiophene-2-carboxamidine trifluoroacetate. ¹H-NMR (CD₃Cl₃/TFA-d) 300 MHz) δ 8.48 (s, 1H), 7.78 (s, 1H), 7.71-7.73 (m, 1H), 7.65-7.67 (d, 1H), 7.36-7.41 (t, 1H), 7.00-7.04 (d, 1H) 4.68 (s, 2H), 4.43 (s, 2H), 3.75 (s, 3H). 3.56 (s, 3H). 2.78 (s, 3H). Mass Spectrum (LC-Q ESI, m/z) Calcd. for C₂₆H₂₆N₄O₄S₃: 554.6 (M+H), found 555.2

### Example 50

### Synthesis of 5-methylthio-4-{4-[3-({N-[2-(phenylamino)ethyl]carbamoyl}methoxy)phenyl](1,3-thiazol-2-yl)}thiophene-2-carboxamidine trifluoroacetate

Dissolved 100 mg (0.197 mmol) of 2-{3-[2-(5-{[(tert-butoxy)carbonylamino]iminomethyl}-2-methylthio-3-thienyl)-1,3-thiazol-4-yl]phenoxy}acetic acid, prepared in a manner similar to Example 48, step (c), in 1 mL of anhydrous DMF and added PyBOP (0.396 mmol, 206 mg), N-phenylethylenediamine (0.396 mmol, 54 mg), and diisopropylethylamine (0.494 mmol; 86 µL) and let stir for 18 hrs after which solution was concentrated and purified on a 2 g silica SPE column and deprotected with 50% trifluoroacetic acid/ methylene chloride to afford 65 mg (63% yield) 5-methylthio-4-{4-[3-({N-[2-(phenylamino)ethyl]carbamoyl}methoxy)phenyl](1,3-thiazol-2-yl)}thiophene-2-carboxamidine trifluoroacetate ¹H-NMR (CD₃Cl₃/TFA-d 300 MHz) δ 8.50 (s, 1H), 7.82 (s, 1H), 7.77 (s, 1H), 7.65-7.67 (d, 1H), 7.36-7.41 (t,1H), 7.00-7.04 (d, 1H) 4.68 (s, 2H), 4.43 (s, 2H), 3.75 (s, 3H). 3.56 (s, 3H). 2.78 (s, 3H). Mass Spectrum (LC-Q ESI, m/z) Calcd. for C₂₅H₂₅N₅O₂S₃: *523.6* (M+H), found 524.1

### Example 51

### Synthesis of 5-methylthio-4-[4-(3-{[N-(2-morpholin-4-ylethyl)carbamoyl]methoxy}phenyl)(1,3-thiazol-2-yl)]thiophene-2-carboxamidine trifluoroacetate

83 mg (0.164 mmol) of 2-{3-[2-(5-{[(tert-butoxy)carbonylamino]iminomethyl}-2-methylthio-3-thienyl)-1,3-thiazol-4-yl]phenoxy}acetic acid, prepared in a manner similar to Example 40, step (c), was reacted with 2-morpholin-4-ylethylamine (0.328 mmol, 43 µL) in a manner similar to Example 48 to afford 46 mg (54% yield) of 5-methylthio-4-[4-(3-{[N-(2-morpholin-4-ylethyl)carbamoyl]methoxy}phenyl)(1,3-thiazol-2-yl)]thiophene-2-carboxamidine trifluoroacetate. ¹H-NMR (DMSO-d₆, 300 MHz) δ 9.38 (bs, 2H), 9.08 (bs, 2H), 8.61 (s, 1H), 8.45 (t, 1H), 8.27 (s, 1H), 7.69-7.74 (m, 2H) 7.42-7.47 (t, 1H), 7.00-7.03 (d, J= 8 Hz, 1H), 4.62 (s, 2H), 3.53-3.64 (m, 5H), 3.24-3.38 (m, 5H), 2.80 (s, 3H), 1.1 (t, 2H). Mass Spectrum (ESI, m/z) Calcd. for C₂₃H₂₇N₅O₃S₃: 517.6 (M+H), found 518.2.

### Example 52

### Synthesis of 5-methylthio-4-{4-[3-(2-morpholin-4-yl-2-oxoethoxy)phenyl](1,3-thiazol-2-yl)}thiophene-2-carboxamidine trifluoroacetate

73 mg (0.144 mmol) of 2-{3-[2-(5-{[(tert-butoxy)carbonylamino]iminomethyl}-2-methylthio-3-thienyl)-1,3-thiazol-4-yl]phenoxy}acetic acid, prepared in a manner similar to Example 48, step (c), was reacted with morpholine (0.288 mmol; 25 µL) in a manner similar to Example 48 step (b) to afford 50 mg (75% yield) 5-methylthio-4-{4-[3-(2-morpholin-4-yl-2-oxoethoxy)phenyl](1,3-thiazol-2-yl)}thiophene-2-carboxamidine trifluoroacetate. ¹H-NMR (DMSO-d₆/TFA-d 300 MHz) δ 9.38 (bs, 1H), 9.08 (bs, 2H), 8.66 (s, 1H), 8.22 (s, 1H), 7.69-7.74 (m, 2H) 7.39-7.45 (t, 1H), 6.98-7.00 (dd, J=2.3 Hz and 8.2 Hz, 1H), 4.95 (s, 2H), 3.53-3.67 (m, 8H), 2.82 (s, 3H). Mass Spectrum (ESI, m/z) Calcd. for C₂₁H₂₂N₄O₃S₃: 474.6 (M+H), found 475.2.

### Example 53

### Synthesis of 5-methylthio-4-{4-[3-(2-oxo-2-piperazinylethoxy)phenyl](1,3-thiazol-2-yl)}thiophene-2-carboxamidine trifluoroacetate

100 mg (0.198 mmol) of 2-{3-[2-(5-{[(tert-butoxy)carbonylamino}iminomethyl}-2-methylthio-3-thienyl)-1,3-thiazol-4-yl]phenoxy}acetic acid, prepared in a manner similar to Example 48, step (c), was reacted with tert-butyl piperazinecarboxylate (0.396 mmol; 74 mg) in a manner similar to Example 48 step (b) to afford 40 mg (43% yield) of 5-methyithio-4-{4-[3-(2-oxo-2-piperazinylethoxy)phenyl](1,3-thiazol-2-yl)}thiophene-2-carboxamidine trifluoroacetate. ¹H-NMR (DMSO-d₆/TFA-d); 300 MHz) δ 8.68 (s, 1H), 8.20(s, 1H), 7.75 (m, 2H) 7.40-7.46 (t, 1H), 6.99-7.03 (dd, J= 2.3 Hz and 8.1 Hz, 1H), 5.02 (s, 2H), 3.76 (bs, 4H), 3.17-3.26 (m, 4H). 2.82 (s, 3H). Mass Spectrum (LC-Q ESI, m/z) Calcd. for C₂₁H₂₃N₅O₂S₃: 473.6 (M+H), found 474.2.

### Example 54

### Synthesis of 4-[4-(3-{[N-(2-aminoethyl)carbamoyl]methoxy}phenyl)(1,3-thiazol-2-yl)]-5-methylthiothiophene-2-carboxamidine hydrochloride

51 mg (0.101 mmol) of 2-{3-[2-(5-{[(tert-butoxy)carbonylamino]iminomethyl}-2-methylthio-3-thienyl)-1,3-thiazol-4-yl]phenoxy}acetic acid, prepared in a manner similar to Example 48, step (c), was reacted with N-(2-aminoethyl)(tert-butoxy)carboxamide (0.202 mmol; 32 mg) in a manner similar to Example 48 step (b) to afford 80 mg (80% yield) of 4-(4-{3-[(N-{2-[(tert-butoxy}carbonylamino]ethyl}carbamoyl)methoxy]phenyl}(1,3-thiazol-2-yl))-5-methylthiothiophene-2-carboxamidine which was then deprotected with 4N HCl in dioxane to afford 36 mg (68% yield) of4-[4-(3-{[N-(2-aminoethyl)carbamoyl]methoxy}phenyl)(1,3-thiazol-2-yl)]-5-methylthiothiophene-2-carboxamidine hydrochloride. ¹H-NMR (CD₃OD); 300 MHz) δ 8.55 (s, 1H), 7.95 (s, 1H), 7.69-7.76 (m, 2H) 7.38-7.44 (t, 1H), 7.03-7.06 (m, 1H), 4.80 (s, 2H), 3.43-3.59 (m, 2H), 3.13-3.31 (m, 2H), 2.83 (s, 3H). Mass Spectrum (ESI, m/z) Calcd. for C₁₉H₂₁N₅O₂S₃: 447.5 (M+H), found 448.2.

### Example 55

### Synthesis of 4-(4-{3-[2-(4-acetylpiperazinyl)-2-oxoethoxy]phenyl}(1,3-thiazol-2-yl))-5-methylthiothiophene-2-carboxamidine trifluoroacetate

52 mg (0.103 mmol) of 2-{3-[2-(5-{[(tert-butoxy)carbonylamino]iminomethyl}-2-methylthio-3-thienyl)-1,3-thiazol-4-yl]phenoxy}acetic acid, prepared in a manner similar to Example 48, step (c), was reacted with 1-acetyl piperazine (0.154 mmol, 20 mg), 1-hydroxy-7-azabenzotriazole (HOAt)) (0.154 mmol, 21 mg), O-(7-azabenzotriazol-1-yl)-1,1,3,3-tetramethyluronium hexafluorophosphate) HATU (0.154 mmol, 58 mg) and diisopropylethylamine (0.258 mmol, 44 µL) in DMF to afford crude product which was then purified on 1 mm silica prep plates eluting with 3% methanol/methylene chloride to afford 28 mg (53% yield) of N-{[4-(4-{3-[2-(4-acetylpiperazinyl)-2-oxoethoxy]phenyl}(1,3-thiazol-2-yl))-5-methylthio(2-thienyl)]iminomethyl}(tert-butoxy)carboxamide. This was subsequently reacted with a solution of trifluoroacetic acid: methylene chloride: water (47.5%: 47.5%: 2.5%) for 1 hour, concentrated and purified on a silica SPE column eluting with 15% methanol/methylene chloride to afford 20 mg (80% yield) of4-(4-{3-[2-(4-acetylpiperazinyl)-2-oxoethoxy]phenyl}(1,3-thiazol-2-yl))-5-methylthiothiophene-2-carboxamidine trifluoroacetate. ¹H-NMR (CD₃OD); 300 MHz) δ 8.48 (s, 1H), 7.91 (s, 1H), 7.66-7.71 (m, 2H) 7.35-7.41 (t, 1H), 6.97-7.00 (dd, J= 2 Hz and 8.1 Hz, 1H), 4.93 (s, 2H), 3.52-3.67 (m, 8H), 2.78 (s, 3H), 2.12 (s, 3H). Mass Spectrum (ESI, m/z) Calcd. for C₂₃H₂₅N₅O₃S₃:515.6(M+H), found 516.2.

### Example 56

### Synthesis of 4-(4-{3-[2-(4-methylpiperazinyl)-2-oxoethoxy]phenyl}(1,3-thiazol-2-yl))-5-methylthiothiophene-2-carboxamidine trifluoroacetate

54 mg (0.107 mmol) of 2-{3-[2-(5-{[(tert-butoxy)carbonylamino]iminomethyl}-2-methylthio-3-thienyl)-1,3-thiazol-4-yl]phenoxy}acetic acid, prepared in a manner similar to Example 48, step (c), was reacted with N-methyl piperazine (0.128 mmol, 14 µL), 1-hydroxy-7-azabenzotriazole (HOAt) (0.128 mmol, 17 mg), O-(7-azabenzotriazol-1-yl)-1,1,3,3-tetramethyluronium hexafluorophosphate) HATU (0.128 mmol, 49 mg) and diisopropylethylamine (0.268 mmol, 56 µL) in DMF to afford crude product which was then partitioned between methylene chloride and 1N NaOH and washed. The organic layer was obtained and similarly washed with 10 % citric acid and saturated aq. sodium chloride, dried over sodium sulfate and concentrated to a yellow oil. The oil was then purified on 1 mm silica prep plates eluting with 5% methanol/methylene chloride to afford (tert-butoxy)-N-{imino[4-(4-{3-[2-(4-methylpiperazinyl)-2-oxoethoxy]phenyl}(1,3-thiazol-2-yl))-5-methylthio(2-thienyl)]methyl}carboxamide. This was subsequently reacted with a solution of trifluoroacetic acid: methylene chloride: water (47.5%: 47.5%: 2.5%) for 1 hour, concentrated and purified on a silica SPE column eluting with 10-15% methanol/methylene chloride to afford 17 mg (33% yield) of 4-(4-{3-[2-(4-methylpiperazinyl)-2-oxoethoxy]phenyl}(1,3-thiazol-2-yl))-5-methylthiothiophene-2-carboxamidine trifluoroacetate. ¹H-NMR (CD₃OD); 300 MHz) δ 8.52 (s, 1H), 7.91 (s, 1H), 7.66-7.70 (m, 2H) 7.35-7.40 (t, 1H), 6.96-6.99 (dd, J= 2 Hz and 8.1 Hz, 1H), 4.90 (s, 2H), 3.64-3.68 (t, 4H), 2.78 (s, 3H), 2.49-2.57 (m, 4H), 2.35 (s, 3H). Mass Spectrum (ESI, m/z) Calcd. for C₂₂H₂₅N₅O₂S₃: 487.6 (M+H), found 488.2

### Example 57

### Synthesis of 5-methylthio-4-[4-(3-{2-oxo-2-[4-benzylpiperazinyl]ethoxy}phenyl)(1,3-thiazol-2-yl)]thiophene-2-carboxamidine trifluoroacetate

54 mg (0.107 mmol) of 2-{3-[2-(5-{[(tert-butoxy)carbonylamino]iminomethyl}-2-methylthio-3-thienyl)-1,3-thiazol-4-yl]phenoxy}acetic acid, prepared in a manner similar to Example 48, step (c), was reacted with N-benzylpiperazine (0.128 mmol, 22 µL), 1-hydroxy-7-azabenzotriazole (HOAt) (0.128 mmol, 17 mg), O-(7-azabenzotriazol-1-yl)-1,1,3,3-tetramethyluronium hexafluorophosphate) HATU (0.128 mmol, 48 mg) and diisopropylethylamine (0.267 mmol, 50 µL) in DMF to afford crude product which was then partitioned between methylene chloride and 1N NaOH and washed. The organic layer was obtained and similarly washed with 10% citric acid and saturated aq. sodium chloride, dried over sodium sulfate and concentrated to a yellow oil. The oil was then purified on 1 mm silica prep plates eluting with 5% methanol/methylene chloride to afford (tert-butoxy)-N-(imino{5-methylthio-4-[4-(3-{2-oxo-2-[4-benzylpiperazinyl]ethoxy}phenyl)(1,3-thiazol-2-yl)](2-thienyl)}methyl)carboxamide. This was subsequently reacted with a solution of trifluoroacetic acid: methylene chloride: water (47.5%: 47.5%: 2.5%) for 1 hour, concentrated and purified on a 5 g silica SPE column eluting with 10-15% methanol/methylene chloride to afford 36 mg (60% yield) of 5-methylthio-4-[4-(3-{2-oxo-2-[4-benzylpiperazinyl]ethoxy}phenyl)(1,3-thiazol-2-yl)]thiophene-2-carboxamidine trifluoroacetate. ¹H-NMR (CD₃OD); 300 MHz) δ 8.54 (s, 1H), 7.93 (s, 1H), 7.69-7.72 (m, 2H), 7.50 (s, 5H) 7.36-7.41 (t, 1H), 6.97-7.01 (dd, J= 2 Hz and 8.1 Hz, 1H), 4.94(s, 2H), 4.37(s, 2H), 3.3 (m, 4H), 2.81 (s, 3H), 2.49-2.57 (m, 4H), 2.35 (s, 3H). Mass (ESI, m/z) Calcd. for C₂₈H₂₉N₅O₂S₃: 563.7 (M+H), found 564.3.

### Example 58

### Synthesis of (D,L)-4-(4-{3-[2-(2-aminopyrrolidinyl)-2-oxoethoxy]phenyl}(1,3-thiazol-2-yl))-5-methylthiothiophene-2-carboxamidine trifluoroacetate

41 mg (0.081 mmol) of 2-{3-[2-(5-{[(tert-butoxy)carbonylamino]iminomethyl}-2-methylthio-3-thienyl)-1,3-thiazol-4-yl]phenoxy}acetic acid, prepared in a manner similar to Example 48, step (c), was reacted with (D,L) (tert-butoxy)-N-pyrrolidin-3-ylcarboxamide (0.122 mmol, 23 mg), O-(7-azabenzotriazol-1-yl)-1,1,3,3-tetramethyluronium hexafluorophosphate) HATU (0.122 mmol, 46 mg), 1-hydroxy-7-azabenzotriazole (HOAt) (0.122 mmol, 17 mg) and diisopropylethylamine (0.203 mmol, 35µL) in a manner similar to Example 56 to afford 20 mg (53% yield) of (D,L)- 4-(4-{3-[2-(3-aminopyrrolidinyl)-2-oxoethoxy]phenyl}(1,3-thiazol-2-yl))-5-methylthiothiophene-2-carboxamidine trifluoroacetate. ¹H-NMR (CD₃OD); 300 MHz) δ 8.54 (s, 1H), 7.94 (s, 1H), 7.69-7.72 (m, 2H) 7.36-7.41 (t, 1H), 6.97-7.01 (dd, J= 2 Hz and 8.1 Hz, 1H), 4.85 (s, 2H), 4.37(s, 2H), 3.60-4.01 (m, 5H), 2.81 (s, 3H), 2.15-2.71 (m, 2H). Mass Spectrum (ESI, m/z) Calcd. for C₂₁H₂₃N₅O₂S₃: 473.6 (M+H), found 474.3.

### Example 59

### Synthesis of 5-methylthio-4-{4-[3-(2-oxo-2-piperidylethoxy)phenyl](1,3-thiazol-2-yl)}thiophene-2-carboxamidine trifluoroacetate

33 mg (0.065 mmol) of 2-{3-[2-(5-{[(tert-butoxy)carbonylamino]iminomethyl}-2-methylthio-3-thienyl)-1,3-thiazol-4-yl]phenoxy}acetic acid, prepared in a manner similar to Example 40, step (c), was reacted with piperidine (0.078 mmol, 8 µL), O-(7-azabenzotriazol-1-yl)-1,1,3,3-tetramethyluronium hexafluorophosphate) HATU (0.078 mmol, 30 mg), 1-hydroxy-7-azabenzotriazole (HOAt) (0.078 mmol, 11 mg) and diisopropylethylamine (0.163 mmol, 56µL) in a manner similar to Example 57 to afford 15 mg (41% yield) of 5-methylthio-4-{4-[3-(2-oxo-2-piperidylethoxy)phenyl](1,3-thiazol-2-yl)}thiophene-2-carboxamidine trifluoroacetate. ¹H-NMR (CD₃OD); 300 MHz) δ 8.54 (s, 1H). 7.92 (s, 1H), 7.65-7.71 (m, 2H) 7.35-7.40 (t, 1H), 6.96-6.99 (dd, J= 2 Hz and 8.1 Hz, 1H), 4.95 (s, 2H), 3.52-3.60 (m, 4H), 2.80 (s, 3H), 1.57-1.70 (m, 6H). Mass Spectrum (ESI, m/z) Calcd. for C₂₂H₂₄N₄O₂S₃: 472.6 (M+H), found 473.2.

### Example 60

### Synthesis of 2-(3-{2-[5-(imino{[(4-polystyryloxyphenyl)methoxy]carbonylamino}methyl)-2-methylthio-3-thienyl]-1,3-thiazol-4-yl}phenoxy)acetic acid

2 g (1.86 mmol) of *p*-Nitrophenyl carbonate Wang resin (0.93 mmol/g) (Calbiochem-Novabiochem, San Diego, CA) was suspended in 9 mL of a 2:1 mixture of anhydrous DMSO:DMF. 2 g (4.93 mmol) of 2-{3-[2-(5-amidino-2-methylthio-3-thienyl)-1,3-thiazol-4-yl]phenoxy}acetic acid was added to suspension followed by the addition of 1 mL of 1,8-diazabicyclo[5.4.0]undec-7-ene, (DBU, Aldrich Chemical Co., 6.69 mmol) and let shake vigorously for 5 days after which resin was washed thoroughly with DMF, MeOH, and diethyl ether and dried in *vacuo* to afford 2 g of resin-bound 2-{3-[2-(5-amidino-2-methylthio-3-thienyl)-1,3-thiazol-4-yl]phenoxy}acetic acid.

### Example 61

### Synthesis of (D,L)-ethyl 1-(2-{3-[2-(5-amidino-2-methylthio-3-thienyl)-1,3-thiazol-4-yl]phenoxy}acetyl)piperidine-2-carboxylate trifluoroacetate

100 mg (0.093 mmol) of resin-bound 2-{3-[2-(5-amidino-2-methylthio-3-thienyl)-1,3-thiazol-4-yl]phenoxy}acetic acid (0.93 mmol/g), as prepared in a manner similar to Example 60, was suspended 1 mL of anhydrous DMF. O-(7-azabenzotriazol-1-yl)-1,1,3,3-tetramethyluronium hexafluorophosphate) HATU (0.5 M, 190 mg), 1-hydroxy-7-azabenzotriazole (HOAt) (0.5 M; 68 mg), ethyl piperidine-2-carboxylate (0.5 M; 78 µL) and diisopropylethylamine (0.233 mmol, 40 µL) were added and allowed to shake vigorously for 18 hrs, after which the resin was washed thoroughly with DMF, methanol, methylene chloride, and diethyl ether. After drying, crude product was removed from resin by reaction with a solution of trifluoroacetic acid: methylene chloride: water (47.5%: 47.5%: 2.5%) for 1 hour. The solution was filtered and concentrated to a yellow oil. After purification on a 2 g silica SPE column, eluting with a gradient of 3%-10% MeOH/methylene chloride, 15 mg (30% yield) of (D,L)-ethyl 1-(2-{3-[2-(5-amidino-2-methylthio-3-thienyl)-1,3-thiazol-4-yl]phenoxy}acetyl)piperidine-2-carboxylate trifluoroacetate was obtained. Mass Spectrum (ESI, m/z) Calcd. for C₂₅H₂₈N₄O₄S₃: 544.70 (M+H). found 545.2

### Example 62

### Synthesis of 5-methylthio-4-{4-[3-(2-oxo-2-pyrrolidinylethoxy)phenyl](1,3-thiazol-2-yl)}thiophene-2-carboxamidine trifluoroacetate

100 mg (0.093 mmol) of resin-bound 2-{3-[2-(5-amidino-2-methylthio-3-thienyl)-1,3-thiazol-4-yl]phenoxy}acetic acid (0.93 mmol/g), as prepared in a manner similar to Example 60, was suspended 1 mL of anhydrous DMF. O-(7-azabenzotriazol-1-yl)-1,1,3,3-tetramethyluronium hexafluorophosphate) HATU (0.5 M, 190 mg), 1-hydroxy-7-azabenzotriazole (HOAt) (0.5 M; 68 mg), pyrrolidine (0.5 M; 42 µL) and diisopropylethylamine (0.233 mmol, 40 µL) were added and allowed to shake vigorously for 18 hours, after which the resin was washed thoroughly with DMF, methanol, methylene chloride, and diethyl ether. After drying, crude product was removed from resin by reaction with a solution of trifluoroacetic acid: methylene chloride: water (47.5%: 47.5%: 2.5%) for 1 hour. After trituration with diethyl ether and drying, 18 mg (42% yield) of 5-methylthio-4-{4-[3-(2-oxo-2-pyrrolidinylethoxy)phenyl](1,3-thiazol-2-yl)}thiophene-2-carboxamidine trifluoroacetate was obtained. Mass Spectrum (ESI, m/z) Calcd. for C₂₁H₂₂N₄O₂S₃: 458.6 (M+H), found 459.2

### Example 63

### Synthesis of 5-methylthio-4-[4-(3-{2-oxo-2-[4-benzylpiperidyl]ethoxy}phenyl)(1,3-thiazol-2-yl)]thiophene-2-carboxamidine trifluoroacetate

80 mg (0.074 mmol) of resin-bound 2-{3-[2-(5-amidino-2-methylthio-3-thienyl)-1,3-thiazol-4-yl]phenoxy}acetic acid (0.93 mmol/g), as prepared in a manner similar to Example 60, was suspended in 1 mL of anhydrous DMF. O-(7-azabenzotriazol-1-yl)-1,1,3,3-tetramethyluronium hexafluorophosphate) HATU (0.5 M, 190 mg), 1-hydroxy-7-azabenzotriazole (HOAt) (0.5 M; 68 mg), 4-benzyl piperidine (0.5 M; 88 µL) and diisopropylethylamine (0.185 mmol, 32 µL) were added and allowed to shake vigorously for 18 hrs, after which the resin was washed thoroughly with DMF, methanol, methylene chloride, and diethyl ether. After drying, crude product was removed from resin by reaction with a solution of trifluoroacetic acid: methylene chloride: water (47.5%: 47.5%: 2.5%) for 1 hour. After trituration with diethyl ether and drying, 17 mg (40% yield) of 5-methylthio-4-[4-(3-{2-oxo-2-[4-benzylpiperidyl]ethoxy}phenyl)(1,3-thiazol-2-yl)]thiophene-2-carboxamidine trifluoroacetate was obtained. Mass Spectrum (ESI, m/z) Calcd. for C₂₉H₃₀N₄O₂S₃: 562.7 (M+H), found 563.3.

### Example 64

### Synthesis of (D,L)-4-(4-{3-[2-(3-methylpiperidyl)-2-oxoethoxy]phenyl}(1,3-thiazol-2-yl))-5-methylthiothiophene-2-carboxamidine trifluoroacetate

80 mg (0.074 mmol) of resin-bound 2-{3-[2-(5-amidino-2-methylthio-3-thienyl)-1,3-thiazol-4-yl]phenoxy}acetic acid (0.93 mmol/g), as prepared in a manner similar to Example 60, was reacted with (+/-)-3-methyl piperidine (0.5 M, 59 µL) and O-(7-azabenzotriazol-1-yl)-1,1,3,3-tetramethyluronium hexafluorophosphate) HATU (0.5 M, 190 mg), 1-hydroxy-7-azabenzotriazole (HOAt) (0.5 M; 68 mg) and diisopropylethylamine (0.185 mmol, 32 µL) in 1 mL of anhydrous DMF in a manner similar to Example 63 to afford 10 mg (28% yield) of 4-(4-{3-[2-(3-methylpiperidyl)-2-oxoethoxy]phenyl}(1,3-thiazol-2-yl))-5-methylthiothiophene-2-carboxamidine trifluoroacetate. Mass Spectrum (ESI, m/z) Calcd. for C₂₃H₂₆N₄O₂S₃: 486.6 (M+H), found 487.3.

### Example 65

### Synthesis of 4-(4-{3-[2-(4-methylpiperidyl)-2-oxoethoxy]phenyl}(1,3-thiazol-2-yl))-5-methylthiothiophene-2-carboxamidine trifluoroacetate

80 mg (0.074 mmol) of resin-bound 2-{3-[2-(5-amidino-2-methylthio-3-thienyl)-1,3-thiazol-4-yl]phenoxy}acetic acid (0.93 mmol/g), as prepared in a manner similar to Example 60, was reacted with 4-methyl piperidine (0.5M, 59 µL) and O-(7-azabenzotriazol-1-yl)-1,1,3,3-tetramethyluronium hexafluorophosphate) HATU (0.5 M, 190 mg), 1-hydroxy-7-azabenzotriazole (HOAt) (0.5 M; 68 mg) and diisopropylethylamine (0.185 mmol, 32 µL) in 1 mL of anhydrous DMF in a manner similar to Example 63 to afford 12 mg (33% yield) of 4-(4-{3-[2-(4-methylpiperidyl)-2-oxoethoxy]phenyl}(1,3-thiazol-2-yl))-5-methylthiothiophene-2-carboxamidine trifluoroacetate. Mass Spectrum (ESI, m/z) Calcd. for C₂₃H₂₆N₄O₂S₃ : 486.6 (M+H), found 487.3.

### Example 66

### Synthesis of 4-(4-{3-[2-(2-azabicyclo[4.4.0]dec-2-yl)-2-oxoethoxy]phenyl}(1,3-thiazol-2-yl))-5-methylthiothiophene-2-carboxamidine trifluoroacetate

80 mg (0.074 mmol) of resin-bound 2-{3-[2-(5-amidino-2-methylthio-3-thienyl)-1,3-thiazol-4-yl]phenoxy} acetic acid (0.93 mmol/g), as prepared in a manner similar to Example 60, was reacted with decahydroquinoline (0.5M, 75 µL) and O-(7-azabenzotriazol-1-yl)-1,1,3,3-tetramethyluronium hexafluorophosphate) HATU (0.5 M, 190 mg), 1-hydroxy-7-azabenzotriazole (HOAt) (0.5 M; 68 mg) and diisopropylethylamine (0.185 mmol, 32 µL) in 1 mL of anhydrous DMF in a manner similar to Example 63 to afford 16 mg (41% yield) of 4-(4-{3-[2-(2-azabicyclo[4.4.0]dec-2-yl)-2-oxoethoxy]phenyl}(1,3-thiazol-2-yl))-5-methylthiothiophene-2-carboxamidine trifluoroacetate. Mass Spectrum (ESI, m/z) Calcd. for C₂₆H₃₀N₄O₂S₃: 526.7 (M+H), found 527.2.

### Example 67

### Synthesis of (D,L)-ethyl 1-(2-{3-[2-(5-amidino-2-methylthio-3-thienyl)-1,3-thiazol-4-yl]phenoxy}acetyl)piperidine-3-carboxylate trifluoroacetate

80 mg (0.074 mmol) of resin-bound 2-{3-[2-(5-amidino-2-methylthio-3-thienyl)-1,3-thiazol-4-yl]phenoxy}acetic acid (0.93 mmol/g), as prepared in a manner similar to Example 60, was reacted with ethyl nipecotate (0.5M, 78 µL) and O-(7-azabenzotriazol-1-yl)-1,1,3,3-tetramethyluronium hexafluorophosphate) HATU (0.5 M, 190 mg), 1-hydroxy-7-azabenzotriazole (HOAt) (0.5 M; 68 mg) and diisopropylethylamine (0.185 mmol, 32 µL) in 1 mL of anhydrous DMF in a manner similar to Example 63 to afford 18 mg (45% yield) of ethyl 1-(2-{3-[2-(5-amidino-2-methylthio-3-thienyl)-1,3-thiazol-4-yl]phenoxy}acetyl)piperidine-3-carboxylate trifluoroacetate. Mass Spectrum (ESI, m/z) Calcd. for C₂₅H₂₈N₄O₄S₃: 545.7 (M+H), found 545.2.

### Example 68

### Synthesis of 5-methylthio-4-{4-[3-(2-oxo-2-(1,2,3,4-tetrahydroquinolyl)ethoxy)phenyl](1,3-thiazol-2-yl)}thiophene-2-carboxamidine trifluoroacetate

100 mg (0.093 mmol) of resin-bound 2-{3-[2-(5-amidino-2-methylthio-3-thienyl)-1,3-thiazol-4-yl]phenoxy}acetic acid (0.93 mmol/g), as prepared in a manner similar to Example 60, was reacted with 1,2,3,4-tetrahydroisoquinoline (0.5M) and O-(7-azabenzotriazol-1-yl)-1,1,3,3-tetramethyluronium hexafluorophosphate) HATU (0.5M, 190 mg), 1-hydroxy-7-azabenzotriazole (HOAt) (0.5 M; 68 mg) and diisopropylethylamine (0.233 mmol, 40 µL) in 1 mL of anhydrous DMF in a manner similar to Example 63 to afford 20 mg (42% yield) of 5-methylthio-4-{4-[3-(2-oxo-2-(1,2,3,4-tetrahydroquinolyl)ethoxy)phenyl](1,3-thiazol-2-yl)}thiophene-2-carboxamidine trifluoroacetate. Mass Spectrum (ESI, m/z) Calcd. for C₂₆H₂₄N₄O₂S₃: 520.7 (M+H), found 521.2.

### Example 69

### Synthesis of ethyl 1-(2-{3-[2-(5-amidino-2-methylthio-3-thienyl)-1,3-thiazol-4-yl]phenoxy}acetyl)piperidine-4-carboxylate trifluoroacetate

100 mg (0.093 mmol) of resin-bound 2-{3-[2-(5-amidino-2-methylthio-3-thienyl)-1,3-thiazol-4-yl]phenoxy}acetic acid (0.93 mmol/g), as prepared in a manner similar to Example 60, was reacted with ethyl isonipecotate (0.5M, 77 mg) and O-(7-azabenzotriazol-1-yl)-1,1,3,3-tetramethyluronium hexafluorophosphate) HATU (0.5M, 190 mg), 1-hydroxy-7-azabenzotriazole (HOAt) (0.5 M; 68 mg) and diisopropylethylamine (0.233 mmol, 40 µL) in 1 mL of anhydrous DMF in a manner similar to Example 63 to afford 21 mg (42% yield) of ethyl 1-(2-{3-[2-(5-amidino-2-methylthio-3-thienyl)-1,3-thiazol-4-yl]phenoxy}acetyl)piperidine-4-carboxylate trifluoroacetate. Mass Spectrum (ESI, m/z) Calcd. for C₂₅H₂₈N₄O₄S₃: 545.7 (M+H), found 545.3.

### Example 70

### Synthesis of 4-(4-{3-[2-((3R)-3-hydroxypiperidyl)-2-oxoethoxy]phenyl}(1,3-thiazol-2-yl))-5-methylthiothiophene-2-carboxamidine trifluoroacetate

100 mg (0.093 mmol) of resin-bound 2-{3-[2-(5-amidino-2-methylthio-3-thienyl)-1,3-thiazol-4-yl]phenoxy}acetic acid (0.93 mmol/g), as prepared in a manner similar to Example 60, was reacted with R-(+)-3-hydroxy piperidine (0.5M, 69 mg) and O-(7-azabenzotriazol-1-yl)-1,1,3,3-tetramethyluronium hexafluorophosphate) HATU (0.5M, 190 mg), 1-hydroxy-7-azabenzotriazole (HOAt) (0.5M; 68 mg) and diisopropylethylamine (0.233 mmol, 40 µL) in 1 mL of anhydrous DMF in a manner similar to Example 63 to afford 16 mg (36% yield) of 4-(4-{3-[2-((3R)-3-hydroxypiperidyl)-2-oxoethoxy]phenyl}(1,3-thiazol-2-yl))-5-methylthiothiophene-2-carboxamidine trifluoroacetate. Mass Spectrum (ESI, m/z) Calcd. for C₂₂H₂₃N₄O₃S₃: 489.7 (M+H), found 489.2.

### Example 71

### Synthesis of D,L-4-(4-{3-[2-(2-ethylpiperidyl)-2-oxoethoxy]phenyl}(1,3-thiazol-2-yl))-5-methylthiothiophene-2-carboxamidine trifluoroacetate

100 mg (0.093 mmol) of resin-bound 2-{3-[2-(5-amidino-2-methylthio-3-thienyl)-1,3-thiazol-4-yl]phenoxy}acetic acid (0.93 mmol/g), as prepared in a manner similar to Example 60, was reacted with 2-ethyl piperidine (0.5M) and O-(7-azabenzotriazol-1-yl)-1,1,3,3-tetramethyluronium hexafluorophosphate) HATU (0.5M, 190 mg), 1-hydroxy-7-azabenzotriazole (HOAt) (0.5M; 68 mg) and diisopropylethylamine (0.233 mmol, 40 µL) in 1 mL of anhydrous DMF in a manner similar to Example 63 to afford 11 mg (23% yield) of D,L-4-(4-{3-[2-(2-ethylpiperidyl)-2-oxoethoxy]phenyl}(1,3-thiazol-2-yl))-5-methylthiothiophene-2-carboxamidine trifluoroacetate. Mass Spectrum (ESI, m/z) Calcd. for C₂₄H₂₇N₄O₂S₃: 501.4 (M+H), found 501.4.

### Example 72

### Synthesis of 4-(4-{3-[2-((3S)-3-hydroxypyrrolidinyl)-2-oxoethoxy]phenyl}(1,3-thiazol-2-yl))-5-methylthiothiophene-2-carboxamidine trifluoroacetate

100 mg (0.093 mmol) of resin-bound 2-{3-[2-(5-amidino-2-methylthio-3-thienyl)-1,3-thiazol-4-yl]phenoxy}acetic acid (0.93 mmol/g), as prepared in a manner similar to Example 60, was reacted with R-(-)-3-pyrrolidinol (0.5M, 62 mg) and O-(7-azabenzotriazol-1-yl)-1,1,3,3-tetramethyluronium hexafluorophosphate) HATU (0.5M, 190 mg), 1-hydroxy-7-azabenzotriazole (HOAt) (0.5M; 68 mg) and diisopropylethylamine (0.233 mmol, 40 µL) in 1 mL of anhydrous DMF in a manner similar to Example 63 to afford 10 mg (23% yield) of 4-(4-{3-[2-((3S)-3-hydroxypyrrolidinyl)-2-oxoethoxy]phenyl}(1,3-thiazol-2-yl))-5-methylthiothiophene-2-carboxamidine trifluoroacetate. Mass Spectrum (ESI, m/z) Calcd. for C₂₁H₂₂N₄O₃S₃: 475.2 (M+H), found 475.2.

### Example 73

### Synthesis of 5-methylthio-4-(4-{3-[(N-(5,6,7,8-tetrahydronaphthyl)carbamoyl)methoxy]phenyl}(1,3-thiazol-2-yl))thiophene-2-carboxamidine trifluoroacetate

100 mg (0.093 mmol) of resin-bound 2-{3-[2-(5-amidio-2-methylthio-3-thienyl)-1,3-thiazol-4-yl]phenoxy}acetic acid (0.93 mmol/g), as prepared in a manner similar to Example 60, was reacted with 5,6,7,8-tetrahydro-1-naphthylamine (0.5M, 73 mg) and O-(7-azabenzotriazol-1-yl)-1,1,3,3-tetramethyluronium hexafluorophosphate) HATU (0.5M, 190 mg), 1-hydroxy-7-azabenzotriazole (HOAt) (0.5 M; 68 mg) and diisopropylethylamine (0.233 mmol, 40 µL) in 1 mL of anhydrous DMF in a manner similar to Example 63 to afford 15 mg (30% yield) of 5-methylthio-4-(4-{3-[(N-(5,6,7,8-tetrahydronaphthyl)carbamoyl)methoxy]phenyl}{1,3-thiazol-2-yl))thiophene-2-carboxamidine trifluoroacetate. Mass Spectrum (ESI, m/z) Calcd. for C₂₇H₂₆N₄O₂S₃: 535.2 (M+H), found 535.3.

### Example 74

### Synthesis of D,L-4-[4-(3-{2-[3-(hydroxymethyl)piperidyl]-2-oxoethoxy}phenyl)(1,3-thiazol-2-yl)]-5-methylthiothiophene-2-carboxamidine trifluoroacetate

100 mg (0.093 mmol) of resin-bound 2-{3-[2-(5-amidino-2-methylthio-3-thienyl)-1,3-thiazol-4-yl]phenoxy}acetic acid (0.93 mmol/g), as prepared in a manner similar to Example 60, was reacted with 3-piperidine methanol (0.5M, 58 mg) and O-(7-azabenzotriazol-1-yl)-1,1,3,3-tetramethyluronium hexafluorophosphate) HATU (0.5M, 190 mg), 1-hydroxy-7-azabenzotriazole (HOAt) (0.5M; 68 mg) and diisopropylethylamine (0.233 mmol, 40 µL in 1 mL of anhydrous DMF in a manner similar to Example 40 to afford to 19 mg (40% yield) of D,L-4-[4-(3-{2-[3-(hydroxymethyl)piperidyl]-2-oxoethoxy}phenyl)(1,3-thiazol-2-yl)]-5-methylthiothiophene-2-carboxamidine trifluoroacetate. Mass Spectrum (ESI, m/z) Calcd. for C₂₃H₂₅N₄O₃S₃: 503.2 (M+H), found 503.2.

### Example 75

### Synthesis of 4-{4-[3-(2-{(2R)-2-[(phenylamino)methyl]pyrrolidinyl}-2-oxoethoxy)phenyl](1,3-thiazol-2-yl)}-5-methylthiothiophene-2-carboxamidine trifluoroacetate

100 mg (0.093 mmol) of resin-bound 2-{3-[2-(5-amidino-2-methylthio-3-thienyl)-1,3-thiazol-4-yl]phenoxy}acetic acid (0.93 mmol/g), as prepared in a manner similar to Example 60, was reacted with (S)-(+)-2-anilino methyl pyrrolidine (0.5M, 88 mg) and O-(7-azabenzotriazol-1-yl)-1,1,3,3-tetramethyluronium hexafluorophosphate) HATU (0.5M, 190 mg), 1-hydroxy-7-azabenzotriazole (HOAt) (0.5M; 68 mg) and diisopropylethylamine (0.233 mmol, 40 µL) in 1 mL of anhydrous DMF in a manner similar to Example 63 to afford 13 mg (25% yield) of 4-{4-[3-(2-{(2R)-2-[(phenylamino)methyl]pyrrolidinyl}-2-oxoethoxy)phenyl](1,3-thiazol-2-yl)}-5-methylthiothiophene-2-carboxamidine trifluoroacetate. Mass Spectrum (ESI, m/z) Calcd. for C₂₈H₂₈N₅O₂S₃: 563.8 (M+H), found 564.2.

### Example 76

### Synthesis of 4-[4-(3-{2-[(3R)-3-(methoxymethyl)pyrrolidinyl]-2-oxoethoxy}phenyl)(1,3-thiazol-2-yl)]-5-methylthiothiophene-2-carboxamidine trifluoroacetate

100 mg (0.093 mmol) of resin-bound 2-{3-[2-(5-amidino-2-methylthio-3-thienyl)-1,3-thiazol-4-yl]phenoxy}acetic acid (0.93 mmol/g), as prepared in a manner similar to Example 60, was reacted with (S)-(+)-2-methoxymethyl pyrrolidine (0.5M, 58 mg) and O-(7-azabenzotriazol-1-yl)-1,1,3,3-tetramethyluronium hexafluorophosphate) HATU (0.5M, 190 mg), 1-hydroxy-7-azabenzotriazole (HOAt) (0.5M; 68 mg) and diisopropylethylamine (0.233 mmol, 40 µL) in 1 mL of anhydrous DMF in a manner similar to Example 63 to afford 16 mg (35% yield) of 4-[4-{3-{2-[(3R)-3-(methoxymethyl)pyrrolidinyl]-2-oxoethoxy}phenyl)(1,3-thiazol-2-yl)]-5-methylthiothiophene-2-carboxamidine trifluoroacetate. Mass Spectrum (ESI, m/z) Calcd. for C₂₃H₂₆N₄O₃S₃: 503.2 (M+H), found 503.3.

### Example 77

### Synthesis of 1-(2-{3-[2-(5-amidino-2-methylthio-3-thienyl)-1,3-thiazol-4-yl]phenoxy}acetyl)piperidine-3-carboxamide trifluoroacetate

100 mg (0.093 mmol) of resin-bound 2-{3-[2-(5-amidino-2-methylthio-3-thienyl)-1,3-thiazol-4-yl]phenoxy}acetic acid (0.93 mmol/g), as prepared in a manner similar to Example 60, was reacted with nipecotamide (0.5M, 64 mg) and O-(7-azabenzotriazol-1-yl)-1,1,3,3-tetramethyluronium hexafluorophosphate) HATU (0.5M, 190 mg), 1-hydroxy-7-azabenzotriazole (HOAt) (0.5M; 68 mg) and diisopropylethylamine (0.233 mmol, 40 µL) in 1 mL of anhydrous DMF in a manner similar to Example 63 to afford 11 mg (23% yield) 1-(2-{3-[2-(5-amidino-2-methylthio-3-thienyl)-1,3-thiazol-4-yl]phenoxy}acetyl)piperidine-3-carboxamide trifluoroacetate. Mass Spectrum (ESI, m/z) Calcd. for C₂₃H₂₅N₄O₃S₃: 516.2 (M+H), found 516.3.

### Example 78

### Synthesis of 5-methylthio-4-{4-[3-(trifluoromethoxy)phenyl](1,3-thiazol-2-yl)}thiophene-2-carboxamidine hydrochloride

***a) Synthesis of methyl 5-methylthio-4-{4-[3-(trifluoromethoxy)phenyl](1,3-thiazol-2-yl)}thiophene-2-carboxylate:*** 435 mg (1.76 mmol) of methyl 4-(aminothioxomethyl)-5-methylthiothiophene-2-carboxylate was dissolved in 10 mL of reagent grade acetone. 2-bromo-3'-trifluoromethoxy acetophenone, prepared in a manner similar to Example 95, step (a), (1.76 mmol; 497 mg) was added and the solution was allowed to reflux for 3 h. The solution was allowed to cool and concentrated to an oil which was then dissolved in 150 mL of methylene chloride and washed with 50 mL of 10% HCl (aq.) and 50 mL of 2N NaOH (aq.). The organic layer was obtained and dried over magnesium sulfate and concentrated affording 877 mg (90% yield) of a methyl- 5-methylthio-4-{4-[3-(trifluoromethoxy)phenyl](1,3-thiazol-2-yl)}thiophene-2-carboxylate.

***b) Synthesis of 5-methylthio-4-{4-[3-(trifluoromethoxy)phenyl](1,3-thiazol-2-yl)}thiophene-2-carboxamidine hydrochloride:*** To a stirred suspension of 19.4 mmol ( 1.04 g) of ammonium chloride (Fisher Scientific) in 20 mL of anhydrous toluene (Aldrich Chemical Co.) placed under nitrogen atmosphere at 0°C, 9.7 mL ( 19.4 mmol) of 2M trimethylaluminum in toluene (Aldrich Chemical Co.) was added via syringe over 15 min and then let stir at 0°C for 30 min after which 837 mg (1.94 mmol) of methyl- 5-methylthio-4-{4-[3-(trifluoromethoxy)phenyl](1,3-thiazol-2-yl)}thiophene-2-carboxylate was added to solution and allowed to reflux for 3 h. The reaction mixture was quenched by pouring over a slurry of 10 g of silica in 50 mL of chloroform. The silica was poured onto a sintered glass funnel and washed with ethyl acetate and eluting with a 15% methanol/CH₂Cl₂ solution and concentrated. The crude product was purified on 1 mm silica prep plates eluting with 15%. methanol/CH₂Cl₂ and treated with 4N HCl/dioxane to afford 37 mg (5% yield) of 5-methylthio-4-{4-[3-(trifluoromethoxy)phenyl](1,3-thiazol-2-yl)}thiophene-2-carboxamidine hydrochloride. ¹H-NMR (DMSO-d₆; 300 MHz) δ 9.43 (bs, 1.9 H), 9.05 (bs, 1.9 H), 8.67 (s, 1H), 8.43 (s, 1H), 8.05-8.14 (m, 2H), 7.62-7.67 (t, 1H), 7.38-7.42 (m, 1H), 2.8 (s, 3H). Mass Spectrum (LCQ-ESI, m/z) Calcd. for C₁₆H₁₂F₃N₃OS₃: 415.5(M+H), found 416.2.

### Example 79

### 5-Methylthio-4-(5-phenyl(1,3-oxazol-2-yl))thiophene-2-carboxamidine hydrochloride

***a) Methyl 5-methylthio-4-[N-(2-oxo-2-phenylethyl)carbamoyl]thiophene-2-carboxylate:*** To a stirred suspension of 300 mg (1.29 mmol) of 5-(methoxycarbonyl)-2-methylthiothiophene-3-carboxylic acid (as prepared in Example 95) in 10 mL of anhyd CH₂Cl₂ (under a CaSO₄ drying tube) was added 135 µL (1.55 mmol) of oxalyl chloride followed by 30 µL of anhyd DMF. After stirring for 2 h at room temperature, the mixture was concentrated *in vacuo.* The resulting yellow solid was dissolved in 10 mL of anhyd CH₂Cl₂, cooled (0°C) and 266 mg (1.55 mmol) of 2-aminoacetophenone was added. *N,N*-diisopropylethylamine (DIEA) (756 µL, 4.34 mmol) was added dropwise over 3 min and the mixture stirred for 1 h at room temperature. The mixture was concentrated to an oil and partitioned between 125 mL of EtOAc and 80 mL of 1 M HCl. The aqueous layer was extracted with ethyl acetate (2 x 30 mL) and the combined organic phases were washed with 1 M HCl (60 mL), saturated NaHCO₃ (120 mL), and brine (120 mL) and dried over Na₂SO₄. After removing the solvent *in vacuo*, the residue was recrystallized from MeOH to afford the title compound as a cream-colored powder (314 mg, 70%). ¹H-NMR (300 MHz, DMSO-d₆) δ 8.82 (t, 1H, J = 6 Hz), 8.43 (s, 1H), 8.02 (d, 2H, J = 7 Hz), 7.69 (t, 1H, J = 7 Hz), 7.57 (t, 2H, J = 7 Hz), 4.72 (d, 2H, J = 6 Hz), 3.84 (s, 3H) and 2.57 (s, 3H). Mass spectrum (MALDI-TOF, α-cyano-4-hydroxycinnamic acid matrix) calcd. for C₁₆H₁₅NO₄S₂: 372.0 (M + Na). Found: 372.1.

***b) Methyl 5-methylthio-4-(5-phenyl(1,3-oxazol-2-yl))thiophene-2-carboxylate:*** To a cooled (0°C) solution of 80.1 mg (0.229 mmol) of methyl 5-methylthio-4-[N-(2-oxo-2-phenylethyl)carbamoyl]thiophene-2-carboxylate (as prepared in the previous step) in 2 mL of anhyd DMF was added 26.7 µL (0.286 mmol) of phosphorus oxychloride. After stirring for 20 h at room temperature, the mixture was concentrated *in vacuo.* The resulting yellow solid was recrystallized twice from MeOH to afford the title compound as a beige powder (48.8 mg, 64 %). ¹H-NMR (300 MHz, DMSO-d₆) δ 8.26 (s, 1H), 7.88 (s, 1H), 7.86 (d, 2H, J = 7 Hz), 7.51 (m, 2H), 7.40 (m, 1H), 3.86 (s, 3H), and 2.79 (s, 3H). Mass spectrum (MALDI-TOF, α-cyano-4-hydroxycinnamic acid matrix) calcd. for C₁₆H₁₃NO₃S₂: 332.0 (M + H). Found: 331.9.

***c) 5-Methylthio-4-(5-phenyl(1,3-oxazol-2-yl))thiophene-2-carboxamidine hydrochloride:*** Methyl 5-methylthio-4-(5-phenyl(1,3-oxazol-2-yl))thiophene-2-carboxylate (37.0 mg, 0.112 mmol, as prepared in the previous step) was treated according to the procedure in Example 10, step (b) using 59.9 mg (1.12 mmol) of ammonium chloride in 0.50 mL of toluene and 0.560 mL (1.12 mmol) of 2 M trimethylaluminum in toluene. The resulting residue was chromatographed on a 5 g silica SPE column (Waters Sep-Pak) with 10% MeOH-CH₂Cl₂ to elute an impurity followed by 20% MeOH-CH₂Cl₂ to give 39 mg of a light yellow glass. Crystallization from MeOH-MeCN afforded the title compound as a cream-colored solid (33.4 mg, 85 %). ¹H-NMR (300 MHz. DMSO-d₆) δ 9.45 (broad s, 2H), 9.13 (broad s, 2H), 8.72 (s, 1H), 7.93 (s, 1H), 7.84 (d, 2H, J = 7 Hz), 7.53 (t, 2H, J = 7 Hz), 7.42 (t, 1H, J = 7 Hz), and 2.80 (s, 3H). Mass spectrum (MALDI-TOF, α-cyano-4-hydroxycinnamic acid matrix) calcd. for C₁₅H₁₃N₃OS₂: 316.1 (M + H). Found: 316.5.

### Examples 80 and 81

### 5-Methylthio-4-(4-phenylimidazol-2-yl)thiophene-2-carboxamidine trifluoroacetate and 5-Methylthio-4-[N-(2-oxo-2-phenylethyl)carbamoyl]thiophene-2-carboxamidine trifluoroacetate

Methyl 5-methylthio-4-[N-(2-oxo-2-phenylethyl)carbamoyl]thiophene-2-carboxylate (39.4 mg, 0.100 mmol, as prepared in Example 79, step (a)) was treated according to the procedure in Example 10, step (b) using 64.2 mg (1.20 mmol) of ammonium chloride in 0.2 mL of toluene and 0.600 mL (1.20 mmol) of 2 M trimethylaluminum in toluene. The resulting residue was chromatographed on a 5 g silica SPE column (Waters Sep-Pak) with a gradient of 5-20% MeOH-CH₂Cl₂ to elute an impurity followed by 20% MeOH-CH₂Cl₂ to give a yellow resin. Crystallization from MeOH-Et₂O-MeCN afforded 16 mg of a yellow solid consisting of two products by ¹H-NMR spectra. A portion of the mixture (11 mg) was purified by reverse-phase HPLC (5µ C₈ column, 4.6 x 100 mm, gradient 5-100% solvent B over 15 min, solvent A = 0.1% TFA/H₂O, solvent B = 0.1%TFA/MeCN, detection at 215 nm) to afford 6 mg of 5-methylthio-4-(4-phenylimidazol-2-yl)thiophene-2-carboxamidine trifluoroacetate as a colorless glass. ¹H-NMR (300 MHz, CD₃OD) δ 8.23 (s, 1H), 7.80 (s, 1H), 7.79 (d, 2H, J = 7 Hz), 7.48 (m, 2H), 7.39 (m, 1H), and 2.78 (s, 3H). Mass spectrum (electrospray ionization) calcd. for C₁₅H₁₄N₄S₂: 315.1 (M + H). Found: 315.3. Also isolated was 4 mg of 5-methylthio-4-[N-(2-oxo-2-phenylethyl)carbamoyl]-thiophene-2-carboxamidine trifluoroacetate as a colorless glass. ¹H-NMR (300 MHz, DMSO-d₆) δ 9.30 (broad s, 2H), 8.86 (broad s, 2H), 8.68 (t, 1H, J = 5.4 Hz), 8.43 (s, 1H), 8.04 (d, 2H, J = 7 Hz), 7.70 (t, 1H, J = 7 Hz), 7.58 (t, 2H, J = 7 Hz), 4.78 (d, 2H, J = 5.4 Hz), and 2.63 (s, 3H). Mass spectrum (electrospray ionization) calcd. for C₁₅H₁₅N₃O₂S₂: 334.1 (M + H). Found: 334.3.

### Example 82

### 4-(4-Phenyl-1,3-thiazol-2-yl)thiophehe-2-carboxamidine hydrochloride

***a) 4-Bromothiophene-2-carboxylic acid:*** To a cooled (0°C) solution of 10.0 g (47.1 mmol based on 90 % purity) of 4-bromothiophene-2-carbaldehyde (Aldrich Chemical Company, Milwaukee, WI) in 200 mL of t-butanol was added 100 mL of 20 % (w/v) NaH₂PO₄ followed by 60 mL (0.566 mol) of 2-methyl-2-butene. Sodium chlorite (70.8 mmol based on 80 % purity) in 60 mL of water was added with stirring. After stirring the two-phase mixture vigorously for 16 h at room temperature, the pH of the aqueous layer was adjusted to 1-2 with 20 % HCl. The layers were separated and the aqueous layer extracted with EtOAc (2 x 120 mL). The combined organic layers were dried (Na₂SO₄) and concentrated *in vacuo* to afford 9.8 g of an off-white solid. Recrystallization from a minimum of MeCN (three crops) gave the title compound as a white solid (9.02 g, 93%). ¹H-NMR (300 MHz, CDCl₃) δ 7.79 (d, 1H, J = 1.5 Hz), and 7.55 (d, 1H, J = 1.5 Hz).

***b) Methyl 4-bromothiophene-2-carboxylate*:** To a cooled (-20°C) solution of 6.02 g (29.1 mmol) of 4-bromothiophene-2-carboxylic acid (as prepared in the previous step) in 100 mL of anhyd MeOH under nitrogen was added 2.55 mL (34.9 mmol) of thionyl chloride dropwise at a rate to keep the temperature below -5°C (ca. 8-10 min). After stirring for 1 h at room temperature, the mixture was refluxed for 8 h, cooled, and concentrated *in vacuo.* The resulting 6.7 g of pale amber oil was passed through a 150 g pad of silica gel with ca. 600 mL of CH₂Cl₂ (discarding the first 120 mL which contained a minor impurity) to afford, after concentration *in vacuo,* the title compound as a colorless oil (6.11 g, 95 %). ¹H-NMR (300 MHz, CDCl₃) δ 7.69 (d, 1H, J = 1.5 Hz), 7.45 (d, 1H, J = 1.5 Hz), and 3.90 (s, 3H).

***c) Methyl 4-cyanothiophene-2-carboxylate:*** To a solution of 3.82 g (17.3 mmol) methyl 4-bromothiophene-2-carboxylate (as prepared in the previous step) in 10 mL of anhyd DMF was added 3.10 g (34.6 mmol) of copper (I) cyanide. The mixture was heated to reflux with stirring for 18 h, cooled and poured into 100 mL of 10 % (w/v) KCN. The mixture was extracted with EtOAc (3 x 60 mL) and the combined extracts were washed with 150 mL each of water and brine. The dark solution was dried over Na₂SO₄, treated with decolorizing carbon, filtered and the resulting colorless solution concentrated *in vacuo*. The resulting light yellow solid was recrystallized from MeOH to afford the title compound as a cream-colored solid (1.67 g, 58 %). ¹H-NMR (300 MHz, CDCl₃) δ 8.09 (d, 1H, J = 1.4 Hz), 7.93 (d, 1H, J = 1.4 Hz), and 3.93 (s, 3H). IR (film): 2235 and 1712 cm⁻¹.

***d) Methyl 4-(aminothioxomethyl)thiophene-2-carboxylate:*** A solution of 1.32 g (7.89 mmol) of methyl 4-cyanothiophene-2-carboxylate (as prepared in the previous step) in 200 mL of reagent grade MeOH was degassed with nitrogen through a fritted gas dispersion tube for 10 min. Triethylamine (5.50 mL, 39.5 mmol) was added and hydrogen sulfide gas was bubbled into the solution at a vigorous rate for 5 min and then at a minimal rate (as measured through an outlet oil bubbler) for 5 h with stirring. The gas introduction was stopped and the mixture was capped and stirred for 19 h at room temperature. The mixture was concentrated *in vacuo* to a yellow solid which was suspended in 10 mL of EtOH, cooled to -20°C, and filtered washing with 5 mL of cold (-20°C) EtOH. The resulting solid was dried under suction followed by high vacuum to afford the title compound as a beige solid (1.31 g, 82 %). ¹H-NMR (300 MHz, DMSO-d₆) δ 9.85 (broad s, 1H), 9.51 (broad s, 1H), 8.50 (d, 1H, J = 1.5 Hz), 8.28 (d, 1H, J = 1.5 Hz), and 3.84 (s, 3H).

***e) Methyl 4-(4-phenyl-1,3-thiazol-2-yl)thiophene-2-carboxylate*:** To a solution of 150 mg (0.745 mmol) of methyl 4-(aminothioxomethyl)-thiophene-2-carboxylate (as prepared in the previous step) in 6 mL of acetone was added 148 mg (0.745 mmol) of 2-bromoacetophenone. After refluxing for 2 h, the mixture was concentrated by boiling to a volume of ca. 2 mL. The resulting mixture was cooled (-10°C) and filtered washing with cold acetone (2 x 0.5 mL). A second crop was obtained from the mother liquors and the combined crops dried to afford the title compound as a beige solid (202 mg, 90 %). ¹H-NMR (300 MHz, DMSO-d₆) δ 8.56 (d, 1H, J = 1.5 Hz), 8.25 (d, 1H, J = 1.5 Hz), 8.18 (s, 1H), 8.04 (d, 2H, J = 7 Hz), 7.48 (t, 2H, J = 7 Hz), 7.38 (t, 1H, J = 7 Hz), and 3.89 (s, 3H). Mass spectrum (MALDI-TOF, a-cyano-4-hydroxycinnamic acid matrix) calcd. for C₁₅H₁₁NO₂S₂: 302.0 (M + H). Found: 301.8.

***f) 4-(4-Phenyl-1,3-thiazol-2-yl)thiophene-2-carboxamidine hydrochloride:*** Methyl 4-(4-phenyl-1,3-thiazol-2-yl)thiophene-2-carboxylate (160 mg, 0.531 mmol, as prepared in the previous step) was treated according to the procedure in Example 10, step (b) using 284 mg (5.31 mmol) of ammonium chloride in 2.6 mL of toluene and 2.65 mL (5.30 mmol) of 2 M trimethylaluminum in toluene. The resulting light yellow solid was chromatographed on a 10 g silica SPE column (Waters Sep-Pak) with a gradient of 5-20% MeOH-CH₂Cl₂. The resulting pale amber glass was triturated with CH₂Cl₂-MeCN and concentrated *in vacuo* to afford the title compound as a beige solid (68 mg, 45 %). ¹H-NMR (300 MHz, DMSO-d₆) δ 9.51 (broad s, 2H), 9.09 (broad s, 2H), 8.71 (d, 1H, J = 1.5 Hz), 8.61 (d, 1H, J = 1.5 Hz), 8.21 (s, 1H), 8.05 (d, 2H, J = 7 Hz), 7.50 (t, 2H, J = 7 Hz), and 7.40 (t, 1H, J = 7 Hz). Mass spectrum (MALDI-TOF, α-cyano-4-hydroxycinnamic acid matrix) calcd. for C₁₄H₁₁N₃S₂: 286.0 (M + H). Found: 286.3.

### Example 83

### 5-Methylthio-4-[4-benzyl(1,3-thiazol-2-yl)]thiophene-2-carboxamidine hydrochloride

***a) Bromo-3-phenylacetone:*** To a solution of 132 µL (1.00 mmol) of phenylacetyl chloride in 1.0 mL of anhyd MeCN was added 1.05 mL (2.10 mmol) of a 2 M solution of trimethylsilyldiazomethane in hexane. After stirring 1 h at room temperature, the mixture was cooled (0°C) and 300 µL (1.50 mmol) of 30 wt % HBr in acetic acid was added dropwise (gas evolution). After stirring 15 min, the mixture was concentrated *in vacuo* and rapidly chromatographed on a 2 g silica SPE column (Waters Sep-Pak) with 50 % CH₂Cl₂-hexane to afford the title compound as a pale yellow oil (201 mg, 94 %). ¹H-NMR (300 MHz, CDCl₃) δ 7.2-7.4 (m, 5H), 3.95 (s, 2H), 3.92 (s, 2H).

***b) Methyl 5-methylthio-4-[4-benzyl(1,3-thiazol-2-yl)]thiophene-2-carboxylate*:** Using a procedure similar to that of Example 10 with 171 mg (0.690 mmol) of methyl 4-(aminothioxomethyl)-5-methylthiothiophene-2-carboxylate (as prepared in Example 82, step (e)) in 4 mL of acetone and 147 mg (0.690 mmol) of 1-bromo-3-phenylacetone (as prepared in the previous step) afforded the title compound as a light tan powder (236 mg, 95 %). ¹H-NMR (300 MHz, DMSO -d₆) δ 8.11 (s, 1H), 7.2-7.4 (m, 5H), 4.11 (s, 2H), 3.84 (s, 3H), and 2.72 (s, 3H). Mass spectrum (MALDI-TOF, α-cyano-4-hydroxycinnamic acid matrix) calcd. for C₁₇H₁₅NO₂S₃: 362.0 (M + H). Found: 362.3.

***c) 5-Methylthio-4-[4-benzyl(1,3-thiazol-2-yl)]thiophene-2-carboxamidine hydrochloride:*** Methyl 5-methylthio-4-[4-benzyl(1,3-thiazol-2-yl)]thiophene-2-carboxylate (60 mg, 0.166 mmol, as prepared in the previous step) was treated according to the procedure in Example 10, step (b) using 88.8 mg (1.66 mmol) of ammonium chloride in 0.5 mL of toluene and 0.830 mL (5.30 mmol) of 2 M trimethylaluminum in toluene to afford, after trituration from MeOH with Et₂O, the title compound as a yellow solid (38.2 mg, 60 %). ¹H-NMR (300 MHz, CD₃OD) δ 8.43 (s, 1H), 7.16-7.33 (m, 5H), 4.15 (s, 2H), and 2.75 (s, 3H). Mass spectrum (MALDI-TOF, α-cyano-4-hydroxycinnamic acid matrix) calcd. for C₁₆H₁₅N₃S₃: 346.0 (M + H). Found: 346.0.

### Example 84

### 5-Methylthio-4-(4-phenyl(1,3-oxazol-2-yl))thiophene-2-carboxamidine hydrochloride HCl

***a) Methyl 4-[N-(2-hydroxy-1-phenylethyl)carbamoyl]-5-methylthiothiophene-2-carboxylate*:** To a stirred suspension of 1.23 g (5.29 mmol) of 5-(methoxycarbonyl)-2-methylthiothiophene-3-carboxylic acid (as prepared in Example 79, step (a)) in 20 mL of anhyd CH₂Cl₂ (under a CaSO₄ drying tube) was added 1.85 mL (21.2 mmol) of oxalyl chloride followed by 30 µL of anhyd DMF. After stirring for 2 h at room temperature, the mixture was concentrated *in vacuo.* The resulting yellow solid was dissolved in 20 mL of anhyd CH₂Cl₂, cooled (0°C) and 1.85 mL of *N,N*-diisopropylethylamine (10.6 mmol) and 1.02 g (7.41 mmol) of phenylglycinol was added and the mixture stirred for 1 h at room temperature. The mixture was concentrated to an oil and partitioned between 200 mL of EtOAc and 200 mL of saturated NaHCO₃. The organic phase was washed with saturated NaHCO₃ (200 mL), 10 % (w/v) citric acid, and brine (200 mL), and dried over Na₂SO₄. After removing the solvent *in vacuo,* the residue was chromatographed on a 10 g silica SPE column (Waters Sep-Pak) with a gradient of 0-20 % EtOAc-CH₂Cl₂ to afford the title compound as a light yellow solid (1.26 g, 68 %). ¹H-NMR (300 MHz, CDCl₃) δ 8.00 (s, 1H), 7.30-7.42 (m, 5H), 7.08 (d, 1H, J = 7.2 Hz), 5.26 (m, 1H), 3.99 (t, 2H, J = 5.4 Hz), 3.89 (s, 3H), 2.60 (s, 3H), and 2.33 (t, 1H J = 6.1 Hz). Mass spectrum (electrospray ionization) calcd. for C₁₆H₁₇NO₄S₂: 352.1 (M + H). Found: 352.0.

***b) Methyl 5-methylthio-4-[N-(2-oxo-1-phenylethyl)carbamoyl]thiophene-2-carboxylate*:** To a solution of 505 mg (1.44 mmol) methyl 4-[N-(2-hydroxy-1-phenylethyl)carbamoyl]-5-methylthiothiophene-2-carboxylate (as prepared in the previous step) in 20 mL of anhydrous CH₂Cl₂ was added 856 mg (2.02 mmol) of Dess Martin reagent (Omega Chemical Company, Inc., Levis (Qc) Canada). After stirring in an open flask for 1.5 h at room temperature, the mixture was concentrated *in vacuo.* to ca. 10 % volume and partitioned between 50 mL of EtOAc and 50 mL of saturated NaHCO₃-brine (1:1). The organic phase were washed with brine (200 mL), dried over Na₂SO₄ and concentrated *in vacuo.* Concentrated again from CH₂Cl₂ followed by high vacuum afforded the title compound as a light yellow foam (495 mg, 98 %) which was used in the next step without further purification. ¹H-NMR (300 MHz, CDCl₃) δ 9.64 (s, 1H), 8.04 (s, 1H), 7.59 (d, 1H, J = 5 Hz), 7.36-7.46 (m, 5H), 5.76 (d, 1H, J = 5 Hz), 3.90 (s, 3H), and 2.62 (s, 3H).

***c) Methyl 5-methylthio-4-(4-phenyl(1,3-oxazol-2-yl))thiophene-2-carboxylate*:** To a cooled (0°C) solution of 465 mg (1.33 mmol) methyl 5-methylthio-4-[N-(2-oxo-1-phenylethyl)carbamoyl]thiophene-2-carboxylate (as prepared in the previous step) in 6 mL of anhyd DMF was added 186 µL (2.00 mmol) of phosphorus oxychloride. After stirring for 14 h at room temperature, the mixture was treated with 10 mL of saturated NaHCO₃ and concentrated to dryness under high vacuum. The resulting residue was partitioned between 80 mL of EtOAc and 60 mL of water. The aqueous layer was extracted with EtOAc (2 x 10 mL) and the combined organic phases washed with brine (60 mL), and dried over Na₂SO₄. The resulting 406 mg of amber-colored solid was recrystallized from CH₂Cl₂-Et₂O to remove the majority of a polar impurity as a cream-colored solid. The remaining mother liquors were chromatographed on a 10 g silica SPE column (Waters Sep-Pak) with a gradient of 40-100 % CH₂Cl₂-hexane and the resulting residue triturated with Et₂O-hexane (2:1)) to afford the title compound as a light beige solid (114 mg, 26 %). ¹H-NMR (300 MHz, CDCl₃) δ 8.24 (s, 1H), 7.93 (s, 1H), 7.83 (m, 2H), 7.43 (m, 2H), 7.33 (m, 1H), 3.91 (s, 3H), and 2.72 (s, 3H). Mass spectrum (ESI) calcd. for C₁₆H₁₃NO₃S₂: 332.0 (M + H). Found: 332.2.

***d) 5-Methylthio-4-(4-phenyl(1,3-oxazol-2-yl))thiophene-2-carboxamidine hydrochloride:*** Methyl 5-methylthio-4-(4-phenyl(1,3-oxazol-2-yl))thiophene-2-carboxylate (80.3 mg, 0.242 mmol, as prepared in the previous step) was treated according to the procedure in Example 10, step (b) using 155 mg (2.90 mmol) of ammonium chloride in 1.45 mL of toluene and 1.45 mL (2.90 mmol) of 2 M trimethylaluminum in toluene. The resulting light yellow solid was chromatographed on a 5 g silica SPE column (Waters Sep-Pak) with 10% MeOH-CH₂Cl₂ to give a light yellow resin. Crystallization from MeOH- Et₂O (ca. 1:3) afforded the title compound as a yellow solid (62.2 mg, 82 %). ¹H-NMR (300 MHz, DMSO-d₆) δ 9.39 (broad s, 2H), 8.97 (broad s, 2H), 8.78 (s, 1H), 8.60 (s, 1H), 7.89 (d, 2H, J = 7 Hz), 7.49 (t, 2H, J = 7 Hz), 7.38 (t, 1H, J = 7 Hz), and 2.80 (s, 3H). Mass spectrum (ESI) calcd. for C₁₅H₁₃N₃OS₂: 316.1 (M+H). Found: 316.2.

### Example 85

### 4-[4-(4-hydroxy-3-methoxyphenyl)(1,3-thiazol-2-yl)]-5-methylthiothiophene-2-carboxamidine hydrochloride

***a) 4-(Chlorocarbonyl)-2-methoxyphenyl acetate:*** To a stirred suspension of 1.00 g (4.76 mmol) of 4-acetoxy-3-methoxybenzoic acid (Pfaltz and Bauer, Inc.) in 4 mL of anhyd CH₂Cl₂ (under a CaSO₄ drying tube) was added 4.15 mL (47.6 mmol) of oxalyl chloride followed by 25 µL of anhyd DMF. After stirring for 4 h at room temperature, the mixture was concentrated *in vacuo* to afford the title compound as light yellow crystals (1.12 g, 103%). ¹H-NMR (300 MHz, CDCl₃) δ 7.81 (dd, 1H, J = 8.4, 2.1 Hz), 7.66 (d, 1H, 2.1 Hz), 7.19 (d, 1H, 8.4 Hz), 3.91 (s, 3H), and 2.35 (s, 3H).

***b) 4-(2-Bromoacetyl)-2-methoxyphenyl acetate*:** To a solution of 1.09 g (4.6 mmol) of 4-(chlorocarbonyl)-2-methoxyphenyl acetate (as prepared in the precious step) in 10 mL of anhyd CH₂Cl₂ was added 10.0 mL (20.0 mmol) of a 2 M solution of trimethylsilyldiazomethane in hexane. After stirring 2 h at room temperature, the mixture was cooled (0°C) and 3.20 mL (16.0 mmol) of 30 wt % HBr in acetic acid was added dropwise (gas evolution). After stirring 5 min, the mixture was concentrated *in vacuo* and rapidly chromatographed on a 10 g silica SPE column (Waters Sep-Pak) with CH₂Cl₂ to afford the title compound as a light yellow crystalline solid (1.28 g, 97 %). ¹H-NMR (300 MHz, CDCl₃) δ 7.63 (d, 1H, 1.9 Hz), 7.59 (dd, 1H, J = 8.2, 1.9 Hz), 7.16 (d, 1H, 8.2 Hz), 4.43 (s, 2H), 3.91 (s, 3H), and 2.35 (s, 3H).

***c) 2-Methoxy-4-{2-[5-(methoxycarbonyl)-2-methylthio(3-thienyl)](1,3-thiazol-4-yl)}phenyl acetate:*** Using a procedure similar to that of Example 82, step (e) with 1.00 g (4.04 mmol) of methyl 4-(aminothioxomethyl)-5-methylthiothiophene-2-carboxylate (Maybridge Chemical Company, Cornwall, UK) in 15 mL of reagent acetone and 1.16 g (4.04 mmol) of 4-(2-bromoacetyl)-2-methoxyphenyl acetate (as prepared in the previous step) afforded the title compound as 1.42 g of a yellow solid which, according to the ¹H-NMR spectrum, consisted of a ca. 1:1 mixture of the title compound and the corresponding compound resulting from partial loss of the acetate. ¹H-NMR (300 MHz, DMSO -d₆) δ 8.27 (s, 1H), 8.22 (s, 1H), 8.19 (s, 1H), 8.00 (s, 1H), 7.78 (d, 1H, 1.9 Hz), 7.67 (dd, 1H, J = 8.2, 1.9 Hz), 7.61 (d, 1H, 1.9 Hz), 7.51 (dd, 1H, J = 8.2, 1.9 Hz), 7.19 (d, 1H, 8.2 Hz), 6.86 (d, 1H, 8.2 Hz), 8.87 (m, 12H), 2.76 (s, 3H), 2.75 (s, 3H), and 2.28 (s, 3H). Mass spectrum (ESI) calcd. for C₁₉H₁₇NO₅S₃ and C₁₇H₁₅NO₃S₃ 436.0 (M + H) and 394.1 (M + H). Found: 436.1 and 394.2. The mixture was used without further purification in the following step where formation of the amidine involves concomitant removal of the acetate.

***d) 4-[4-(4-hydroxy-3-methoxyphenyl)(1,3-thiazol-2-yl)]-5-methylthiothiophene-2-carboxamidine hydrochloride:*** A portion of the mixture (500 mg, ca. 1.21 mmol as based on the ¹H-NMR integration) containing the 2-methoxy-4-{2-[5-(methoxycarbonyl)-2-methylthio(3-thienyl)](1,3-thiazol-4-yl)}phenyl acetate (as prepared in the previous step) was treated according to the procedure in Example 10, step (b) using 610 mg (11.4 mmol) of ammonium chloride in 5.7 mL of toluene and 5.70 mL (11.4 mmol) of 2 M trimethylaluminum in toluene. After chromatography of the resulting residue on a 10 g silica SPE column (Waters Sep-Pak) with a gradient of 5-20 % MeOH-CH₂Cl₂ to obtain a yellow glass which was recrystallized from MeOH-CH₂Cl₂ to afford the title compound as a pale yellow solid (192 mg, 42 %). ¹H-NMR (300 MHz, DMSO-d₆) δ 9.35 (broad s, 2H), 9.27 (s, 1H), 8.97 (broad s, 2H), 8.62 (s, 1H), 8.04 (s, 1H), 7.62 (s, 1H), 7.54 (d, 1H J = 8.2 Hz), 6.88 (d, 1H, J = 8.2 Hz), 3.87 (s, 3H), and 2.79 (s, 3H). Mass spectrum (ESI) calcd. for C₁₆H₁₅N₃O₂S₃: 378.0 (M + H). Found: 378.1.

### Example 86

### 4-[4-(3-Hydroxy-4-methoxyphenyl)(1,3-thiazol-2-yl)]-5-methylthiothiophene-2-carboxamidine hydrochloride

***a) 3-Acetyloxy-4-methoxybenzoic acid:*** To a suspension of 600 mg (3.57 mmol) of 3-hydroxy-4-methoxybenzoic acid (Aldrich Chemical Company, Milwaukee, WI) in 5 mL of anhyd CH₂Cl₂ was added 1.31 mL (7.50 mmol) of *N, N*-diisopropylethylamine and the mixture stirred until homogeneous (ca. 5 min). Acetyl chloride (305 µL, 4.28 mmol) was added dropwise over 2 min followed by 2.0 mg ((0.016 mmol) of 4-dimethylaminopyridine. After stirring at room temperature for 1 h, the mixture was poured into 50 mL of EtOAc and washed with 1 M HCl (3 x 25 mL). The organic phase was extracted with saturated NaHCO₃ (6 x 15 mL) and the combined extracts saturated with solid NaCl and acidified to pH 2 with cone HCl. The resulting suspension was extracted with EtOAc (3 x 20mL) and the combined extracts were dried over Na₂SO₄ and concentrated *in vacuo* to afford the title compound as a light beige powder (463 mg, 62 %). ¹H-NMR (300 MHz, CDCl₃) δ 8.00 (dd, 1H, J = 8.7, 2.0 Hz), 7.79 (d, 1H, 2.0 Hz), 7.00 (d, 1H, 8.7 Hz), 3.91 (s, 3H), and 2.34 (s, 3H).

***b) 3-(Chlorocarbonyl)-6-methoxyphenyl acetate:*** Using the procedure in Example 85, step (a), 400 mg (1.90 mmol) of 3-acetyloxy-4-methoxybenzoic acid (as prepared in the previous step) was treated with 663 µL (7.60 mmol) of oxalyl chloride and 25 µL of anhyd DMF for 2 h to afford, after workup, the title compound as a beige crystalline solid which was used in the following step without further purification.

***c) 5-(2-bromoacetyl)-2-methoxyphenyl acetate:*** Using the procedure in Example 85, step (b), the entire sample of 3-(chlorocarbonyl)-6-methoxyphenyl acetate (as prepared in the previous step) in 5 mL of anhyd CH₂Cl₂ was treated with 2.09 mL (4.18 mmol) of a 2 M solution of trimethylsilyldiazomethane in hexane and 456 µL (2.28 mmol) of 30 wt % HBr in acetic acid. Chromatography as in Example 85, step (b) followed by recrystallization from CH₂Cl₂-hexane afforded the title compound as a faintly yellow solid (366 mg, 67 %). ¹H-NMR (300 MHz, CDCl₃) δ 7.79 (dd, 1H, J = 8.6, 2.2 Hz), 7.70 (d, 1H, 2.2 Hz), 7.03 (d, 1H, 8.6 Hz), 4.38 (s, 2H), 3.92 (s, 3H), and 2.34 (s, 3H).

***d) 2-Methoxy-5-{2-[5-(methoxycarbonyl)-2-methylthio(3-thienyl)](1,3-thiazol-4-yl)}phenyl acetate:*** Using a procedure similar to that of Example 82, step (e) with 282 mg (1.14 mmol) of methyl 4-(aminothioxomethyl)-5-methylthiothiophene-2-carboxylate (Maybridge Chemical Company, Cornwall, UK) in 4 mL of acetone and 3.27 mg (1.14 mmol) of 5-(2-bromoacetyl)-2-methoxyphenyl acetate (as prepared in the previous step) afforded a yellow solid (374 mg) which, according to the ¹H-NMR spectrum, consisted of a 3:7 mixture of the title compound and the corresponding compound resulting from partial loss of the acetate. Mass spectrum (ESI) calcd. for C₁₉H₁₇NO₅S₃ and C₁₇H₁₅NO₃S₃ 436.0 (M + H) and 394.1 (M + H). Found: 436.0 and 394.0. The mixture was used without further purification in the following step where formation of the amidine involves concomitant removal of the acetate.

***e) 4-[4-(3-Hydroxy-4-methoxyphenyl)(1,3-thiazol-2-yl)]-5-methylthiothiophene-2-carboxamidine hydrochloride:*** A portion of the mixture (320 mg, ca. 0.788 mmol as based on the ¹H-NMR spectrum) containing the 2-methoxy-5-{2-[5-(methoxycarbonyl)-2-methylthio(3-thienyl)](1,3-thiazol-4-yl)}phenyl acetate (as prepared in the previous step) was treated according to the procedure in Example 10, step (b), using 415 mg (7.76 mmol) of ammonium chloride in 3.5 mL of toluene and 3.88 mL (7.66 mmol) of 2 M trimethylaluminum in toluene. After chromatography of the resulting residue on a 10 g silica SPE column (Waters Sep-Pak) with 10-40 % MeOH-CH₂Cl₂, a light yellow solid was obtained which was dissolved in 45 mL of DMF and filtered to remove silica gel. Concentration under high vacuum and recrystallization from MeOH-Et₂O afforded the title compound as a light tan solid (132 mg, 44 %). ¹H-NMR (300 MHz, DMSO-d₆) δ 9.49 (broad s, 2H), 9.16 (broad s, 2H), 8.67 (s, 1H), 7.98 (s, 1H), 7.5 (obscured m, 3H), 7.00 (obscured d, 1H, J = 8.3 Hz), 3.82 (s, 3H), and 2.79 (s, 3H). Mass spectrum (ESI) calcd. for C₁₆H₁₅N₃O₂S₃: 378.0 (M + H). Found: 378.1.

### Example 87

### 5-Methylthio-4-(N-phenylcarbamoyl)thiophene-2-carboxamidine hydrochloride

***a) Methyl 5-methylthio-4-(N-phenylcarbamoyl)thiophene-2-carboxylate:*** To 182 mg (0.785 mmol) of 5-(methoxycarbonyl)-2-methylthiothiophene-3-carboxylic acid (as prepared in Example 95) in 4 mL of anhyd CH₂Cl₂ was treated with 275 µL (3.15 mmol) of oxalyl chloride and 6 µL of anhyd DMF for 2 h similar to Example 79, step (a); followed by 206 µL (1.18 mmol) of *N,N*-diisopropylethylamine and 85.9 µL (0.942 mmol) of aniline in 3 mL of anhyd CH₂Cl₂ for 20 min. The mixture was poured into 25 mL of EtOAc and washed with 1 M HCl (2 x 25 mL), saturated NaHCO₃ (2 x 25 mL), and brine (25 mL), and dried over Na₂SO₄. Removal of the solvent *in vacuo*, afforded the pure title compound as a light yellow solid (163 mg, 68 %). ¹H-NMR (300 MHz, CDCl₃) δ 8.23 (broad s, 1H), 8.10 (s, 1H), 7.63 (d, 2H, J = 7 Hz), 7.36 (t, 2H, J = 7 Hz), 7.15 (t, 2H, J = 7 Hz), 3.90 (s, 3H), and 2.64 (s, 3H).

***b) 5-Methylthio-4-(N-phenylcarbamoyl)thiophene-2-carboxamidine hydrochloride:*** Methyl 5-methylthio-4-(N-phenylcarbamoyl)thiophene-2-carboxylate (60.0 mg, 0.195 mmol, as prepared in the previous step) was treated similarly to the procedure in Example 10, step (b) using 310 mg (5.80 mmol) of ammonium chloride in 2 mL of toluene and 2.90 mL (5.80 mmol) of 2 M trimethylaluminum in toluene for 6 h. Chromatography of the resulting residue on a 2 g silica SPE column (Waters Sep-Pak) with a gradient of 5-20 % MeOH-CH₂Cl₂, followed by crystallization from MeOH-Et₂O afforded the title compound as a beige solid (40.3 mg, 71 %). ¹H-NMR (300 MHz, DMSO-d₆) δ 10.24 (s, 1H), 9.34 (broad s, 2H), 9.05 (broad s, 2H), 8.75 (s, 1H), 7.73 (d, 2H, J = 8 Hz), 7.36 (t, 2H, J = 8 Hz), 7.11 (m, 1H), and 2.67 (s, 3H). Mass spectrum (ESI) calcd. for C₁₃H₁₃N₃OS₂: 292.1 (M + H). Found: 292.4.

### Example 88 and 89

### 5-Methylthio-4-[N-benzylcarbamoyl]thiophene-2-carboxamidinehydrochloride and 4-{Imino[benzylamino]methyl}-5-methylthiothiophene-2-carboxamidine hydrochloride

***a) Methyl 5-methylthio-4-[N-benzylcarbamoyl]thiophene-2-carboxylate:*** The identical procedure of Example 87, step (a) was used with 103 µL (0.942 mmol) of benzylamine and the same amounts of all other reagents to afford the title compound as a light yellow solid (167 mg, 66 %). ¹H-NMR (300 MHz, CDCl₃) δ 7.93 (s, 1H), 7.28-7.38 (m, 5H), 6.58 (broad s, 1H), 4.62 (s, 2H, J = 5.7 Hz), 3.87 (s, 3H), and 2.60 (s, 3H).

***b) 5-Methylthio-4-[N-benzylcarbamoyl]thiophene-2-carboxamidinehydrochloride and 4-{Imino[benzylamino]methyl}-5-methylthiothiophene-2-carboxamidinehydrochloride:*** Methyl 5-methylthio-4-[N-benzylcarbamoyl]thiophene-2-carboxylate (62.7 mg, 0.195 mmol, as prepared in the previous step) was treated similarly to the procedure in Example 10, step (b) using 310 mg (5.80 mmol) of ammonium chloride in 2 mL of toluene and 2.90 mL (5.80 mmol) of 2 M trimethylaluminum in toluene for 6 h.

Chromatography of the resulting residue on a 2 g silica SPE column (Waters Sep-Pak) with a gradient of 5-20 % MeOH-CH₂Cl₂, followed by crystallization from MeOH-Et₂O afforded 5-methylthio-4-[N-benzylcarbamoyl]thiophene-2-carboxamidinehydrochloride as a beige solid (21.1 mg, 35 %). ¹H-NMR (300 MHz, DMSO-d₆) δ 7.93 (s, 1H), 7.28-7.38 (m, 5H), 6.58 (broad s, 1H), 4.62 (s, 2H, J = 5.7 Hz), 3.87 (s, 3H), and 2.60 (s, 3H). Mass spectrum (ESI) calcd. for C₁₄H₁₅N₃OS₂: 306.1(M+H). Found: 306.6.

Also isolated and crystallized from MeOH-Et₂O was the more polar 4-{imino[benzylamino]methyl }-5-methylthiothiophene-2-carboxamidinehydrochloride as a beige solid (32.0 mg, 54 %). ¹H-NMR (300 MHz, DMSO-d₆) consistent with desired product as broad mixture of rotomers. Mass spectrum (ESI) calcd. for C₁₄H₁₆N₄S₂: 305.1 (M + H). Found: 305.8.

### Example 90 and 91

### 4-[N-Methyl-N-benzylcarbamoyl]-5-methylthiothiophene-2-carboxamidine hydrochloride and 4-{Imino[methylbenzylamino]methyl}-5-methylthiothiophene-2-carboxamidinehydrochloride

***a) Methyl 4-[N-methyl-N-benzylcarbamoyl]-5-methylthiothiophene-2-carboxylate:*** The identical procedure of Example 87, step (a) was used with 122 µL (0.942 mmol) of *N*-benzylmethylamine and the same amounts of all other reagents to afford the title compound as a light yellow solid (169 mg, 64 %). ¹H-NMR (300 MHz, CDCl₃) δ 7.68 (s, 1H), 7.34 (m, 5H), 4.6 (broad m, 2H), 3.86 (s, 3H), 2.91 (m, 3H), and 2.60 (s, 3H).

***b) 4-[N-Methyl-N-benzylcarbamoyl]-5-methylthiothiophene-2-carboxamidine hydrochloride and 4-{Imino[methylbenzylamino]methyl}-5-methylthiothiophene-2-carboxamidine hydrochloride:*** Methyl 4-[N-methyl-N-benzylcarbamoyl]-5-methylthiothiophene-2-carboxylate (65.4 mg, 0.195 mmol, as prepared in the previous step) was treated similarly to the procedure in Example 10, step (a) using 310 mg (5.80 mmol) of ammonium chloride in 2 mL of toluene and 2.90 mL (5.80 mmol) of 2 M trimethylaluminum in toluene for 6 h.

Chromatography of the resulting residue on a 2 g silica SPE column (Waters Sep-Pak) with a gradient of 5-20 % MeOH-CH₂Cl₂ afforded 4-[N-methyl-N-benzylcarbamoyl]-5-methylthiothiophene-2-carboxamidine hydrochloride as a amber-colored glass (34.3 mg, 55 %). ¹H-NMR (300 MHz, DMSO-d₆) δ 9.32 (broad s, 2H), 9.06 (broad s, 2H), 8.11 (s, 1H), 7.36 (m, 5H), 4.66 (m, 2H), 2.88 (s, 3H) and 2.66 (s, 3H). Mass spectrum (ESI) calcd. for C₁₅H₁₇N₃OS₂: 320.1(M + H). Found: 320.4.

Also isolated and then crystallized from MeOU-Et₂O was the more polar 4-{imino[methylbenzylamino]methyl}-5-methylthiothiophene-2-carboxamidine hydrochloride as a beige solid (19.8 mg, 32 %). ¹H-NMR (300 MHz, DMSO-d₆) consistent with desired product as broad mixture of rotomers. Mass spectrum (ESI) calcd. for C₁₅H₁₈N₄S₂: 319.1 (M + H). Found: 319.6.

### Example 92 and 93

### 5-Methylthio-4-[N-(2-phenylethyl)carbamoyl]thiophene-2-carboxamidine hydrochloride and 4-{Imino[(2-phenylethyl)amino]methyl}-5-methylthiothiophene-2-carboxamidine hydrochloride

***a) Methyl 5-methylthio-4-[N-(2-phenylethyl)carbamoyl]thiophene-2-carboxylate:*** The identical procedure of Example 87, step (a) was used with 118 µL (0.942 mmol) of phenethylamine and the same amounts of all other reagents to afford the title compound as a light yellow solid (165 mg, 63 %). ¹H-NMR (300 MHz, CDCl₃) δ 7.86 (s, 1H), 7.30-7.35 (m, 5H), 6.44 (m, 1H), 3.87 (s, 3H), 3.70 (q, 2H, J = 7 Hz), 2.93 (t, 2H, J = 7 Hz), and 2.53 (s, 3H).

***b) 5-Methylthio-4-[N-(2-phenylethyl)carbamoyl]thiophene-2-carboxamidine hydrochloride and 4-{Imino[(2-phenylethyl)amino]methyl}-5-methylthiothiophene-2-carboxamidine hydrochloride:*** Methyl 5-methylthio-4-[N-(2-phenylethyl)carbamoyl]thiophene-2-carboxylate (65.4 mg, 0.195 mmol, as prepared in the previous step) was treated similarly to the procedure in Example 10, step (a) using 310 mg (5.80 mmol) of ammonium chloride in 2 mL of toluene and 2.90 mL (5.80 mmol) of 2 M trimethylaluminum in toluene for 6 h.

Chromatography of the resulting residue on a 2 g silica SPE column (Waters Sep-Pak) with a gradient of 5-20 % MeOH-CH₂Cl₂, followed by crystallization from MeOH-Et₂O afforded 5-methylthio-4-[N-(2-phenylethyl)carbamoyl]thiophene-2-carboxamidine hydrochloride as a beige solid (17.4 mg, 28 %). ¹H-NMR (300 MHz, DMSO-d₆) δ 8.8-9.3 (broad m, 4H), 8.48 (m, 1H), 8.35 (s, 1H), 7.26 (m, 5H), 3.44 (m, 2H), 2.82 (t, 3H, J = 7.5 Hz), and 2.61 (s, 3H). Mass spectrum (ESI) calcd. for C₁₅H₁₇N₃OS₂: 320.1(M + H). Found: 320.4.

Also isolated and crystallized from MeOH-Et₂O was the more polar 4-{imino[(2-phenylethyl)amino]methyl}-5-methylthiothiophene-2-carboxamidine hydrochloride as a beige solid (19.1 mg, 31 %). ¹H-NMR (300 MHz, DMSO-d₆) δ 8.37 (s, 1H), 7.2-7.4 (m, 5H), 3.70 (t, 2H, J = 7.6 Hz), 2.96 (t, 2H, J = 7.6 Hz), and 2.71 (s, 3H). Mass spectrum (ESI) calcd. for C₁₅H₁₈N₄S₂: 319.1 (M + H). Found: 319.5.

### Example 94

### 3-Amino-2-aza-3-[5-methylthio-4-(4-phenyl(1,3-thiazol-2-yl))(2-thienyl)]prop-2-enenitrile

To 100 mg (0.302 mmol) of 5-methylthio-4-(4-phenyl(1,3-thiazol-2-yl))thiophene-2-carboxamidine (as prepared in Example 10, step b) in 3 mL of EtOH was added 29.6 mg (0.604 mmol) of cyanamide as a solution in 0.3 mL of water. The mixture was heated to reflux and 0.302 mL (0.302 mmol) of 1 M aqueous KOH was added. After 3 h, the mixture was cooled (0°C) and filtered washing with ice-cold EtOH. The resulting solid was dried *in vacuo* to afford the title compound as a light yellow powder (78.4 mg, 73 %). ¹H-NMR (300 MHz. DMSO-d₆) δ 9.31 (broad s, 1H), 8.70 (broad s, 1H), 8.63 (s, 1H), 8.19 (s, 1H), 8.09 (d, 2H, J = 7 Hz), 7.49 (t, 2H, J = 7 Hz), 7.39 (t, 1H, J = 7 Hz), and 2.75 (s, 3H). Mass spectrum (MALDI-TOF, α-cyano-4-hydroxycinnamic acid matrix) calcd. for C₁₆H₁₂N₄S₃: 357.0 (M + H). Found: 357.1.

### Example 95

### 5-(Methoxycarbonyl)-2-methylthiothiophene-3-carboxylic acid

Methyl 4-cyano-5-methylthiothiophene-2-carboxylate (2.20 g, 10.3 mmol, Maybridge Chemical Company, Cornwall, UK) and tetrafluorophthalic acid (2.45 g, 10.3 mmol) in an 8-mL sealable pressure tube (Ace Glass Company) with stir bar was heated to 160°C. The molten mixture was stirred for 4 days, cooled and the resulting residue broken up and extracted by refluxing with 80 mL chloroform. The mixture was cooled, decolorizing carbon (ca. 0.5 g) was added and the mixture filtered (Celite). The resulting solution was extracted with saturated NaHCO₃ (4 x 30 mL) and the combined aqueous extracts acidified to pH 1-2 with conc HCl and filtered to provide a light tan solid. After dissolving the solid in a minimum of 1 M K₂CO₃ (35-40 mL) and filtering (washing with 10-20mL of water) to clarify the solution, it was slowly acidified to pH 6.5-7.0 with stirring and filtered (Celite) to remove a brown precipitate. The pH adjustment and filtration was repeated and the resulting solution was saturated with solid NaCl and acidified to pH 1-2 with conc HCl. The precipitate was filtered, washed with water (3 x 10 mL) and dried over P₂O₅ under high vacuum to afford the title compound as a cream-colored powder (1.24 g, 52 %). ¹H-NMR (300 MHz, DMSO-d₆) δ 13.14 (broad s, 1H), 7.89 (s, 1H), 3.82 (s, 3H) and 2.64 (s, 3H). Mass spectrum (ESI, negative mode) calcd. for C₈H₈O₄S₂: 232.0 (M-). Found: 231.7.

### Example 96

### 5-Ethylthio-4-(4-phenyl(1,3-thiazol-2-yl))thiophene-2-carboxamidine hydrochloride

***a) Methyl 4-(4-phenyl(1,3-thiazol-2-yl))-5-(methylsulfonyl)thiophene-2-carboxylate:*** Using the procedure of Example 141, step (a) with 600 mg (1.73 mmol) of methyl 5-methylthio-4-(4-phenyl(1,3-thiazol-2-yl))thiophene-2-carboxylate as prepared in Example 10, step (a) afforded 642 mg (98 %) of the title compound as a light yellow powder. ¹H-NMR (300 MHz, CDCl₃) δ 7.93 (s, 1H), 7.90 (m, 2H), 7.63 (s, 1H), 7.47 (m, 2H), 7.39 (m, 1H), 3.98 (s, 3H) and 3.73 (s, 3H). Mass spectrum (ESI, m/z): calcd. for C₁₆H₁₃NO₄S₃ 380.0 (M+H), found 380.2.

***b) 4-(4-Phenyl)(1,3-thiazol-2-yl))-5-(methylsulfonyl)thiophene-2-carboxamidine hydrochloride:*** Using the procedure of Example 141, step (b) with 560 mg (1.48 mmol) methyl 4-[4-(4-chlorophenyl)(1,3-thiazol-2-yl)]-5-(methylsulfonyl)thiophene-2-carboxylate as prepared in the previous step afforded 392 mg (66 %) of the title compound as a off-white solid. ¹H-NMR (300 MHz, DMSO-d₆) δ 9.7 (broad s, 2H), 9.4 (broad s, 2H), 8.58 (s, 1H), 8.43 (s, 1H), 8.02 (d, 2H, J = 7 Hz), 7.52 (t, 2H, J = 7 Hz), 7.43 (t, 1H, J = 7 Hz), and 3.90 (s, 3H). Mass spectrum (ESI, m/z): calcd. for C₁₅H₁₃N₃O₂S₃ 364.0 (M+H), found 364.1.

***c) 5-Ethylthio-4-(4-phenyl(1,3-thiazol-2-yl))thiophene-2-carboxamidine hydrochloride:*** Using the procedure of Example 141, step (c) with 23.1 mg (0.0578 mmol) of the 4-(4-phenyl)(1,3-thiazol-2-yl))-5-(methylsulfonyl)thiophene-2-carboxamidine hydrochloride (as prepared in the previous step), 64.1 µL( 0.867 mmol) of ethanethiol (in 2 portions over 2 h) and 40.3 µL (0.231 mmol) of DIEA in 3 mL of methanol gave a yellow resin which was chromatographed on a 2 g silica SPE column (Waters Sep-Pak) with a gradient of 0-15 % MeOH-CH₂Cl₂, followed by trituration with CH₂Cl₂ to afford the title compound as an off-white solid (21.7 mg, 98 %). ¹H-NMR (300 MHz, DMSO-d₆) δ 9.45 (broad s, 2H), 9.07 (broad s, 2H), 8.68 (s, 1H), 8.28 (s, 1H), 8.09 (d, 2H, J = 7 Hz), 7.51 (t, 2H, J = 7 Hz), 7.40 (t, 1H, J = 7 Hz), 3.23 (q, 2H J = 7 Hz) and 1.42 (t, 3H, J = 7 Hz). Mass spectrum (ESI) calcd. for C₁₆H₁₅N₃S₃: 346.1 (M+H). Found: 346.2.

### Example 97

### 5-Methylthio-4-[4-(phenoxymethyl)(1,3-thiazol-2-yl)]thiophene-2-carboxamidine hydrochloride

***a) 3-Bromo-1-phenoxyacetone:*** To a solution of 6.c (0.050 mmol) of phenoxyacetyl chloride in 250 µL of anhyd MeCN in a 1-dram short vial (Wheaton Glass) was added 50 µL (0.100 mmol) of a 2 M solution of trimethylsilyldiazomethane in hexane and the vial capped with a PTFE-lined cap. After stirring 1 h at room temperature on a vortex shaker, the mixture was cooled (0 C) and 21 µL (0.105 mmol) of 30 wt % HBr in acetic acid was added dropwise (gas evolution). After vortexing for 10 min, the mixture was concentrated *in vacuo* on a vacuum centrifuge concentrator (Speed-Vac, Savant Instruments, Inc.) to provide an amber-colored oil which was used directly in the following step.

***b) Methyl 5-methylthio-4-[4-(phenoxymethyl)(1,3-thiazol-2-yl)]thiophene-2-carboxylate:*** To the 3-bromo-1-phenoxyacetone (as prepared in the previous step in a 1-dram vial) was added 14.8 mg (0.060 mmol) of methyl 4-(aminothioxomethyl)-5-methylthiothiophene-2-carboxylate (Maybridge Chemical Company, Cornwall, UK) as 1.48 mL of a 10 mg/mL solution in acetone. The vial was tightly capped and placed on a heated platform shaker (Innova model 4080, New Brunswick Scientific Co., Inc.) and vortexed at 55 C and 250 rpm for 4 h. To the resulting mixture was added 50 mg (0.150 mmol) of diethylaminomethyl-polystyrene resin (Fluka Chemika-Biochemika, 3.0 mmol / g) as 0.50 mL of a 100 mg / mL suspension in acetone and the mixture vortexed briefly. Chloroacetylpolystyrene resin (30 mg, 0.150 mmol, Advanced ChemTech Inc., 5.0 mmol / g) was then added followed by (0.750 mg, 0.005 mmol) NaI as 100 µL of a 7.5 mg / mL solution in acetone. The mixture was again capped tightly and placed on a heated platform shaker and vortexed at 55°C and 250 rpm for 22 h. The mixture was filtered through a 2 mL fritted column (BioRad Biospin minicolumn) washing with acetone (2 x 0.5 mL) and MeOH (2 x 0.5 mL) into a 2 dram vial and concentrated on a vacuum centrifuge concentrator to afford 21.0 mg of the title compound as an off-white solid. ¹H-NMR (300 MHz, DMSO-d₆) δ 8.17 (s, 1H), 7.82 (s, 1H), 7.13 (m, 2H), 7.07 (m, 2H), 6.96 (m, 1H), 5.22 (s, 2H), 3.85 (s, 3H), and 2.74 (s, 3H). Mass spectrum (MALDI-TOF, α-cyano-4-hydroxycinnamic acid matrix) calcd. for C₁₇H₁₅NO₃S₃: 378.0 (M + H). Found: 378.3.

***c) 5-Methylthio-4-[4-(phenoxymethyl)(1,3-thiazol-2-yl)]thiophene-2-carboxamidine hydrochloride:*** The methyl 5-methylthio-4-[4-(phenoxymethyl)(1,3-thiazol-2-yl)]thiophene-2-carboxylate (as prepared in the previous step)under nitrogen in a 2 dram vial with a micro magnetic stir bar) was capped with an open-top phenolic cap containing a PTFE-backed silicone septum. A 1 M solution of the reagent freshly prepared from trimethylaluminum and ammonium chloride in toluene according to the procedure in Example 10, step b (0.750 mL, 0.750 mmol) was added by syringe by puncturing the septum once with the needle to allow venting of gas followed by a second puncture to inject the reagent. The vial was placed in an aluminum heating block under nitrogen (Fisher Scientific Dry Bath Incubator fitted with a custom-made nitrogen manifold cover). The manifold was flushed with nitrogen and the reaction stirred by means of a large magnetic stir motor placed inverted on top of the manifold. The reaction was heated to 100°C for 4 h, and cooled to room temperature over ca. 2 h. The contents of the vial were quenched carefully into 0.5 g of silica gel in 2 mL of CH₂Cl₂, capped and shaken to homogeneity. The slurry was filtered through a 4-mL fritted column (Isolab microcolumn) into a 2-dram vial washing with CH₂Cl₂ (2 x 1 mL), CH₂Cl₂-MeOH (1:1, 1 x 1 mL) and MeOH(2 x 1 mL) and the filtrate concentrated on a vacuum centrifuge concentrator to a yellow solid. Filtration through a 500 mg silica SPE column (Supelco LC-Si) with 10 % MeOH -CH₂Cl₂ afforded the title compound as a yellow solid (14.8 mg). ¹H-NMR (300 MHz, DMSO-d₆) δ 9.45 (d, 2H, J = 8.2 Hz), 9.11 (d, 2H, J = 8.2 Hz), 8.97 (broad s, 2H), 8.65 (s, 1H), 7.90 (s, 1H), 7.0-7.5 (m, 5H), 5.25 (s, 2H), and 2.79 (s, 3H). Mass spectrum (MALDI-TOF, gentisic acid matrix) calcd. for C₁₇H₁₅NO₃S₃: 362.0 (M + H). Found: 361.7.

### Examples 98-126

Examples 98-104 were carried out using the procedure of Example 97, steps (b) and (c) using 0.050 mmol of the reagent specified in the table. Examples 105-126 were carried out using the procedure of Example 97, steps (a), (b) and (c) using 0.05 mmol of reagent.

| | | | | Mass Spectrum (ESI) | |
|---|---|---|---|---|---|
| Example | Reagent | Compound | Formula | Calcd(M+H) | Found |
| 98 | 1-bromopinacolone | 4-[4-(tert-butyl)(1,3-thiazol-2-yl)]-5-methylthiothiophene-2-carboxamidine hydrochloride | C13 H17 N3 S3 | 312.1 | 312.2 |
| 99 | 4-fluorophenacyl bromide | 4-[4-(4-fluorophenyl)(1,3-thiazol-2-yl)]-5-methylthiothiophene-2-carboxamidine hydrochloride | C15 H12 F N3 S3 | 350.0 | 350.2 |
| 100 | 4-cyano-phenacyl bromide | 4-[4-(4-amidinophenyl)(1,3-thiazol-2-yl)]-5-methylthiothiophene-2-carboxamidine hydrochloride | C16 H15 N5 S3 | 374.1 | 374.2 |
| 101 | 3-fluorophenacyl bromide | 4-[4-(3-fluorophenyl)(1,3-thiazol-2-yl)]-5-methylthiothiophene-2-carboxamidine hydrochloride | C15 H12 F N3 S3 | 350.0 | 350.2 |
| 102 | 4-(diethylamino)-phenacyl bromide | 4-{4-[4-(diethylamino)-phenyl](1,3-thiazol-2-yl)}-5-methylthiothiophene-2-carboxamidine hydrochloride | C19 H22 N4 S3 | 403.1 | 403.2 |
| 103 | 3-chlorophenacyl bromide | 4-[4-(3-chlorophenyl)(1,3-thiazol-2-yl)]-5-methylthiothiophene-2-carboxamidine hydrochloride | C15 H12 Cl N3 S3 | 366.0 | 366.1 |
| 104 | 3,4-difluorophenacyl bromide | 4-[4-(3,4-difluorophenyl)(1,3-thiazol-2-yl)]-5-methylthiothiophene-2-carboxamidine hydrochloride | C15 H11 F2 N3 S3 | 368.0 | 368.2 |
| 105 | 2,6-difluorobenzoyl chloride | 4-[4-(2,6-difluorophenyl)(1,3-thiazol-2-yl)]-5-methylthiothiophene-2-carboxamidine hydrochloride | C15 H11 F2 N3 S3 | 368.0 | 368.2 |
| 106 | 4-ethoxy-benzoyl chloride | 4-[4-(4-ethoxyphenyl)(1,3-thiazol-2-yl)]-5-methylthiothiophene-2-carboxamidine hydrochloride | C17 H17 N3 O S3 | 376.1 | 376.2 |
| 107 | 4-chlorophenoxyacetyl chloride | 4-{4-[(4-chlorophenoxy)-methyl](1,3-thiazol-2-yl)}-5-methylthiothiophene-2-carboxamidine hydrochloride | C16 H14 Cl N3 O S3 | 396.0 | 396.1 |
| 108 | cyclopentane-carbonyl chloride | 4-(4-cyclopentyl(1,3-thiazol-2-yl))-5-methylthiothiophene-2-carboxamidine hydrochloride | C14 H17 N3 S3 | 324.1 | 324.2 |
| 109 | 1-naphthoyl chloride | 5-methylthio-4-(4-naphthyl(1,3-thiazol-2-yl))thiophene-2-carboxamidine hydrochloride | C19 H15 N3 S3 | 382.1 | 382.2 |
| 110 | 3,5-dichlorobenzoyl chloride | 4-[4-(3,5-dichlorophenyl)(1,3-thiazol-2-yl)]-5-methylthiothiophene-2-carboxamidine hydrochloride | C15 H11 C12 N3 S3 | 400.0 | 400.1 |
| 111 | 2,5-difluorobenzoyl chloride | 4-[4-(2,5-difluorophenyl)(1,3-thiazol-2-yl)]-5-methylthiothiophene-2-carboxamidine hydrochloride | C15 H11 F2 N3 S3 | 368.0 | 368.2 |
| 112 | 9-fluorenone-4-carbonyl chloride | 5-methylthio-4-[4-(9-oxofluoren-4-yl)(1,3-thiazol-2-yl)]thiophene-2-carboxamidine hydrochloride | C22 H15 N3 O S3 | 434.1 | 434.2 |
| 113 | 3-methoxyphenylacetyl chloride | 4-{4-[(3-methoxyphenyl)methyl](1,3-thiazol-2-yl)}-5-methylthiothiophene-2-carboxamidine hydrochloride | C17 H17 N3 O S3 | 376.1 | 376.2 |
| 114 | 4-methyl valeroyl chloride | 4-[4-(3-methylbutyl)(1,3-thiazol-2-yl)]-5-methylthiothiophene-2-carboxamidine hydrochloride | C14 H19 N3 S3 | 326.1 | 326.2 |
| 115 | 3-(2-chlorophenyl)-5-methylisoxazole-4-carbonyl chloride | 4-{4-[3-(2-chlorophenyl)-5-methylisoxazol-4-yl](1,3-thiazol-2-yl)}-5-methylthiothiophene-2-carboxamidine hydrochloride | C19H15 C1 N4 O S3 | 447.0 | 447.1 |
| 116 | 4-n-amyloxybenzoyl chloride | 5-methylthio-4-[4-(4-pentyloxyphenyl)(1,3-thiazol-2-yl)]thiophene-2-carboxamidine hydrochloride | C20 H23 N3 O S3 | 418.1 | 418.2 |
| 117 | 1-(4-chlorophenyl)-1-cyclopentanecarb onyl-chloride | 4-{4-[(4-chlorophenyl)-cyclopentyl](1,3-thiazol-2-yl)}-5-methylthiothiophene-2-carboxamidine hydrochloride | C20 H20 C1 N3 S3 | 434.1 | 434.3 |
| 118 | 4-(trifluoromethoxy)benzoyl chloride | 5-methylthio-4-{4-[4-(trifluoromethoxy)phenyl](1,3-thiazol-2-yl)}thiophene-2-carboxamidine hydrochloride | C16 H12 F3 N3 O S3 | 416.0 | 416.1 |
| 119 | 3-chlorobenzo[b] thiophene-2-carbonyl chloride | 4-[4-(3-chlorobenzo[b]thiophen-2-yl)(1,3-thiazol-2-yl)]-5-methylthiothiophene-2-carboxamidine hydrochloride | C17 H12 C1 N3 S4 | 422.0 | 422.1 |
| 120 | 3-(2-chloro-6-fluorophenyl)-5-methylisoxazole-4-carbonyl chloride | 4-{4-[3-(6-chloro-2-fluorophenyl)-5-methylisoxazol-4-yl](1,3-thiazol-2-yl)}-5-methylthiothiophene-2-carboxamidine hydrochloride | C19 H14 Cl F N4 O S3 | 465.0 | 465.1 |
| 121 | 3-cyanobenzoyl chloride | 4-[4-(3-amidinophenyl)(1,3-thiazol-2-yl)]-5-methylthiothiophene-2-carboxamidine hydrochloride | C16 H15 N5 S3 | 374.1 | 374.7 |
| 122 | 4-methoxyphenylacetyl chloride | 4-{4-[(4-methoxyphenyl)-methyl](1,3-thiazol-2-yl)}-5-methylthiothiophene-2-carboxamidine hydrochloride | C17 H17 N3 O S3 | 376.1 | 376.2 |
| 123 | 3-(t-butyl)-1-benzylpyrazole-5-carbonyl chloride_ | 4-{4-[3-(tert-butyl)pyrazol-5-yl](1,3-thiazol-2-yl)}-5-methylthiothiophene-2-carboxamidine hydrochloride | C16 H19 N5 S3 | 378.1 | 378.2 |
| 124 | 3-(4-chlorophenyl)-2,2-dimethylpropanoyl chloride | 5-methylthio-4-[4-(1-methylvinyl)(1,3-thiazol-2-yl)]thiophene-2-carboxamidine hydrochloride | C12 H13 N3 S3 | 296.0 | 296.2 |
| 125 | n-(1-naphthalene-sulfonyl)-1-phenylalanyl chloride | 5-methylthio-4-(4-{1-[(naphthylsulfonyl)amino ]-2-phenylethyl}(1,3-thiazol-2-yl))thiophene-2-carboxamidine hydrochloride | C27 H24 N4 O2 S4 | 565.1 | 565.1 |
| 126 | 2-bromo-5-methoxybenzoyl chloride | , 4-[4-(2-bromo-5-methoxyphenyl)(1,3-thiazol-2-yl)]-5-methylthiothiophene-2-carboxamidine hydrochloride | C16 H14 Br N3 O S3 | 440.0 | 440.2 |

### Example 127

***a) 1-[3,5-bis(trifluoromethyl)phenyl]-2-bromoethan-1-one:*** A stirred suspension of 1 g (3.9 mmol) of 3,5-bis(trifluoromethyl)acetophenone (Lancaster, Windham, NH, USA) in dry methanol (20 mL) and 1 g (15 mmol, 2.6 eq) of poly(4-vinyl pyridinium tribromide) (Aldrich, Milwaukee, WI, USA) was protected from moisture with dry nitrogen, and heated at reflux for 70 min. The polymer was filtered from the cooled solution and washed with methanol and twice with dichloromethane. The solvents were removed *in vacuo* to give 1-[3,5-bis(trifluoromethyl)phenyl]-2-bromoethan-1-one (1.2 g, 92 %). ¹H-NMR (DMSO-d₆; 300 MHz) δ 8.43 (m, 2H), 8.12 (m, 1H), 4.46 (s, 3H).

***b) Methyl 4-{4-[3,5-bis(trifluoromethyl)phenyl](1,3-thiazol-2-yl)}-5-methylthiothiophene-2-carboxylate:*** A solution of 75 mg (0.3 mmol) of methyl 4-(aminothioxomethyl)-5-methylthiothiophene-2-carboxylate (Maybridge, Cornwall, UK) was reacted with 101 mg (0.3 mmol) of 1-[3,5-bis(trifluoromethyl)phenyl]-2-bromoethane-1-one in a manner similar Example 8, step (a) to give methyl 4-{4-[3,5-bis(trifluoromethyl)phenyl](1,3-thiazol-2-yl)}-5-methylthiothiophene-2-carboxylate (7 mg, 5 %) as a solid. ¹H-NMR (DMSO-d₆; 300 MHz) δ 8.75 (s, 1H), 8.73 (m, 2H), 8.29 (s, 1H), 8.13 (m, 1H), 3.87 (s, 3H), 2.79 (s, 3H). Mass spectrum (MALDI-TOF, CHCA matrix, m/z): Calcd. for C₁₈H₁₁NO₂S₃F₆, 484.0 (M+H), found 484.0.

***c) 4-{4-[3,5-bis(trifluoromethyl)phenyl](1,3-thiazol-2-yl)}-5-methylthiothiophene-2-carboxamidine:*** Methyl 4-[4-3,5-bis(trifluoromethyl)phenyl](1,3-thiazol-2-yl))-5-methylthiothiophene-2-carboxylate (7 mg, 14.5 mmol) was treated in a manner similar to that for Example 10, step (b), to give 4-{4-[3,5-bis(trifluoromethyl)phenyl](1,3-thiazol-2-yl)}-5-methylthiothiophene-2-carboxamidine (6 mg, 89 %) as a yellow solid. ¹H-NMR (DMSO-d₆; 300 MHz) 8.78 (s, 1H), 8.74 (s, 2H), 8.62 (s, 1H), 8.15 (s, 1H), 2.82 (s, 3H). Mass spectrum (MALDI-TOF, CHCA matrix, m/z): Calcd, for C₁₇H₁₁N₃S₃F₆, 468.0 (M+H), found 468.0.

### Example 128

***a) 2-Bromo-1-[3-fluoro-5-(trifluoromethyl)phenyl]ethan-1-one:*** A stirred suspension of 1 g (4.5 mmol) of 3-fluoro-5-(trifluoromethyl)acetophenone (Lancaster, Windham, NH, USA) was treated in a manner similar to that for Example 127, step (a) to give of a 1:1 mixture of 2-bromo-1-[3-fluoro-5-(trifluoromethyl)phenyl]ethan-1-one and dibrominated product (1.6 g, 100%). ¹H-NMR (DMSO-d₆; 300 MHz) δ 8.25-7.52 (m, 6H), 6.54 (s, 1H), 4.42 (s, 2H).

***b) Methyl 4-{4-[3-fluoro-5-(trifluoromethyl)phenyl](1,3-thiazol-2-yl)}-5-methylthiothiophene-2-carboxylate:*** A solution of 75 mg (0.3 mmol) of methyl 4-(aminothioxomethyl)-5-methylthiothiophene-2-carboxylate (Maybridge, Cornwall, UK) was reacted with of 86 mg (0.3 mmol) 2-bromo-1-[3-fluoro-5-(trifluoromethyl)phenyl]ethan-1-one in a manner similar to Example 8, step (a) to give, methyl 4-{4-[3-fluoro-5-(trifluoromethyl)phenyl](1,3-thiazol-2-yl)}-5-methylthiothiophene-2-carboxylate (41 mg, 31 %) as a solid. ¹H-NMR (DMSO-d₆; 300 MHz) δ 8.59 (s, 1H), 8.29 (m, 1H), 8.27 (s, 1H), 8.25 and 8.21 (m, 1H, 1:1 ratio conformers), 7.73 and 7.70 (m, 1H, 1:1 ratio conformers). Mass spectrum (MALDI-TOF, CHCA matrix, m/z): Calcd. for C₁₇H₁₁NO₂S₃F₄, 434.0 (M+H), found 434.0.

***c) 4-{4-[3-Fluoro-5-(trifluoromethyl)phenyl](1,3-thiazol-2-yl)}-5-methylthiothiophene-2-carboxamidine:*** Methyl 4-{4-[3-fluoro-5-(trifluoromethyl)phenyl](1,3-thiazol-2-yl)}-5-methylthiothiophene-2-carboxylate (40 mg, 0.92 mmol) was treated in a manner similar to that for Example 10, step (b), to give 4-{4-[3-fluoro-5-(trifluoromethyl)phenyl](1,3-thiazol-2-yl)}-5-methylthiothiophene-2-carboxamidine (31 mg, 81 %) as a yellow solid. ¹H-NMR (DMSO-d_{g}; 300 MHz) δ 9.36 (br s, 2H), 9.01 (br s, 2H), 8.68 (s, 1H), 8.63 (s, 1H), 8.30 (m, 1H), 8.25 and 8.22 (m, 1H, 1:1 ratio conformers), 7.75 and 7.73 (m, 1H, 1:1 ratio conformers), 2.82 (s, 3H). Mass spectrum (MALDI-TOF, CHCA matrix, m/z): Calcd. for C₁₆H₁₁N₃S₃F₄, 418.5 (M+H), found 418.0.

### Example 129

***a) 2-Bromo-1-[3-fluoro-5-(trifluoromethyl)phenyl]propan-1-one:*** A stirred suspension of 1 g (4.5 mmol) of 1-[3-fluoro-5-(trifluoromemyl)phenyl]propan-1-one (Lancaster, Windham, NH, USA) was treated in a manner similar to that for Example 127, step (a) to give 2-bromo-1-[3-fluoro-5-(trifluoromethyl)phenyl]propan-1-one (1.33 g, 99 %). ¹H-NMR (DMSO-d₆; 300 MHz) δ 8.07 (m, 1H), 7.92 and 7.89 (m, 1H, 1:1 ratio conformers), 7.57 and 7.55 (m, 1H, 1:1 ratio conformers), 5.20 (q, 1H, J=6.6Hz), 1.93 (d, 3H, J=6.6 Hz).

***b) Methyl 4-{4-[3-fluoro-5-(trifluoromethy)phenyl]-5-methyl(1,3-thiazol-2-yl)}-5-methylthiothiophene-2-carboxylate:*** A solution of 75 mg (0.3 mmol) of methyl 4-(aminothioxomethyl)-5-methylthiothiophene-2-carboxylate (Maybridge, Cornwall, UK) was reacted with 90 mg (0.3 mmol) of 2-bromo-1-[3-fluoro-5-(trifluoromethyl)phenyl]propan-1-one in a manner similar to Example 8, step (a) to give, methyl 4-{4-[3-fluoro-5-(trifluoromethyl)phenyl]-5-methyl(1,3-thiazol-2-yl)}-5-methylthiothiophene-2-carboxylate (31.9 mg, 24 %) as a solid. ¹H-NMR (DMSO-d₆; 300 MHz) δ 8.17 (s, 1H), 7.98 (m, 1H), 7.95 and 7.92 (m, 1H, 1:1 ratio conformers), 7.77 and 7.74 (m, 1H, 1:1 ratio conformers), 3.87 (s, 3H), 2.75 (s, 3H), 2.70 (s, 3H). Mass spectrum (MALDI-TOF, CHCA matrix, m/z): Calcd. for C₁₈H₁₃NO₂S₃F₄, 448.0 (M+H), found 448.0.

***c) 4-{4-[3-Fluoro-5-(trifluoromethyl)phenyl]-5-methyl(1,3-thiazol-2-yl)}-5-methylthiothiophene-2-carboxamidine:*** Methyl 4-{4-[3-fluoro-5-(trifluoromethyl)phenyl]-5-methyl(1,3-thiazol-2-yl)}-5-methylthiothiophene-2-carboxylate (30 mg, 0.067 mmol) was treated in a manner similar to that for Example 10, step (b), to give 4-{4-[3-fluoro-5-(trifluoromethyl)phenyl]-5-methyl(1,3-thiazol-2-yl)}-5-methylthiothiophene-2-carboxamidine (32 mg, quantitive yield) as a yellow solid. ¹H-NMR (DMSO-d₆; 300 MHz) δ 9.42 (br s, 2H), 9.03 (br s, 2H), 8.60 (s, 1H), 7.98 (m, 1H), 7.95 and 7.92 (m, 1H, 1:1 ratio conformers), 7.79 and 7.76 (m, 1H, 1:1 ratio conformers), 2.78 (s, 3H), 2.71 (s, 3H). Mass spectrum (MALDI-TOF, CHCA matrix, m/z): Calcd. for C₁₇H₁₃N₃S₃F₄, 432.0 (M+H), found 432.6.

### Example 130

***a) 1-[3,5-Bis(trifluoromethyl)phenyl]-2-bromopropan-1-one:*** *A stirred* suspension of 1 g (3.7 mmol) of 1-[3,5-bis(trifluoromethyl)phenyl]-propan-1-one (Lancaster, Windham, NJ, USA) treated in a manner similar to that for Example 127, step (a) to give 2-bromo-1-[3-fluoro-5-(trifluoromethyl)phenyl]propan-1-one (1.1 g, 86 %). ¹H-NMR (DMSO-d₆; 300 MHz) δ 8.46 (m, 2H), 8.09 (m, 1), 5.26 (q, 1H, J=6.6Hz), 1.96 (d, 3H, J=6.5 Hz). Mass spectrum (MALDI-TOF, CHCA matrix, m/z): Calcd. for C₁₁H₇OBrF₆, 349.0 (M+H), found 348.9.

***b) Methyl 4-{4-[3,5-bis(trifluoromethyl)phenyl]-5-methyl(1,3-thiazol-2-yl)}-5-methylthiothiophene-2-carboxylate:*** A solution of 75 mg (0.3 mmol) of methyl 4-(aminothioxomethyl)-5-methylthiothiophene-2-carboxylate (Maybridge, Cornwall, UK) was reacted with 105 mg 1-[3,5-Bis(trifluoromethyl)phenyl)-2-bromopropan-1-one in a manner similar to Example 8, step (a) to give, after preparative thin-layer chromatrography purification, methyl 4-{4-[3,5-bis(trifluoromethyl)phenyl]-5-methyl(1,3-thiazol-2-yl)}-5-methylthiothiophene-2-carboxylate (16.2 mg, 11 %) as a solid. ¹H-NMR (DMSO-d₆; 300 MHz) δ 8.41 (m, 2H), 8.18 (m, 2H), 3.86 (s, 3H), 2.75 (s, 3H), 2.71 (s, 3H). Mass spectrum (MALDI-TOF, CHCA matrix, m/z): Calcd. for C₁₉H₁₃NO₂S₃F₆, 498.0 (M+H), found 497.6.

***c) 4-{4-[3,5-Bis(trifluoromethyl)phenyl]-5-methyl(1,3-thiazol-2-yl)}-5-methylthiothiophene-2-carboxamidine:*** Methyl 4-{4-[3,5-bis(trifluoromethyl)phenyl]-5-methyl(1,3-thiazol-2-yl)}-5-methylthiothiophene-2-carboxylate (15 mg, 0.031 mmol) was treated in a manner similar to that for Example 10, step (b), to give 4-{4-[3,5-bis(trifluoromethyl)phenyl]-5-methyl(1,3-thiazol-2-yl)}-5-methylthiothiophene-2-carboxamidine (13 mg, 88 %) as a yellow solid. ¹H-NMR (DMSO-d₆; 300 MHz) δ 9.39 (br s, 2H), 8.94 (br s, 2H), 8.58 (s, 1H), 8.40 (m, 2H), 8.19 (m, 1H), 2.79 (s, 3H), 2.73 (s, 3H). Mass spectrum (MALDI-TOF, CHCA matrix, m/z): Calcd. for C₁₈H₁₃N₃S₃F₆, 482.0 (M+H), found 482.5.

### Example 131

***a) 2-Bromo-1,2-diphenylethan-1-one:*** A stirred suspension of 0.2 g (1 mmol) of deoxybenzoin was treated in a manner similar to that for Example 127, step (a) to give 2-bromo-1,2-diphenylethan-1-one (270 mg, 98 %). ¹H-NMR (DMSO-d₆; 300 MHz) δ 8.10-8.06 (m, 2H), 7.95-7.31 (m, 8H), 7.21 (s, 1H).

***b) Methyl 4-(4,5-diphenyl(1,3-thiazol-2-yl))-5-methylthiothiophene-2-carboxylate:*** A solution of 75 mg (0.3 mmol) of methyl 4-(aminothioxomethyl)-5-methylthiothiophene-2-carboxylate (Maybridge, Cornwall, UK) was reacted with 92 mg, 0.3 mmol) of 2-bromo-1,2-diphenylethan-1-one in a manner similar to Example 8, step (a) to give, after preparative thin-layer chromatrography purification, methyl 4-(4,5-diphenyl(1,3-thiazol-2-yl))-5-methylthiothiophene-2-carboxylate (9 mg, 7 %) as a solid. ¹H-NMR (DMSO-d₆; 300 MHz) δ 8.94 (br s, 0.4H), 8.66 (s, 1H), 8.60 (br s, 0.3 H), 8.08 (s, 1H), 7.93 and 7.20 (AB quartet, 2H, J = 8.7 Hz), 7.68 and 7.35 (AB quartet, 2H, J = 8.2 Hz), 2.77 (s, 3H),), 2.33 (s, 3H). Mass spectrum (MALDI-TOF, CHCA matrix, m/z): Calcd. for C₂₂H₁₇NO₂S₃, 424.0 (M+H), found 424.3.

***c) 4-(4,5-Diphenyl(1,3-thiazol-2-yl))-5-methylthiothiophene-2-carboxamidine:*** Methyl 4-(4,5-diphenyl(1,3-thiazol-2-yl))-5-methylthiothiophene-2-carboxylate (9 mg, 0.021 mmol) was treated in a manner similar to that for Example 10, step (b), to give 4-(4,5-diphenyl(1,3-thiazol-2-yl))-5-methylthiothiophene-2-carboxamidine (3 mg, 35 %) as a brown oil. Mass spectrum (MALDI-TOF, CHCA matrix, m/z): Calcd. for C₂₁H₁₇N₃S₃, 408.1 (M+H), found 408.0.

### Example 132

***a) Methyl 4-(4-benzo[b]thiophen-2-yl(1,3-thiazol-2-yl))-5-methylthiothiophene-2-carboxylate:*** A solution of 75 mg (0.3 mmol) of methyl 4-(aminothioxomethyl)-5-methylthiothiophene-2-carboxylate was reacted with 77 mg (0.3 mmol) of3-bromoacetylbenzo[b]thiophene (Maybridge, Cornwall, UK) in a manner similar to Example 8, step (a) to give, after preparative thin-layer chromatrography purification, methyl 4-(4-benzo[b]thiophen-2-yl(1,3-thiazol-2-yl))-5-methylthiothiophene-2-carboxylate (28 mg, 23 %) as a solid. ¹H-NMR (DMSO-d₆; 300 MHz) δ 8.63 (d, 1H, J=7.4 Hz), 8.30 (s, 1H), 8.25 (s, 1H), 8.22 (s, 1H), 7.53-7.46 (m, 2H), 3.87 (s, 3H), 2.78 (s, 3H).

***b) 4-(4-Benzo[b]thiophen-2-yl(1,3-thiazol-2-yl))-5-methylthiothiophene-2-carboxamidine:*** Methyl 4-(4-benzo[b]thiophen-2-yl(1,3-thiazol-2-yl))-5-methylthiothiophene-2-carboxylate (28 mg, 0.69 mmol) was treated in a manner similar to that for Example 10, step (b), to give 4-(4-benzo[b]thiopben-2-yl(1,3-thiazol-2-yl))-5-methylthiothiophene-2-carboxamidine (17 mg, 64 %) as a brown solid. ¹H-NMR (DMSO-d₆; 300 MHz) δ 9.22 (br s, 4H), 8.68 (s, 1H), 8.66 (d, 1H, J=7.6 Hz), 8.30 (s, 1H), 8.25 (s, 1H), 8.10 (d, 1H, J=7.3 Hz), 7.55-7.45 (m, 2H), 2.81 (s, 3H). Mass spectrum (MALDI-TOF, GA matrix, m/z): Calcd. for C₁₇H₁₃N₃S₄, 388.0 (M+H), found 388.2.

### Example 133

***a) Methyl 4-(4-benzo[d]benzo[3,4-b]furan-3-yl(1,3-thiazol-2-yl))-5-methylthiothiophene-2-carboxylate:*** A solution of 75 mg (0.3 mmol) of methyl 4-(aminothioxomethyl)-5-methylthiothiophene-2-carboxylate (Maybridge, Cornwall, UK) was reacted with 86 mg (0.3 mmol) of 2-(bromoacetyl)-dibenzofuran (Aldrich, Milwaukee, WI, USA) in a manner similar to Example 8, step (a) to give, after preparative thin-layer chromatrography purification, methyl 4-(4,5-diphenyl(1,3-thiazol-2-yl))-5-methylthiothiophene-2-carboxylate (45 mg, 36 %) as a solid. ¹H-NMR (DMSO-d₆; 300 MHz) δ 8.83-7.44 (m, 7H), 8.29 (s, 1H), 8.27 (s, 1 H), 3.88 (s, 3H), 2.80 (s, 3H). Mass spectrum (MALDI-TOF, CHCA matrix, m/z): Calcd. for C₂₂H₁₅NO₃S₃, 438.0 (M+H), found 438.5.

***b) 4-4-Benzo[d]benzo[3,4-b]furan-3-yl(1,3-thiazol-2-yl))-5-methylthiothiophene-2-carboxamidine:*** Methyl 4-(4-benzo[d]benzo[3,4-b]furan-3-yl(1,3-thiazol-2-yl))-5-methylthiothiophene-2-carboxylate (45 mg, 0.11 mmol) was treated in a manner similar to that for Example 10, step (b), to give 4-4-benzo[d]benzo[3,4-b]furan-3-yl(1,3-thiazol-2-yl))-5-methylthiothiophene-2-carboxamidine (16.8 mg, 36 %) as a yellow solid. ¹H-NMR (DMSO-d₆; 300 MHz) δ 9.72-9.10 (m, 3H), 8.84 - 7.31 (m, 9H), 2.84 (s, 3H). Mass spectrum (MALDI-TOF, CHCA matrix, m/z): Calcd. for C₂₁H₁₅N₃OS₃, 422.0 (M+H), found 421.9.

### Example 134

***a) Methyl 4-(4-(4-nitrophenyl)(1,3-thiazol-2-yl))-5-methylthiothiophene-2-carboxylate:*** A solution of 1 g (4 mmol) of methyl 4-(aminothioxomethyl)-5-methylthiothiophene-2-carboxylate (Maybridge, Cornwall, UK) was reacted with 987 mg (4 mmol) of 2-bromo-4'-nitroacetophenone in a manner similar to Example 8, step (a) to give methyl 4-(4-(4-nitrophenyl)(1,3-thiazol-2-yl))-5-methylthiothiophene-2-carboxylate (1.7 g, quantitive yield) as a brown solid. ¹H-NMR (DMSO-d₆; 300 MHz) δ 8.57 (s, 1H), 8.34 (s, 4H), 8.25 (s, 1H), 3.94 (s, 3H), 3.81 (s, 3H). Mass spectrum (MALDI-TOF, CHCA matrix, m/z): Calcd. for C₁₆H₁₂N₂O₄S₃ 393.0 (M+H), found 392.8.

***b) Methyl 4-(4-(4-aminophenyl)(1,3-thiazol-2-yl))-5-methylthiothiophene-2-carboxylate:*** Methyl 4-(4-(4-nitrophenyl)(1,3-thiazol-2-yl))-5-methylthiothiophene-2-carboxylate (800 mg, 2 mmol) was dissolved in 150 mL tetrahydrofuran and treated with 20 % titanium chloride solution (Fisher Scientific, Pittsburgh, PA, USA) for 1 h. The mixture was poured into 2 M sodium hydroxide solution (100 mL), extracted with dichloromethane (4 x 50 mL). The combined organic layers were washed with saturated brine solution and dried over anhydrous sodium sulfate. The solid was filtered off, and the solvent removed *in vacuo*. This material was purified by column chromatography on silica gel (30 g) eluting with dichloromethane:methanol 98/2 (v:v) to give methyl 4-(4-(4-aminophenyl)(1,3-thiazol-2-yl))-5-methylthiothiophene-2-carboxylate (500 mg, 69 %) as a solid. ¹H-NMR (DMSO-d₆; 300 MHz) δ 8.17 (s, 1H), 7.77 (s, 1H), 7.74 and 6.62 (AB quartet, 2H, J = 8.6 Hz), 5.35 (s, 2H), 3.86 (s, 3H), 2.74 (s, 3H).). Mass spectrum (MALDI-TOF, CHCA matrix, m/z): Calcd. for C₁₆H₁₄N₂O₂S₃ 363.0 (M+H), found 362.4.

***c) Methyl 4-(4-{4-[(methylsulfonyl)amino]phenyl}(1,3-thiazol-2-yl))-5-methylthiothiophene-2-carboxylate:*** Methyl 4-(4-(4-aminophenyl)(1,3-thiazol-2-yl))-5-methylthiothiophene-2-carboxylate (200 mg; 0.55 mmol) was dissolved in dry dichloromethane (20 mL). To this, *N*-methyl morpholine (150 µL, 1.38 mmol) and dimethylaminopyridine (6.1 mg, 0.055 mmol) were added, the mixture was cooled on an ice bath, and methanesulfonyl chloride (43 µL, 0.55 mmol) was added dropwise. The mixture was then stirred for 8 days at room temperature. The mixture was partitioned between saturated sodium bicarbonate (50 mL) and dichloromethane (20 mL). The aqueous layer was extracted with dichloromethane (3 x 20 mL), and the combined organic layers were washed with saturated sodium bicarbonate (20 mL), brine (2 x 20 mL), and dried over anhydrous sodium sulfate. The solvent was remove *in vacuo.* Column chromatrography on silica gel (100 g) eluting with dichloromethane:methanol 99/1 (v:v), gave methyl 4-(4-{4-[(methylsulfonyl)amino]phenyl}(1,3-thiazol-2-yl))-5-methylthiothiophene-2-carboxylate (155 mg, 64 %) as a solid. ¹H-NMR (DMSO-d₆; 300 MHz) δ 9.92 (s, 1H), 8.22 (s, 1H), 8.11 (s, 1H), 8.40 and 6.90 (AB quartet, 2H, J = 8.7 Hz), 3.87 (s, 3H), 3.05 (s, 3H), 2.76 (s, 3H). Mass spectrum (MALDI-TOF, CHCA matrix m/z): Calcd. for C₁₇H₁₆N₂O₄S₄ 441.0 (M+H), found 441.2.

***d) 4-[4-{4-[(Methylsulfonyl)amino]phenyl}(1,3-thiazol-2-yl))-5-methylthiothiophene-2-carboxamidine:*** Methyl 4-(4-{4-[(methylsulfonyl)amino]phenyl}(1,3-thiazol-2-yl))-5-methylthiothiophene-2-carboxylate (81 mg, 0.184 mmol) was treated in a manner similar to that for Example 10, step (b), to give 4-(4-{4-[(methylsulfonyl)amino]phenyl}(1,3-thiazol-2-yl))-5-methylthiothiophene-2-carboxamidine (24.9 mg, 32 %) as a light brown solid. ¹H-NMR (DMSO-d₆; 300 MHz) δ 10.0 (br s, 1H), 9.3 (br s, 2H), 8.98 (s, 1H), 8.65 (s, 1H), 8.21 (s, 1H), 7.98 and 7.5 (AB quartet, 2H, J = 8.6 Hz), 3.05 (s, 3H), 2.79 (s, 3H). Mass spectrum (MALDI-TOF, CHCA matrix, m/z): Calcd. for C₁₆H₁₆N₄O₂S₄ 425.0 (M+H), found 425.1.

### Example 135

***a) Methyl 4-(4-{4-[(phenylsulfonyl)amino]phenyl}(1,3-thiazol-2-yl))-5-methylthiothiophene-2-carboxylate:*** Methyl 4-(4-(4-aminophenyl)(1,3-thiazol-2-yl))-5-methylthiothiophene-2-carboxylate (100 mg, 0.28 mmol) was dissolved in dry dichloromethane (10 mL). To this, N-methyl morpholine (46 µL, 0.42 mmol) and dimethylaminopyridine (3.4 mg, 0.028 mmol) were added, the mixture was cooled on an ice bath, and benzenesulfonyl chloride 35 µL, 0.28 mmol) was added dropwise. The mixture was then stirred for 24 h at room temperature. Workup was carried out as in Example 134, step (c). Trituration with dichloromethane and methanol gave methyl 4-(4-{4-[(phenylsulfonyl)amino]phenyl}(1,3-thiazol-2-yl))-5-methylthiothiophene-2-carboxylate (44 mg, 31 %) as a crystalline solid. ¹H-NMR (DMSO-d₆; 300 MHz) δ 10.46 (s, 1H), 8.19 (s, 1H), 8.05 (s, 1H), 7.91 and 7.19 (AB quartet, 2H, J = 8.7 Hz), 7.81 (m, 2H), 7.64-7.54 (m, 3H) 3.85 (s, 3H), 2.74 (s, 3H). Mass spectrum (MALDI-TOF, CHCA matrix, m/z): Calcd. for C₂₂H₁₈N₂O₄S₄ 504.2 (M+H), found 504.1

***b) 4-(4-{4-[(Phenylsulfonyl)amino]phenyl}(1,3-thiazol-2-yl))-5-methylthiothiophene-2-carboxamidine:*** Methyl 4-(4-{4-[(phenylsulfonyl)amino]phenyl}(1,3-thiazol-2-yl))-5-methylthiothiophene-2-carboxylate (30 mg, 0.060 mmol) was treated in a manner similar to that for Example 10, step (b), to give 4-(4-{4-[(phenylsulfonyl)amino]phenyl}(1,3-thiazol-2-yl))-5-methylthiothiophene-2-carboxamidine (12.6 mg, 43 %) as a yellow solid. ¹H-NMR (DMSO-d₆; 300 MHz) δ 9.13 (br s, 3H), 8.60 (s, 1H), 8.08 (s, 1H) 7.93 and 7.20 (AB quartet, 2H, J = 8.7 Hz), 7.82-7.79 (m, 2H), 7.65-7.53 (m, 3H) 3.85 (s, 3H), 2.74 (s, 3H). Mass spectrum (MALDI-TOF, CHCA matrix, m/z): Calcd. for C₂₁H₁₈N₄O₂S₄, 87.0 (M+H), found 487.7.

### Example 136

***a) Methyl 4-(4-{4-[(trifluoromethylsulfonyl)amino]phenyl}(1,3-thiazol-2-yl))-5-methylthiothiophene-2-carboxylate:*** Methyl 4-(4-(4-aminophenyl)(1,3 -thiazol-2-yl))-5-methylthiothiophene-2-carboxylate (200 mg, 0.55 mmol) was dissolved in dry pyridine (20 mL). The mixture was cooled on an ice bath, and trifluoromethanesulfonic anhydride (0.5 mL, 3 mmol) was added. The mixture was then stirred for 1.5 h at room temperature. Workup was carried out as in Example 134, step (c). Column chromatrography on silica gel (30 g) eluting with hexanes:ethyl acetate 7/3 (v:v), followed by preparative thin layer chromatography eluting with dichloromethane:methanol 99/1 (v:v) gave methyl 4-(4-{4-[(trifluoromethylsulfonyl)amino]phenyl} (1,3-thiazol-2-yl))-5-methylthiothiophene-2-carboxylate (160 mg, 59 %) as a solid. ¹H-NMR (DMSO-d₆; 300 MHz) δ 8.48 and 7.87(s, 3/2 ratio conformers, 1H), 8.23 (s, 1H), 8.21 (s, 1H), 8.29 and 7.84 (AB quartet, 2H, 2/3 ratio conformers, J=8.7 Hz), 8.10 and 7.37 (AB quartet, 2H, J=8.7 Hz), 3.87 and 3.86 (s, 2/3 ratio conformers, 3H), 2.77 and 2.76 (s, 2/3 ratio conformers, 3H). Mass spectrum (MALDI-TOF, CHCA matrix, m/z): Calcd. for C₁₇H₁₃N₂O₄S₄F₄ 495.0 (M+H), found 495.6

***b) 4-(4-{4-[(Trifluoromethylsulfonyl)amino]phenyl}(1,3-thiazol-2-yl))-5-methylthiothiophene-2-carboxamidine:*** Methyl 4-(4-{4-[(trifluoromethylsulfonyl)amino]phenyl}(1,3-thiazol-2-yl))-5-methylthiothiophene-2-carboxylate (30 mg, 0.061 mmol) was treated in a manner similar to that for Example 10, step (b), to give of 4-(4-{4-[(trifluoromethylsulfonyl)amino]phenyl}(1,3-thiazol-2-yl))-5-methylthiothiophene-2-carboxamidine (21.6 mg, 74 %) as a light brown solid. ¹H-NMR (DMSO-d₆; 300 MHz) δ 9.39 (br s, 2H), 8.97 (br s, 2H), 8.64 (s, 1H), 8.24 (s, 1H), 8.12 and 7.39 (AB quartet, 2H, J = 8.7 Hz), 4.78 (br s, 1H), 2.79 (s, 3H). Mass spectrum (MALDI-TOF, CHCA matrix, m/z): Calcd. for C₁₆H₁₃N₄O₂S₄F₃, 479.0 (M+H), found 479.5.

### Example 137

***a) Methyl 4-(4-{4-[(toluenesulfonyl)amino]phenyl}(1,3-thiazol-2-yl))-5-methylthiothiophene-2-carboxylate:*** Methyl 4-(4-(4-aminophenyl)(1,3-thiazol-2-yl))-5-methylthiothiophene-2-carboxylate (33 mg, 0.09 mmol) was dissolved in dry dichloromethane (5 mL). To this, *N*-methyl morpholine (10 µL, 0.09 mmol) and *p*-toluenesulfonyl chloride (17 mg, 0.09 mmol) was added and the mixture was stirred at room temperature for 5 days. Workup was carried out as in Example 134, step (c). Trituration with dichloromethane and methanol gave methyl 4-(4-{4-[(toluenesulfonyl)amino]phenyl}(1,3-thiazol-2-yl))-5-methylthiothiophene-2-carboxylate (20 mg, 43 %) as a brown solid. ¹H-NMR (DMSO-d₆; 300 MHz) δ 10.39 (s, 1H), 8.19 (s, 1H), 8.05 (s, 1H), 7.91 and 7.18 (AB quartet, 2H, J = 8.7 Hz), 7.68 and 7.35 (AB quartet, 2H, J = 8.2 Hz), 3.85 (s, 3H), 2.74 (s, 3H), 2.27 (s, 3H). Mass spectrum (MALDI-TOF, CHCA matrix, m/z): Calcd. for C₂₃H₂₀N₂O₄S₄, 517.2 (M+H), found 517.0.

***b) 4-(4-{4-[(Toluenesulfonyl)amino]phenyl}(1,3-thiazol-2-yl))-5-methylthiothiophene-2-carboxamidine:*** Methyl 4-(4-{4-[(toluenesulfonyl)amino]phenyl}(1,3-thiazol-2-yl))-5-methylthiothiophene-2-carboxylate (15 mg, 0.029 mmol) was treated in a manner similar to that for Example 10, step (b), to give 4-(4-{4-[(toluenesulfonyl)amino]phenyl}(1,3-thiazol-2-yl))-5-methylthiothiophene-2-carboxamidine (17.9 mg, 81 %) as a light brown solid. ¹H-NMR (DMSO-d₆; 300 MHz) δ 8.94 (br s, 0.4H), 8.66 (s, 1H), 8.60 (br s, 0.3 H), 8.08 (s, 1H), 7.93 and 7.20 (AB quartet, 2H, J = 8.7 Hz), 7.68 and 7.35 (AB quartet, 2H, J = 8.2 Hz), 2.77 (s, 3H), 2.33 (s, 3H). Mass spectrum (MALDI-TOF, CHCA matrix, m/z): Calcd. for C₂₂H₂₀N₄O₂S₄: 501.1 (M+H), found 501.1.

### Example 138

***a) Methyl 4-[4-(4-chlorophenyl)(1,3-thiazol-2-yl)]-5-(methylsulfinyl)thiophene-2-carboxylate:*** To a stirred solution of 764 mg (2 mmol) of methyl 4-[4-(4-chlorophenyl)(1,3-thiazol-2-yl)]-5-methylthiothiophene-2-carboxylate (Maybridge, Cornwall, UK) dissolved in 1,1,1,3,3,3-hexafluoroisopropanol (2.5 mL) was added 30% hydrogen peroxide (0.45 mL, 4 mmol). This solution was stirred for 45 h at room temperature. Dichloromethane (10 mL) was added after 2 hours. Additional hydrogen peroxide (2 x 0.45 mL portions) was added after 4 hours and 24 hours. The mixture was quenched with 10% sodium sulfite in brine (4 mL). The organic layer was separated, dried over anhydrous sodium sulfate, and the solvents removed in vacuum. Column chromatography on silica gel (45 g), eluting with dichloromethane:methanol 99/1 (v:v) gave methyl 4-[4-(4-chlorophenyl)(1,3-thiazol-2-yl)]-5-(methylsulfinyl)thiophene-2-carboxylate (720 mg, 90 %) as a solid. ¹H-NMR (DMSO-d₆; 300 MHz) δ 8.37 (s, 1H), 8.30 (s, 1H), 8.05 and 7.52 (AB quartet, 2H, J = 8.6 Hz), 3.91 (s, 3H), 3.16 (s, 3H). Mass spectrum (MALDI-TOF, GA matrix, m/z): Calcd. for C₁₆H₁₂NO₃S₃Cl:398.0 (M+H), found 397.8.

***b) 4-[4-(4-Chlorophenyl)(1,3-thiazol-2-yl)]-5-(methylsulfinyl)thiophene-2-carboxamidine:*** Methyl 4-[4-(4-chlorophenyl)(1,3-thiazol-2-yl)]-5-(methylsulfinyl)thiophene-2-carboxylate (100 mg, 0.25 mmol) was treated in a manner similar to that for Example 10, step (b), to give, after preparative thin layer chromatography purification eluting with dichloromethane:methanol:acetic acid 9/1/0.5 (v:v:v), 4-[4-(4-chlorophenyl)(1,3-thiazol-2-yl)]-5-(methylsulfinyl)thiophene-2-carboxamidine (18.2 mg, 19 %) as a solid. ¹H-NMR (DMSO-d₆; 300 MHz) δ 8.33 (s, 1H), 8.22 (s, 1H), 8.05 and 7.57 (AB quartet, 2H, J = 8.6 Hz), 3.12 (s, 3H). Mass spectrum (MALDI-TOF, CHCA matrix m/z): Calcd. for C₁₅H₁₂N₃OS₃Cl 382.0 (M+H), found 382.1.

### Example 139

***a) Methyl 4-cyano-5-(methylsulfonyl)thiophene-2-carboxylate:*** To a stirred solution of (4.5 g, 21 mmol) of methyl 4-cyano-5-methylthiothiophene-2-carboxylate (Maybridge, Cornwall, UK) was dissolved in dichloromethane (250 mL) and treated with m-chloroperbenzoic acid (15.3 g, 90 mmol) at room temperature for 2.25 h. The mixture was filtered and the solid washed with dichloromethane (2 x 50 mL). The filtrate was washed with sodium bicarbonate (2 x 100 mL), sodium thiosulfate (100 mL), sodium bicarbonate (100 mL), water (100 mL), brine (100 mL), and dried over anhydrous sodium sulfate. The solvent was removed *in vacuo* to give methyl 4-cyano-5-(methylsulfonyl)thiophene-2-carboxylate (4.91 g, 95%) as a solid. ¹H-NMR (DMSO-d₆; 300 MHz) δ 8.44 (s, 1H), 3.91 (s, 3H), 3.58 (s, 3H).

***b) Methyl 4-cyano-5-methoxythiophene-2-carboxylate:*** Methyl 4-cyano-5-(methylsulfonyl)thiophene-2-carboxylate (2 g, 8 mmol) was refluxed with 0.5 M sodium methoxide in methanol (16 mL) for 15 minutes. The solution was cooled, the crystallized solid collected on a Büchner funnel and washed with methanol (50 mL) to give methyl 4-cyano-5-methoxythiophene-2-carboxylate (1.145 g, 73%) as a solid. ¹H-NMR (DMSO-d₆; 300 MHz) δ 8.87 (s, 1H) 4.19 (s, 3H), 3.82 (s, 3H).

***c) Methyl 4-(aminothioxomethyl)-5-methoxythiophene-2-carboxylate:*** Methyl 4-cyano-5-methoxythiophene-2-carboxylate (1 g, 5 mmol) was dissolved in dry methanol (150 mL) and triethylamine (3.5 mL, 25.4 mmol) was added. After degassing the solution with argon for 10 minutes, hydrogen sulfide gas was bubbled through the solution for 5 h. After stirring 18 h at room temperature, the solution was degassed by bubbling argon (6 h), concentrated to 20 mL and acetone (20 mL) was added. The dark solid was collected on a Büchner funnel and washed with acetone. Recrystallize solid from hot ethanol (15 mL) to give methyl 4-(aminothioxomethyl)-5-methoxythiophene-2-carboxylate (683 mg, 59 %) as a brown oil. Mass spectrum (MALDI-TOF, CHCA matrix, m/z): Calcd. for C₈H₉NO₃S₂ 232.0 (M+H), found 232.4

***d) Methyl 5-methoxy-4-(4-phenyl(1,3-thiazol-2-yl))thiophene-2-carboxylate:*** A solution of 400 mg (1.73 mmol) of methyl 4-(aminothioxomethyl)-5-methoxythiophene-2-carboxylate was reacted with 345 mg (1.73 mmol) of 2-bromoacetophenone (Aldrich, Milwaukee, WI, USA) in a manner similar to Example 8, step (a) to give methyl 5-methoxy-4-(4-phenyl(1,3-thiazol-2-yl))thiophene-2-carboxylate (56 mg, 10 %) as a solid. ¹H-NMR (DMSO-d₆; 300 MHz) δ 8.22 (s, 1H), 8.14 (s, 1H), 8.05 (m, 2H), 7.47 (m, 2H), 7.36 (m, 1H), 4.26 (s, 3H), 3.85 (s, 3H).

***e) 5-Methoxy-4-(4-phenyl(1,3-thiazol-2-yl))thiophene-2-carboxamidine:*** Methyl 5-methoxy-4-(4-phenyl(1,3-thiazol-2-yl))thiophene-2-carboxylate (55 mg, 0.16 mmol) was treated in a manner similar to that for Example 10, step (b), to give 5-methoxy-4-(4-phenyl(1,3-thiazol-2-yl))thiophene-2-carboxamidine (36 mg, 69 %) as a yellow solid. ¹H-NMR (DMSO-d₆; 300 MHz) δ 9.34 (br s, 2H), 8.94 (br s, 2H), 8.70 (s, 1H), 8.20 (s, 1H), 8.07 (m, 2H), 7.49 (m, 2H), 7.38 (m, 1H), 4.32 (s, 3H). Mass spectrum (MALDI-TOF, CHCA matrix, m/z): Calcd. for C₁₅H₁₃N₃OS₂ 316.5 (M+H), found 316.1

### Example 140

***a) Methyl 4-cyano-5-[(4-methoxyphenyl)methylthio]thiophene-2-carboxylate:*** To a stirred solution of 2.5 g (10 mmol) of methyl 4-cyano-5-(methylsulfonyl)thiophene-2-carboxylate (Example 139, step (a)) in dry methanol (15 mL) was added *p*-methoxybenzylmercaptan (3.8 mL, 28 mmol) and triethylamine (1.4 mL, 10 mmol). This solution was refluxed for 15 min and cooled. The resulting solid was collected on a büchner funnel and washed with methanol (2 x 25 mL) to methyl 4-cyano-5-[(4-methoxyphenyl)methylthio]thiophene-2-carboxylate (2.84 g, 89 %) as a solid.

***b) Methyl 4-(aminothioxomethyl)-5-[(4-methoxyphenyl)methylthio]thiophene-2-carboxylate:*** Methyl 4-cyano-5-[(4-methoxyphenyl)methylthio]thiophene-2-carboxylate (2.5 g, 7.8 mmol) was treated as in Example 139, step (c) to give methyl 4-(aminothioxomethyl)-5-[(4-methoxyphenyl)methylthio]thiophene-2-carboxylate (1.32 g, 48 %) as a solid. ¹H-NMR (DMSO-d₆; 300 MHz) δ 9.64 (s, 1H), 9.28 (s, 1H), 8.08 (s, 1H), 7.35 and 6.92 (AB quartet, 2H, J=8.7 Hz), 4.27 (s, 2H), 3.82 (s, 3H), 3.75 (s, 3H).

***c) Methyl 5-(methoxyphenylthio)-4-(4-phenyl(1,3-thiazol-2-yl))thiophene-2-carboxylate:*** A solution of 1.2 g (3.4 mmol) of methyl 4-(aminothioxomethyl)-5-[(4-methoxyphenyl)methylthio]thiophene-2-carboxylate was reacted with 676 mg (3.4 mmol) of 2-bromoacetophenone (Aldrich, Milwaukee, WI, USA) in a manner similar to Example 8, step (a) to give methyl 5-(methoxyphenylthio)-4-(4-phenyl(1,3-thiazol-2-yl))thiophene-2-carboxylate (755 mg, 49 %) as a solid. ¹H-NMR (DMSO-d₆; 300 MHz) δ 8.26 (s, 1H), 8.22 (s, 1H), 8.04 (m, 2H), 7.48 (m, 2H), 7.38 (m, 1H), 7.33 and 6.89 (AB quartet, 2H, J=8.7 Hz), 4.40 (s, 2H), 3.86 (s, 3H), 3.72 (s, 3H).

***d) 5-(Methoxyphenylthio)-4-(4-phenyl(1,3-thiazol-2-yl))thiophene-2-carboxamidine:*** Methyl 5-(methoxyphenylthio)-4-(4-phenyl(1,3-thiazol-2-yl))thiophene-2-carboxylate (100 mg, 0.22 mmol) was treated in a manner similar to that for Example 10, step (b), to give 5-methoxy-4-(4-phenyl(1,3-thiazol-2-yl))thiophene-2-carboxamidine (94 mg, 91 %) as an orange solid. ¹H-NMR (DMSO-d₆; 300 MHz) δ 9.49 (br s, 2H), 9.15 (br s, 2H), 8.70 (s, 1H), 8.26 (s, 1H), 8.07 (m, 2H), 7.49 (m, 2H), 7.40 (m, 1H), 7.35 and 6.90 (AB quartet, 2H, J=8.7 Hz), 4.41 (s, 2H), 3.73 (s, 3H). Mass spectrum (MALDI-TOF, CHCA matrix, m/z): Calcd. for C₂₂H₁₉N₃OS₃ 438.5 (M+H), found 438.1.

### Example 141

***a) Methyl 4-[4-(4-chlorophenyl)(1,3-thiazol-2-yl)]-5-(methylsulfonyl)thiophene-2-carboxylate:*** To a stirred solution of 1 g (2.6 mmol) of methyl 4-[4-(4-chlorophenyl)(1,3-thiazol-2-yl)]-5-methylthiothiopliene-2-carboxylate (Maybridge, Cornwall, UK) was dissolved in dry dichloromethane (50 mL) and treated with m-chloroperbenzoic acid (1.94 g, 11.3 mmol) at room temperature for 1.5 h. The solution was filtered and the solid washed with dichloromethane. The filtrate was washed with sodium bicarbonate solution (2 x 20 mL), sodium thiosulfate solution (20 mL), sodium bicarbonate solution (20 mL), brine (20 mL), and dried over anhydrous sodium sulfate. The solvent was removed *in vacuo* to give methyl 4-[4-(4-chlorophenyl)(1,3-thiazol-2-yl)]-5-(methylsulfonyl)thiophene-2-carboxylate (826 mg, 77%) as a tan solid. Mass spectrum (MALDI-TOF, CHCA matrix, m/z): Calcd. for C₁₆H₁₂NO₄S₃Cl 414.0 (M+H), found 414.8.

***b) 4-[4-(4-Chlorophenyl)(1,3-thiazol-2-yl)]-5-(methylsulfonyl)thiophene-2-carboxamidine:*** Methyl 4-[4-(4-chlorophenyl)(1,3-thiazol-2-yl)]-5-(methylsulfonyl)thiophene-2-carboxylate (200 mg, 0.4 mmol) was treated in a manner similar to that for Example 10, step (b), to give 4-[4-(4-chlorophenyl)(1,3-thiazol-2-yl)]-5-(methylsulfonyl)thiophene-2-carboxamidine (85 mg, 53 %) as a yellow solid.

***c) 4-[4-(4-Chlorophenyl)(1,3-thiazol-2-yl)]-5-(phenylmethylthio)thiophene-2-carboxamidine:*** A stirred solution of 80 mg (0.2 mmol) of 4-[4-(4-chlorophenyl)(1,3-thiazol-2-yl)]-5-(methylsulfonyl)thiophene-2-carboxamidine benzyl mercaptan (115 µl, 0.980 µmol) was treated in a manner similar to that for Example 140, step (a) to give, after silica gel column chromatography (20 g) eluting with dichloromethane:methanol:acetic acid 9/1/0.5 (v:v:v), 4-[4-(4-chlorophenyl)(1,3-thiazol-2-yl)]-5-(phenylmethylthio)thiophene-2-carboxamidine (75 mg, 85 %) as a pale orange solid. ¹H-NMR (DMSO-d₆; 300 MHz) δ 9.44 (br s, 2H), 9.03 (br s, 2H), 8.67 (s, 1H), 8.33 (s, 1H), 8.08 and 7.56 (AB quartet, 2H, J=8.7 Hz), 7.54-7.17 (m, 5H), 4.45 (s, 2H). Mass spectrum (MALDI-TOF, CHCA matrix, m/z): Calcd. for C₂₁H₁₆N₃S₃Cl 442.0 (M+H), found 442.7.

### Example 142

***a) 1-[5-(tert-butyl)-2-methyl(3-furyl)]-2-bromoethan-1-one:*** A solution of 1 g (5 mmol) of 5-(tert-butyl)-2-methylfuran-3-carbonyl chloride (Maybridge, Cornwall, UK) dissolved in dry acetonitrile (4 mL) and 6.25 mL (12.5 mmol) of 2 M trimethylsilyldiazomethane in hexanes (Aldrich, Milwaukee, WI) was stirred 1.75 h at room temperature and the mixture was cooled on an ice bath for 5 min. To this, 30% hydrogen bromide in acetic acid (2 mL, 10 mmol) was added dropwise over 10 min. This was stirred an additional 20 minutes on an ice bath. Evaporation of the solvents gave 1-[5-(tert-butyl)-2-methyl(3-furyl)]-2-bromoethan-1-one (1 g, 77 %) as a brown oil. ¹H-NMR (DMSO-d₆; 300 MHz) δ 6.50 (s, 1H), 4.57(s, 2H), 2.52 (s, 1H), 1.24 (s, 9H). Mass spectrum (LCA, m/z): Calcd. for C₁₁H₁₅O₂Br, 259.1 and 261.1 (M+H), found 259. and 261.1.

***b) Methyl 4-{4-[5-(tert-butyl)-2-methyl(3-furyl)](1,3-thiazol-2-yl)}-5-methylthiothiophene-2-carboxylate:*** A solution of 955 mg (3.86 mmol) of methyl 4-(aminothioxomethyl)-5-methylthiothiophene-2-carboxylate (Maybridge, Cornwall, UK) was reacted with 1 g (3.86 mmol) of 1-[5-(tert-butyl)-2-methyl(3-furyl)]-2-bromoethan-1-one (1 g) in a manner similar to Example 8, step (a) to give methyl 4-{4-[5-(tert-butyl)-2-methyl(3-furyl)](1,3-thiazol-2-yl) }-5-methylthiothiophene-2-carboxylate (999 mg, 64 %) as a red-brown solid. ¹H-NMR (DMSO-d₆; 300 MHz) δ 8.14 (s, 1H), 7.74 (s, 1H), 6.46 (s, 1H), 3.86 (s, 3H), 2.74 (s, 3H), 2.66 (s, 3H), 1.27 (s, 9H). Mass spectrum (MALDI-TOF, CHCA matrix, m/z): Calcd. for C₁₉H₂₁NO₃S₃, 408.1 (M+H), found 408.0.

***c) 4-{4-[5-(tert-Butyl)-2-methyl(3-furyl)}(1,3-thiazol-2-yl)}-5-methylthiothiophene-2-carboxamidine:*** Methyl 4-{4-[5-(tert-butyl)-2-methyl(3-furyl)](1,3-thiazol-2-yl)}-5-methylthiothiophene-2-carboxylate (940 mg, 2.3 mmol) was treated in a manner similar to that for Example 10, step (b) to give 4-{4-[5-(tert-butyl)-2-methyl(3-furyl)](1,3-thiazol-2-yl)}-5-methylthiothiophene-2-carboxamidine (930 mg, quantitive yield) as a yellow solid. ¹H-NMR (DMSO-d₆; 300 MHz) δ 9.42 (br s, 2H), 9.03 (br s, 2H), 8.59 (s, 1H), 7.77 (s, 1H), 6.47 (s, 1H), 2.78 (s, 3H), 2.68 (s, 3H), 1.27 (s, 9H). Mass spectrum (MALDI-TOF, CHCA matrix, m/z): Calcd. for C₁₈H₂₁N₃OS₃, 392.1 (M+H), found 392.1.

### Example 143

***a) 1-[3-(tert-butyl)-1-benzylpyrazol-5-yl]-2-bromoethan-1-one:*** A solution of 1 g (3.6 mmol) of 3-(tert-butyl)-1-benzylpyrazole-5-carbonyl chloride (Maybridge, Cornwall, UK) was dissolved in dry acetonitrile (4 mL) and 4.5 mL (9 mmol) of 2 M trimethylsilyldiazomethane in hexanes (Aldrich, Milwaukee, WI, USA) was added. After stirring 1 h 20 min at room temperature, the mixture was cooled on an ice bath for 5 min. To this, 30% hydrogen bromide in acetic acid (2 mL, 10 mmol) was added dropwise over 15 min. This was stirred an additional 15 minutes on an ice bath. Filtration of the precipitated solid and evaporation of the solvents gave 1-[3-(tert-butyl)-1-benzylpyrazol-5-yl]-2-bromoethan-1-one (1.47 g, quantitive yield) as an orange solid. ¹H-NMR (DMSO-d₆; 300 MHz) δ 7.33-7.06 (m, 5H), 7.08 (s, 1H), 5.64 (s, 2H), 4.57 (s, 2H), 1.28 (s, 9H). Mass spectrum (MALDI-TOF, CHCA matrix, m/z): Calcd. for C₁₆H₁₉N₂OBr, 335.1 and 337.1 (M+H), found 335.6 and 337.6.

***b) Methyl 4-{4-[3-(tert-butyl)-1-benzylpyrazol-5-yl](1,3-thiazol-2-yl)}-5-methylthiothiophene-2-carboxylate:*** A solution of 823 mg (3.3 mmol of methyl 4-(aminothioxomethyl)-5-methylthiothiophene-2-carboxylate (Maybridge, Cornwall, UK) was reacted with 1.36 g (3.3 mmol) of 1-[3-(tert-butyl)-1-benzylpyrazol-5-yl]-2-bromoethan-1-one in a manner similar to Example 8, step (a) to give methyl 4-{4-[3-(tert-butyl)-1-benzylpyrazol-5-yl](1,3-thiazol-2-yl)}-5-methylthiothiophene-2-carboxylate (1.25 g, 79 %) as a crystalline solid. ¹H-NMR (DMSO-d₆; 300 MHz) δ 8.11 (s, 1H), 8.05 (s, 1H), 7.28-6.99 (m, 5H), 6.70 (s, 1H), 5.88 (s, 2H), 3.86 (s, 3H), 2.70 (s, 3H), 1.30 (s, 9H). Mass spectrum (MALDI-TOF, CHCA matrix, m/z): Calcd. for C₂₄H₂₅N₃O₂S₃, 484.1 (M+H), found 483.9.

***c) 4-{4-[3-(Tert-butyl)-1-benzylpyrazol-5-yl](1,3-thiazol-2-yl)}-5-methylthiothiophene-2-carboxamidine:*** Methyl 4-{4-[3-(tert-butyl)-1-benzylpyrazol-5-yl](1,3-thiazol-2-yl)}-5-methylthiothiophene-2-carboxylate (1.2 mg, 2.6 mmol) was treated in a manner similar to that for Example 10, step (b) to give 4-{4-[3-(tert-butyl)-1-benzylpyrazol-5-yl](1,3-thiazol-2-yl)}-5-methylthiothiophene-2-carboxamidine (1.21 g, quantitive yield) as a yellow solid. ¹H-NMR (DMSO-d₆; 300 MHz) δ 9.43 (br s, 1H), 9.07 (br s, 1H), 8.60 (s, 1H), 8.04 (s, 1H), 7.37-6.97 (m, 5H), 6.70 (s, 1H), 5.92 (s, 2H), 2.73 (s, 3H), 1.30 (s, 9H). Mass spectrum (MALDI-TOF, CHCA matrix, m/z): Calcd. for C₂₃H₂₅N₅S₃, 468.1 (M+H), found 468.1.

### Example 144

***a) 4-Bromo-5-methylthiophene-2-carboxylic acid:*** A stirred solution of 1 g (3.9 mmol) of 2-methyl-3,5-dibromothiophene (prepared by the method of Kano, *S.et al., Heterocycles* 20(10):2035, 1983) in dry tetrahydrofuran (10 mL) was cooled to -78°C and 2 M *n*-butyllithium in cyclohexane (1.93 mL, 3.87 mmol) was added over 3 min. After stirring 3 min at -78°C, the mixture was added to tetrahydrofuran (100 mL) with dry ice suspended. This mixture was allowed to stir and warm to room temperature. To this, 6 N hydrochloric acid (50 mL) was added carefully. Then, water (50 mL) was added and the layers were separated. The aqueous layer was extracted with diethyl ether (4 x 30 mL). The combined organic layers were washed with water, brine, and dried over anhydrous sodium sulfate. The solvents were removed *in vacuo* to give an 85/15 mixture of 4-bromo-5-methylthiophene-2-carboxylic acid and 5-bromothiophene-2-carboxylic acid (780 mg, 90 %) as a tan solid. ¹H-NMR (DMSO-d₆; 300 MHz) δ 13.33 (br s, 1H), 7.62 (s, 1H), 7.56 and 7.34 (AB quartet, 0.35H, J=3.9 Hz), 2.41 (s, 3H). Gas Chromotography/Mass spectroscopy (m/z): Calcd. for C₆H₅O₂SBr, 220.9 and 222.9 (M+H), found 221.3 and 223.3. Calcd. for C₅H₃O₂SBr, 206.9 and 208.9 (M+H), found 207.3 and 209.3.

***b) Methyl 4-bromo-5-methylthiophene-2-carboxylate:*** A solution of 780 mg (3.5 mmol) of an 85/15 mixture of 4-bromo-5-methylthiophene-2-carboxylic acid and 5-bromothiophene-2-carboxylic acid was dissolved in methanol (50 mL) and treated with 9 ml (18 mmol) 2 M trimethylsilyldiazomethane in hexanes (Aldrich, Milwaukee, WI, USA). Evaporation of the solvents gave an 8/2 mixture of methyl 4-bromo-5-methylthiophene-2-carboxylate and methyl 5-bromothiophene-2-carboxylate (858 mg, quantitive yield) as a brown oil. Gas Chromotography/Mass spectroscopy (m/z): Calcd. for C₇H₇O₂SBr, 234.9 and 236.9 (M+H), found 235.3 and 237.3. Calcd. for C₆H₄O₂SBr, 220.9 and 222.9 (M+H), found 221.3 and 223.3.

***c) Methyl 4-cyano-5-methylthiophene-2-carboxylate:*** A solution of an 8/2 mixture of 823 mg (3.5 mmol) of methyl 4-bromo-5-methylthiophene-2-carboxylate and methyl 5-bromothiophene-2-carboxylate was dissolved in dry dimethylformamide (5 mL) and refluxed with copper cyanide (345 mg, 3.9 mmol) for 7 hours. The cooled solution was poured into 0.1 M aqueous sodium cyanide solution (200 mL) and extracted with diethyl ether (5 x 30 mL). The organic layers were washed with brine (2 x 30 mL), dried over anhydrous sodium sulfate, and the solvents removed *in vacuo.* The resulting brown solid was purified by column chromatography on silica gel eluting with hexanes:ethyl acetate 9/1 (v:v) to give a 95/5 mixture of methyl 4-cyano-5-methylthiophene-2-carboxylate and methyl 5-methylthiophene-2-carboxylate (369 mg, 68 %) as a yellow solid. ¹H-NMR (DMSO-d₆; 300 MHz) δ 8.06 (s, 1H), 8.05 and 7.90 (2H, 0.1 H, J=4.0 Hz, minor component), 3.87 (s, 3H, minor component), 3.84 (s, 3H) 2.68 (s, 3H).

***d) Methyl 4-(aminothioxomethyl)-5-methylthiophene-2-carboxylate:*** A stirred solution of 804 mg (4.4 mmol) of methyl 4-cyano-5-methylthiophene-2-carboxylate was treated in a manner similar to Example 139, step (c) to give, after fractional crystallization ethanol of the unreacted starting nitrile, a 2:3 ratio of methyl 4-(aminothioxomethyl)-5-methylthiophene-2-carboxylate and methyl 4-cyano-5-methylthiophene-2-carboxylate (457 mg, 48 %) as a light brown solid. ¹H-NMR (DMSO-d₆; 300 MHz) δ 9.93 (br s, 1H, minor), 9.34 (br s, 1H, minor), 8.06 (s, 1H, major), 7.77 (s, 1H, minor component), 3.84 (s, 3H, minor), 3.81 (s, 3H, major), 2.68 (s, 3H, major), 2.61 (s, 2H, minor). Mass spectrum (MALDI-TOF, CHCA matrix, m/z): Calcd. for C₈H₉NO₂S₂ 216.0 (M+H), found 216.4.

***e) Methyl 5-methyl-4-(4-phenyl(1,3-thiazol-2-yl))thiophene-2-carboxylate:*** A solution of 200 mg (0.93 mmol) of methyl 4-(aminothioxomethyl)-5-methylthiophene-2-carboxylate was reacted with 185 mg (0.93 mmol) of 2-bromoacetophenone in a manner similar to Example 8, step (a) to give, after purification by preparative thin layer chromatography eluting with hexanes:ethyl acetate 7/3 (v:v), a mixture of methyl 5-methyl-4-(4-phenyl(1,3-thiazol-2-yl))thiophene-2-carboxylate and methyl 4-cyano-5-methylthiophene-2-carboxylate (96 mg, 36 %) as a solid.

***f) 5-Methyl-4-(4-phenyl(1,3-thiazol-2-yl))thiophene-2-carboxamidine:*** Methyl-4-(4-phenyl(1,3-thiazol-2-yl))thiophene-2-carboxylate (64 mg, 0.23 mmol) was treated in a manner similar to Example 10, step (b) to give, after preparative high pressure liquid chromatography (Dynamax C18 column, 300Å pore size, 10 µm particle size, 40% to 100% acetonitrile over 30 minutes in 0.1% aqueous trifluoroacetic acid) 5-methyl-4-(4-phenyl(1,3-thiazol-2-yl))thiophene-2-carboxamidine (0.6 mg, 0.9 %) as an an off-white solid. ¹H-NMR (Methanol-d₄; 300 MHz) δ 8.44 (s, 1H), 8.02 (m, 2H), 7.92 (s, 1H), 7.45 (m, 2H), 7.36 (m, 1H), 2.96 (s, 3H). Mass spectrum (MALDI-TOF, CHCA matrix, m/z): Calcd. for C₁₅H₁₃N₃S₂ 300.1 (M+H), found 300.6.

***g) 5-(4-phenyl-1,3-thiazol-2-yl)thiophene-2-carboxamide:*** From the HPLC purified mixture in the previous step was isolated 5-(4-phenyl-1,3-thiazol-2-yl)thiophene-2-carboxamide as an off-white solid (2 mg). ¹H-NMR (Methanol-d₄; 300 MHz) δ 7.99 (m, 2H), 7.97 (s, 1H), 7.95 and 7.78 (AB quartet, 2H, J=4.2 Hz), 7.48-7.35 (m, 3H). Mass spectrum (MALDI-TOF, CHCA matrix, m/z): Calcd. for C₁₄H₁₁N₃S₂ 286.0 (M+H), found 286.2.

### Example 145

***a) Methyl 4-[4-(3,4-dimethoxyphenyl)(1,3-thiazol-2-yl)]-5-methylthiophene-2-carboxylate:*** A solution of 257 mg (0.48 mmol, based on a mixture containing 60% nitrile) of methyl 4-(aminothioxomethyl)-5-methylthiophene-2-carboxylate was reacted with 124 mg (0.48 mmol) of 2-bromo-(3',4'-dimethoxy)-acetophenone (Example 31, step (a)) was reacted in a manner similar to Example 8, step (a) to give methyl 4-[4-(3,4-dimethoxyphenyl)(1,3-thiazol-2-yl)]-5-methylthiophene-2-carboxylate (95 mg, 53 %) as a solid. Mass spectrum (MALDI-TOF, CHCA matrix, m/z): Calcd. for C₁₈H₁₇NO₄S₂ 376.1 (M+H), found 376.3.

***b) 4-[4-(3,4-dimethoxyphenyl)(1,3-thiazol-2-yl)]-5-methylthiophene-2-carboxamide:*** Methyl 4-[4-(3,4-dimethoxyphenyl)(1,3-thiazol-2-yl)]-5-methylthiophene-2-carboxylate (95 mg, 0.25 mmol) was treated in a manner similar to Example 10, step (b) to give 4-[4-(3,4-dimethoxyphenyl)(1,3-thiazol-2-yl)]-5-methylthiophene-2-carboxamide (8 mg, 9 %) as a yellow solid. 'H-NMR (Methanol-d₄; 300 MHz) δ 8.42 (s, 1H), 7.81 (s, 1H), 7.61 (m, 2H), 7.03 (m, 1H), 3.92 (s, 3H), 3.88 (s, 3H), 2.95 (s, 3H). Mass spectrum (MALDI-TOF, CHCA matrix, m/z): Calcd. for C₁₇H₁₇N₃O₂S₂ 360.1 (M+H), found 360.2.

### Example 146

***a) 4-Bromo-5-methylthiophene-2-carboxylic acid:*** A solution of 27.65 g (108 mmol) of 2-methyl-3,5-dibromothiophene (prepared by the method of Kano, *S.et al., Heterocycles* 20(10):2035, 1983) was dissolved in dry tetrahydrofuran (280 mL), cooled to -78°C and 2 M n-butyl lithium in cyclohexane (54 mL, 108 mmol) was added over 10 min. After stirring 20 min at -78°C, dry carbon dioxide gas was bubbled through the solution for 1.5 h as the mixture was allowed to warm to room temperature. To this 6 N hydrochloric acid (100 mL) was added carefully. The layers were separated and the aqueous layer was extracted with diethyl ether (4 x 50 mL). The combined organic layers were washed with brine, and dried over anhydrous sodium sulfate. The solvents were removed *in vacuo* to give 4-bromo-5-methylthiophene-2-carboxylic acid (22.4 g, 94 %) as an off-white solid. ¹H-NMR (DMSO-d₆; 300 MHz) δ 13.34 (br s, 1H), 7.61 (s, 1H), 2.41 (s, 3H).

***b) Isopropyl 4-bromo-5-methylthiophene-2-carboxylate:*** A solution of 5 g (22.6 mmol) of 4-bromo-5-methylthiophene-2-carboxylic acid was dissolved in dry dichloromethane (200 mL) and reacted with oxalyl chloride (2 mL, 22.6 mmol) and dimethylformamide (100 µL) stirring on an ice bath for 30 min and then at room temperature for 2.5 h. The solvents were removed *in vacuo* and the residue was passed through silica gel, eluting off with hexanes:ethyl acetate 7/3 (v:v), ethyl acetate, and dichloromethane. The solvents were removed *in vacuo* and the resulting oil dissolved in dry dichloromethane (100 mL). This solution was reacted with dry pyridine (9 mL, 113 mmol) and dry isopropanol (40 mL, 522 mmol) for 88 h. The solvents were removed *in vacuo* and the residue partitioned between sodium bicarbonate (150 mL) and dichloromethane (75 mL). The aqueous layers were extracted with dichloromethane (2 x 20 mL), and the combined organic layers were washed with sodium bicarbonate (30 mL), brine (30 mL), and dried over anhydrous sodium sulfate. The solvents were removed *in vacuo*. The residue was purified by column chromatography eluting with hexanes:ethyl acetate 9/1 (v:v) to give isopropyl 4-bromo-5-methylthiophene-2-carboxylate (1.91 g, 32 %) as a pale yellow oil. ¹H-NMR (DMSO-d₆; 300 MHz) δ 7.66 (s, 1H), 5.07 (septet, 1H, J=6.2 Hz), 2.42 (s, 3H), 1.29 (d, 6H, J=6.2 Hz). Mass spectrum (ESI, m/z): Calcd. for C₉H₁₁O₂SBr 264.2 (M+H), found 264.8.

***c) Isopropyl 4-cyano-5-methylthiophene-2-carboxylate:*** A stirred solution of 1.9 g (7.3 mmol) of isopropyl 4-bromo-5-methylthiophene-2-carboxylate was dissolved in dry dimethylformamide (30 mL) and refluxed with copper cyanide (785 mg, 8.8 mmol) for 16 hours. The cooled solution was poured into 0.1 M aqueous sodium cyanide solution (300 mL) and extracted with diethyl ether (4 x 40 mL). The organic layers were washed with brine (2 x 40 mL), dried over anhydrous sodium sulfate, and the solvents removed *in vacuo.* Column chromatography on silica gel eluting with hexanes:ethyl acetate 9/1 (v:v), gave isopropyl 4-cyano-5-methylthiophene-2-carboxylate (960 mg, 63 %) as a yellow crystalline solid ¹H-NMR (DMSO-d₆; 300 MHz) δ 8.01 (s, 1H), 5.09 (septet, 1H, J=6.2 Hz), 2.67 (s, 3H), 1.29 (d, 6H, J=6.2 Hz).

***d) Isopropyl 4-(aminothioxomethyl)-5-methylthiophene-2-carboxylate:*** A stirred solution of 960 mg (4.59 mmol) of isopropyl 4-cyano-5-methylthiophene-2-carboxylate was treated in a manner similar to Example 139, step (c) to give, after crystallization from diethyl ether, isopropyl 4-(aminothioxomethyl)-5-methylthiophene-2-carboxylate (623 mg, 56 %) as a solid. ¹H-NMR (DMSO-d₆; 300 MHz) δ 9.93 (br s, 1H), 9.34 (br s, 1H), 7.54 (s, 1H), 5.07 (septet, 1H, J=6.2 Hz), 2.60 (s, 3H), 1.29 (d, 6H, J=6.2 Hz). Mass spectrum (MALDI-TOF, GA matrix, m/z): Calcd. for C₁₀H₁₃NO₂S₂ 244.0 (M+H), found 243.8.

***e) Isopropyl 5-methyl-4-(4-phenyl(1,3-thiazol-2-yl))thiophene-2-carboxylate:*** A solution of 375 mg (1.54 mmol) of isopropyl 4-(aminothioxomethyl)-5-methylthiophene-2-carboxylate was reacted with 307 mg (1.54 mmol) of 2-bromoacetophenone (Aldrich, Milwaukee, WI, USA) in a manner similar to Example 8, step (a) to give, after crystallization from methanol, isopropyl 5-methyl-4-(4-phenyl(1,3-thiazol-2-yl))thiophene-2-carboxylate (347 mg, 66%) as light brown needles. ¹H-NMR (DMSO-d₆; 300 MHz) δ 8.23 (s, 1H), 8.09 (s, 1H), 8.05 (m, 2H), 7.49 (m, 2H), 7.38 (m, 1H), 5.13 (septet, 1H, J=6.2 Hz), 2.86 (s, 3H), 1.33 (d, 6H, J=6.2 Hz). Mass spectrum (ESI, m/z): Calcd. for C₁₈H₁₇NO₂S₂ 344.1 (M+H), found 344.1.

***f) 5-methyl-4-(4-phenyl(1,3-thiazol-2-yl))thiophene-2-carboxamidine:*** Isopropyl 5-methyl-4-(4-phenyl(1,3-thiazol-2-yl))thiophene-2-carboxylate (340 mg, 0.99 mmol) was treated in a manner similar to Example 10, step (b) to give 5-methyl-4-(4-phenyl(1,3-thiazol-2-yl))thiophene-2-carboxamidine (360 mg, quantitive yield) as a yellow solid. This material was dissolved in dry methanol (20 mL) and treated with 1 M HCl (g) in diethyl ether. Evaporation of the solvents *in vacuo* and recrystallization from methanol gave the hydrochloride salt of 5-methyl-4-(4-phenyl(1,3-thiazol-2-yl))thiophene-2-carboxamidine (252 mg, 76 %) as a light brown crystalline solid. ¹H-NMR (DMSO-d₆; 300 MHz) δ 9.45 (br s, 2H), 9.10 (br s, 2H), 8.56 (s, 1H), 8.27 (s, 1H), 8.06 (m, 2H), 7.50 (m, 2H), 7.40 (m, 1H), 2.93 (s, 3H). Mass spectrum (ESI, m/z): Calcd. for C₁₅H₁₃N₃S₂ 300.1 (M+H), found 300.2.

### Example 147

***a) 2-Methyl-5-[(methylethyl)oxycarbonyl]thiophene-3-carboxylic acid:*** A stirred mixture of 500 mg (2.39 mmol) of isopropyl 2-methyl-3-cyanothiophene-5-carboxylate and tetrafluorophthalic acid (570 mg, 2.39 mmol) was heated in a glass bomb at 160°C for 66 hours. The cooled residue was digested in hot chloroform (30 mL), treated with norite, and filtered through celite. The celite was washed with hot chloroform (30 mL). The cooled chloroform extracts were filtered and extracted with saturated sodium bicarbonate (4 x 10 mL). The basic extracts were washed with chloroform, filtered through celite, and acidified to pH 1 with concentrated hydrochloric acid. The solid was collected by filtration and washed with water (3 x 10 mL) to give 2-methyl-5-[(methylethyl)oxycarbonyl]thiophene-3-carboxylic acid (288 mg, 53 %) as a light brown solid. ¹H-NMR (DMSO-d₆; 300 MHz) δ 13.03 (br s, 1H), 7.85 (s, 1H), 5.08 (septet, 1H, J=6.2 Hz), 2.71 (s, 3H), 1.29 (d, 6H, J=6.2 Hz). Mass spectrum (ESI, m/z): Calcd. for C₁₀H₁₂O₄S 229.1 (M+H), found 228.8

***b) Isopropyl 4-(2-bromoacetyl)-5-methylthiophene-2-carboxylate:*** A stirred solution of 300 mg (1.3 mmol) of 2-methyl-5-[(methylethyl)oxycarbonyl]thiophene-3-carboxylic acid was dissolved in dry dichloromethane (10 mL) and treated with oxalyl chloride (174 µL, 2 mmol) and dimethylformamide (50 µL). The mixture was stirred at room temperature for 1.25 h, the solvents removed *in vacuo,* and the residue passed through silica gel (1 inch in a 60 mL sintered-glass Büchner funnel) and eluted off with dichloromethane (150 mL). This material was treated in a manner similar to Example 142, step (a) to give isopropyl 4-(2-bromoacetyl)-5-methylthiophene-2-carboxylate (266 mg, 67 %) as a solid.

***c) Isopropyl 4-(2-amino(1,3-thiazol-4-yl))-5-methylthiophene-2-carboxylate:*** A solution of 260 mg (0.85 mmol) of isopropyl 4-(2-bromoacetyl)-5-methylthiophene-2-carboxylate was reacted with 65 mg (0.85 mmol) of thiourea in a manner similar to Example 8, step (a) to give isopropyl 4-(2-amino(1,3-thiazol-4-yl))-5-methylthiophene-2-carboxylate (257 mg, quantitive yield) as a white solid. ¹H-NMR (DMSO-d₆; 300 MHz) δ 7.90 (s, 1H), 6.93 (s, 1H), 5.09 (septet, 1H, J=6.2 Hz), 2.61 (s, 3H), 1.29 (d, 6H, J=6.2 Hz). Mass spectrum (ESI, m/z): Calcd. for C₁₂H₁₄N₂O₂S₂ 283.1 (M+H), found 283.1

***d) 4-(2-Amino(1,3-thiazol-4-yl))-5-methylthiophene-2-carboxamidine:*** Isopropyl 4-(2-amino(1,3-thiazol-4-yl))-5-methylthiophene-2-carboxylate (240 mg, 0.85 mmol) was treated in a manner similar to Example 10, step (b) to give 4-(2-amino(1,3-thiazol-4-yl))-5-methylthiophene-2-carboxamidine (20 mg, 10 %) as a solid. ¹H NMR (DMSO-d₆, 300 MHz): δ 9.30 (br s, 2H), 8.99 (bs, 2H), 8.28 (s, 1H), 6.78 (s, 1H), 2.71 (s, 3H); Mass Spectrum (ESI, m/z) calcd. for C₉H₁₀N₄S₂, 238.8 (M+H), found 239.2.

### Example 148

***a) 4-Bromo-5-ethylthiophene-2-carboxylic acid:*** A stirred solution of 10 g (35 mmol) of 4,5-dibromothiophene-2-carboxylic acid (Lancaster, Windham, NH, USA) in dry THF (100 mL) was cooled to -78°C. To this, 35 mL (70 mmol) of 2.0 M n-butyllithium in cyclohexane (Aldrich, Milwaukee, WI, USA) was added dropwise over 15 min, and the reaction was allowed to stir for 15 min at -78°C. The mixture was quenched with ethyl iodide (2.8 mL, 35 mmol) and allowed to warm to room temperature. The mixture was carefully poured into 6N hydrochloric acid (100 mL) and extracted with diethyl ether (4 x 50 mL). The organic layers were washed with water (2 x 50 mL), brine (50 mL), and dried over anhydrous sodium sulfate. The solvents were removed *in vacuo* to give 2-ethyl-3-bromo-thiophene-5-carboxylate (7 g, 85 %) as a dark solid. ¹H-NMR (DMSO-d₆; 300 MHz) δ 13.25 (br s, 1H), 7.62 (s, 1H), 2.80 (q, 2H, J=7.5 Hz), 1.23 (t, 3H, J=7.5 Hz).

***b) Isopropyl 4-bromo-5-ethylthiophene-2-carboxylate:*** A solution of 7 g (30 mmol) of 4-bromo-5-ethylthiophene-2-carboxylic acid was dissolved in dry dichloromethane (200 mL) and treated with oxalyl chloride (3.2 mL, 36 mmol) and dimethylformamide (0.5 mL) for 18.5 h. The solvents were removed *in vacuo* and the residual brown oil was passed through silica gel (2 inches in a 350 mL scintered-glass Büchner funnel) and eluted with 700 mL of hexanes:ethyl acetate 9/1 (v:v). The elutate was concentrated *in vacuo* and the oil dissolved in dry dichloromethane (200 mL). This solution was treated with pyridine (12 mL, 150 mmol) and dry isopropanol (60 mL, 750 mmol) for 4 h at room temperature. The solvents were removed *in vacuo* and the residue partioned between dichloromethane (100 mL) and water (200 mL). The aqueous layers were extracted with dichloromethane (2 x 30 mL). The combined organic layers were extracted with sodium bicarbonate (2 x 30 mL), brine (30 mL), and dried over anhydrous sodium sulfate. The solvent was removed *in vacuo.* Purification by column chromatography on silica gel (250 g) eluting with hexanes:ethyl acetate 95/5 (v:v) gave isopropyl 2-ethyl-3-bromo-thiophene-5-carboxylate (4 g, 48 %) as a yellow oil. ¹H-NMR (DMSO-d₆; 300 MHz) δ 7.66 (s, 1H), 5.89 (septet, 1H, J=6.2 Hz), 2.80 (q, 2H, J=7.5 Hz), 1.29 (d, 6H, J=6.0 Hz), 1.24 (t, 3H, J=7.5 Hz).

***c) Isopropyl 4-cyano-5-ethylthiophene-2-carboxylate:*** A stirred solution of 4 g (14.4 mmol) of isopropyl 4-bromo-5-ethylthiophene-2-carboxylate was refluxed in dry dimethylformamide (50 mL) with copper cyanide (1.94 g, 22 mmol) for 8 hours. The cooled mixture was poured into 0.1 M sodium cyanide (500 mL) and extracted with diethyl ether (4 x 50 mL). The organic layers were washed twice with brine (50 mL) and dried over anhydrous sodium sulfate. The solvents were removed *in vacuo*. Column chromatography on silica gel (400 g), eluting with hexanes:ethyl acetate 9/1 (v:v) gave isopropyl 2-ethyl-3-cyano-thiophene-5-carboxylate (1.7 g, 53 %) as a pale yellow oil. ¹H-NMR (DMSO-d₆; 300 MHz) δ 8.03 (s, 1H), 5.10 (septet, 1H, J=6.2 Hz), 3.04 (q, 2H, J=7.5 Hz), 1.31 (t, 3H, J=7.5 Hz), 1.30 (d, 6H, J=6.2 Hz). Mass spectrum (ESI m/z): Calcd. for C₁₁H₁₃NO₂S 224.1 (M+H), found 224.0.

***d) Isopropyl 4-(aminothioxomethyl)-5-ethylthiophene-2-carboxylate:*** A stirred solution of 1.7 g (7.6 mmol) of isopropyl 4-cyano-5-ethylthiophene-2-carboxylate was treated as in Example 139, step (c) to give isopropyl 5-ethyl-4-(aminothioxomethyl)-5-ethylthiophene-2-carboxylate (1.45 g, 74 %) as a yellow solid. ¹H-NMR (DMSO-d₆; 300 MHz) δ 9.93 (br s, 1H), 9.39 (br s, 1H), 8.04 (s, 1H), 5.08 (septet, 1H, J=6.2 Hz), 3.08 (q, 2H, J=7.5 Hz), 1.29 (d, 6H, J=6.2 Hz), 1.24 (t, 3H, J=7.5 Hz).

***e) Isopropyl 5-ethyl-4-(4-phenyl(1,3-thiazol-2-yl))thiophene-2-carboxylate:*** A solution of 450 mg (1.75 mmol) of isopropyl 5-ethyl-4-(aminothioxomethyl)-5-ethylthiophene-2-carboxylate was reacted with 348 mg (1.75 mmol) of 2-bromoacetophenone (Aldrich, Milaukee, WI, USA) in a manner similar to Example 8, step (a) to give isopropyl 5-ethyl-4-(4-phenyl(1,3-thiazol-2-yl))thiophene-2-carboxylate (303 mg, 49%) as an off-white solid. ¹H-NMR (DMSO-d₆; 300 MHz) δ 8.22 (s, 1H), 8.07 (s, 1H), 8.03 (m, 2H), 7.49 (m, 2H), 7.38 (m, 1H), 5.13 (septet, 1H, J=6.2 Hz), 3.34 (q, 2H, J=7.4 Hz), 1.39 (t, 3H, J=7.4 Hz), 1.33 (d, 6H, J=6.2 Hz). Mass spectrum (ESI, m/z): Calcd. for C₁₉H₁₉NO₂S₂ 358.1 (M+H), found 358.1.

***f) 5-Ethyl-4-(4-phenyl(1,3-thiazol-2-yl))thiophene-2-carboxamidine:*** Isopropyl 5-ethyl-4-(4-phenyl(1,3-thiazol-2-yl))thiophene-2-carboxylate (250 mg, 0.70 mmol) was treated in a manner similar to that for Example 10, step (b), to give 5-ethyl-4-(4-phenyl(1,3-thiazol-2-yl))thiophene-2-carboxamidine (148 mg, 67 %) as a yellow solid. ¹H-NMR (DMSO-d₆; 300 MHz) δ 9.44 (br s, 2H), 9.07 (br s, 2H), 8.54 (s, 1H), 8.26 (s, 1H), 8.05 (m, 2H), 7.50 (m, 2H), 8.70 (s, 1H), 7.40 (m, 1H), 3.44 (q, 2H, J=7.4 Hz), 1.42 (t, 3H, J=7.4 Hz). Mass spectrum (ESI, m/z): Calcd. for C₁₆H₁₅N₃S₂ 314.1 (M+H), found 314.2.

### Example 149

***a) Isopropyl 4-[4-(3-hydroxyphenyl)(1,3-thiazol-2-yl)]-5-methylthiophene-2-carboxylate:*** A solution of 1.97 g (8.1 mmol) of isopropyl 4-(aminothioxomethyl)-5-methylthiophene-2-carboxylate was reacted with 1.74 g (8.1 mmol) of 3'-hydroxy-2-bromoacetophenone (Example 40, step (a)) were reacted in a manner similar to Example 8, step (a) to give, after column chromatography on silica gel eluting with hexane:ethyl acetate 7/3 (v:v), crystallization from acetonitrile, and recrystallization from hexanes, isopropyl 4-[4-(3-hydroxyphenyl)(1,3-thiazol-2-yl)]-5-methylthiophene-2-carboxylate (1.4 g, 48%) as brown solid. ¹H-NMR (DMSO-d₆; 300 MHz) δ 9.57 (br s, 1H), 8.14 (s, 1H), 8.08 (s, 1H), 7.46 (m, 2H), 7.26 (m, 1H),), 6.78 (m, 1H), 5.12 (septet, 1H, J=6.2 Hz), 2.85 (s, 3H), 1.33 (d, 6H, J=6.2 Hz). Mass spectrum (ESI, m/z): Calcd. for C₁₈H₁₇NO₃S₂ 360.1 (M+H), found 360.1.

***b) 4-[4-(3-Hydroxyphenyl)(1,3-thiazol-2-yl)]-5-methylthiophene-2-carboxamide:*** Isopropyl 4-[4-(3-hydroxyphenyl)(1,3-thiazol-2-yl)]-5-methylthiophene-2-carboxylate (1.4 g, 3.89 mmol) was treated in a manner similar to Example 10, step (b) to give 4-[4-(3-hydroxyphenyl)(1,3-thiazol-2-yl)]-5-methylthiophene-2-carboxamide (360 mg, 31 %) as a brown solid. ¹H-NMR (DMSO-d₆; 300 MHz) δ 9.62 (br s, 1H), 9.45 (br s, 2H), 9.09 (br s, 2H), 8.53 (s, 1H), 8.16 (s, 1H), 7.47 (m, 2H), 7.27 (m, 1H), 6.80 (m, 1H), 2.93 (s, 3H). Mass spectrum (ESI, m/z): Calcd. for C₁₅H₁₃N₃ OS₂ 316.1 (M+H), found 316.2.

### Example 150

***a) (Tert-butoxy)-N-({4-[4-(3-hydroxyphenyl)(1,3-thiazol-2-yl)]-5-methyl(2-thienyl)}iminomethyl)carboxamide:*** A stirred solution of 320 mg (1 mmol) of 4-[4-(3 -hydroxyphenyl)(1,3-thiazol-2-yl)]-5-methylthiophene-2-carboxamide was dissolved in dry dimethylformamide (50 mL) and treated with 262 mg (1.2 mmol) of di-tert-butyl-dicarbonate (Acros, Pittsburgh, PA, USA) and diisopropylethylamine (261 µL, 1.5 mmol) for 64 hours at room temperature. The mixture was poured into sodium bicarbonate solution (200 mL) and extracted with dichloromethane (6 x 30 mL). The organic extracts were washed twice with brine (50 mL) and dried over anhydrous sodium sulfate. The solvents were *in vacuo* and column chromatography on silica gel (100 g) eluting with dichloromethane:methanol 95/5 (v:v) gave (tert-butoxy)-N-({4-[4-(3-hydroxyphenyl)(1,3-thiazol-2-yl)]-5-methyl(2-thienyl)}iminomethyl)carboxamide (247 mg, 59 %) as a yellow oil. ¹H-NMR (DMSO-d₆; 300 MHz) δ 9.56 (s, 1H), 9.12 (br s, 2H), 8.47 (s, 1H), 8.09 (s, 1H), 7.46 (m, 2H), 7.26 (m, 1H), 6.78 (m, 1H), 2.83 (s, 3H), 1.45 (s, 9H). Mass spectrum (ESI, m/z): Calcd. for C₂₀H₂₁N₃O₃S₂ 416.1 (M+H), found 415.7

***b) Methyl 2-{3-[2-(5-{[(tert-butoxy)carbonylamino]iminomethyl}-2-methyl-3-thienyl)-1,3-thiazol-4-yl]phenoxy}acetate:*** A stirred solution of 247 mg (0.595 mmol) of (tert-butoxy)-N-({4-[4-(3-hydroxyphenyl)(1,3-thiazol-2-yl)]-5-methyl(2-thienyl)}iminomethyl)carboxamide was dissolved in dry dimethylformamide (4 mL) and treated with cesium carbonate (291 mg, 0.89 mmol) and methyl bromoacetate (136 mg, 0.89 mmol) for 3 h at 60°C. The mixture was poured into water (50 mL) and extracted with dichloromethane (9 x 10 mL). The organic extracts were washed with brine (10 mL) and dried over anhydrous sodium sulfate. The solvents were removed *in vacuo* and column chromatography on silica gel (50 g) eluting with dichloromethane:methanol 98/2 (v:v) gave methyl 2-{3-[2-(5-{[(tert-butoxy)carbonylamino]iminomethyl}-2-methyl-3-thienyl)-1,3-thiazol-4-yl]phenoxy}acetate (178 mg, 61 %) as an oil. Mass spectrum (ESI, m/z): Calcd. for C₂₃H₂₅N₃O₅S₂ 488.1 (M+H), 388.1 ((M-BOC)+H), found 487.8, 388.2.

***c) Methyl 2-{3-[2-(5-amidino-2-methyl-3-thienyl)-1,3-thiazol-4-yl]phenoxy}acetate:*** Methyl 2-{3-[2-(5-{[(tert-butoxy)carbonylamino]iminomethyl}-2-methyl-3-thienyl)-1,3-thiazol-4-yl]phenoxy}acetate (15 mg, 0.031 mmol) treated with dichloromethane:trifluoroacetic acid 1/1 (v:v) with 2.5% water added at room temperature for 1.5 h. Removal of the solvents *in vacuo* gave methyl 2-{3-[2-(5-amidino-2-methyl-3-thienyl)-1,3-thiazol-4-yl]phenoxy}acetate (8.1 mg, 52 %) as a brown solid. ¹H-NMR (DMSO-d₆; 300 MHz) δ 9.38 (br s, 2H), 8.94 (br s, 2H), 8.51 (s, 1H), 8.31 (s, 1H), 7.62 (m, 2H), 7.41 (m, 1H), 6.96 (m, 1H), 4.89 (s, 2H), 3.72 (s, 3H), 2.92 (s, 3H). Mass spectrum (ESI, m/z): Calcd. for C₁₈H₁₇N₃O₃S₂ 388.1 (M+H), found 388.3.

### Example 151

***a) 2-{3-[2-(5-{[(tert-Butoxy)carbonylamino]iminomethyl}-2-methyl-3-thienyl)-1,3-thiazol-4-yl]phenoxy}acetic acid:*** A stirred solution of 50 mg (0.11 mmol) of methyl 2-{3-[2-(5-{[(tert-butoxy)carbonylamino]iminomethyl}-2-methyl-3-thienyl)-1,3-thiazol-4-yl]phenoxy}acetate was dissolved in tetrahydrofuran (10 mL) and treated 2M aqueous sodium hydroxide solution (2 mL) at room temperature for 1 h 10 min. The solvents were removed *in vacuo.* Purification by passing the solid through silica gel (1 inch in a 60 mL scintered-glass Büchner funnel) eluting with dichloromethane:methanol 8/2 (v:v) gave 2-{3-[2-(5-{[(tert-butoxy)carbonylamino]iminomethyl}-2-methyl-3-thienyl)-1,3-thiazol-4-yl]phenoxy}acetic acid (44 mg, 88 %) as a yellow solid. 'H-NMR (DMSO-d₆; 300 MHz) δ 9.38 (br s, 2H), 8.94 (br s, 2H), 8.51 (s, 1H), 8.31 (s, 1H), 7.62 (m, 2H), 7.41 (m, 1H), 6.96 (m, 1H), 4.89 (s, 2H), 3.72 (s, 3H), 2.92 (s, 3H). Mass spectrum (ESI, m/z): Calcd. for C₂₂H₂₃N₃O₅S₂ 474.1 (M+H), 374.1 ((M-BOC)+H) found 374.2, 473.7.

***b) 2-{3-[2-(5-Amidino-2-methyl-3-thienyl)-1,3-thiazol-4-yl]phenoxy}acetic acid:*** Methyl 2-{3-[2-(5-{[(tert-butoxy)carbonylamino]iminomethyl}-2-methyl-3-thienyl)-1,3-thiazol-4-yl]phenoxy}acetate (4 mg, 0.0084 mmol) was treated with dichloromethane:trifluoroacetic acid 1/1 (v:v) with 2.5% water added at room temperature for 2 h 35 min. Removal of the solvents *in vacuo* gave 2-{3-[2-(5-amidino-2-methyl-3-thienyl)-1,3-thiazol-4-yl]phenoxy}acetic acid (2.9 mg, 71 %) as a solid. Mass spectrum (ESI, m/z): Calcd. for C₁₇H₁₅N₃O₃S₂ 373.1 (M+H), found 374.2.

***c) Tert-butyl 4-(2-{3-[2-(5-{[(tert-butoxy)carbonylamino]iminomethyl}-2-methyl-3-thienyl)-1,3-thiazol-4-yl]phenoxy}acetyl)piperazinecarboxylate:*** A stirred solution of 40 mg (0.084 mmol) of 2-{3-[2-(5-{[(tert-butoxy)carbonylamino]iminomethyl }-2-methyl-3-thienyl)-1,3-thiazol-4-yl]phenoxy}acetic acid dissolved in dry dimethylformamide (5 mL) was treated with hydroxybenzotriazole (23 mg, 0.17 mmol), 32 mg (0.17 mmol) of *N*-tert-butoxycarbonyl-piperazine (Lancaster, Windham, NH, USA), 65 mg (0.17 mmol) of *O*-(7-azabenzotriazol-1-yl)-*N,N,N',N'*-tetramethyluronium hexafluorophosphate (HATU) at room temperature for 20 h. The mixture was partitioned between dichloromethane (50 mL) and brine (50 mL). The aqueous layers were extracted twice with dichloromethane (50 mL) and the combined organic layers were washed with brine (50 mL) and dried over anhydrous sodium sulfate. The solvents were removed *in vacuo.* Purification preparative thin layer chromatography eluting with dichloromethane:methanol 95/5 (v:v) gave tert-butyl 4-(2-{3-[2-(5-{[(tert-butoxy)carbonylamino]iminomethyl}-2-methyl-3-thienyl)-1,3-thiazol-4-yl]phenoxy}acetyl)piperazinecarboxylate (25 mg, 46%) as a white solid. ¹H-NMR (DMSO-d₆; 300 MHz) δ 9.13 (br s, 2H), 8.50 (s, 1H), 8.20 (s, 1H), 7.63 (m, 2H), 7.39 (m, 1H), 6.95 (m, 1H), 4.93 (s, 2H), 3.47-3.34 (m, 8H), 2.82 (s, 3H), 1.45 (s, 9H), 1.42 (s, 9H). Mass spectrum (ESI, m/z): Calcd. for C₃₁H₃₉N₅O₆S₂ 642.3 (M+H), 542.3 ((M-BOC)+H), 442.3 ((M-2 BOC)+H), found 642.0, 542.2, 442.3.

***d) 5-Methyl-4-{4-[3-(2-oxo-2-piperazinylethoxy)phenyl](1,3-thiazol-2-yl)}thiophene-2-carboxamidine:*** tert-Butyl 4-(2-{3-[2-(5-{[(tert-butoxy)carbonylamino]iminomethyl}-2-methyl-3-thienyl)-1,3-thiazol-4-yl)phenoxy}acetyl)piperazinecarboxylate (25 mg, 0.039 mmol) treated with dichloromethane:trifluoroacetic acid 1/1 (v:v) with 2.5% water added at room temperature for 2 h. Removal of the solvents *in vacuo* gave 5-methyl-4-{4-[3-(2-oxo-2-piperazinylethoxy)phenyl](1,3-thiazol-2-yl)}thiophene-2-carboxamidine (27.4 mg, quantitive yield) as an off-white solid. ¹H-NMR(Methanol-d₄; 300 MHz) δ 8.41 (s, 1H), 7.94 (s, 1H), 7.67 (m, 2H), 7.39 (m, 1H), 7.00 (m, 1H), 4.96 (s, 2H), 3.88 (m, 4H), 3.25 (m, 4H), 2.95 (s, 3H). Mass spectrum (ESI, m/z): Calcd. for C₂₁H₂₃N₅O₂S₂ 442.1 (M+H), found 442.4.

### Example 152

### Methyl 4-(2-bromoacetyl)-5-methylthiothiophene-2-carboxylate

To a stirring slurry of 2-methylthio-(5-carbomethoxy)-thiophene-3-carboxylic acid (2.0 g, 8.61 mmol) in 28 mL of CH₂Cl₂ under N₂ containing 0.8 mL DMF at 0°C was added oxalyl chloride (1.9 equiv, 16.3 mmol) slowly via syringe. The reaction was allowed to warm to ambient temperature after 1 h, and then stirred an additional 1 h. The reaction mixture was filtered through a 20 cm pad of silica gel in a 30 mL sintered glass funnel wetted with 50% ethyl acetate-hexanes and further eluted with the same solvent system until the eluent showed no product by UV visualization. The solvent was concentrated *in vacuo*, azeotroped with toluene (1x), and dried under vacuum to afford the acid chloride (1.52 g) as a light yellow solid. The acid chloride was dissolved in 20 mL of CH₃CN, cooled to 0°C, and treated with TMSCHN₂ (2.1 equiv, 6.3 mL, 2 M in hexanes) dropwise via syringe. The reaction was allowed to warm to ambient temperature (0.5 h), cooled back to 5°C and immediately treated with 30% HBr-acetic acid (0.66 mL) dropwise via an addition funnel. After 15 min. at 0°C, the reaction diluted with 20 mL of ether, filtered and thoroughly washed with ether (3x20 mL). The yellow solids were dried under vacuum to afford methyl 4-(2-bromoacetyl)-5-methylthiothiophene-2-carboxylate (1.0 g, 37% yield) as a yellow powder. ¹H NMR (DMSO-d₆, 300 MHz) δ 2.66 (s, 3H), 3.84 (s, 3H), 5.03 (s, 2H), 8.29 (s, 1H).

### Example 153

### Isopropyl-4-(2-bromoacetyl)-5-methylthiophene-2-carboxylate

To a stirring slurry of 2-methyl-(5-carboisopropoxy)-thiophene-3-carboxylic acid (0.40 g, 1.75 mmol) in 15 mL of CH₂Cl₂ under N₂ containing 0.3 mL DMF at 0°C was added oxalyl chloride (1.9 equiv, 3.32 mmol,) slowly via syringe. The reaction was allowed to warm to ambient temperature after 1 h, and then stirred an additional 1 h. The solvent was concentrated *in vacuo*, azeotroped with toluene (1x), and dried under vacuum to afford the acid chloride (0.397 g, 1.60 mmol) as a light yellow solid. The acid chloride was dissolved in 7 mL of CH₃CN, cooled to 0°C, and treated with TMSCHN₂ (2.1 equiv, 1.68 mL, 2 M in hexanes) dropwise via syringe. The reaction was allowed to warm to ambient temperature (0.5 h), cooled back to 5°C and immediately treated with 30% HBr-acetic acid (0.5 mL) dropwise via an addition funnel. After 15 min. at 0°C, the reaction mixture was filtered through a 10 cm pad of silica gel in a 15 mL sintered glass funnel wetted with 50% ethyl acetate-hexanes and further eluted with the same solvent system until the eluent showed no product by UV visualization. The solvent was concentrated *in vacuo* dried under vacuum to afford isopropyl- 4-(2-bromoacetyl)-5-methylthiophene-2-carboxylate (0.329 g, 61% yield) as an oil which solidified upon standing to a tan solid. ¹H NMR (DMSO-d₆, 300 MHz) δ 1.31 (d, 6H, J=6.3 Hz), 2.71 (s, 3H), 4.60 (s, 2H), 5.09 (m, 1H), 8.08 (s, 1H).

### Example 154

***a) Methyl 5-methylthio-4-[2-(phenylamino)-(1,3-thiazol-4-yl)]-thiophene-2-carboxylate hydrobromide:*** Methyl 4-(2-bromoacetyl)-5-methylthiothiophene-2-carboxylate (60.5 mg, 0.19 mmol) was slurried in 4 mL of acetone with phenyl thiourea (1 equiv, 30 mg) and heated to 70°C . After 3 h the reaction was allowed to cool to room temperature, filtered, and dried *in vacuo* to give 62.5 mg (69% yield) of methyl 5-methylthio-4-[2-(phenylamino)-(1,3-thiazol-4-yl)]-thiophene-2-carboxylate hydrobromide. ¹H NMR (DMSO-d₆, 300 MHz) δ 2.65 (s, 3H), 3.83 (s, 3H), 6.95-6.99 (m, 1H), 7.28-7.35 (m, 4H), 7.67 (d, 1H, J= 1.4, 7.7 Hz), 8.06 (s, 1H), 10.54 (s, 1H); Mass Spectrum (ESI) m/z calcd. for C₁₆H₁₄N₂O₂S₃, 362.49 (M+H), found 363.7.

***b) 5-Methylthio-4-[2-(phenylamino)(1,3-thiazol-4-yl)]thiophene-2-carboxamidine hydrochloride:*** To a flame dried flask containing 57.8 mg (8 equiv, 1.08 mmol) of NH₄Cl under N₂ was charged 1.3 mL of toluene. AlMe₃ (8 equiv, 2M/hexanes, 0.54 mL) was added dropwise to the stirring slurry over a 3 min. period, and allowed to stir another 5 min. At this time methyl 5-methylthio-4-[2-(phenylamino)-(1,3-thiazol-4-yl)]-thiophene-2-carboxylate hydrobromide (1 equiv, 60 mg, 0.135 mmol) was quickly added in one portion and the resultant mixture was immersed in a 120°C oil bath. After 2 h 10 min. at this temperature TLC (silica gel 60 F₂₅₄, Merck KGaA, Darmstadt, Germany, 9:1:0.5 CH₂Cl₂-MeOH-AcOH eluent) indicated the reaction to be complete by disappearance of the starting material. The reaction was allowed to cool to ambient temperature, then added via pipette to a stirring slurry of 1.3 g of SiO₂ in 20 mL of CHCl₃. The residual residue in the flask was rinsed with 4 mL of MeOH, briefly sonicated and added to the SiO₂ slurry. The slurry was stirred for 10 min. and then filtered through a 15 mL sintered glass funnel containing 20 cm of SiO₂ with 50% CHCl₃-MeOH. The yellow fraction is collected, discarding the forerun. TLC indicated the product was essentially pure. The solvent was removed *in vacuo,* and the residue triturated with 10% MeOH-CH₂Cl₂. The solids were removed by filtration. The solvent was concentrated *in vacua* to give 30.1 mg (66% yield) of 5-methylthio-4-[2-(phenylamino)-(1,3-thiazol-4-yl)]thiophene-2-carboxamidine hydrochloride as a red-brown powder. ¹H NMR (DMSO-d₆, 300 MHz) δ 2.73 (s, 3H), 6.94- 7.00 (m, 1H), 7.15 (s, 1H), 7.30-7.35 (m, 1H), 7.78 (d, 1H, J=8.7 Hz), 8.49 (s, 1H), 8.87 (bs, 2H), 9.31 (bs, 2H), 10.38 (s, 1H); Mass Spectrum (ESI) m/z calcd. for C₁₅H₁₄N₄S₃, 346.50 (M+H), found 347.2.

### Example 155

***a) Methyl 4-{2-[(2-chlorophenyl)amino](1,3-thiazol-4-yl)}-5-methylthiothiophene-2-carboxylate hydrobromide:*** Methyl 4-(2-bromoacetyl)-5-methylthiothiophene-2-carboxylate (50 mg) was allowed to react with 2-chlorophenyl thiourea (26.7 mg) as described in Example 154, step (a), to give 58 mg (75%) of methyl 4-{2-[(2-chlorophenyl)amino]-(1,3-thiazol-4-yl)}-5-methylthiothiophene-2-carboxylate hydrobromide. ¹H NMR (DMSO-d₆, 300 MHz) δ 2.66 (s, 3H), 3.82 (s, 3H), 7.04 (m, 1H), 7.32-7.38 (m, 2H), 7.47 (dd, 1H, J= 1.4, 8.7 Hz), 8.12 (s, 1H), 8.56 (dd, 1H, J=1.4, 8.3 Hz), 9.75 (s, 1H); Mass Spectrum (ESI) m/z calcd. for C₁₆H₁₃ClN₂O₂S₃, 396.94 (M+H), found 397.1.

***b) 4-{2-[(2-Chlorophenyl)amino](1,3-thiazol-4-yl)}-5-methylthiothiophene-2-carboxamidine hydrochloride:*** Methyl 4-{2-[(2-chlorophenyl)amino]-(1,3-thiazol-4-yl)}-5-methylthiothiophene-2-carboxylate hydrobromide (40 mg, 0.08 mmol) was treated as described in Example 154, step (b) to give 24 mg (71.8%)of 4-{2-[(2-chlorophenyl)amino]-(1,3-thiazol-4-yl)}-5-methylthiothiophene-2-carboxamidine hydrochloride. ¹H NMR (DMSO-d₆, 300 MHz) δ 2.71 (s, 3H), 7.04 (td, 1H, J=1.4, 7.8 Hz), 7.21 (s, 1H), 7.35 (t, 1H, J=8.5 Hz), 8.42 (s, 1H), 8.57 (dd, 1H, J=1.3, 8.3 Hz), 8.80 (bs, 2H), 9.26 (bs, 2H), 9.79 (s, 1H); Mass Spectrum (ESI) m/z calcd. for C₁₅H₁₄N₄S₃Cl, 380.94 (M+H), found 381.1.

### Example 156

***a) Methyl 4-(2-amino(1,3-thiazol-4-yl))-5-methylthiothiophene-2-carboxylate hydrobromide:*** Methyl 4-(2-bromoacetyl)-5-methylthiothiophene-2-carboxylate (50 mg, 0.16 mmol) was allowed to react with thiourea (12 mg) as described in Example 154, step (a), to give 54 mg (70% yield) of methyl 4-(2-amino-(1,3-thiazol-4-yl))-5-methylthiothiophene-2-carboxylatehydrobromide. ¹H NMR (DMSO-d₆, 300 MHz) δ 2.69 (s, 3H), 3.83 (s, 3H), 7.00 (s, 1H), 8.05 (s, 1H); Mass Spectrum (ESI) m/z calcd. for C₁₀H₁₀O₂S₃N₂, 286.41 (M+H), found 287.1;

***b) 4-(2-Amino-(1,3-thiazol-4-yl))-5-methylthiothiophene-2-carboxamidine hydrochloride:*** Methyl 4-(2-amino-(1,3-thiazol-4-yl))-5-methylthiothiophene-2-carboxylate hydrobromide (110 mg, 0.29 mmol) was treated as described in Example 154, step (b). The resultant amidine (74 mg) was stirred in 3 mL of dry methanol under N₂ and treated with ca. 1mL of ether saturated with dry HCl gas. Dry ether (1.5 mL) was then added and the result was allowed to sit for 2 h at ambient temperature and then filtered to give 40 mg (45% yield) of 4-(2-amino-(1,3-thiazol-4-yl))-5-methylthiothiophene-2-carboxamidine hydrochloride. ¹H NMR (DMSO-d₆, 300 MHz) δ 2.69 (s, 3H), 6.90 (s, 1H), 8.44 (s, 1H), 9.20, 9.42 (s, 4H, NH); Mass Spectrum (ESI) m/z calcd.C₉H₁₀N₄S₃, 270.4 (M+H), found 271.2.

### Example 157

***a) Methyl 4-{2-[(2,5-dimethoxyphenyl)amino](1,3-thiazol-4-yl)}-5-methylthiothiophene-2-carboxylate hydrobromide:*** Methyl 4-(2-bromoacetyl)-5-methylthiothiophene-2-carboxylate (49.4 mg, 0.15 mmol) was allowed to react with 2,5-dimethoxy phenyl thiourea (37.2 mg) as described in Example 154, step (a), to give 65.5 mg (87% yield) of methyl 4-{2-[(2,5-dimethoxyphenyl)amino](1,3-thiazol-4-yl)}-5-methylthiothiophene-2-carboxylate hydrobromide. ¹H NMR (DMSO-d₆, 300 MHz) δ 2.66 (s, 3H), 3.76 (s, 3H), 3.81 (s, 3H), 3.83 (s, 3H), 6.49 (dd, 1H, J=3.0, 8.8 Hz), 6.92 (d, 1H, J=8.9 Hz), 7.26 (s, 1H), 8.17 (s, 1H), 8.37 (d, 1H, J=3.1 Hz), 9.70 (s, 1H); Mass Spectrum (ESI) m/z calcd. for C₁₈H₁₈N₂O₄S₃, 422.54 (M+H), found 423.1.

***b) 4-{2-[(2,5-Dimethoxyphenyl)amino](1,3-thiazol-4-yl)}-5-methylthiothiophene-2-carboxamidine:*** Methyl 4-{2-[(2,5-dimethoxyphenyl)amino]-(1,3-thiazol-4-yl)}-5-methylthiothiophene-2-carboxylate hydrobromide (45.5 mg, 0.09 mmol) was treated as described in Example 154, step (b), followed by preparative thin layer chromatography (500 µm silica gel plate, J.T. Baker, Phillipsburg, NJ, 10%-methanol-CH₂Cl₂-sat'd. NH₃ eluent) to give 9.9 mg (27% yield of 4-{2-[(2,5-dimethoxyphenyl)amino](1,3-thiazol-4-yl)}-5-methylthiothiophene-2-carboxamidine. ¹H NMR (DMSO-d₆, 300 MHz) δ 2.60 (s, 3H), 3.73 (s, 3H), 3.81 (s, 3H), 6.48 (dd, 1H, J=3.1, 8.8 Hz), 6.92 (d, 1H, J=7.9 Hz), 7.05 (s, 1H), 7.5 (bs, 2H), 8.04 (s, 1H), 8.34 (d, 1H, J=1.0 Hz), 9.6 (bs, 1H); Mass Spectrum (ESI) m/z calcd. for C₁₇H₁₈N₄O₂S₃, 406.55 (M+H), found 407.1.

### Example 158

***a) Methyl 4-{2-[(3-methoxyphenyl)amino](1,3-thiazol-4-yl)}-5-methylthiothiophene-2-carboxylate hydrobromide:*** Methyl 4-(2-bromoacetyl)-5-methylthiothiophene-2-carboxylate (53.3 mg, 0.17 mmol) was allowed to react with 2-methoxy phenyl thiourea (34.5 mg) as described in Example 154, step (a), to give 61 mg (76% yield) of methyl 4-{2-[(3-methoxyphenyl)amino](1,3-thiazol-4-yl)}-5-methylthiothiophene-2-carboxylate hydrobromide. ¹H NMR (DMSO-d₆, 300 MHz) δ 2.67 (s, 3H), 3.78 (s, 3H), 3.83 (s, 3H), 6.53 (d, 1H, J=6.8 Hz), 7.13-7.24 (m, 2H), 7.29 (s, 3H), 7.59 (m, 1H), 8.16 (s, 3H), 10.32 (s, 1H); Mass Spectrum (ESI) m/z calcd. for C₁₇H₁₆N₂O₃S₃, 392.52 (M+H), found 393.2.

***b) 4-{2-[(3-Methoxyphenyl)amino](1,3-thiazol-4-yl)}-5-methylthiothiophene-2-carboxamidine hydrochloride:*** Methyl 4-{2-[(3-methoxyphenyl)amino]-(1,3-thiazol-4-yl)}-5-methylthiothiophene-2-carboxylate hydrobromide (54.6 mg, 0.11 mmol) was treated as described in Example 154, step (b) to give 25.2 mg (56%) of 4-{2-[(3-methoxyphenyl)amino](1,3-thiazol-4-yl)}-5-methylthiothiophene-2-carboxamidine hydrochloride. ¹H NMR (DMSO-d₆, 300 MHz) δ 2.71 (s, 3H), 3.77 (s, 3H), 6.54 (m, 1H), 7.15 (s, 3H), 7.19-7.28 (m, 2H), 7.47 (m, 1H), 8.46 (s, 1H), 8.86 (bs, 2H), 9.28 (bs, 2H), 10.36 (s, 1H); Mass Spectrum (ESI) m/z calcd. for C₁₆H₁₆N₄OS₃, 376.52 (M+H), found 377.2.

### Example 159

***a) Methyl 4-{2-[(4-methoxyphenyl)amino](1,3-thiazol-4-yl)}-5-methylthiothiophene-2-carboxylate hydrobromide:*** Methyl 4-(2-bromoacetyl)-5-methylthiothiophene-2-carboxylate (41.3 mg, 0.13 mmol) was allowed to react with 5-methoxy phenyl thiourea (26.8 mg) as described in Example 154, step (a) to give 25 mg (41% yield) of methyl 4-{2-[(4-methoxyphenyl)amino](1,3-thiazol-4-yl)}-5-methylthiothiophene-2-carboxylate hydrobromide. ¹H NMR (DMSO-d₆, 300 MHz) δ 2.64, 2.68 (s, 3H *rotomer)* ), 3.72, 3.73 (s, 3H *rotomer*), 3.83 (s, 3H), 6.91 (dd, 2H, J=6.7, 8.8 Hz), 7.21 (s, 1H), 7.59 (d, 1H, J=9.0 Hz), 7.67 (d, 1H, J=9.0 Hz), 8.05, 8.13 (s, 1H *rotomer),* 10.16, 10.34(bs, 1H, *rotomer);* Mass Spectrum (ESI) m/z calcd. for C₁₇H₁₆N₂O₂S₃, 392.52 (M+H), found 393.1.

***b) 4-{2-[(4-Methoxyphenyl)amino](1,3-thiazol-4-yl)}-5-methylthiothiophene-2-carboxamidine hydrochloride:*** Methyl 4-{2-[(4-methoxyphenyl)amino]-(1,3-thiazol-4-yl)}-5-methylthiothiophene-2-carboxylate hydrobromide (22 mg, 0.046 mmol) was treated as described in Example 154, step (b) to give 11.5 mg (61% yield) of 4-{2-[(4-methoxyphenyl)amino](1,3-thiazol-4-yl)}-5-methylthiothiophene-2-carboxamidine hydrochloride. ¹H NMR (DMSO-d₆, 300 MHz) δ 2.72 (s, 3H), 3.73 (s, 3H), 6.91 (d, 2H, J=9.0 Hz), 7.08 (s, 1H), 7.69 (d, 2H, J=9.1 Hz), 8.44 (s, 1H), 8.83 (bs, 2H), 9.28 (bs, 2H), 10.15 (s, 1H);Mass Spectrum (ESI) m/z calcd. for C₁₆H₁₆N₄OS₃, 376.52 (M+H), found 377.1.

### Example 160

***a) Methyl 4-(2-{[4-(dimethylamino)phenyl]amino}(1,3-thiazol-4-yl))-5-methylthiothiophene-2-carboxylate hydrobromide:*** Methyl 4-(2-bromoacetyl)-5-methylthiothiophene-2-carboxylate (50 mg, 0.16 mmol) was allowed to react with 4-N,N-dimethylaminophenyl thiourea (31.5 mg) as described in Example 154, step (a), to give 53.2 mg (75% yield) of methyl 4-(2-{[4-(dimethylamino)phenyl]amino}(1,3-thiazol-4-yl))-5-methylthiothiophene-2-carboxylate hydrobromide. ¹H NMR (DMSO-d₆, 300 MHz) δ 2.69 (s, 3H), 3.15 (s, 6H), 3.83 (s, 3H), 7.36 (s, 1H), 7.55 (bs, 2H), 7.88 (d, 2H, J=8.3 Hz), 8.16 (s, 1H), 10.56 (bs, 1H); Mass Spectrum (ESI) m/z calcd. for C₁₈H₁₉N₃O₂S₃, 405.56 (M+H), found 406.1.

***b) 4-(2-{[4-(Dimethylamino)phenyl]amino}(1,3-thiazol-4-yl))-5-methylthiothiophene-2-carboxamidine hydrochloride:*** Methyl 4-(2-{[4-(dimethylamino)phenyl]amino}(1,3-thiazol-4-yl))-5-methylthiothiophene-2-carboxylate hydrobromide (50 mg, 0.10 mmol) was treated as described in Example 154, step (b) to give 9.4 mg (22% yield) of 4-{2-[(4-methoxyphenyl)amino](1,3-thiazol4-yl)}-5-methylthiothiophene-2-carboxamidine hydrochloride. ¹H NMR (DMSO-d₆, 300 MHz) 2.70 (s, 3H), 2.84 (s, 6H), 6.75 (d, 2H, J=9.2 Hz), 7.00 (s, 1H), 7.56 (d, 2H, J=9.1 Hz), 8.31 (s, 1H), 8.68 (bs, 3H), 9.92 (bs, 1H).

### Example 161

***a) Methyl 4-{2-[(4-chloro-2-methylphenyl)amino](1,3-thiazol-4-yl)}-5-methylthiothiophene-2-carboxylate hydrobromide:*** Methyl 4-(2-bromoacetyl)-5-methylthiothiophene-2-carboxylate (50 mg, 0.16 mmol) was allowed to react with 2-methyl-4-chlorophenyl thiourea (32.1 mg) as described in Example 154, step (a), to give 62.2 mg (79% yield) of methyl 4-{2-[(4-chloro-2-methylphenyl)amino](1,3-thiazol-4-yl)}-5-methylthiothiophene-2-carboxylate hydrobromide. ¹H NMR (DMSO-d₆, 300 MHz) δ 2.28, 2.29 (s, 3H *rotomer),* 2.62, 2.66 (s, 3H *rotomer),* 3.82 (s, 3H), 7.21-7.29 (m, 3H), 8.04, 8.11 (s, 1H *rotomer),* 8.17 (d, 1H, J=8.8 Hz), 8.30 (d, 1H, J=8.4 Hz), 9.44 (s, 1H), 9.59 (s, 1H); Mass Spectrum (ESI) m/z calcd. for C₁₇H₁₅ClN₂O₂S₃, 410.96 (M+H), found 411.1.

***b) 4-{2-[(4-Chloro-2-methylphenyl)amino](1,3-thiazol-4-yl)}-5-methylthiothiophene-2-carboxamidine hydrochloride:*** Methyl 4-{2-[(4-chloro-2-methylphenyl)amino]-(1,3-thiazol-4-yl)}-5-methylthiothiophene-2-carboxylate hydrobromide (55 mg, 0.17 mmol) was treated as described in Example 154, step (b) to give 16 mg (22% yield) of 4-{2-[(4-chloro-2-methylphenyl)amino](1,3-thiazol-4-yl)}-5-methylthiothiophene-2-carboxamidine hydrochloride. ¹H NMR (DMSO-d₆, 300 MHz) δ 2.30 (s, 3H), 2.70 (s, 3H), 7.15 (s, 1H), 7.23 -7.29 (m, 2H), 8.34 (d, 1H, J=8.6 Hz), 8.44 (s, 1H), 8.86 (bs, 2H), 9.29 (bs, 2H), 9.47 (s, 1H); Mass Spectrum (ESI) m/z calcd. for C₁₆H₁₅ClN₄S₃, 394.97 (M+H), found 395.1.

### Example 162

***a) Methyl 4-{2-[(diphenylmethyl)amino](1,3-thiazol-4-yl)}-5-methylthiothiophene-2-carboxylate hydrobromide:*** Methyl 4-(2-bromoacetyl)-5-methylthiothiophene-2-carboxylate (50 mg, 0.16 mmol) was allowed to react with diphenylmethane thiourea (38 mg) as described in Example 154, step (a), to give 145 mg (100% yield) of methyl 4-{2-[(diphenylmethyl)amino](1,3-thiazol-4-yl)}-5-methylthiothiophene-2-carboxylate hydrobromide after removal of solvent *in vacuo.* ¹H NMR (DMSO-d₆, 300 MHz) δ 2.50 (s, 3H), 2.80 (s, 3H), 6.13, 6.18 (d, 1H *rotomer,* J=7.9 Hz), 7.23-7.41 (m, 11H), 8.00, 8.02 (s, 1H *rotomer),* 8.73, 8.86 (d, 1H, *rotomer,* J=8.0 Hz); Mass Spectrum (ESI) m/z calcd. for C₂₃H₂₀N₂O₂S₃, 452.62 (M+H), found 453.0.

***b) 4-{2-[(diphenylmethyl)amino](1,3-thiazol-4-yl)}-5-methylthiothiophene-2-carboxamidine:*** Methyl 4-{2-[(diphenylmethyl)amino](1,3-thiazol-4-yl)}-5-methylthiothiophene-2-carboxylate hydrobromide. (96.3 mg. 0.18 mmol) was treated as described in Example 154, step (b) to give 16 mg (20% yield) of 4-{2-[(diphenylmethyl)amino](1,3-thiazol-4-yl)}-5-methylthiothiophene-2-carboxamidine hydrochloride. ¹H NMR (DMSO-d₆, 300 MHz) 2.59 (s, 3H), 6.23 (d, 1H, J=7.9 Hz), 6.84 (s, 1H), 7.22-7.40 (m, 10 H), 8.09 (bs, 3H), 8.12 (s, 1H), 8.68 (d, 1H, J=8.4 Hz); Mass Spectrum (ESI) m/z calcd. for C₂₂H₂₀N₄S₃, 436.62 (M+H), found 437.1.

### Example 163

***a) Methyl 5-methylthio-4-{2-[(3-phenylpropyl)amino](1,3-thiazol-4-yl)}thiophene-2-carboxylate hydrobromide:*** Methyl 4-(2-bromoacetyl)-5-methylthiothiophene-2-carboxylate (131 mg, 0.42 mmol) was allowed to react with propylphenyl thiourea (82.3 mg) in DMF as described in Example 154, step (a), then filtered through a 5 cm pad of silica gel in a 15 mL glass fritted funnel with 10% methanol-CHCl₃. Concentration of the solvent *in vacuo* gave 203 mg (100% yield) of methyl -5-methylthio-4-{2-[(3-phenylpropyl)amino](1,3-thiazol-4-yl)}thiophene-2-carboxylate hydrobromide. ¹H NMR (DMSO-d₆, 300 MHz) δ 1.89 (m, 2H), 2.62 (s, 3H), 2.63-2.71 (m, 2H), 3.27-3.39 (m, 2H), 3.82 (s, 3H), 6.97 (s, 1H), 7.15-7.31 (m, 5H), 8.06 (s, 1H); Mass Spectrum (ESI) m/z calcd. for C₁₉H₂₀N₂O₂S₃, 404.57 (M+H)_{,} found 405.1.

***b) 5-Methylthio-4-{2-[(3-phenylpropyl)amino](1,3-thiazol-4-yl)}thiophene-2-carboxamidine hydrochloride:*** Methyl -5-methylthio-4-{2-[(3-phenylpropyl}amino](1,3-thiazol-4-yl)}thiophene-2-carboxylate hydrobromide (112 mg, 0.23 mmol) was treated as described in Example 154, step (b) to give 16 mg (16% yield) of 4-{2-[(diphenylmethyl)amino](1,3-thiazol-4-yl)}-5-methylthiothiophene-2-carboxamidine hydrochloride, which was further purified by preparative thin layer chromatography using 20%-methanol-CH₂Cl₂-sat'd. NH₃ as eluent. ¹H NMR (DMSO-d₆, 300 MHz) δ 1.89 (m, 2H), 2.54 (s, 1H), 2.66 (at, 2H, J=7.3 Hz), 3.31 (m, 2H), 6.69 (bs, 3H), 6.76 (s, 1H), 7.15-7.31 (m, 5H), 7.69 (m, 1H), 7.84 (s, 1H); Mass Spectrum (ESI) m/z calcd. for C₁₈H₂₀N₄S₃, 388.58 (M+H), found 389.2.

### Example 164

***a) Methyl 5-methylthio-4-{2-[(2,4,5-trimethylphenyl)amino](1,3-thiazol-4-yl)}thiophene-2-carboxylate hydrobromide:*** Methyl 4-(2-bromoacetyl)-5-methylthiothiophene-2-carboxylate (60 mg, 0.21 mmol) was allowed to react with 2,4,5-trimethylphenyl thiourea as described in Example 154, step (a) to give 42.3 mg (41% yield) of methyl 5-methylthio-4-{2-[(2,4,5-trimethylphenyl)amino](1,3-thiazol-4-yl)}thiophene-2-carboxylate hydrobromide. ¹H NMR (DMSO-d₆, 300 MHz) δ 2.16 (s, 3H), 2.18 (s, 3H), 2.19 (s, 3H), 2.64 (s, 3H), 3.82 (s, 3H), 6.97 (s, 1H), 7.18 (s, 1H), 7.86 (s, 1H), 8.12 (s, 1H), 9.29 (s, 1H); Mass Spectrum (ESI) m/z calcd. for C₁₉H₂₀N₂O₂S₃, 404.57 (M+H), found 405.1.

***b) 5-Methylthio-4-{2-[(2,4,5-trimethylphenyl)amino](1,3-thiazol-4-yl)}thiophene-2-carboxamidine hydrochloride:*** Methyl -5-methylthio-4-{2-[(2,4,5-trimethylphenyl)amino](1,3-thiazol-4-yl)}thiophene-2-carboxylate hydrobromide (37.3 mg, 0.07 mmol) was treated as described in Example 154, step (b) to give 28.3 mg (95% yield) of 5-methytthio-4-{2-[(2,4,5-trimethylphenyl)amino](1,3-thiazol-4-yl)}thiophene-2-carboxamidine hydrochloride. ¹H NMR (DMSO-d₆, 300 MHz) δ 2.16 (s, 3H), 2.19 (s, 3H), 2.20 (s, 3H), 2.68 (s, 3H), 6.97 (s, 1H), 7.03 (s, 1H), 7.84 (s, 1H), 8.41 (s, 1H), 8.84 (bs, 2H), 9.26 (bs, 3H); Mass Spectrum (ESI) m/z calcd. for C₁₈H₂₀N₄S₃, 388.58 (M+H), found 389.2.

### Example 165

***a) Methyl 4-{2-[(2-fluorophenyl)amino](1,3-thiazol-4-yl)}-5-methylthiothiophene-2-carboxylate hydrobromide:*** Methyl 4-(2-bromoacetyl)-5-methylthiothiophene-2-carboxylate (60 mg, 0.19 mmol) was allowed to react with 2-fluorophenyl thiourea as described in Example 154, step (a) to give 55.6 mg (70% yield) of methyl 4-{2-[(2-fluorophenyl)amino](1,3-thiazol-4-yl)}-5-methylthiothiophene-2-carboxylate hydrobromide. ¹H NMR (DMSO-d₆, 300 MHz) δ 2.68 (s, 3H), 3.83 (s, 3H), 6.96-7.04 (m, 1H), 7.14-7.29 (m, 3H), 7.35 (s, 1H), 8.06, 8.14 (s, 1H *rotomer*), 8.53, 8.8.68 (td, 1H *rotomer*, J=1.5, 8.5 Hz), 10.14, 10.30 (s, 1H *rotomer);* Mass Spectrum (ESI) m/z calcd. for C₁₆H₁₃FN₂O₂S₃, 380.48 (M+H), found 381.1.

***b) 4-{2-[(2-Fluorophenyl)amino](1,3-thiazol-4-yl)}-5-methylthiothiophene-2-carboxamidine hydrochloride:*** Methyl 4-{2-[(2-fluorophenyl)amino](1,3-thiazol-4-yl)}-5-methylthiothiophene-2-carboxylate hydrobromide (55.6 mg, 0.13 mmol)) was treated as described in Example 154, step (b) to give 12.4 mg (24%) of 4-{2-[2-fluorophenyl)amino](1,3-thiazol-4-yl)}-5-methylthiothiophene-2-carboxamidine hydrochloride. ¹H NMR (DMSO-d₆, 300 Mhz); δ 2.72 (s, 3H), 3.16 (s, 3H), 6.97-7.08 (m, 1H), 7.18-7.36 (m, 4H), 8.49 (s, 1H), 8.70 (td, 1H, 1.4, 8.4 Hz), 8.92 (bs, 2H), 9.32 (bs, 2H), 10.18 (d, 1H, J=1.6 Hz); Mass Spectrum (ESI) m/z calcd. for C₁₅H₁₃FN₄S₃, 364.49 (M+H), found 365.1.

### Example 166

***a) Methyl 4-{2-[(3-chloro-2-methylphenyl)amino](1,3-thiazol-4-yl)}-5-methylthiothiophene-2-carboxylate hydrobromide:*** Methyl 4-(2-bromoacetyl)-5-methylthiothiophene-2-carboxylate (60 mg, 0.19 mmol) was allowed to react with 2-methyl-3-chlorophenyl thiourea (39 mg) as described in Example 154, step (a) to give 61.8 mg (66% yield) of methyl 4-{2-[(3-chloro-2-methylphenyl)amino](1,3-thiazol-4-yl)}-5-methylthiothiophene-2-carboxylate hydrobromide. Mass Spectrum (ESI) m/z calcd. for C₁₇H₁₅ClN₂O₂S₃, 410.96 (M+H), found 411.1.

***b) 4-{2-[(3-Chloro-2-methylphenyl)amino](1,3-thiazol-4-yl)}-5-methylthiothiophene-2-carboxamidine hydrochloride:*** Methyl 4-{2-[(3-chloro-2-methylphenyl)amino](1,3-thiazol-4-yl)}-5-methylthiothiophene-2-carboxylate hydrobromide (61.8 mg, 0.12 mmol) was treated as described in Example 154, step (b) to give 46.7 mg (90% yield) of 4-{2-[(3-chloro-2-methylphenyl)amino](1,3-thiazol-4-yl)}-5-methylthiothiophene-2-carboxamidine hydrochloride. ¹H NMR (DMSO-d₆, 300 MHz) δ 2.34 (s, 3H), 2.69 (s, 3H), 7.15 (s, 1H), 7.18-7.26 (m, 2H), 8.12 (d, 1H, J=7.9 Hz), 8.41 (s, 1H), 8.84 (bs, 2H), 9.27 (bs, 2H), 9.61 (s, 1H); Mass Spectrum (ESI) m/z calcd. for C₁₆H₁₅ClN₄S₃, 394.97 (M+H), found 395.1.

### Example 167

***a) Methyl 4-(2-{[2-(methylethyl)phenyl]amino}(1,3-thiazol-4-yl))-5-methylthiothiophene-2-carboxylate hydrobromide:*** Methyl 4-(2-bromoacetyl)-5-methylthiothiophene-2-carboxylate (60 mg, 0.19 mmol) was allowed to react with 2-isopropyl phenyl thiourea (40 mg) as described in Example 154, step (a) to give 33.1 mg (36% yield) of methyl 4-(2-{[2-(methylethyl)phenyl]amino}(1,3-thiazol-4-yl))-5-methylthiothiophene-2-carboxylate hydrobromide. ¹H NMR (DMSO-d₆, 300 MHz) δ 1.17 (d, 6H, J=6.7 Hz), 2.60, 2.65 (s, 3H *rotomer*), 3.27 (s, 1H), 3.82 (s, 3H), 7.13 (s, 1H), 7.14-7.25 (m, 2H), 7.34-7.37 (m, 1H), 7.78 (m, 1H), 7.99, 8.08 (s, 1H *rotomer*), 9.52, 9.61 (bs, 1H *rotomer*); Mass Spectrum (ESI) m/z calcd. for C₁₉H₂₀N₂O₂S₃, 404.57 (M+H), found 405.1.

***b) 4-(2-{[2-(Methylethyl)phenyl]amino}(1,3-thiazol-4-yl))-5-methylthiothiophene-2-carboxamidine hydrochloride:*** Methyl 4-(2-{[2-(methylethyl)phenyl]amino}(1,3-thiazol-4-yl))-5-methylthiothiophene-2-carboxylate hydrobromide (33.1 mg, 0.06 mmol) was treated as described in Example 154, step (b) to give 22.4 mg (88%) of 4-(2-{[2-(methylethyl)phenyl]amino}(1,3-thiazol-4-yl))-5-methylthiothiophene-2-carboxamidine hydrochloride. ¹H NMR (DMSO-d₆, 300 MHz) δ 1.19 (d, 6H, J=6.8 Hz), 2.70 (s, 3H), 3.32 (m, 1H), 7.04 (s, 1H), 7.14-7.25 (m, 2H), 7.35 (dd, 1H, J=1.4, 7.5 Hz), 7.86 (dd, 1H, J=1.4, 7.9 Hz), 8.37 (s, 1H); Mass Spectrum (ESI) m/z calcd. for C₁₈H₂₀N₄S₃, 388.58 (M+H), found 389.2.

### Example 168

***a) Methyl 5-methylthio-4-(2-{[4-(phenylmethoxy)phenyl]amino}(1,3-thiazol***-*4****-yl))thiophene-2-carboxylate:*** Methyl 4-(2-bromoacetyl)-5-methylthiothiophene-2-carboxylate (336.3 mg, 1.08 mmol) was allowed to react with 4-benzyloxyphenyl thiourea (279 mg) as described in Example 154, step (a) to give 450 mg (76% yield) of methyl 4-(2-{[4-phenylmethoxyphenyl]amino}(1,3-thiazol-4-yl))-5-methylthiothiophene-2-carboxylate hydrobromide. Mass Spectrum (ESI) m/z calcd. for C₂₃H₂₀N₂O₃S₃, 468.61 (M+H), found 469.2.

***b) 5-Methylthio-4-(2-{[4-(phenylmethoxy)phenyl]amino}(1,3-thiazol-4-yl))thiophene-2-carboxamidine hydrochloride:*** Methyl 4-(2-{[4-phenylmethoxyphenyl]amino}(1,3-thiazol-4-yl))-5-methylthiothiophene-2-carboxylate hydrobromide (100 mg, 0.18 mmol) was treated as described in Example 154, step (b) to give 23.9 mg (27% yield) 5-methylthio-4-(2-{[4-(phenylmethoxy)phenyl]amino}(1,3-thiazol-4-yl))thiophene-2-carboxamidine hydrochloride. ¹H NMR (DMSO-d₆, 300 MHz) δ 2.73 (s, 3H), 5.08 (s, 2H), 7.00 (d, 2H, J=8.2 Hz), 7.09 (s, 1H), 7.31-7.47 (m, 5H), 7.70 (d, 2H, J=8.0 Hz), 8.47 (s, 1H), 8.88 (bs, 2H), 9.30 (bs, 2H), 10.20 (s, 1H); Mass Spectrum (ESI) m/z calcd. for C₂₂H₂₀N₄OS₃, 452.62 (M+H), found 453.1.

### Example 169

***a) Methyl 4-{2-[(2-bromophenyl)amino](1,3-thiazol-4-yl)}-5-methylthiothiophene-2-carboxylate hydrobromide:*** Methyl 4-(2-bromoacetyl)-5-methylthiothiophene-2-carboxylate (60 mg, 0.19 mmol) was allowed to react with 2-bromophenyl thiourea (44 mg) as described in Example 154, step (a) to give 63.1 mg (64% yield) of methyl 4-{2-[(2-bromophenyl)amino](1,3-thiazol-4-yl)}-5-methylthiothiophene-2-carboxylate hydrobromide. ¹H NMR (DMSO-d₆, 300 MHz) δ 2.65 (s, 3H), 3.82 (s, 3H), 7.00 (m, 1H), 7.33 (s, 1H), 7.40 (m, 1H), 7.64 (dd, 1H, J=1.4, 7.9 Hz), 8.04, 8.11 (s, 1H *rotomer),* 8.27, 8.37 (dd, 1H 9.60, 9.80 (bs, 1H *rotomer,* J=1.5, 8.2 Hz), Mass Spectrum (ESI) m/z calcd. for C₁₆H₁₃BrN₂O₂S₃, 441.39 (M+H), found 441.1, 443.0.

***b) 4-{2-[(2-Bromophenyl)amino]amino](1,3-thiazol-4-yl)}-5-methylthiothiophene-2-carboxamidine hydrochloride:*** Methyl 4-{2-[(2-bromophenyl)amino](1,3-thiazol-4-yl)}-5-methylthiothiophene-2-carboxylate hydrobromide (63.1mg, 0.12 mmol) was treated as described in Example 154, step (b) to give 47.9 mg (86% yield) of 4-{2-[(2-bromophenyl)amino](1,3-thiazol-4-yl)}-5-methylthiothiophene-2-carboxamidine hydrochloride. ¹H NMR (DMSO-d₆, 300 MHz) δ 2.70 (s, 3H), 7.01 (m 1H), 7.20 (s, 1H), 7.40 (m, 1H), 7.65 (dd, 1H, J=1.5, 8.0), 8.38 (dd, 1H, J=1.5, 8.3 Hz), 8.44 (s, 1H), 8.89 (bs, 2H), 9.30 (bs, 2H), 9.62 (s, 1H); Mass Spectrum (ESI) m/z calcd. for C₁₅H₁₃BrN₄S₃, 425.39 (M+H), found 425.1, 427.0.

### Example 170

***a) Methyl 4-{2-[(2,6-dichlorophenyl)amino](1,3-thiazol-4-yl)}-5-methylthiothiophene-2-carboxylate hydrobromide:*** Methyl 4-(2-bromoacetyl)-5-methylthiothiophene-2-carboxylate (60 mg, 0.19 mmol) was allowed to react with 2,6-dichlorophenyl thiourea (42 mg) as described in Example 154, step (a) to give 63.1 mg (65% yield) of methyl 4-{2-[(2,6-dichlorophenyl)amino](1,3-thiazol-4-yl)}-5-methylthiothiophene-2-carboxylate hydrobromide. ¹H NMR (DMSO-d₆, 300 MHz) δ 2.59 (s, 3H), 3.8 (s, 3H), 7.15 (s, 1H), 7.36 (m, 1H), 7.61 (m, 2H), 7.97 (s, 1H); Mass Spectrum (ESI) m/z calcd. for C₁₆H₁₂Cl₂N₂O₂S₃, 431.38 (M+H), found 431.0, 433.0.

***b) 4-{2-[(2,6-Dichlorophenyl)amino](1,3-thiazol-4-yl)}-5-methylthiothiophene-2-carboxamidine hydrochloride:*** Methyl 4-{2-[(2,6-dichlorophenyl)amino](1,3-thiazol-4-yl)}-5-methylthiothiophene-2-carboxylate hydrobromide (43 mg, 0.08 mmol) was treated as described in Example 154, step (b) to give 14.5 mg (40% yield) of 4-{2-[(2,6-dichlorophenyl)amino](1,3-thiazol-4-yl)}-5-methylthiothiophene-2-carboxamidine hydrochloride. ¹H NMR (DMSO-d₆, 300 MHz) δ 2.69 (s, 3H), 7.15 (s, 1H), 7.18-7.26 (m, 2H), 8.13 (d, 1H, J=7.5 Hz), 8.41 (s, 1H), 8.84 (bs, 2H), 9.27 (bs, 2H), 9.61 (bs, 1H); Mass Spectrum (ESI) m/z calcd. for C₁₅H₁₂Cl₂N₄S₃, 415.39 (M+H), found 415.1, 417.1;

### Example 171

***a) Methyl 4-{2-[(2-bromo-4-methylphenyl)amino](1,3-thiazol-4-yl)}-5-methylthiothiophene-2-carboxylate hydrobromide:*** Methyl 4-(2-bromoacetyl)-5-methylthiothiophene-2-carboxylate (60 mg, 0.19 mmol) was allowed to react with 2-bromo-4-methylphenyl thiourea (47 mg) as described in Example 154, step (a) to give 62 mg (61% yield) of methyl 4-{2-[(2-bromo-4-methylphenyl)amino](1,3-thiazol-4-yl)}-5-methylthiothiophene-2-carboxylate hydrobromide. ¹H NMR (DMSO-d₆, 300 MHz) δ 2.28 (s, 3H), 3.82 (s, 3H), 7.19-7.23 (m, 1H), 7.27 (s, 1H), 7.48 (m, 1H), 8.14, 8.17 (s, 1H *rotomer*), 9.52, 9.72 (bs, 1H *rotomer*); Mass Spectrum (ESI) m/z calcd. for C₁₇H₁₅BrN₂O₂S₃, 455.42 (M+H), found 455.0, 457.0.

***b) 4-{2-[(2-Bromo-4-methylphenyl)amino](1,3-thiazol-4-yl)}-5-methylthiothiophene-2-carboxamidine hydrochloride:*** Methyl 4-{2-[(2-bromo-4-methylpheny)amino](1,3-thiazol-4-yl)}-5-methylthiothiophene-2-carboxylate hydrobromide (62 mg, 0.11 mmol) was treated as described in Example 154, step (b) to give 26 mg (50% yield) of 4-{2-[(2-bromo-4-methylphenyl)amino](1,3-thiazol-4-yl)}-5-methylthiothiophene-2-carboxamidine hydrochloride. ¹H NMR (DMSO-d₆, 300 MHz) δ 2.28 (s, 3H), 2.70 (s, 3H), 7.14 (s, 1H), 7.21 (dd, 1H, J=1.6, 8.5 Hz), 7.49 (d, 1H, J=1.5 Hz), 8.16 (d, 1H, 8.3 Hz), 8.41 (s, 1H), 8.85 (bs, 2H), 9.28 (bs, 2H), 9.53 (s, 1H); Mass Spectrum (ESI) m/z calcd. for C₁₆H₁₅BrN₄S₃, 439.42 (M+H), found 439.1, 441.1.

### Example 172

***a) Methyl 5-methylthio-4-{2-[(2-morpholin-4-ylethyl)amino](1,3-thiazol-4-yl)}thiophene-2-carboxylate hydrobromide:*** Methyl 4-(2-bromoacetyl)-5-methylthiothiophene-2-carboxylate (100 mg, 0.32 mmol), was allowed to react with 1-ethylmorpholinothiourea (61.2 mg) as described in Example 154, step (a) to give 120.8 mg (79% yield) methyl 5-methylthio-4-{2-[(2-morpholin-4-ylethyl)amino](1,3-thiazol-4-yl)}thiophene-2-carboxylate hydrobromide. ¹H NMR (CD₃OD, 300 MHz) δ 2.64 (s, 3H), 3.43-3.52 (m, 5H), 3.83-3.86 (m, 10H), 6.95 (s, 1H), 8.04 (s, 1H); Mass Spectrum (ESI) m/z calcd. for C₁₆H₂₁N₃O₃S₃, 399.55 (M+H), found 400.1.

***b) 5-Methylthio-4-{2-[2-morpholin-4-ylethyl)amino](1,3-thiazol-4-yl)}thiophene-2-carboxylate hydrochloride:*** Methyl- 5-methylthio-4-{2-[(2-morpholin-4-ylethyl)amino](1,3-thiazol-4-yl)}thiophene-2-carboxylate hydrobromide (62 mg, 0.12 mmol) was treated as described in Example 154, step (b) to give 26 mg (52% yield) of 5-methylthio-4-{2-[(2-morpholin-4-ylethyl)amino](1,3-thiazol-4-yl)}thiophene-2-carboxylate hydrochloride. ¹H NMR (DMSO-d₆, 300 MHz) δ 2.69 (s, 3H), 3.16-3.95 (m, 15H), 6.96 (s, 1H), 8.01 (bs, 1H), 8.49 (s, 1H), 8.84 (bs, 2H), 9.28 (bs, 2H), 10.49 (bs, 1H); Mass Spectrum (ESI) m/z calcd. for C₁₅H₂₁N₅OS₃, 383.56 (M+H), found 384.2.

### Example 173

***a) Methyl 4-{2-[(2,3-dichlorophenyl)amino](1,3-thiazol-4-yl)}-5-methylthiothiophene-2-carboxylate hydrobromide:*** Methyl 4-(2-bromoacetyl)-5-methylthiothiophene-2-carboxylate (60 mg, 0.19 mmol) was allowed to react with 2,3-dichlorophenylthiourea (42 mg) as described in Example 154, step (a) to give 60.5 mg (62% yield) methyl 4-{2-[(2,3-dichlorophenyl)amino](1,3-thiazol-4-yl)}-5-methylthiothiophene-2-carboxylate hydrobromide. ¹H NMR (DMSO-d₆, 300 MHz) δ 2.66 (s, 3H), 3.82 (s, 3H), 7.27 (dd, 1H, J=1.5, 6.5 Hz), 7.36 (d, 1H, J=8.2 Hz), 7.43 (s, 1H), 8.14 (s, 1H), 8.62 (dd, 1H, J=1.5, 8.4 Hz), 9.95 (bs. 1H); Mass Spectrum (ESI) m/z calcd. for C₁₆H₁₂C₁₂N₂O₂S₃, 431.38 (M+H), found 431.1, 433.0.

***b) 4-{2-[(2,3-Dichlorophenyl)amino](1,3-thiazol-4-yl)}-5-methylthiothiophene-2-carboxamidine hydrochloride:*** Methyl 4-{2-[(2,3-dichlorophenyl)amino](1,3-thiazol-4-yl)}-5-methylthiothiophene-2-carboxylate hydrobromide (60.5 mg, 0.11 mmol) was treated as described in Example 154, step (b) to give 15 mg (30% yield) of 4-{2-[(2,3-dichlorophenyl)amino](1,3-thiazol-4-yl)}-5-methylthiothiophene-2-carboxamidine hydrochloride. ¹H NMR (DMSO-d₆, 300 MHz) δ 2.71 (s, 3H), 7.27-7.28-7.41 (m, 2H), 8.45 (s, 1H), 8.63 (dd, 1H, J=1.5, 8.4 Hz), 8.84 (bs, 2H), 9.29 (bs, 2H), 9.99 (s, 1H); Mass Spectrum (ESI) m/z calcd. for C₁₅H₁₂Cl₂N₄S₃, 415.34 (M+H), found 415.1, 417.1;

### Example 174

***a) Methyl 5-methylthio-4-{2-[(3,4,5-trimethoxyphenyl)amino](1,3-thiazol-4-yl)}thiophene-2-carboxylate hydrobromide:*** Methyl 4-(2-bromoacetyl)-5-methylthiothiophene-2-carboxylate (60 mg, 0.19 mmol) was allowed to react with 2,3,4-trimethoxyphenylthiourea (46 mg) as described in Example 154, step (a) to give 61.8 mg (63% yield) of methyl 5-methylthio-4-{2-[(3,4,5-trimethoxyphenyl)amino](1,3-thiazol-4-yl)}thiophene-2-carboxylate hydrobromide. ¹H NMR (DMSO-d₆, 300 MHz) δ 2.67 (s, 3H), 3.81 (s, 6H), 3.82 (s, 3H), 7.11 (s, 2H), 7.25 (s, 1H), 8.19 (s, 1H), 10.25 (s, 1H); Mass Spectrum (ESI) m/z calcd. for C₁₈H₂₀N₄O₃S₃, 436.56 (M+H). found 437.1.

***b) 5-Methylthio-4-{2-[(3,4,5-trimethoxyphenyl)amino](1,3-thiazol-4-yl)}thiophene-2-carboxamidine hydrochloride:*** Methyl 5-methylthio-4-{2-[(3,4,5-trimethoxyphenyl)amino](1,3-thiazol-4-yl)}thiophene-2-carboxylate hydrobromide (61.8 mg, 0.11 mmol) was treated as described in Example 154, step (b) to give 14 mg (27% yield) of 5-methylthio-4-{2-[(3,4,5-trimethoxyphenyl)amino](1,3-thiazol-4-yl)}thiophene-2-carboxamidine hydrochloride. ¹H NMR (DMSO-d₆, 300 MHz) δ 2.70 (s, 3H), 3.61 (s, 3H), 3.80 (s, 6H), 7.08 (s, 2H), 7.14 (s, 1H), 8.44 (s, 1H), 8.84 (bs, 2H), 9.26 (bs, 2H), 10.29 (s, 1H); Mass Spectrum (ESI) m/z calcd. for C₁₈H₂₀N₄O₃S₃, 436.56 (M+H), found 437.1.

### Example 175

***a) Methyl 5-methylthio-4-{2-[(2-piperidylethyl)amino](1,3-thiazol-4-yl)}thiophene-2-carboxylate hydrobromide:*** Methyl 4-(2-bromoacetyl)-5-methylthiothiophene-2-carboxylate (100 mg, 0.32 mmol) was allowed to react with N-ethylpiperidylthiourea (60.6 mg) as described in Example 154, step (a) to give 90 mg (59% yield) of methyl 5-methylthio-4-{2-[(2-piperidylethyl)amino](1,3-thiazol-4-yl)}thiophene-2-carboxylate hydrobromide. ¹H NMR (DMSO-d₆, 300 MHz) δ 1.41 (m, 2H), 1.70-1.79 (m, 6H), 2.65 (s, 3H), 2.95 (m, 2H), 3.52 (m, 2H), 3.73 (m, 2H), 3.82 (s, 3H), 7.08 (s, 1H), 7.96 (at, 1H, J=5.3 Hz), 8.09 (s, 1H), 9.40 (bs, 1H); Mass Spectrum (ESI) m/z calcd. for C₁₇H₂₃N₃O₂S₃, 397.6 (M+H), found 398.1.

***b) 5-Methylthio-4-{2-[(2-piperidylethyl)amino](1,3-thiazol-4-yl)}thiophene-2-carboxamidine hydrochloride:*** Methyl 5-methylthio-4-{2-[(2-piperidylethyl}amino](1,3-thiazol-4-yl)}thiophene-2-carboxylate hydrobromide (72 mg, 0.15 mmol) was treated as described in Example 154, step (b) to give 26.8 mg (43% yield) of 5-methylthio-4-{2-[(2-piperidylethyl)amino](1,3-thiazol-4-yl)}thiophene-2-carboxamidine hydrochloride. ¹H NMR (DMSO-d₆, 300 MHz) δ 1.40 (m, 2H), 1.72-1.79 (m, 6H), 2.69 (s, 3H), 2.96 (m, 2H), 3.51 (m, 2H), 3.76 (m, 2H), 6.97 (s, 1H), 8.08 (t, 1H, J=5.5 Hz), 8.60 (s, 1H), 8.95 (bs, 1H), 9.35 (bs, 2H), 10.25 (s, 1H); Mass Spectrum (ESI) m/z calcd. for C₁₆H₂₃N₅S₃, 381.1 (M+H), found 382.2.

### Example 176

***a) Methyl 4-(2-{[(4-methylphenyl)methyl]amino}(1,3-thiazol-4-yl))-5-methylthiothiophene-2-carboxylate hydrobromide:*** Methyl 4-(2-bromoacetyl)-5-methylthiothiophene-2-carboxylate (111 mg, 0.35 mmol) was allowed to react with 4-methylphenylmethylthiourea as described in Example 154, step (a) to give 125 mg (81% yield) of methyl 4-(2-{[(4-methylphenyl)methyl]amino}(1,3-thiazol-4-yl))-5-methylthiothiophene-2-carboxylate hydrobromide. Mass Spectrum (ESI) m/z calcd. for C₁₈H₁₈N₂O₂S₂, 358.5 (M+H), found 359.1.

***b) 4-(2-{[(4-Methylphenyl)methyl]amino}(1,3-thiazol-4-yl))-5-methylthiothiophene-2-carboxamidine hydrochloride:*** Methyl 4-(2-{[(4-methylphenyl)methyl]amino}(1,3-thiazol-4-yl))-5-methylthiothiophene-2-carboxylate hydrobromide (118 mg, 0.26 mmol) was treated as described in Example 154, step (b) to give 58.2 mg (54% yield) of 4-(2-{[(4-methylphenyl)methyl]amino}(1,3-thiazol-4-yl))-5-methylthiothiophene-2-carboxamidine hydrochloride. ¹H NMR (DMSO-d₆, 300 MHz) δ 2.27 (s, 3H), 2.66 (s, 3H), 4.49 (d, 2H, J=5.7 Hz), 6.88 (s, 1H), 7.13 (d, 2H, J=7.8 Hz), 7.27 (d, 2H, J=8.0 Hz), 8.20 (t, 1H, J=5.8 Hz), 8.42 (s, 1H), 8.90 (bs, 2H), 9.27 (bs, 2H); Mass Spectrum (ESI) m/z calcd. for C₁₇H₁₈N₄S₃, 374.55 (M+H), found 375.2.

### Example 177

***a) Amino{[4-(4-chlorophenoxy)phenyl]amino}methane-1-thione:*** Unless otherwise indicated, all thioureas, isothiocyanates, thioamides and amines were purchased from Maybridge Chemical Co. Ltd.(Cornwall, U.K.), Transworld Chemical Co. (Rockville, MD), or Aldrich Chemical Co., (Milwaukee, WI). (a) 4-Amino-4'-chlorodiphenylether (TCI America, Portland OR, 520 mg, 2.03 mmol) was slurried in 10 mL of ether and treated with ca. 1 mL of ether saturated with HCl gas. After 5 min. the solvent was removed *in vacuo.* To a stirring biphasic solution amine-HCl salt in 20 mL CHCl₃-sat'd NaHCO₃ (1:1, *v*/*v*) at ambient temperature was added thiophosgene (1.2 equiv, 2.4 mmol) in 5 mL of CHCl₃ dropwise via an addition funnel. The reaction was vigorously stirred for 1 h (TLC, 50% ethyl acetate-hexanes indicates clean conversion to a higher Rf spot), at which time the layers were separated, the aqueous layer extracted with CHCl₃ (1x20 mL), and the combined organic layers washed with brine (1x20 mL) and dried (Na₂SO₄). Concentration of the solvent *in vacuo* yielded the crude 4-(4-chlorophenoxy)-phenylisothiocyanate (414 mg). (b) The 4-(4-chlorophenoxy)-phenylisothiocyanate was transferred to an Ace Glass pressure tube equipped with a Teflon coated stir bar and treated with a 2.0 M solution of NH₃ in 5 ml methanol (Aldrich Chemical Co., Milwaukee, WI)). The tube was sealed and immersed in a 80°C oil bath. After 2 h, the reaction was cooled to 0°C in an ice bath. The precipitates were filtered and dried under vacuum to yield amino{[4-(4-chlorophenoxy)phenyl]amino}methane-1-thione (328 mg, 79%). ¹H NMR (DMSO-d₆, 300 MHz) 7.02 (m, 4H), 7.41 (m, 4H), 9.65 (s, 1H); Mass Spectrum (ESI) m/z calcd. for C₁₃H₁₁ClN₂OS, 278.8 (M+H), found 279.4.

***b) Methyl 4-(2-{[4-(4-chlorophenoxy)phenyl]amino}(1,3-thiazol-4-yl))-5-methylthiothiophene-2-carboxylate hydrobromide:*** Methyl 4-(2-bromoacetyl)-5-methylthiothiophene-2-carboxylate (309 mg, 1.0 mmol) was allowed to react with amino {[4-(4-chlorophenoxy)phenyl]amino}methane-1-thione (297 mg) as described in Example 154, step (a) to give 410 mg (72% yield) of methyl 4-(2-{[4-(4-chlorophenoxy)phenyl]amino} (1,3-thiazol-4-yl))-5-methylthiothiophene-2-carboxylate hydrobromide. Mass Spectrum (ESI) m/z calcd. for C₂₂H₁₇ClN₂O₃S₃, 489.1 (M+H), found 489.1.

***c) 4-(2-{[4-(4-Chlorophenoxy)phenyl]amino}(1,3-thiazol-4-yl))-5-methylthiothiophene-2-carboxamidine hydrochloride:*** Methyl 4-(2-{[4-(4-chlorophenoxy)phenyl]amino}(1,3-thiazol-4-yl))-5-methylthiothiophene-2-carboxylate hydrobromide (300 mg, 0.52 mmol) was treated as described in Example 154, step (b) to give 129.9 mg (49% yield) of 4-(2-{[4-(4-chlorophenoxy)phenyl] amino}(1,3-thiazol-4-yl))-5-methylthiothiophene-2-carboxamidine hydrochloride. ¹H NMR (DMSO-d₆, 300 MHz) 8 2.72 (s, 3H), 6.97 (m, 2H), 7.07 (m, 2H), 7.15 (s, 1H), 7.40 (m, 2H), 7.85 (m, 2H), 8.46 (s, 1H), 8.82 (bs, 2H), 9.27 (bs, 2H), 10.43 (bss, 1H); Mass Spectrum (ESI) m/z calcd. for C₂₁H₁₇ClN₄OS₃, 473.1 (M+H), found 473.2, 475.1.

### Example 178

***a) Methyl 5-methylthio-4-[2-({4-[5-(trifluoromethyl)(2-pyridyloxy)]phenyl}amino)(1,3-thiazol-4-yl)]thiophene-2-carboxylate:*** Methyl 4-(2-bromoacetyl)-5-methylthiothiophene-2-carboxylate (70 mg, 0.23 mmol) was allowed to react with 4-[5-(trifluoromethyl)pyrid-2-yloxy]thiobenzamide (50 mg) as described in Example 154, step (a) to give 115 mg (98% yield) of methyl 5-methylthio-4-[2-({4-[5-(trifluoromethyl)(2-pyridyloxy)]phenyl}amino)(1,3-thiazol-4-yl)]thiophene-2-carboxylate. ¹H NMR (DMSO-d₆, 300 MHz) δ 2.70 (s, 3H), 3.85 (s, 3H), 7.38 (m, 3H), 8.10 (m, 1H), 8.18 (s, 1H), 8.28 (dd, 1H, J=2.7, 8.8 Hz), 8.32 (s, 1H), 8.60 (m, 1H); Mass Spectrum (ESI) m/z calcd. for C₂₂H₁₅F₃N₂O₃S₃, 508.56 (M+H), found 509.2.

***b) 5-Methylthio-4-[2-({4-[5-(trifluoromethyl)(2-pyridyloxy)]phenyl}amino)(1,3-thiazol-4-yl)]thiophene-2-carboxamidine hydrochloride:*** Methyl 5-methylthio-4-[2-({4-[5-(trifluoromethyl)(2-pyridyloxy)]phenyl}amino)(1,3-thiazol-4-yl)]thiophene-2-carboxylate (95 mg, 0.18 mmol) was treated as described in Example 154, step (b) to give 30.3 mg (32% yield) of 5-methylthio-4-[2-({4-[5-(trifluoromethyl)(2-pyridyloxy)]phenyl}amino)(1,3-thiazol-4-yl)]thiophene-2-carboxamidine hydrochloride. ¹H NMR (DMSO-d₆, 300 MHz) δ 2.75 (s, 3H), 7.34 (d, 1H, J=8.7 Hz), 7.41 (m, 2H), 8.01 (s, 1H), 8.10-8.14 (m, 2H), 8.29 (dd, 1H, J=2.5, 8.4 Hz), 8.60 (m, 1H), 8.63 (s, 1H), 8.91 (bs, 2H), 9.31 (bs, 2H); Mass Spectrum (ESI) m/z calcd. for C₂₁H₁₅F₃N₄OS₃, 492.6 (M+H), found 493.1.

### Example 179

***a) Methyl 4-(2-{[4-phenoxyphenyl]amino}(1,3-thiazol-4-yl))-5-methylthiothiophene-2-carboxylate hydrobromide:*** Methyl 4-(2-bromoacetyl)-5-methylthiothiophene-2-carboxylate (200 mg, 0.64 mmol) was allowed to react with 4-phenoxyphenylthiourea (158 mg) as described in Example 154, step (a) to give 300 mg (88% yield) of methyl 4-(2-{[4-(phenoxy)phenyl]amino}(1,3-thiazol-4-yl))-5-methylthiothiophene-2-carboxylate hydrobromide. Mass Spectrum (ESI) m/z calcd. for C₂₂H₁₈N₂O₃S₃, 454.6 (M+H), found 455.2.

***b) 4-(2-{[4-Phenoxyphenyl]amino}(1,3-thiazol-4-yl))-5-methylthiothiophene-2-carboxamidine hydrochloride:*** Methyl 4-(2-{[4-(phenoxy)phenyl]amino}(1,3-thiazol-4-yl))-5-methylthiothiophene-2-carboxylate hydrobromide (230 mg, 0.42 mmol) was treated as described in Example 154, step (b) and purified by preparative thin layer chromatography (20% methanol-CH₂Cl₂-sat'd. NH₃, 500 µm silica gel plate, J.T. Baker, Phillipsburg, NJ) to give 86 mg (47% yield) of the product. A 46 mg aliquot was dissolved in 1 mL of methanol, treated with 3 drops of ether saturated with HCl gas, and concentrated *in vacuo* with toluene (2x5mL) to give 42.3 mg (21% yield) of 4-(2-{[4-phenoxyphenyl]amino}(1,3-thiazol-4-yl))-5-methylthiothiophene-2-carboxamidine hydrochloride. ¹H NMR (DMSO-d₆, 300 MHz) δ 2.71 (s, 3H), 6.97-7.11 (m, 4H), 7.15 (s, 1H), 7.36 (m, 2H), 7.72, 7.85 (d, 2H rotomer, J=8.7 Hz), 8.36, 8.55 (s, 1H rotomer), 9.00 (bs, 2H), 9.35 (bs, 2H), 10.49 (s, 1H); , Mass Spectrum (ESI) m/z calcd. for C₂₁H₁₈N₄OS₃, 438.6 (M+H), found 439.2.

### Example 180

***a) Amino{[4-(phenylamino)phenyl]amino}methane-1-thione:*** 4-Aminodiphenylamine (500 mg, 2.71 mmol) was treated as described in Example 177, step (a) and recrystallized from toluene to give 350 mg (53% yield) of amino{[4-(phenylamino)phenyl]amino}methane-1-thione. ¹H NMR (DMSO-d₆, 300 MHz) δ 6.80 (m, 1H), 7.01-7.24 (m, 8H), 8.15 (s, 1H), 9.45 (s, 1H); Mass Spectrum (ESI) m/z calcd. for C₁₃H₁₃N₃S, 243.33 (M+H), found 244.2.

***b) Methyl 5-methylthio-4-(2-{[4-(phenylamino)phenyl]amino}(1,3-thiazol-4-yl))thiophene-2-carboxylate hydrobromide:*** Methyl 4-(2-bromoacetyl)-5-methylthiothiophene-2-carboxylate (90 mg, 0.28 mmol) was allowed to react with amino{[4-(phenylamino)phenyl]amino}methane-1-thione (70.8 mg) as described in Example 154, step (a) to give 71 mg (47% yield) of methyl 5-methylthio-4-(2-{[4-(phenylamino)phenyl]amino}(1,3-thiazol-4-yl))thiophene-2-carboxylate hydrobromide. ¹H NMR (DMSO-d₆, 300 MHz) δ 2.66 (s, 3H), 3.82 (s, 3H), 6.73 (m, 1H), 6.96-7.24 (m, 9H), 7.63 (d, 1H, J=8.6 Hz), 8.12 (s, 1H), 10.13 (bs, 1H); Mass Spectrum (ESI) m/z calcd. for C₂₂H₁₉N₃O₂S₃, 453.60 (M+H), found 454.2.

***c) 5-Methylthio-4-(2-{[4-(phenylamino)phenyl]amino}(1,3-thiazol-4-yl))thiophene-2-carboxamidine hydrochloride:*** Methyl 5-methylthio-4-(2-{[4-(phenylamino)phenyl]amino}(1,3-thiazol-4-yl))thiophene-2-carboxylate hydrobromide (71 mg, 0.13 mmol) was treated as described in Example 154, step (b) to give 23.3 mg (38% yield) of 5-methylthio-4-{2-{[4-(phenylamino)phenyl]amino}(1,3-thiazol-4-yl))thiophene-2-carboxamidine hydrochloride. ¹H NMR (DMSO-d₆, 300 MHz) δ 2.72 (s, 3H), 6.74 (t, 1H, J=7.3 Hz), 6.98 (d, 1H, J=7.6 Hz), 7.08 (m, 2H), 7,18 (m, 2H), 7.66 (d, 2H, J=8.9 Hz), 7.99 (s, 1H), 8.45 (s, 1H), 9.03 (bs, 4H), 10.17 (s, 1H); Mass Spectrum (ESI) m/z calcd. for C₂₁H₁₉N₅S₃, 437.59 (M+H), found 438.2.

### Example 181

***a) Amino{[4-benzylphenyl]amino}methane-1-thione:*** 4-Benzylphenylamine (500 mg, 2.73 mmol) was treated as described in Example 177, step (a) to give 410 mg (62% yield) of amino{[4-benzylphenyl]amino}methane-1-thione. ¹H NMR (DMSO-d₆, 300 MHz) δ 3.89 (s, 2H), 7.14-7.28 (m, 9H), 9.59 (s, 1H); Mass Spectrum (ESI) m/z calcd. for C₁₄H₁₄N₂S₃, 242.1 (M+H), found 243.2.

***b) Methyl 5-methylthio-4-(2-{[4-benzylphenyl]amino}(1,3-thiazol-4-yl))thiophene-2-carboxylate hydrobromide:*** Methyl 4-(2-bromoacetyl)-5-methylthiothiophene-2-carboxylate (90 mg, 0.28 mmol) was allowed to react with amino{[4-benzylphenyl]amino}methane-1-thione (70.5 mg) as described in Example 154, step (a) to give 70.1 (47% yield) of methyl 5-methylthio-4-(2-{[4-benzylphenyl]amino}(1,3-thiazol-4-yl))thiophene-2-carboxylate hydrobromide. ¹H NMR (DMSO-d₆, 300 MHz) δ 2.66 (s, 3H), 3.82 (s, 3H), 3.87 (s, 2H), 7.14-7.30 (m, 8H), 7.66 (d, 2H, J=8.5 Hz), 8.12 (s, 1H), 10.23 (s, 1H); (Mass Spectrum (ESI) m/z calcd. for C₂₂H₁₉N₃O₂S₃, 453.6 (M+H), found 454.2.

***c) 5-Methylthio-4-(2-{[4-benzylphenyl]amino}(1,3-thiazol-4-yl))thiophene-2-carboxamidine hydrochloride:*** Methyl 5-methylthio-4-(2-{[4-benzylphenyl]amino}(1,3-thiazol-4-yl))thiophene-2-carboxylate hydrobromide (82.2 mg, 0.15 mmol) was treated as described in Example 154, step (b) to give 33.4 mg (47% yield) of 5-methylthio-4-(2-{[4-benzylphenyl]amino}(1,3-thiazol-4-yl))thiophene-2-carboxamidine hydrochloride. ¹H NMR (DMSO-d₆, 300 MHz) δ 2.72 (s, 3H), 3.89 (s, 2H), 7.12 (s, 1H), 7.16-7.29 (m, 7H), 7.69 (d, 2H, J=8.6 Hz), 8.43 (s, 1H), 9.02 (bs, 4H), 10.28 (s, 1H); Mass Spectrum (ESI) m/z calcd. for C₂₂H₂₀N₄S₃, 436.6 (M+H), found 437.2.

### Example 182

***a) ({4-[(Aminothioxomethyl)amino]phenyl}sulfonyl)piperidine:*** 4-Aminophenylsulphonylpiperidine (500 mg, 2.08 mol) was treated as described in Example 177, step (a) to give 382 mg (61% yield) of ({4-[(aminothioxomethyl)amino]phenyl}sulfonyl)piperidine. ¹H NMR (DMSO-d₆, 300 MHz) δ 1.34 (m, 2H), 1.53 (m, 4H), 2.85 (m, 4H), 7.62 (m, 2H), 7.78 (m, 2H), 10.10 (bs, 1H); Mass Spectrum (ESI) m/z calcd. for C₁₂H₁₇N₃O₂S₂, 299.4 (M+H), found 300.2.

***b) Methyl 5-methylthio-4-(2-{[4-(piperidylsulfonyl)phenyl]amino}(1,3-thiazol-4-yl))thiophene-2-carboxylate hydrobromide:*** Methyl 4-(2-bromoacetyl)-5-methylthiothiophene-2-carboxylate (90 mg, 0.28 mmol) was allowed to react with ({4-[(aminothioxomethyl)amino]phenyl}sulfonyl)piperidine (87.1 mg) as described in Example 154, step (a) to give 105 mg (63% yield) of methyl 5-methylthio-4-(2-{[4-(piperidylsulfonyl)phenyl]amino}(1,3-thiazol-4-yl))thiophene-2-carboxylate hydrobromide. ¹H NMR (DMSO-d₆, 300 MHz) δ 1.33 (m, 2H), 1.52 (m, 4H), 2.69 (s, 3H), 2.84 (m, 4H), 3.82 (s, 3H), 7.43 (s, 1H), 7.66 (m, 2H), 7.98 (m, 2H), 8.16 (s, 1H), 10.85 (s, 1H); (Mass Spectrum (ESI) m/z calcd. for C₂₁H₂₃N₃O₄S₄, 509.69 (M+H), found 510.2.

***c) 5-Methylthio-4-(2-{[4-(piperidylsulfonyl)phenyl]amino}(1,3-thiazol-4-yl))thiophene-2-carboxamidine hydrochloride:*** Methyl 5-methylthio-4-(2-{[4-(piperidylsulfonyl)phenyl]amino}(1,3-thiazol-4-yl))thiophene-2-carboxylate hydrobromide (105 mg, 0.17 mmol) was treated as described in Example 154, step (b) to give 30.3 mg (34% yield) of 5-methylthio-4-(2-{[4-(piperidylsulfonyl)phenyl]amino}(1,3-thiazol-4-yl))thiophene-2-carboxamidine hydrochloride. ¹H NMR (DMSO-d₆, 300 MHz) δ 1.36 (m, 2H), 1.54 (m, 4H), 2.76 (s, 3H), 2.86 (m, 4H), 7.30 (s, 1H), 7.68 (d, 2H, J=8.8 Hz), 8.03 (d, 2H, J=8.8 Hz), 8.51 (s, 1H), 8.84 (bs, 2H), 9.28 (bs, 2H), 10.94 (s, 1H); Mass Spectrum (ESI) m/z calcd. for C₂₀H₂₃N₅O₂S₅, 493.69 (M+H), found 494.2.

### Example 183

***a) Amino(3-quinolylamino)methane-1-thione:*** 3-Aminooquinoline (500 mg, 3.46 mmol) was treated as described in Example 177, step (a) to give 285 mg (41% yield) of amino(3-quinolylamino)methane-1-thione. ¹H NMR (DMSO-d₆, 300 MHz) δ 7.57 (m, 1H), 7.67 (m, 1H), 7.94 (m, 2H), 8.41 (d, 1H, J=2.4 Hz), 8.85 (d, 1H, J=2.5 Hz), 10.03 (s, 1H); Mass Spectrum (ESI) m/z calcd. for C₁₀H₉N₃S, 203.3 (M+H), found 204.1.

***b) Methyl 5-methylthio-4-[2-(3-quinolylamino)(1,3-thiazol-4-yl)]thiophene-2-carboxylate:*** Methyl 4-(2-bromoacetyl)-5-methylthiothiophene-2-carboxylate (90 mg, 0.28 mmol) was allowed to react with amino(3-quinolylamino)methane-1-thione (59.1 mg) as described in Example 154, step (a) to give 107.5 mg (78% yield) of methyl 5-methylthio-4-[2-(3-quinolylamino)(1,3-thiazol-4-yl)]thiophene-2-carboxylate hydrobromide. ¹H NMR (DMSO-d₆, 300 MHz) δ 2.75 (s, 3H), 3.84 (s, 3H), 7.52 (s, 1H), 7.92-8.05 (m, 2H), 8.22 (s, 1H), 9.22 (m, 2H); Mass Spectrum (ESI) m/z calcd. for C₁₉H₁₅N₃O₂S₃, 413.54 (M+H), found 414.1.

***c) 5-Methylthio-4-[2-(3-quinolylamino)(1,3-thiazol-4-yl)]thiophene-2-carboxamidine hydrochloride:*** Methyl 5-methylthio-4-[2-(3-quinolylamino)(1,3-thia2ol-4-yl)]thiophene-2-carboxylate hydrobromide (107.5 mg, 0.21 mmol) was treated as described in Example 154, step (b) to give 4.5 mg (4.9% yield) of 5-methylthio-4-[2-(3-quinolylamino)(1,3-thiazol-4-yl)]thiophene-2-carboxamidine hydrochloride. ¹H NMR (DMSO-d₆, 300 MHz) δ 2.80 (s, 3H), 7.29 (s, 1H), 7.59 (m, 2H), 7.93 (m, 2H), 8.54 (s, 1H), 8.89 (bs, 2H), 8.91 (m, 1H), 9.16 (m, 1H), 9.29 (bs, 2H), 10.97 (s, 1H); Mass Spectrum (ESI) m/z calcd. for C₁₈H₁₅N₅S₃, 397.5 (M+H), found 398.1.

### Example 184

***a) Methyl 5-methylthio-4-[2-(2-naphthylamino)(1,3-thiazol-4-yl)]thiophene-2-carboxylate hydrobromide:*** Methyl 4-(2-bromoacetyl)-5-methylthiothiophene-2-carboxylate (65 mg, 0.21 mmol) was allowed react with 2-napthylthiourea (42.4 mg) as described in Example 154, step (a) to give 82.5 mg (80% yield)of methyl 5-methylthio-4-[2-(2-naphthylamino)(1,3-thiazol-4-yl)]thiophene-2-carboxylate hydrobromide. ¹H NMR (DMSO-d₆, 300 MHz) δ 2.67 (s, 3H), 3.83 (s, 3H), 7.31 (s, 1H), 7.50-7.67 (m, 4H), 7.93 (m, 1H), 8.15 (s, 1H), 8.31-8.35 (m, 1H), 8.46 (d, 1H, J=7.6), 10.22 (s, 1H)); Mass Spectrum (ESI) m/z calcd. for C₂₀H₁₆N₂O₂S₃, 412.6 (M+H), found 413.1.

***c) 5-Methylthio-4-[2-(2-naphthylamino)(1,3-thiazol-4-yl)]thiophene-2-carboxamidine hydrochloride:*** Methyl 5-methylthio-4-[2-(2-naphthylamino)(1,3-thiazol-4-yl)]thiophene-2-carboxylate hydrobromide (42.7 mg, 0.086 mmol) was treated as described in Example 154, step (b) to give 5.8 mg (16% yield) of 5-methylthio-4-[2-(2-naphthylamino)(1,3-thiazol-4-yl)]thiophene-2-carboxamidine hydrochloride. ¹H NMR (DMSO-d₆, 300 MHz) δ 2.72 (s, 3H), 7.12-7.27 (m, 3H), 7.50-7.68 (m, 3H), 7.94 (m, 1H), 8.32-8.35 (m, m, 1H), 8.51 (s, 1H), 8.97 (bs, 2H), 9.34 (bs, 2H), 10.26 (s, 1H); Mass Spectrum (ESI) m/z calcd. for C₁₉H₁₆N₄S₃, 396.6 (M+H), found 397.2.

### Example 185

***a) Methyl 4-[2-(2H-benzo[3,4-d]1,3-dioxolan-5-ylamino)(1,3-thiazol-4-yl)]-5-methylthiothiophene-2-carboxylate hydrobromide:*** Methyl 4-(2-bromoacetyl)-5-methylthiothiophene-2-carboxylate (65 mg, 0.21 mmol) was allowed to react with 2,3-methylenedioxyphenylthiourea (41.2 mg) as described in Example 154, step (a) to give 51 mg (50% yield) of methyl 4-[2-(2H-benzo[3,4-d]1,3-dioxolan-5-ylamino)(1,3-thiazol-4-yl)]-5-methylthiothiophene-2-carboxylate hydrobromide. ¹H NMR (DMSO-d₆, 300 MHz) δ 2.66 (s, 3H), 3.83 (s, 3H), 5.98 (s, 2H), 6.84-6.89 (m, 1H), 6.96, 7.04 (dd, 1H *rotomer*, J=2.2, 8.5 Hz), 7.25 (s, 1H), 7.46, 7.60 (d, 1H *rotomer*, J=2.1 Hz), 8.05, 8.13 (s, 1H *rotomer),* 10.19, 10.34 (s, 1H, *rotomer);* Mass Spectrum (ESI) m/z calcd. for C₁₇H₁₄N₂O₄S₃, 406.5 (M+H), found 407.1.

***b) 4-[2-(2H-Benzo[3,4-d]1,3-dioxolan-5-ylamino)(1,3-thiazol-4-yl)]-5-methylthiothiophene-2-carboxamidine hydrochloride:*** Methyl 4-[2-(2H-benzo[3,4-d] 1,3-dioxolan-5-ylamino)(1,3-thiazol-4-yl)]-5-methylthiothiophene-2-carboxylate hydrobromide (51 mg, 0.10 mmol) was treated as described in Example 154, step (b) to give 16.6 mg (39% yield) of 4-[2-(2H-benzo[3,4-d]1,3-dioxolan-5-ylamino)(1,3-thiazol-4-yl)]-5-methylthiothiophene-2-carboxamidine hydrochloride. ¹H NMR (DMSO-d₆, 300 MHz) δ 2.71(s, 3H), 5.98 (s, 2H), 6.87 (d, 1H, J=8.2 Hz), 7.09-7.13 (m, 2H), 7.67 (d, 1H, J=2.4 Hz), 8.50 (s, 1H), 8.95 (bs, 2H), 9.33 (bs, 2H), 10.30 (s, 1H); Mass Spectrum (ESI) m/z calcd. for C₁₆H₁₄N₄O₂S3, 390.51 (M+H), found 391.2;

### Example 186

***a) Amino[(7-bromofluoren-2-yl)amino]methane-1-thione:*** 2-Amino-7-bromofluorene (500 mg, 1.90 mmol) was treated as described in Example 177, step (a) to give 128 mg (21% yield) of amino[(7-bromofluoren-2-yl)amino]methane-1-thione. ¹H NMR (DMSO-d₆, 300 MHz) δ 3.35 (s, 2H), 7.35 (d, 1H, J=8.3 Hz), 7.54 (d, 1H, J=8.0 Hz), 7.66 (s, 1H), 7.77-7.87 (m, 3H), 9.80 (s, 1H); Mass Spectrum (ESI) m/z calcd. for C₁₄H₁₁BrN₂S, 319.2 (M+H), found 320.1, 321.1.

***b) Methyl 4-{2-[(7-bromofluoren-2-yl)amino](1,3-thiazol-4-yl)}-5-methylthiothiophene-2-carboxylate hydrobromide:*** Methyl 4-(2-bromoacetyl)-5-methylthiothiophene-2-carboxylate (90 mg, 0.28 mmol) was allowed to react with amino[(7-bromofluoren-2-yl)amino]methane-1-thione (92.8 mg) as described in Example 154, step (a) to give 141 mg (82% yield) of methyl 4-{2-[(7-bromofluoren-2-yl)amino](1,3-thiazol-4-yl)}-5-methylthiothiophene-2-carboxylate hydrobromide. ¹H NMR (DMSO-d₆, 300 MHz) δ 2.70 (s, 3H), 3.83 (s, 3H), 3.93 (s, 2H), 7.33 (s, 1H), 7.51 (dd, 1H, J=1.9, 8.0 Hz), 7.65 (dd, 1H, J=2.0, 8.4 Hz), 7.74 (ad, 2H, J=8.3 Hz), 7.83 (ad, 1H, J=8.4 Hz), 8.18 (s, 1H), 8.23 (d, 1H, J=1.4 Hz), 10.47 (s, 1H).

***c) 4-{2-[(7-Bromofluoren-2-yl)amino](1,3-thiazol-4-yl)}-5-methylthiothiophene-2-carboxamidine hydrochloride:*** Methyl 4-{2-[(7-bromofluoren-2-yl)amino](1,3-thiazol-4-yl)}-5-methylthiothiophene-2-carboxylate hydrobromide (100 mg, 0.15 mmol) was treated as described in Example 154, step (b) to give 3.3 mg (4% yield) of 4-{2-[(7-bromofluoren-2-yl)amino](1,3-thiazol-4-yl)}-5-methylthiothiophene-2-carboxamidine hydrochloride. ¹H NMR (DMSO-d₆, 300 MHz) δ 2.76 (s, 3H), 3.95 (s, 2H), 7.18 (s, 1H), 7.54 (dd, 1H, J=1.8, 10.0 Hz), 7.67-7.76 (m, 3H), 7.85 (d, 1H, J=8.2 Hz), 8.23 (s, 1H), 8.50 (s, 1H), 10.53 (s, 1H); Mass Spectrum (ESI) m/z calcd. for C₂₂H₁₇BrN₄S₃, 513.5 (M+H), found 513.1, 515.1.

### Example 187

***a) Methyl 4-{2-[(4-cyclohexylphenyl)amino](1,3-thiazol-4-yl)}-5-methylthiothiophene-2-carboxylate hydrobromide:*** Methyl 4-(2-bromoacetyl)-5-methylthiothiophene-2-carboxylate (65 mg, 0.21 mmol) was allowed to react with 4-cyclohexylphenylthiourea (49.2 mg) as described in Example 154, step (a) to give 45 mg (41% yield) of methyl 4-{2-[(4-cyclohexylphenyl)amino](1,3-thiazol-4-yl)}-5-methylthiothiophene-2-carboxylate hydrobromide. ¹H NMR (DMSO-d₆, 300 MHz) δ 1.23-1.39 (m, 5H), 1.71-1.79 (m, 5H), 2.68 (s, 3H), 3.83 (s, 3H), 7.16 (d, 2H, J=8.6 Hz), 7.26 (s, 1H), 7.65 (d, 2H, J=8.7 Hz), 8.14 (s, 1H), 10.19 (s, 1H); Mass Spectrum (ESI) m/z calcd. for C₂₂H₂₄N₂O₂S₃, 444.64 (M+H), found 445.2.

***b) 4-{2-[(4-Cyclohexylphenyl)amino](1,3-thiazol-4-yl)}-5-methylthiothiophene-2-carboxamidine hydrochloride:*** Methyl 4-{2-[(4-cyclohexylphenyl}amino](1,3-thiazol-4-yl)}-5-methylthiothiophene-2-carboxylate hydrobromide (31.1 mg, 0.059 mmol) was treated as described in Example 154, step (b) to give 12.8 mg (47% yield) of 4-{2-[(4-cyclohexylphenyl)amino](1,3-thiazol-4-yl)}-5-methylthiothiophene-2-carboxamidine hydrochloride. ¹H NMR (DMSO-d₆, 300 MHz) δ 1.33-1.40 (m, 5H), 1.68-1.79 (m, 5H), 2.44 (m, 1H), 2.73 (s, 3H), 7.12 (s, 1H), 7.18 (d, 2H, J=8.7 Hz), 7.68 (d, 2H, J=8.7 Hz), 8.47 (s, 1H), 8.85 (bs, 2H), 9.32 (bs, 2H), 10.28 (s, 1H); Mass Spectrum (ESI) m/z calcd. for C₂₁H₂₄N₄S₃, 428.64 (M+H), found 429.2.

### Example 188

***a) Amino{[4-(phenyldiazenyl)phenyl]amino}methane-1-thione:*** 4-Phenylazophenylisothiocyanate (314 mg, 1.30 mmol) was treated as described in Example 177, step (a), part (b), to give 295 mg (88% yield) of amino {[4-(phenyldiazenyl)phenyl]amino}methane-1-thione. ¹H NMR (DMSO-d₆, 300 MHz) δ 6.84 (m, 1H), 7.57 (m, 2H), 7.73 (m, 2H), 7.85-7.89 (m, 4H), 10.04 (s, 1H); Mass Spectrum (ESI) m/z calcd. for C₁₃H₁₂N₄S, 256.3 (M+H), found 257.2.

***b) Methyl 5-methylthio-4-(2-{[4-(phenyldiazenyl)phenyl]amino}(1,3-thiazol-4-yl))thiophene-2-carboxylate hydrobromide:*** Methyl 4-(2-bromoacetyl)-5-methylthiothiophene-2-carboxylate (65 mg, 0.21 mmol) was allowed to react with amino{[4-(phenyldiazenyl)phenyl]amino}methane-1-thione (53.8 mg) as described in Example 154, step (a) to give 80.6 mg (70% yield) of methyl 5-methylthio-4-(2-{[4-(phenyldiazenyl)phenyl]amino}(1,3-thiazol-4-yl))thiophene-2-carboxylate hydrobromide. ¹H NMR (DMSO-d₆, 300 MHz) δ 2.72 (s, 3H), 3.84 (s, 3H), 7.46 (s, 1H), 7.49-7.61 (m, 3H), 7.84 (m, 2H), 7.91-8.02 (m, 4H), 8.20 (s, 1H), 10.83 (s, 1H); Mass Spectrum (ESI) m/z calcd. for C₂₂H₁₈N₄O₂S₃, 466.6 (M+H), found 467.1.

***c) 5-Methylthio-4-(2-{[4-(phenyldiazenyl)phenyl]amino}(1,3-thiazol-4-yl))thiophene-2-carboxamidine hydrochloride:*** Methyl 5-methylthio-4-(2-{[4-(phenyldiazenyl)phenyl]amino}(1,3-thiazol-4-yl))thiophene-2-carboxylate hydrobromide (47.7 mg, 0.087 mmol) was treated as described in Example 154, step (b) to give 32.8 mg (77% yield) of 5-methylthio-4-{2-{[4-(phenyldiazenyl)phenyl]amino}(1,3-thiazol-4-yl))thiophene-2-carboxamidine hydrochloride. ¹H NMR (DMSO-d₆, 300 MHz) δ 2.78 (s, 3H), 7.26 (s, 1H), 7.49-7.63 (m, 3H), 7.66-7.74 (m, 3H), 7.84-8.08 (m, 3H), 8.60 (s, 1H), 11.02 (bs, 1H); Mass Spectrum (ESI) m/z calcd. for C₂₁H₁₈N₆S₃, 450.6 (M+H), found 451.1.

### Example 189

***a) {3-[(Aminothioxomethyl)amino]phenyl}methan-1-ol:*** 3-Aminobenzyl alcohol (550 mg, 4.46 mmol) was treated as described in Example 177, step (a) to give 618 mg (76% yield) of {3-[(aminothioxomethyl)amino]phenyl}methan-1-ol. ¹H NMR (DMSO-d₆, 300 MHz) δ 4.47 (d, 2H, J=5.6 Hz), 5.19 (t, 1H, J=5.7 Hz), 7.06 (d, 1H, J=6.2 Hz), 7.18-7.30 (m, 3H), 9.73 (s, 1H).

***b) Methyl-5-methylthio4-(2-{[3-(hydroxymethyl)phenyl]amino}(1,3-thiazol-4-yl))-thiophene-2-carboxylate hydrobromide:*** Methyl 4-(2-bromoacetyl)-5-methylthiothiophene-2-carboxylate (1.01 g, 3.26 mmol) was allowed to react with of {3-[(aminothioxomethyl)amino]phenyl}methan-1-ol as described in Example 154, step (a) to give 1.42 g (92% yield) of methyl-5-methylthio4-(2-{[3-(hydroxymethyl)phenyl]amino}(1,3-thiazol-4-yl))-thiophene-2-carboxylate hydrobromide. ¹H NMR (DMSO-d₆, 300 MHz) δ 2.67 (s, 3H), 3.83 (s, 3H), 4.49 (s, 2H), 6.92 (m, 1H), 7.23-7.31 (m, 2H), 7.60 (m, 1H), 7.81 (bs, 1H), 8.17 (s, 1H), 10.29 (bs, 1H).

***c) 5-Methylthio 4-(2-{[3-(hydroxymethyl)phenyl]amino}(1,3-thiazol-4-yl))-thiophene-2-carboxamidine hydrochloride:*** Methyl-5-methylthio4-(2-{[3-(hydroxymethyl)phenyl]amino}(1,3-thiazol-4-yl))-thiophene-2-carboxylate hydrobromide (700 mg, 1.47 mmol) was treated as described in Example 154, step (b) using 1:9:1 methanol-CH₂Cl₂-DMF as eluent to give 195 mg (32% yield) of 5-methylthio 4-(2-{[3-(hydroxymethyl)phenyl]amino}(1,3-thiazol-4-yl))-thiophene-2-carboxamidine hydrochloride. ¹ H NMR (DMSO-d₆, 300 MHz) δ 2.71 (s, 3H), 4.50 (s, 2H), 6.93 (d, 1H, J=7.6 Hz), 7.15 (s, 1H), 7.21-7.27 (m, 1H), 7.38 (bs, 1H), 7.65 (d, 1H, J=8.1 Hz), 7.80 (s, 1H), 8.53 (s, 1H), 8.94 (bs, 2H), 9.32 (bs, 2H), 10.37 (s, 1H); Mass Spectrum (ESI) m/z calcd. for C₁₆H₁₆N₄OS₃, 376.5 (M+H), found 377.2.

### Example 190

***a) (tert-Butoxy)-N-[(4-{2-[(3-hydroxymethylphenyl)amino](1,3-thiazol-4-yl)}-5-methylthio(2-thienyl))iminomethyl]-carboxamide:*** 5-Methylthio 4-(2-{[3-(hydroxymethyl)phenyl]amino}(1,3-thiazol-4-yl))-thiophene-2-carboxamidine (103 mg, 0.27 mmol) was slurried in THE (4 mL) and treated with 0.5 mL of 0.5 N NaOH. At this time tert-butyldicarbonate (Aldich Chemical Co., Milwaukee, WI, 0.40 mmol) was added in one portion and the result was stirred overnight. The reaction was partitioned in CH₂Cl₂ and water. The organic layer was separated and washed with brine (1x20 mL) and dried (Na₂SO₄). Removal of the solvent *in vacuo*, followed by purification on preparative thin layer chromatography (500 µm silica gel plate, J.T.Baker, Phillipsburg, NJ, 1% methanol-CH₂Cl₂), gave 45 mg (35% yield) of ((tert-Butoxy)-N-[(4-{2-[(3-hydroxymethylphenyl)amino](1,3-thiazol-4-yl)}-5-methylthio(2-thienyl))iminomethyl)-carboxamide. ¹H NMR (DMSO-d₆, 300 MHz) δ 1.44 (s, 9H), 2.66 (s, 3H), 4.49 (d, 2H, J=5.7 Hz), 5.15 (t, 1H, J=5.5 Hz), 6.92 (d, 1H, J=7.5 Hz), 6.96 (s, 1H), 7.26 (m, 1H), 7.66-7.75 (m, 2H), 8.38 (s, 1H), 8.98 (bs, 2H), 10.24 (s, 1H).

***b) (tert-Butoxy)-N-(imino{4-[2-({3-[(3-methylpiperidyl)methyl]phenyl}amino)(1,3-thiazol-4-yl)]-5-methylthio(2-thienyl)}methyl)carboxamide:*** To a stirring solution of ((tert-butoxy)-N-[(4-{2-[(3-hydroxymethylphenyl)amino](1,3-thiazol-4-yl)}-5-methylthio(2-thienyl))iminomethyl]-carboxamide (45 mg, 0.094 mmol) under N₂ was added triethylamine (2 equiv, 26.3 µl), followed by methansulfonyl chloride (Aldrich Chemical Co., Milwaukee, WI, 0.13 mmol, 10.2 µl). The reaction was stirred for 1 h, at which time the reaction was partitioned in CH₂Cl₂-water. The organic layer was washed with brine (1x20 mL), filtered through a 5 cm pad of silica gel in a 15 mL fritted glass funnel and dried (Na₂SO₄). Removal of the solvent in vacuo afforded the crude mesylate (44 mg) which was used immediately without further purification. To 25.3 mg (0.045 mmol) of the mesylate in 0.5 mL of DMF was added 3-methyl piperidine (0.18 mmol, 21.4 µl) and the result was heated to 65°C in an oil bath for 4 h. The reaction was concentrated *in vacuo* and purified by preparative thin layer chromatography (250 µm silica gel plate, 10% methanol-CH₂Cl₂, J.T.Baker, Phillipsburg, NJ) to give 8.2 mg (32% yield) of (tert-butoxy)-N-(imino{4-[2-({3-[(3-methylpiperidyl)methyl]phenyl}amino)(1,3-thiazol-4-yl)]-5-methylthio(2-thienyl)}methyl)carboxamide. Mass Spectrum (ESI) m/z calcd. for C₂₇H₃₅N₅O₂S₃, 557.8 (M+H), found 557.9, 458.2 (-C(O)OC(CH₃)₃.

***c) 4-[2-({3-[(3-methylpiperidyl)methyl]phenyl}amino)(1,3-thiazol-4-yl)]-5-methylthiothiophene-2-carboxamidine hydrochloride:*** (tert-Butoxy)-N-(imino{4-[2-({3-[(3-methylpiperidyl)methyl]phenyl}amino)(1,3-thiazol-4-yl)]-5-methylthio(2-thienyl)}methyl)carboxamide (8.2 mg, 0.014 mmol) was stirred 2 mL of a 10% 3N HCl-ethyl acetate solution at 0°C for 30 min., at which time the solvent was removed *in vacuo* to give 8 mg (100% yield) of the 4-[2-({3-[(3-methylpiperidyl)methyl]phenyl}amino)(1,3-thiazol-4-yl)]-5-methylthiothiophene-2-carboxamidine hydrochloride. ¹H NMR (DMSO-d₆, 300 MHz) δ 0.83 (d, 3H, J=5.6 Hz), 1.54-2.48 (m, 5H), 2.52-2.63 (m, 4H), 2.66 (s, 3H), 4.23 (d, 2H, J=4.8 Hz), 7.15-7.23 (m, 2H), 7.41 (t, 1H, J=7.8 Hz), 7.86-7.92 (m, 2H), 8.63 (s, 1H), 9.01 (bs, 2H), 9.42 (bs, 2H), 10.63 (s, 1H); (Mass Spectrum (ESI) m/z calcd. for C₂₂H₂₇N₅S₃, 457.7 (M+H), found 458.2.

### Example 191

***a) Methyl-5-methylthio-4-{2-[(3-hydroxyphenyl)amino](1,3-thiazol-4-yl)}-thiophene-2-carboxylate hydrobromide:*** Methyl 4-(2-bromoacetyl)-5-methylthiothiophene-2-carboxylate (60 mg, 0.19 mmol) was allowed to react with 3-hydroxyphenylthiourea (32.6 mg) as described in Example 154, step (a) to give 80.2 mg (92% yield) of methyl-5-methylthio-4-{2-[(3-hydroxyphenyl)amino](1,3-thiazol-4-yl)}-thiophene-2-carboxylate hydrobromide. 'H NMR (DMSO-d₆, 300 MHz) δ 2.67 (s, 3H), 3.83 (s, 3H), 6.38 (d, 1H, J=7.6 Hz), 7.06-7.12 (m, 2H), 7.20-7.29 (m, 2H), 8.14 (s, 1H), 10.17 (s, 1H).

***b) 4-{2-[(3-Hydroxyphenyl)amino](1,3-thiazol-4-yl)}-5-methylthiothiophene-2-carboxamidine hydrochloride:*** Methyl-5-methylthio-4-{2-[(3-hydroxyphenyl)amino](1,3-thiazol-4-yl)}-thiophene-2-carboxylate hydrobromide (460 mg, 1.0 mmol) was treated as described in Example 154, step (b) to give 215 mg (54% yield) of 4-{2-[(3-hydroxyphenyl)amino](1,3-thiazol-4-yl)}-5-methylthiothiophene-2-carboxamidine hydrochloride. (Mass Spectrum (ESI) m/z calcd. for C₁₅H₁₄N₄OS₃, 362.5 (M+H), found 363.2.

***c) (tert-Butoxy)-N-[(4-{2-[(4-hydroxyphenyl)amino](1,3-thiazol-4-yl)}-5-methylthio(2-thienyl))iminomethyl]carboxamide:*** To a stirring solution of 4-{2-[(3-hydroxyphenyl)amino](1,3-thiazol-4-yl)}-5-methylthiothiophene-2-carboxamidine hydrochloride (215 mg, 0.48 mmol) in 4 mL of CH₂Cl₂-DMF (3:1, *v*/*v*) was added diisopropylethylamine (1.2 equiv). Di-tert-butoxy dicarbonate (1.2 equiv, 127 mg, Aldrich Chemicals, Milwaukee, WI) was then added dropwise in 1 mL CH₂Cl₂ via an addition funnel. The reaction was allowed to stir overnight, partitioned in CH₂Cl₂-H₂O, and the layers separated. The organic layer was dried (Na₂SO₄) and concentrated *in vacuo*. The residue was purified by flash chromatography (1% methanol-CH₂Cl₂) to give 60 mg (27% yield) of (tert-butoxy)-N-[(4-{2-[(4-hydroxyphenyl)amino](1,3-thiazol-4-yl)}-5-methylthio(2-thienyl))iminomethyl]carboxamide. ¹H NMR (DMSO-d₆, 300 MHz) δ 1.44 (s, 9H), 2.72 (s, 3H), 6.38 (m, 1H), 6.96 (s, 1H), 7.06-7.12 (m, 2H), 7.28 (m, 1H), 8.35 (s, 1H), 9.00 (bs, 2H), 9.28 (s, 1H), 10.11 (s, 1H); Mass Spectrum (ESI) m/z calcd. for C₂₀H₂₂N₄O₃S₃, 462.6 (M+H), found 462.7, 363.2 [-C(O)OC(CH₃)₃].

***d) (tert-Butoxy)-N-{[4-(2-{[3-(carbamoylmethoxy)phenyl]amino}(1,3-thiazol-4-yl))-5-methylthio(2-thienyl)]iminomethyl}carboxamide:*** To stirring solution of (tert-butoxy)-N-[(4-{2-[(4-hydroxyphenyl)amino](1,3-thiazol-4-yl)}-5-methylthio(2-thienyl))iminomethyl]carboxamide (65 mg, 0.14 mmol) in 1.5 mL of DMF was added sequentially Cs₂CO₃ (1.5 equiv, 60.1 mg, Aldrich Chemicals, Milwaukee, WI), bromoacetamide (1.2 equiv, 20.4 mg, Aldrich Chemicals, Milwaukee, WI), and a catalytic amount of KI. The reaction was warmed to 58 °C in an oil bath, stirred for 48 h, at which time another 0.6 equiv of bromoacetamide was added. Stirring was continued for another 24 h, at which time the reaction was filtered and concentrated *in vacuo.* The residue was purified by preparative thin layer chromatography (50% ethyl acetate-hexanes) to give 9 mg (12% yield) of (tert-butoxy)-N-{[4-(2-{(3-(carbamoylmethoxy)phenyl]amino}(1,3-thiazol-4-yl))-5-methylthio(2-thienyl)]iminomethyl}carboxamide. Mass Spectrum (ESI) m/z calcd. for C₂₂H₂₅N₅O₄S₃, 519.7 (M+H), found 519.7, 420.7 [-C(O)OC(CH₃)₃].

***e) 4-(2-{[4-(Carbamoylmethoxy)phenyl]amino}(1,3-thiazol-4-yl))-5-methylthiothiophene-2-carboxamidine trifluoroacetate:*** To a stirring suspension of(tert-butoxy)-N-{[4-(2-{[3-(carbamoylmethoxy)phenyl]amino}(1,3-thiazol-4-yl))-5-methylthio(2-thienyl)]iminomethyl}carboxamide (ca. 4 mg, 0.007 mmol) in CH₂Cl₂-DMF (4 mL, 3:1 *v*/*v*) at 0°C was added 1 mL of trifluoroacetic acid. The homogeneous solution was stirred an additional 40 min. at this temperature, warmed to ambient temperature over a 30 min. period and concentrated *in vacuo* to give 4 mg (100% yield) of 4-(2-{[4-(carbamoylmethoxy)phenyl]amino}(1,3-thiazol-4-yl))-5-methylthiothiophene-2-carboxamidine trifluoroacetate. ¹H NMR (DMSO-d₆, 300 MHz) δ 2.75 (s, 3H), 4.21(d, 2H, J=5.7 Hz), 6.64 (dd, 1H, J=2.4, 8.2 Hz), 6.97 (dd, 1H, J=1.1, 8.2 Hz), 7.16 (s, 1H), 7.22 (m, 1H), 7.60-7.63 (m, 1H), 7.69-7.72 (m, 1H), 7.88 (t, 1H, J=2.1 Hz), 8.42 (s, 1H); Mass Spectrum (ESI) m/z calcd. for C₁₇H₁₇N₅O₂S₃, 419.6 (M+H), found 420.1.

### Example 192

***a) Isopropyl 5-methyl-4-{2-[(3,4,5-trimethoxyphenyl)amino](1,3-thiazol-4-yl)}thiophene-2-carboxylate hydrobromide:*** Isopropyl-4-(2-bromoacetyl)-5-methylthiophene-2-carboxylate (84 mg, 0.27 mmol) was allowed to react with 3,4,5-trimethoxyphenylthiourea (66.5 mg) as described in Example 154, step (a) to give 68 mg (48% yield) of isopropyl 5-methyl-4-{2-[(3,4,5-trimethoxyphenyl)amino](1,3-thiazol-4-yl)}thiophene-2-carboxylate hydrobromide. Mass Spectrum (ESI) m/z calcd. For C₂₁H₂₄N₂O₅S₂, 448.56 (M+H), found 449.0.

***b) 5-Methyl-4-{2-[(3,4,5-trimethoxyphenyl)amino](1,3-thiazol-4-yl)}thiophene-2-carboxamidine*** ***hydrochloride:*** Isopropyl 5-methyl-4-{2-[(3,4,5-trimethoxyphenyl)amino](1,3-thiazol-4-yl)}thiophene-2-carboxylate hydrobromide (59 mg, 0.11 mmol) was treated as described in Example 154, step (b) to give 24.4 mg (50% yield) of 5-methyl-4-{2-[(3,4,5-trimethoxyphenyl)amino](1,3-thiazol-4-yl)}thiophene-2-carboxamidine hydrochloride. ¹H NMR (DMSO-d₆, 300 MHz) δ 2.81 (s, 3H), 3.61 (s, 3H), 3.77 (s, 6H), 7.04 (s, 2H), 7.09 (s, 1H), 8.40 (s, 1H); Mass Spectrum (ESI) m/z calcd. for C₁₈H₂₀N₄O₃S₂, 404.5 (M+H), found 405.2.

### Example 193

***a) Isopropyl 5-methyl-4-{2-[(4-phenoxyphenyl)amino](1,3-thiazol-4-yl)}thiophene-2-carboxylate hydrobromide:*** Isopropyl- 4-(2-bromoacetyl)-5-methylthiophene-2-carboxylate (91 mg, 0.29 mmol) was allowed to react with 4-phenoxyphenylthiourea (72.6 mg) as described in Example 154, step (a) to give 115 mg (75% yield) of isopropyl 5-methyl-4-{2-[(4-phenoxyphenyl)amino](1,3-thiazol-4-yl)}thiophene-2-carboxylate hydrobromide. ¹H NMR (DMSO-d₆, 300 MHz) δ 1.28 (d, 6H, J=6.2 Hz), 2.70 (s, 3H), 6.06 (quintet, 1H, J=6.2 Hz), 6.92-7.09 (m, 5H), 7.15 (s, 1H), 7.30-7.37 (m, 2H), 7.56-7.70 (m, 2H), 7.98 (s, 1H); Mass Spectrum (ESI) m/z calcd. for C₂₄H₂₂N₂O₃S₂, 450.6 (M+H), found 451.2, 409.2 [-CH(CH₃)₂].

***b) 5-Methyl-4-{2-[(4-phenoxyphenyl)amino](1,3-thiazol-4-yl)}thiophene-2-carboxamidine hydrochloride:*** Isopropyl 5-methyl-4-{2-[(4-phenoxyphenyl)amino](1,3-thiazol-4-yl)}thiophene-2-carboxylate hydrobromide (95.5 mg, 0.17 mmol) was treated as described in Example 154, step (b) to give 23.8 mg (32% yield) of 5-methyl-4-{2-[(4-phenoxyphenyl)amino](1,3-thiazol-4-yl)}thiophene-2-carboxamidine hydrochloride. ¹H NMR (DMSO-d₆, 300 MHz) δ 2.76 (s, 3H), 6.95-7.12 (m, 6H), 7.34-7.39 (m, 2H), 7.72-7.78 (m, 2H), 8.33 (s, 1H), 8.98 (bs, 3H), 10.29 (bs, 1H); Mass Spectrum (ESI) m/z calcd. for C₂₁H₁₈N₄O₂S₃, 406.5 (M+H), found 407.2.

### Example 194

***a) Isopropyl 5-methyl-4-[2-(phenylamino)(1,3-thiazol-4-yl)]-thiophene-2-carboxylate hydrobromide:*** Isopropyl 4-(2-bromoacetyl)-5-methylthiophene-2-carboxylate (64 mg, 0.21 mmol) was allowed to react with phenylthiourea (32.1 mg) as described in Example 154, step (a) to give 80 mg (87% yield) of isopropyl 5-methyl-4-[2-(phenylamino)(1,3-thiazol-4-yl)]-thiophene-2-carboxylate hydrobromide. Mass Spectrum (ESI) m/z calcd. for C₁₈H₁₈N₂O₂S₂, 358.5 (M+H), found 359.2.

***b) 5-Methyl-4-[2-(phenylamino)(1,3-thiazol-4-yl)]thiophene-2-carboxamidine hydrochloride:*** Isopropyl 5-methyl-4-[2-(phenylamino)(1,3-thiazol-4-yl)]-thiophene-2-carboxylate hydrobromide (74 mg, 0.16 mmol) was treated with phenylthiourea (24.3 mg) as described in Example 154, step (b) to give 15 mg (28% yield) (of 5-methyl-4-[2-(phenylamino)(1,3-miazol-4-yl)]thiophene-2-carboxamidine hydrochloride, which was further purified by recrystallization from methanol-water. ¹H NMR (DMSO-d₆, 300 MHz) δ 2.79 (s, 3H), 6.96 (t, 1H, J=7.2 Hz), 7.09 (s, 1H), 7.33 (t, 2H, J=7.5 Hz), 7.71 (d, 2H, J=7.7 Hz), 8.39 (s, 1H), 8.95 (bs, 2H), 9.33 (bs, 2H), 10.37 (s, 1H); Mass Spectrum (ESI) m/z calcd. for C₁₅H₁₄N₄S₃, 314.4 (M+H), found 315.2.

### Example 195

***a) Methyl 4-(4-isoxazol-5-yl(1,3-thiazol-2-yl))-5-methylthiothiophene-2-carboxylate:*** Methyl 4-(aminothioxomethyl)-5-methylthiothiophene-2-carboxylate (872 mg, 2.51 mmol) was allowed to react with 2-bromo-1-isoxazol-5-ylethan-1-one (737 mg, prepared from from isoxazole-5-carbonyl chloride [Maybridge Chemicals, Cornwall, UK] as described in Example 177, step (a)) as described in Example 154, step (a) to give 704 mg (83% yield) of methyl 4-(4-isoxazol-5-yl(1,3-thiazol-2-yl))-5-methylthiothiophene-2-carboxylate. ¹H NMR (DMSO-d₆, 300 MHz) δ 2.75 (s, 3H), 3.85 (s, 3H), 6.93 (d, 1H, J=1.8 Hz), 8.22 (s, 1H), 8.38 (s, 1H), 8.70 (d, 1H, J=1.8 Hz).

***b) 4-(4-Isoxazol-5-yl(1,3-thiazol-2-yl))-5-methylthiothiophene-2-carboxamidine hydrochloride:*** Methyl 4-(4-isoxazol-5-yl(1,3-thiazol-2-yl))-5-methylthiothiophene-2-carboxylate(350 mg, 1.03 mmol) was treated as described in Example 154, step (b) to give 290 mg (78% yield) of 4-(4-isoxazol-5-yl(1,3-thiazol-2-yl))-5-methylthiothiophene-2-carboxamidine hydrochloride, of which an aliquot was further purified by recrystallization from methanol-isopropanol-water (3:1:0.2, *v*/*v*/*v*). ¹H NMR (DMSO-d₆, 300 MHz) δ 2.79 (s, 3H), 6.93 (d, 1H, J=1.9 Hz), 8.45 (s, 1H), 8.74 (m, 2H), 9.23 (bs, 2H), 9.53 (bs, 2H); Mass Spectrum (MALDI-TOF, CHCA matrix) m/z calcd. for C₁₂H₁₀N₄OS₃, 322.4 (M+H), found 323.3.

### Example 196

***a) Methyl 4-[4-(2-hydroxyphenyl)(1,3-thiazol-2-yl)]-5-methylthiothiophene-2-carboxylate:*** Methyl 4-(aminothioxomethyl)-5-methylthiothiophene-2-carboxylate (808 mg, 3.26 mmol) was allowed to react with 2-(2-bromoacetyl)hydroxybenzene (925 mg, prepared from 2-(chlorocarbonyl)phenyl acetate [Aldrich Chemicals, Milwaukee, WI] as described in Example 177, step (a)) as described in Example 154, step (a) to give 433 mg (37% yield) of methyl 4-[4 Methyl 4-[4-(2-hydroxyphenyl)(1,3-thiazol-2-yl)]-5-methylthiothiophene-2-carboxylate. ¹H NMR(DMSO-d₆, 300 MHz) δ 2.77 (s, 3H), 3.86 (s, 3H), 6.91-7.00 (m, 2H), 7.18-7.27 (m, 1H), 8.14-8.19 (m, 2H), 8.24 (s, 1H); Mass Spectrum (ESI) m/z calcd. for C₁₆H₁₃NO₃S₃, 363.48 (M+H), found 364.2.

***b) 4-[4-(2-Hydroxyphenyl)(1,3-thiazol-2-yl)]-5-methylthiothiophene-2-carboxamidine hydrochloride:*** Methyl 4-[4-(2-hydroxyphenyl)(1,3-thiazol-2-yl)]-5-methylthiothiophene-2-carboxylate (400 mg, 1.1 mmol) was treated as described in Example 154, step (b) to give 173 mg (41% yield) of 4-[4-(2-hydroxyphenyl)(1,3-thiazol-2-yl)]-5-methylthiothiophene-2-carboxamidine hydrochloride. ¹H NMR (DMSO-d₆, 300 MHz) δ 2.81 (s, 3H), 6.92-7.02 (m, 2H), 7.22 (m, 1H), 8.20 (dd, 1H, J=1.7, 7.8 Hz), 8.27 (s, 1H), 8.65 (s, 1H), 9.00 (bs, 2H), 9.41 (bs, 2H), 10.58 (s, 1H); Mass Spectrum (ESI) m/z calcd. for C₁₅H₁₃N₃OS₃, 347.48 (M+H), found 348.2.

### Example 197

### Tablet Preparation

Tablets containing 25.0, 50.0, and 100.0 mg, respectively, of the following active compounds are prepared as illustrated below:
a. 4-(4-methylthiazol-2-yl)-5-methylthiothiophene-2-carboxamidine;
b. 4-[4-(4-phenylphenyl)thiazol-2-yl]-5-methylthiothiophene-2-carboxamidine.

| **TABLET FOR DOSES CONTAINING FROM 25-100 MG OF THE ACTIVE COMPOUND** | | | |
|---|---|---|---|
| | Amount-mg | | |
| Active Compound | 25.0 | 50.0 | 100.00 |
| Microcrystalline cellulose | 37.25 | 100.0 | 200.0 |
| Modified food corn starch | 37.25 | 4.25 | 8.5 |
| Magnesium stearate | 0.50 | 0.75 | 1.5 |

All of the active compound, cellulose, and a portion of the cornstarch are mixed and granulated to 10% corn starch paste. The resulting granulation is sieved, dried and blended with the remainder of the corn starch and the magnesium stearate. The resulting granulation is then compressed into tablets containing 25.0, 50.0, and 100.0 mg, respectively, of active ingredient per tablet.

### Example 198

### Intravenous Solution Preparation

An intravenous dosage form of the above-indicated active compounds is prepared as follows:

| | |
|---|---|
| Active Compound | 0.5-10.0 mg |
| Sodium Citrate | 5-50 mg |
| Citric Acid | 1-15 mg |
| Sodium Chloride | 1-8 mg |
| Water for Injection (USP) | q.s. to 1 ml |

Utilizing the above quantities, the active compound is dissolved at room temperature in a previously prepared solution of sodium chloride, citric acid, and sodium citrate in Water for Injection (USP, see page 1636 of United States Pharmacopeia/National Formulary for 1995, published by United States Pharmacopeial Convention, Inc., Rockville, Maryland (1994).

### Example 199

### In vitro Inhibition of Purified Enzymes

**Reagents:** All buffer salts were obtained from Sigma Chemical Company (St. Louis, MO), and were of the highest purity available. The enzyme substrates, N-benzoyl-Phe-Val-Arg-*p*-nitroanilide (Sigma B7632), N-benzoyl-Ile-Glu-Gly- Arg-*p*-nitroanilide hydrochloride (Sigma B2291), N-*p*-tosyl-Gly-Pro-Lys-*p*-nitroanilide (Sigma T6140), N-succinyl-Ala-Ala-Pro-Phe-*p*-nitroanilide (Sigma S7388) and N-CBZ-Val-Gly-Arg-*p*-nitroanilide (Sigma C7271) were obtained from Sigma. N-Succinyl-Ala-Ala-Pro-Arg-*p*-nitroanilide (BACHEM L-1720) and N-succinyl-Ala-Ala- Pro-Val-*p*-nitroanilide (BACHEM L-1770) were obtained from BACHEM (King of Prussia, PA).

Human α-thrombin, human factor Xa and human plasmin were obtained from Enzyme Research Laboratories (South Bend, Indiana). Bovine α-chymotrypsin (Sigma C4129), bovine trypsin (Sigma T8642) and human kidney cell urokinase (Sigma U5004) were obtained from Sigma. Human leukocyte elastase was obtained from Elastin Products (Pacific, MO).
**K**_{**i**} **Determinations:** All assays are based on the ability of the test compound to inhibit the enzyme catalyzed hydrolysis of a peptide *p*-nitroanilide substrate. In a typical Kᵢ determination, substrate is prepared in DMSO, and diluted into an assay buffer consisting of 50 mM HEPES, 200 mM NaCl, pH 7.5. The final concentrations for each of the substrates is listed below. In general, substrate concentrations are lower than the experimentally determined value for Kₘ. Test compounds are prepared as a 1.0 mg/ml solution in DMSO. Dilutions are prepared in DMSO yielding 8 final concentrations encompassing a 200 fold concentration range. Enzyme solutions are prepared at the concentrations listed below in assay buffer.

In a typical K, determination, into each well of a 96 well plate is pipetted 280 µL of substrate solution, 10 µL of test compound solution, and the plate allowed to thermally equilibrate at 37°C in a Molecular Devices plate reader for > 15 minutes. Reactions were initiated by the addition of a 10 µL aliquot of enzyme and the absorbance increase at 405 nm is recorded for 15 minutes. Data corresponding to less than 10% of the total substrate hydrolysis were used in the calculations. The ratio of the velocity (rate of change in absorbance as a function of time) for a sample containing no test compound is divided by the velocity of a sample containing test compound, and is plotted as a function of test compound concentration. The data are fit to a linear regression, and the value of the slope of the line calculated. The inverse of the slope is the experimentally determined Kᵢ value.
**Thrombin**: Thrombin activity was assessed as the ability to hydrolyze the substrate N-succinyl-Ala-Ala-Pro-Arg-p-nitroanilide. Substrate solutions were prepared at a concentration of 32 µM (32 µM<<K*m* = 180 µM) in assay buffer. Final DMSO concentration was 4.3%. Purified human α-thrombin was diluted into assay buffer to a concentration of 15 nM. Final reagent concentrations were: [thrombin] = 0.5 nM, [substrate N-succinyl-Ala-Ala-Pro-Arg-p-nitroanilide] = 32 µM.
**Factor X [FXa]:** FXa activity was assessed as the ability to hydrolyze the substrate N-benzoyl-Ile-Glu-Gly-Arg-*p*-nitroanilide hydrochloride. Substrate solutions were prepared at a concentration of 51 µM (51<< Kₘ = 1.3 mM) in assay buffer. Final DMSO concentration was 4.3%. Purified activated human Factor X was diluted into assay buffer to a concentration of 300 nM. Final reagent concentrations were: [FXa] = 10 nM, [N-benzoyl-Ile-Glu-Gly-Arg-*p*-nitroanilide hydrochloride] = 51 µM.
**Plasmin**: Plasmin activity was assessed as the ability to hydrolyze the N-*p*-Tosyl-Gly-Pro-Lys-*p*-nitroanilide. Substrate solutions were prepared at a concentration of 37 µM (37 µM << Kₘ = 243 µM) in assay buffer. Final DMSO concentration was 4.3%. Purified human plasmin was diluted into assay buffer to a concentration of 240 nM. Final reagent concentrations were: [Plasmin] = 8 nM, [N-*p*-Tosyl-Gly-Pro-Lys-*p*-nitroanilide] = 37 µM.
**Chymotrypsin:** Chymotrypsin activity was assessed as the ability to hydrolyze N-succinyl-Ala-Ala-Pro-Phe-*p*-nitroanilide. Substrate solutions were prepared at a concentration of 14 µM (14 µM<< Kₘ= 62 µM) in assay buffer. Final DMSO concentration was 4.3%. Purified bovine chymotrypsin was diluted into assay buffer to a concentration of 81 nM. Final reagent concentrations were: [Chymotrypsin] = 2.7 nM, [N-succinyl-Ala-Ala-Pro-Phe-*p*-nitroanilide] = 14 µM.
**Trypsin**: Trypsin activity was assessed as the ability to hydrolyze N-benzoyl-Phe-Val-Arg-*p*-nitroanilide. Substrate solutions were prepared at a concentration of 13 µM (13 µM<<Kₘ, = 291 µM) in assay buffer. Final DMSO concentration was 4.3%. Purified bovine trypsin was diluted into assay buffer to a concentration of 120 nM. Final reagent concentrations were: [Trypsin] = 4 nM, [N-benzoyl-Phe-Val-Arg-*p*-nitroanilide] = 13 µM.
**Elastase**: Elastase activity was assessed as the ability to hydrolyze N-succinyl-Ala-Ala-Pro-Val-*p*-nitroanilide. Substrate solutions were prepared at a concentration of 19 µM (19 µM<< Kₘ = 89 µM) in assay buffer. Final DMSO concentration was 4.3%. Purified human leukocyte elastase was diluted into assay buffer to a concentration of 750 nM. Final reagent concentrations were: [Elastase] = 25 nM, [N-succinyl-Ala-Ala-Pro-Val-*p*-nitroanilide] = 19 µM.
**Urokinase**: Urokinase activity was assessed as the ability to hydrolyze N-CBZ-Val-Gly-Arg-*p*-nitroanilide. Substrate solutions were prepared at a concentration of 100 µM (100 µM < Kₘ = 1.2mM) in assay buffer. Final DMSO concentration was 4.3%. Purified human kidney urokinase was diluted into assay buffer to a concentration of 1.2 µM. Final reagent concentrations were: [Urokinase] = 40 nM, and N-CBZ-Val-Gly-Arg-*p*-nitroanilide] = 100 mM.

The results of exemplary assays are shown in the following table.

| ***Protease Inhibition Data*** | | |
|---|---|---|
| Protease | Ki | Example# |
| micromolar | | |
| Trypsin | 0.858 | 8 |
| Trypsin | 0.474 | 52 |
| Factor Xa | 2.73 | 94 |
| Factor Xa | 3.00 | 119 |
| Chymotrypsin | 4.90 | 11 |
| tPA | 9.49 | 1 |
| Plasmin | 7.31 | 12 |

Additionally, the following compounds have Ki values below 2.5 micromolar for uPA:
xz Ex. # 28, 40, 53, 79, 84, 89, 131, 138, 139, 140, 143, 145, 172, 187.

Having now fully described this invention, it will be understood to those of ordinary skill in the art that the same can be performed within a wide and equivalent range of conditions, formulations, and other parameters without affecting the scope of the invention or any embodiment thereof. All patents and publications cited herein are fully incorporated by reference herein in their entirety.

## Claims

1. Use in the manufacture of a medicament for treating a disease selected from benign prostatic hypertrophy, prostatic carcinoma, tumor metastasis, restenosis and psoriasis, of a compound of Formula I: 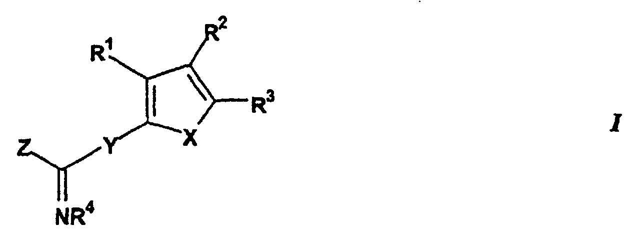 or a solvate, hydrate or pharmaceutically-acceptable salt thereof, wherein:
X is sulfur;
Y is a covalent bond, CH₂ or NH;
Z is NR⁵R⁶, hydrogen or alkyl provided that Y is NH whenever Z is hydrogen or alkyl;
R¹ is a hydrogen, amino, hydroxy, halogen, cyano, C₁₋₄alkyl, -CH₂R where R is hydroxyamino, or C₁₋₃ alkoxy;
R² and R³ are independently:
1. hydrogen;
2. halogen;
3. hydroxy;
4. nitro;
5. cyano;
6. amino, monoalkylamino, dialkylamino, monoarylamino, diarylamino, monoalkylmonoarylamino, monoaralkylamino, diaralkylamino, alkarylamino, alkoxycarbonylamino, aralkoxycarbonylamino, aryloxycarbonylamino, alkylsulfonylamino, aralkylsulfonylamino, arylsulfonylamino, formylamino, acylamino, H(S)CNH―, or thioacylamino;
7. aminocarbonyl, monoalkylaminocarbonyl, dialkylaminocarbonyl, acyl, arylaminocarbonyl, or aminoacyl;
8. aminothiocarbonyl, monoalkylaminothiocarbonyl, dialkylaminothiocarbonyl, thioacyl, or aminothioacyl;
9. aminocarbonylamino, monoalkylaminocarbonylamino, dialkylaminocarbonylamino, monoarylaminocarbonylamino, diarylaminocarbonylamino, monoaralkylaminocarbonylamino, or diaralkylaminocarbonylamino,
10. aminocarbonyloxy, monoalkylaminocarbonyloxy, dialkylaminocarbonyloxy, monoarylaminocarbonyloxy, diarylaminocarbonyloxy, monoaralkylaminocarbonyloxy, or diaralkylaminocarbonyloxy,
11. aminosulfonyl, monoalkylaminosulfonyl, dialkylaminosulfonyl, monoarylaminosulfonyl, diarylaminosulfonyl, or monoaralkylaminosulfonyl, or diaralkylaminosulfonyl,
12. alkoxy or alkylthio, wherein said alkyl portion of said alkoxy or alkylthio group may be optionally substituted;
13. aralkoxy, aryloxy, aralkylthio, or arylthip, wherein the aryl portion of said aralkoxy, aryloxy, aralkylthio or arylthio group may be optionally substituted;
14. alkylsulfonyl, wherein the alkyl portion may be optionally substituted;
15. aralkylsulfonyl, or arylsulfonyl, wherein the aryl portion of each group can be optionally substituted;
16. alkenyl or alkynyl;
17. optionally substituted aryl;
18. optionally substituted alkyl;
19. optionally substituted aralkyl;
20. optionally substituted heterocycle; or
21. optionally substituted cycloalkyl; and
R⁴, R⁵ and R⁶ are independently hydrogen, C₁₋₄ alkyl, aryl, hydroxyalkyl, aminoalkyl, monoalkylamino(C₂₋₁₀)alkyl, dialkylamino(C₂₋₁₀)alkyl, carboxyalkyl, cyano, amino, alkoxy, or hydroxy,
and wherein, unless otherwise stated, alkyl represents a straight or branched chain alkyl radical having up to 12 carbon atoms; alkenyl represents a straight or branched chain alkenyl radical of 2 to 20 carbon atoms; alkynyl represents a straight or branched chain alkynyl radical of 2 to 20 carbon atoms; alkylthio represents a straight or branched chain alkyl radical bonded to a sulfur atom; alkoxy represents a straight or branched chain alkoxy radical of up to 20 carbon atoms bonded to an oxygen atom; cycloalkyl represents a cyclic alkyl group containing from 3 to 9 carbon atoms; aryl represents a monocyclic or bicyclic aromatic group containing from 6 to 14 carbons in the ring portion; aralkyl and arylalkyl represent C₁₋₆ groups having an aryl substituent; heterocyclic represents a saturated or wholly or partially unsaturated 3 to 7 membered monocyclic, or 7 to 10 membered bicyclic ring system which consists of carbon atoms and from one to four heteroatoms independently selected from the group consisting of O, N, and S, and including any bicyclic group in which any ofthe above-defined heterocyclic rings is fused to a benzene ring and wherein the heterocyclic ring can be substituted on a carbon or on a nitrogen atom; heteroatom represents O, S or N, and wherein when heteroatom represents nitrogen, it may form an NR^{y}R^{z} moiety wherein R^{y} and R^{z} are independently selected from hydrogen or C₁₋₈ alkyl, or together with the nitrogen form a saturated or unsaturated 5-, 6- or 7-membered ring; heteroaryl represents groups having. 5 to 14 ring atoms, 6, 10 or 14 π electrons shared in a cyclic array, and containing carbon atoms and 1, 2 or 3 oxygen, nitrogen or sulfur heteroatoms; acyl represents the group -C(O)R^{g} where R^{g} is alkyl, alkenyl, alkynyl, aryl, or aralkyl; thioacyl represents the group -C(S)R^{g} where R^{g} is alkyl, alkenyl, alkynyl, aryl, or aralkyl; monoalkylamine represents an amino group substituted with one alkyl group having up to 6 carbon atoms; dialkylamine represents an amino group substituted with two allyl groups having up to 6 carbon atoms; and wherein optionally substituted alkyl represents an alkyl group substituted with 1 to 4 groups independently selected from halogen, hydroxy, thiol, amino, monoalkylamino, dialkylamino, formylamino, aminoiminomethyl, acylamino, aminoacyl, mono- or di-alkylaminocarbonyl, thiocarbonylamino, thioacylamino, aminothiocarbonyl, alkyoxy, aryloxy, aminocarbonyloxy, mono- or di-alkylaminocarbonyloxy, mono- or di-arylsulfonyl, aralkylsulfonyl, alkylsulfonylamino, arylsulfonylamino, aralkylsulfonylamino, mono- or di-alkylaminothiocarbonyl, aralkoxy, carboxy, carboxyalkyl, alkoxycarbonyl, alkoxycarbonylalkyl, nitro, cyano, trifluoromethyl, alkylthio and arylthio; optionally substituted aryl or heterocycle represents an aryl or heterocyclic group having from 1 to 4 groups independently selected from chloro, hydroxy, amino, mono(C₁₋₄)alkylamino, di(C₁₋₄)alkylamino, formylamino, C₂₋₆ acylamino, aminocarbonyl, C₂₋₈ aminoacyl, C₃₋₇ cycloalkyl, C₁₋₆ alkyl, C₁₋₆ alkoxy, C₆₋₁₄ aryloxy, carboxy, carboxy(C₁₋₆)alkyl, C₂₋₈ alkoxycarbonyl, nitro, cyano, trifluoromethyl, C₁₋₆ alkylthio, C₆₋₁₄ arylthio, C₆₋₁₄ aryl, substituted phenyl, tetrazolyl, thienyl optionally substituted by one, two or three of chloro, hydroxy, C₁₋₄ alkyl, C₁₋₄ alkoxy, amino or carboxy), 3,4-methylenedioxy, 3,4-ethylenedioxy, 3,4-propylenedioxy, C₁₋₆ alkylsulfonylamino, C₇₋₁₅ aralkylsulfonylamino, C₁₋₆ arylsulfonylamino, mono- or di-(C₁₋₆)alkylaminocarbonyloxy, mono- or di- (C₆₋₁₀)arylaminocarbonyloxy, mono- or di-(C₇₋₁₅)aralkylcarbonyloxy, C₁₋₆ alkoxycarbonylamino, C₇₋₁₅ aralkoxycarbonylamino, C₆₋₁₀ aryloxycarbonylamino, C₂₋₆ thioacylamino, aminothiocarbonyl and C₂₋₈ aminothioacyl.

2. Use according to claim 1, wherein said compound is for administration in an amount between 0.01 and 50 milligrams per kilogram per day.

3. Use according to claim 1, wherein said compound is for administration in an amount between 0.1 and 20 milligrams per kilogram per day.

4. A compound having the Formula I as defined in claim 1 and wherein X, Y, R¹, R², R³, R⁴, R⁵ and R⁶ are as defined therein, provided that at least one of R² or R³ is selected from the group consisting of:
(a) an optionally substituted alkyl group;
(b) alkoxy, aryloxy, alkylthio or arylthio, any of which is optionally substituted;
(c) optionally substituted C₆-C₁₄ aryl, or optionally substituted aralkyl, except that R³ is not nitrophenyl or aminophenyl, when R¹ and R² are both hydrogen or methyl;
(d) optionally substituted heterocycle; and
(e) optionally substituted cycloalkyl; provided that the compound is not 5-(1,1-dimethylethyl)-N-hydroxy-2-thiophenethanimidamide or 5-carbamimidoyl-3,4-dimethyl-thiophene-2-carboxylic acid amide.

5. A compound of claim 4, wherein R² or R³ is alkyl, cycloalkyl, alkoxy, alkylthio or alkylsulfonyl, and the alkyl portion of said alkyl, cycloalkyl, alkoxy, alkylthio or alkylsulfonyl is optionally substituted with 1 to 4 substituents selected from the group consisting of halogen, hydroxy, thiol, amino, monoalkylamino, dialkylamino, formylamino, acylamino, aminoacyl, monoalkylaminocarbonyl, dialkylaminocarbonyl, thiocarbonylamino, thioacylamino, aminothiocarbonyl, alkoxy, aryloxy, aminocarbonyloxy, monoalkylaminocarbonyloxy, dialkylaminocarbonyloxy, monoarylaminocarbonyloxy, diarylaminocarbonyloxy, monoaralkylaminocarbonyloxy, diaralkylaminocarbonyloxy, alkylsulfonyl, arylsulfonyl, aralkylsulfonyl, alkylsulfonylamino, arylsulfonylamino, aralkylsulfonylamino, alkoxycarbonylamino, aralkoxycarbonylamino, aryloxycarbonylamino, monoalkylaminothiocarbonyl, dialkylaminothiocarbonyl, aralkoxy, carboxy, carboxyalkyl, alkoxycarbonyl, alkoxycarbonylalkyl, nitro, cyano, trifluoromethyl, alkylthio and arylthio.

6. A compound of claim 4, wherein R³ is optionally substituted alkyl or alkylthio.

7. A compound of claim 5, wherein said 1 to 4 substituents are selected from the group consisting of chloro, hydroxy, amino, mono(C₁₋₄)alkylamino, di(C₁₋₄)alkylamino, formylamino, C₂₋₆ acylamino, aminocarbonyl, C₂₋₈ aminoacyl, C₂₋₆ thioacylamino, aminothiocarbonyl, C₂₋₈ aminothioacyl, C₁₋₆ alkoxy, C₆₋₁₄ aryloxy, carboxy, carboxy(C₁₋₆)alkyl, C₂₋₈ alkoxycarbonyl, nitro, cyano, trifluoromethyl, C₁₋₆ alkylthio, C₆₋₁₄ arylthio, C₁₋₆ aralkylsulfonylamino, C₁₋₆ arylsulfonylamino, monoalkylaminocarbonyloxy, dialkylaminocarbonyloxy, mono(C₆₋₁₀)arylaminocarbonyloxy, di(C₆₋₁₀)arylaminocarbonyloxy, monoaralkylcarbonyloxy, diaralkylcarbonyloxy, C₁₋₆ alkoxycarbonylamino, C₇₋C₁₅ aralkoxycarbonylamino, and C₆-C₁₀ aryloxycarbonylamino.

8. A compound of claim 4, wherein at least one of R² and R³ is aryl, aralkoxy, arylthio, aralkyl, aryloxy, aralkylthio, aralkylsulfonyl, arylsulfonyl, heterocycle or heterocycloalkyl optionally substituted with 1 to 4 substituents selected from the group consisting of halogen, hydroxy, thiol, amino, monoalkylamino, dialkylamino, formylamino, acylamino, aminoacyl, mono alkylaminocarbonyl, dialkylaminocarbonyl, thiocarbonylamino, thioacylamino, aminothiocarbonyl, alkoxy, aryloxy, aminocarbonyloxy, mono alkylaminocarbonyloxy, dialkylaminocarbonyloxy, monoarylaminocarbonyloxy, diarylaminocarbonyloxy, monoaralkylaminocarbonyloxy, diaralkylaminocarbonyloxy, alkylsulfonyl, arylsulfonyl, aralkylsulfonyl, alkylsulfonylamino, arylsulfonylamino, aralkylsulfonylamino, alkoxycarbonylamino, aralkoxycarbonylamino, aryloxycarbonylamino, mono alkylaminothiocarbonyl, dialkylaminothiocarbonyl, aralkoxy, carboxy, carboxyalkyl, alkoxycarbonyl, alkoxycarbonylalkyl, nitro, cyano, trifluoromethyl, alkylthio and arylthio.

9. A compound of claim 8, wherein said 1 to 4 substituents are selected from the group consisting of chloro, hydroxy, amino, mono(C₁₋₄)alkylamino, di(C₁₋₄)alkylamino, formylamino, C₂₋₆ acylamino, aminocarbonyl, C₂₋₈ aminoacyl, C₃₋₇ cycloalkyl, C₁₋₆ alkyl, C₁₋₆ alkoxy, C₆₋₁₄ aryloxy, carboxy, carboxy(C₁₋₆)alkyl, C₂₋₈ alkoxycarbonyl, nitro, cyano, trifluoromethyl, C₁₋₆ alkylthio, C₆₋₁₄ arylthio, C₆₋₁₄ aryl, tetrazolyl, thienyl, 3,4-methylenedioxy, 3,4-ethylenedioxy, 3,4-propylenedioxy, C₁₋₆ alkylsulfonylamino, C₁₋₆ aralkylsulfonylamino, C₁₋₆ arylsulfonylamino, mono- or dialkylaminocarbonyloxy, mono- or di- C₆₋₁₀ arylaminocarbonyloxy, mono- or diaralkylcarbonyloxy, C₁₋₆ alkoxycarbonylamino, C₇-C₁₅ aralkoxycarbonylamino, C₆-C₁₀ aryloxycarbonylamino, C₂₋₆ thioacylamino, aminothiocarbonyl, and C₂₋₈ aminothioacyl.

10. A compound of claim 4, wherein
X is sulfur;
Y is a covalent bond or -NH-;
R¹ is hydrogen, amino, hydroxy or halogen;
one of R² or R³ is hydrogen, C₁₋₆ alkylthio, C₁₋₆ alkyl, or C₁₋₆ alkoxy, and the other of R² or R³ is aminoacyl, acylamino, aminosulfonyl, sulfonylamino, aminocarbonylamino, alkoxycarbonylamino, optionally substituted oxazolyl, optionally substituted isoxazolyl, optionally substituted benzothienyl, optionally substituted furanyl, optionally substituted pyrazolyl or optionally substituted pyridyl.

11. A compound of claim 10, wherein R⁴, R⁵ and R⁶ are hydrogen.

12. A compound of claim 4, wherein
X is sulfur;
Y is a covalent bond or -NH-;
Z is NR⁵R⁶;
R¹ is hydrogen, amino, hydroxy or halogen;
one of R² and R³ is hydrogen, C₁₋₆ alkylthio, C₁₋₆ alkyl or C₁₋₆ alkoxy, and the other of R² and R³ is 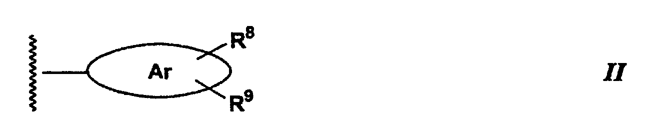 where
Ar is phenyl, thiazolyl, thiazolinyl, oxazolyl, isothiazolyl, isoxazolyl, furanyl, imidazolyl, pyridyl, pyrimidinyl, pyrazinyl, thienyl, tetrazolyl, pyrrolyl, pyrazolyl, oxadiazolyl, oxazolinyl, isoxazolinyl, imidazolinyl, triazolyl, pyrrolinyl, benzothiazolyl, benzothienyl, benzimidazolyl, 1,3-oxazolidin-2-onyl, and imidazolin-2-onyl;
R⁸ and R⁹ are independently selected from the group consisting of hydrogen, halogen, amino, mono(C₁₋₄)alkylamino, arylamino, mono C₆₋₁₄ arylamino, di(C₆₋₁₄)arylamino, mono(C₆₋₁₄)ar(C₁₋₆)alkylamino, di(C₆₋₁₄)ar(C₁₋₆)alkylamino, di(C₁₋₄)alkylamino, formylamino, C₂₋₆ acylamino, aminocarbonyl, C₂₋₈ aminoacyl, C₂₋₆ thioacylamino, aminothiocarbonyl, C₂₋₈ aminothioacyl, C₁₋₆ alkyl, C₃₋₆ cycloalkyl, C₁₋₆ alkoxy, carboxy, carboxy(C₁₋₆)alkyl, C₂₋₈ alkoxycarbonyl, nitro, cyano, trifluoromethyl, tetrazolyl, thienyl, C₆₋₁₄ aryloxy, C₁₋₆ alkylthio, C₆₋₁₄ arylthio, C₆₋₁₄ aryl, and C₆₋₁₄ ar(C₁₋₆)alkyl, wherein the aryl portions of any of said groups may be optionally substituted with 1 to 3 substituents independently selected from the group consisting of halogen, hydroxy, amino, mono(C₁₋₄)alkylamino, di(C₁₋₄)alkylamino, formylamino, C₁₋₄acylamino, C₁₋₄aminoacyl, mono(C₁₋₄)alkylaminocarbonyl, di(C₁₋₄)alkylaminocarbonyl, thiocarbonylamino, C₁₋₄thioacylamino, aminothiocarbonyl, C₁₋₄alkoxy, C₆₋₁₀aryloxy, aminocarbonyloxy, mono(C₁₋₄)alkylaminocarbonyloxy, di(C₁₋₄) alkylaminocarbonyloxy, mono(C₆₋₁₀)arylaminocarbonyloxy, di(C₆₋₁₀)arylaminocarbonyloxy, mono(C₄₋₁₂)aralkylaminocarbonyloxy, di(C₄₋₁₂)aralkylaminocarbonyloxy, C₁₋₄alkylsulfonyl, C₆₋₁₀arylsulfonyl, (C₇₋₁₂) aralkylsulfonyl, C₁₋₄alkylsulfonylamino, C₆₋₁₀arylsulfonylamino, (C₇₋₁₂) aralkylsulfonylamino, C₁₋₄alkoxycarbonylamino, C₇₋₁₂aralkoxycarbonylamino, C₆₋₁₀aryloxycarbonylamino, mono(C₁₋₄)alkylaminothiocarbonyl, di(C₁₋₄)alkylaminothiocarbonyl, C₇₋₁₂ aralkoxy, carboxy; carboxy(C₁₋₄)alkyl, C₁₋₄alkoxycarbonyl, C₁₋₄ alkoxycarbonylalkyl, nitro, cyano, trifluoromethyl, C₁₋₄alkylthio, C₆₋₁₀arylthio, 3,4-methylenedioxy, 3,4-ethylenedioxy, and 3,4-propylenedioxy; and
R⁴, R⁵, R⁶ are independently hydrogen, C₁₋₄ alkyl, amino, C₁₋₄ alkoxy or hydroxy.

13. A compound of Claim 12, wherein
X is sulfur;
Y is a covalent bond;
Z is NR⁵R⁶;
R¹ is hydrogen;
R² is 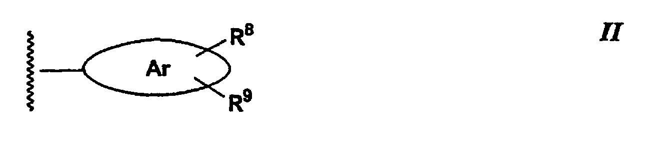
where
Ar is phenyl, thiazolyl, oxazolyl, pyridyl or imidazolyl;
R⁸ and R⁹ are independently selected from the group consisting of hydrogen and C₆₋₁₀ aryl optionally substituted with 1 to 3 substituents independently selected from the group consisting of chloro, hydroxy, C₁₋₄ alkyl, C₃₋₆ cycloalkyl, C₁₋₄ alkoxy, amino, carboxy, phenyl, naphthyl, biphenyl, hydroxyphenyl, methoxyphenyl, chlorophenyl, dichlorophenyl, aminophenyl, carboxyphenyl, nitrophenyl, 3,4-ethylenedioxy, 3,4-methylenedioxy, and 3,4-propylenedioxy;
R³ is methylthio or methyl; and
R⁴, R⁵, R⁶ are hydrogen.

14. A compound of claim 4, wherein
X is sulfur;
Y is a direct covalent bond;
Z is NR⁵R⁶;
R¹ is hydrogen;
R² is alkyl, ar(alkyl), alkylsulfonyl, -SO₂-alkyl, amido, amidino, or 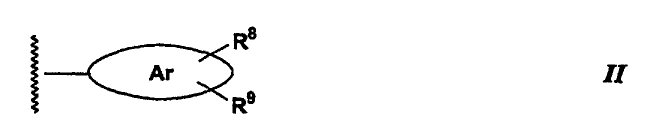
where
Ar is an aromatic or heteroaromatic group selected from the group consisting of phenyl, thiazolyl, oxazolyl, imidazolyl and pyridyl;
R⁸ and R⁹ are independently selected from the group consisting of hydrogen, carboxy, phenyl, naphthyl, alkyl, pyridyl, oxazolyl, furanyl, cycloalkyl and amino, any of which may be optionally substituted with 1 to 3 substituents independently selected from the group consisting of halogen, alkyl, haloalkyl, alkaryl, heteroaryl, phenyl, naphthyl, alkoxy, aryloxy, hydroxy, amino nitro, thiophenyl, benzothiophenyl, fluorenyl, 3,4-ethylenedioxy, 3,4-methylenedioxy, 3,4-propylenedioxy, arylsulfonamido, alkylsulfonamido and aryloxy, each of said 1 to 3 substituents may be further optionally substituted with one or more groups selected from alkoxy, haloalkyl, halogen, alkyl, amino, acetyl, hydroxy, dialkylamino, dialkylamino acyl, monoalkylaminoacyl, -SO₂-heteroaryl, -SO₂-aryl, or aryl;
R³ is -SO₂-alkyl, trifluoromethyl, S(O)-alkyl, hydrogen, alkoxy, alkylthio, alkyl, aralkylthio; and
R⁴, R⁵, R⁶ are hydrogen.

15. A compound of claim 14, wherein Ar is thiazolyl and at least one of R⁷ and R⁸ is phenyl.

16. A compound of claim 14 or 15, wherein said thiazolyl is thiazol-2-yl.

17. A compound of claim 13, wherein R² is a 4-phenylthiazol-2-yl group, wherein said phenyl is further optionally substituted.

18. A compound of claim 14 or 15, wherein said thiazolyl is thiazol-4-yl.

19. A compound of claim 18, wherein R² is a 2-aminothiazol-4-yl group.

20. A compound of claim 14, wherein said oxazolyl is oxazol-2-yl.

21. A compound of claim 14, wherein said oxazolyl is oxazol-4-yl.

22. A compound of claim 14, wherein R³ is methylthio.

23. A compound having the Formula I as defined in claim 1 wherein
X is sulfur;
Y is a covalent bond;
Z is NR⁵R⁶;
R¹ is hydrogen;
R² is 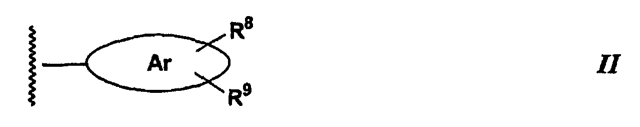
where
Ar is phenyl, thiazolyl, or oxazolyl;
R⁸ and R⁹ are independently selected from the group consisting of hydrogen and C₆₋₁₀ aryl optionally substituted with 1 to 3 substituents independently selected from the group consisting of chloro, hydroxy, C₁₋₄ alkyl, C₁₋₄ alkoxy, phenyl, 3,4-ethylenedioxy, 3,4-methylenedioxy, and 3,4-propylenedioxy;
R³ is methylthio; and
R⁴, R⁵, R⁶ are hydrogen.

24. A compound having the Formula I as defined in Claim 1 wherein
X is sulfur;
Y is a covalent bond;
R¹ is hydrogen;
R² is 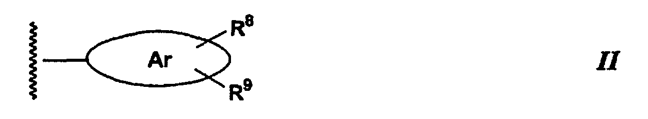
where
Ar is thiazolyl;
R⁸ and R⁹ are independently selected from the group consisting of hydrogen and C₆₋₁₀ aryl substituted with a sulfonamide group;
R³ is methylthio; and
R⁴, R⁵, R⁶ are hydrogen.

25. A compound of claim 24, wherein said sulfonamide group is a C₆₋₁₀ arylsulfonamide, alkylsulfonamide, alkoxysulfonamide orheteroarylsulfonamide.

26. A compound of claim 25, wherein said sulfonamide group is selected from the group consisting of 4-methylphenylsulfonamide, methylsulfonamide, phenylsulfonamide, trifluoromethylsulfonamide, 4-fluorophenylsulfonamide, 4-chlorophenylsulfonamide, 3-chlorophenylsulfonamide, 4-methoxysulfonamide, 2,4-difluorophenylsulfonamide, 2-(thiophene)sulfonamide, 2-(5-chlorothiophene) sulfonamide, butylsulfonamide, and isopropylsulfonamide.

27. A compound having the Formula I as defined in Claim 1 wherein
X is sulfur;
Y is a covalent bond;
Z is NR⁵R⁶;
R¹ is hydrogen;
R² is 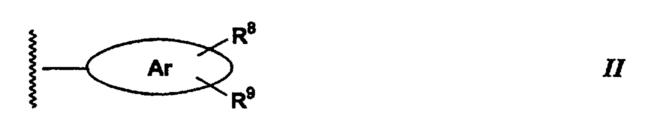
where
Ar is thiazolyl;
R⁸ and R⁹ are independently selected from the group consisting of hydrogen and C₆₋₁₀ aryl substituted with a group selected from -OCH₂C(O)-alkoxy, -OCH₂C(O)-amino, -OCH₂C(O)-NH-alkyl or -OCH₂C(O)-N(alkyl)₂;
R³ is methylthio; and
R⁴, R⁵, R⁶ are hydrogen.

28. A compound of claim 14, wherein
Ar is thiazolyl; and
one of R⁸ and R⁹ is hydrogen and the other of R⁸and R⁹ is amino, said amino group optionally substituted with halogen, hydroxy, amino, monoalkylamino, dialkylamino, formylamino, acylamino, aminoacyl, monoalkylaminocarbonyl, dialkylaminocarbonyl, thiocarbonylamino, thioacylamino, aminothiocarbonyl, alkoxy, aryloxy, aminocarbonyloxy, monoalkylaminocarbonyloxy, dialkylaminocarbonyloxy, monoarylaminocarbonyloxy, diarylaminocarbonyloxy, monoaralkylaminocarbonyloxy, diaralkylaminocarbonyloxy, alkylsulfonyl, arylsulfonyl, aralkylsulfonyl, alkylsulfonylamino, arylsulfonylamino, aralkylsulfonylamino, alkoxycarbonylamino, aralkoxycarbonylamino, aryloxycarbonylamino, monoalkylaminothiocarbonyl, dialkylaminothiocarbonyl, aralkoxy, carboxy, carboxyalkyl, alkoxycarbonyl, alkoxycarbonylalkyl, nitro, cyano, trifluoromethyl, alkylthio and arylthio.

29. A compound of claim 4 which is one of: 4-[4-(4-chlorophenyl)thiazol-2-yl]-5-methylthiothiophene-2-carboxamidine; 4-phenyl-5-methylthiothiophene-2-carboxamidine; 4-[4-(2,4-dichlorophenyl)thiazol-2-yl]-5-methylthiothiophene-2-carboxamidme; 4-(4-methylthiazol-2-yl)-5-methylthiothiophene-2-carboxamidine; 4-[4-(3-methoxyphenyl)thiazol-2-yl]-5-methylthiothiophene-2-carboxamidine; 4-[4-(3-hydroxyphenyl)thiazol-2-yl]-5-methylthiothiophene-2-carboxamidine; 4-(4-phenylthiazol-2-yl)-5-methylthiothiophene-2-carboxamidine; 4-[4-(4-nitrophenyl)thiazol-2-yl]-5-methylthiothiophene-2-carboxamidine; 4-[4-(3,4-ethylenedioxyphenyl)thiazol-2-yl]-5-methylthiothiophene-2-carboxamidine; 4-[4-(3,4-propylenedioxyphenyl)thiazol-2-yl]-5-methylthiothiophene-2-carboxamidine; 4-[4-(4-(naphth-2-yl)thiazol-2-yl]-5-methylthiothiophene-2-carboxamidine; and 4-isopropylsulfonyl-5-methylthiothiophene-2-carboxamidine; or a hydrate, solvate or pharmaceutically acceptable salt thereof.

30. A compound of claim 14, which is one of: 4-phenyl-5-methylthiothiophene-2-carboxamidine; 4-[4-(4-chlorophenyl)thiazol-2-yl]-5-methylthiothiophene-2-carboxamidine; 4-[4-(4-phenylphenyl)thiazol-2-yl]-5-methylthiothiophene-2-carboxamidine; 4-[4-(3-methoxyphenyl)thiazol-2-yl]-5-methylthiothiophene-2-carboxamidine; 4-[4-(3-hydroxyphenyl)thiazol-2-yl]-5-methylthiothiophene-2-carboxamidine; 4-(4-phenylthiazol-2-yl)-5-methylthiothiophene-2-carboxamidine; 4-[4-(4-nitrophenyl)thiazol-2-yl]-5-methylthiothiophene-2-carboxamidine; 4-[4-(3,4-ethylenedioxyphenyl)thiazol-2-yl]-5-methylthiothiophene-2-carboxamidine; 4-[4-(4-methoxyphenyl)thiazol-2-yl]-5-methylthiothiophene-2-carboxamidine; 4-[4-(3,4-propylenedioxyphenyl)thiazol-2-yl]-5-methylthiothiophene-2-carboxamidine; 4-isopropylsulfonyl-5-methylthiothiophene-2-carboxamidine; 4-(4-methylthiazol-2-yl)-5-methylthiothiophene-2-carboxamidine; 4-[4-(2,4-dichlorophenyl)thiazol-2-yl]-5-methylthiothiophene-2-carboxamidine; 4-(2-naphthylthiazol-2-yl)-5-methylthiothiophene-2-carboxamidine; 4-[4-(4-chloro-3-methylphenyl)thiazol-2-yl]-5-methylthiothiophene-2-carboxamidine; 4-(5-methyl-4-phenylthiazol-2-yl)-5-methylthiothiophene-2-carboxamidine; 4-[4-(4-chloro-3-nitrophenyl)thiazol-2-yl]-5-methylthiothiophene-2-carboxamidine; 4-(5-phenyloxazol-2-yl)-5-methylthiothiophene-2-carboxamidine; 4-[4-(3-fluoro-5-trifluoromethylphenyl)-5-methylthiazol-2-yl]-5-methylthiothiophene-2-carboxamidine; 4-[4-(3,5-bis(trifluoromethyl)phenyl)-5-methyl-thiazol-2-yl]-5-methylthiothiophene-2-carboxamidine; 4-[4-(3-fluoro-5-trifluoromethylphenyl)thiazol-2-yl]-5-methylthiothiophene-2-carboxamidine; 4-[4-(3-bromophenyl)thiazol-2-yl]-5-methylthiothiophene-2-carboxamidine; 4-[4-(3,4-methylenedioxyphenyl)thiazol-2-yl]-5-methylthiothiophene-2-carboxamidine; 4-[4-(4-methylphenyl)thiazol-2-yl]-5-methylthiothiophene-2-carboxamidine; 4-[4-(3,5-bis(trifluoromethyl)phenyl)thiazol-2-yl] -5 -methylthiothiophene-2-carboxamidine; 4-[4-(2-methoxyphenyl)thiazol-2-yl]-5-methylthiothiophene-2-carboxamidine; 4-(4-phenylimidazol-2-yl)-5-methylthiothiophene-2-carboxamidine; 4-[4-(2,4-dimethoxyphenyl)thiazol-2-yl]-5-methylthiothiophene-2-carboxamidine; 4-(4-benzylthiazol-2-yl)-5-methylthiothiophene-2-carboxamidine; 4-[4-(3,4-dichlorophenyl)thiazol-2-yl]-5-methylthiothiophene-2-carboxamidine; 4-[4-(3-methylphenyl)thiazol-2-yl]-5-methylthiothiophene-2-carboxamidine; 4-[4-(3,5-dimethoxyphenyl)thiazol-2-yl]-5-methylthiothiophene-2-carboxamidine; 4-[4-(2-methylphenyl)thiazol-2-yl]-5-methylthiothiophene-2-carboxamidine; 4-[4-(2,5-dimethoxyphenyl)thiazol-2-yl]-5-methylthiothiophene-2-carboxamidine; 4-(4,5-diphenyl)thiazol-2-yl-5-methylthiothiophene-2-carboxamidine; 4-(2-phenyl)thiazol-4-yl-5-methylthiothiophene-2-carboxamidine; 4-[4-(2-chloro-3-pyridyl)thiazol-2-yl]-5-methylthiothiophene-2-carboxamidine; 4-[4-(phenoxymethyl)thiazol-2-yl]-5-methylthiothiophene-2-carboxamidine; 4-(4-cyclohexylthiazol-2-yl)-5-methylthiothiophene-2-carboxamidine; 4-[4-(4-chlorophenyl)thiazol-2-yl]-5-methylthiothiophene-2-carboxamidine; 4-[4-(2-hydroxyphenyl)thiazol-2-yl]-5-methylthiothiophene-2-carboxamidine; 4-[4-(3-trifluoromethoxyphenyl)thiazol-2-yl]-5-methylthiothiophene-2-carboxamidine; 4-[4-(2-chloro-4-pyridyl)thiazol-2-yl]-5-methylthiothiophene-2-carboxamidine; 4-(5-phenyl-2-pyridyl)-5-methylthiothiophene-2-carboxamidine; 4-[2-(2-chlorophenylamino)thiazol-4-yl]-5-methylthiothiophene-2-carboxamidine; 4-[2-(3-methoxyphenylamino)thiazol-4-yl]-5-methylthiothiophene-2-carboxamidine; 4-[2-(phenylamino)thiazol-4-yl]-5-methylthiothiophene-2-carboxamidine; 4-[2-(2,5-dimethoxyphenylamino)thiazol-4-yl]-5-methylthiothiophene-2-carboxamidine; 4-(2-aminothiazol-4-yl)-5-methylthiothiophene-2-carboxamidine; 4-[2-(4-chloro-2-methylphenylamino)thiazol-4-yl]-5-methylthiothiophene-2-carboxamidine; 4-[2-(4-dimethylaminophenylamino)thiazol-4-yl]-5-methylthiothiophene-2-carboxamidine; 4-[2-(4-methoxyphenylamino)thiazol-4-yl]-5-methylthiothiophene-2-carboxamidine; 4-[4-(4-hydroxy-3-methoxyphenyl)thiazol-2-yl]-5-methylthiothiophene-2-carboxamidine; 4-[4-(3-hydroxy-4-methoxyphenyl)thiazol-2-yl]-5-methylthiothiophene-2-carboxamidine; 4-[2-(2--fluorophenylamino)thiazol-4-yl]-5-methylthiothiophene-2-carboxamidine; 4-[2-(2,4,5-trimethylphenyl)aminothiazol-4-yl]-5-methylthiothiophene-2-carboxamidine; 4-[2-(3-chloro-2-methylphenyl)aminothiazol-4-yl]-5-methylthiothiophene-2-carboxamidine; 4-[2-(2-isopropylphenyl)aminothiazol-4-yl]-5-methylthiothiophene-2-carboxamidine; 4-[2-(4-benzyloxyphenyl)aminothiazol-4-yl]-5-methylthiothiophene-2-carboxamidine; 4-[2-(2-bromophenyl)aminothiazol-4-yl]-5-methylthiothiophene-2-carboxamidine; 4-[2-(2,5-dichlorophenyl)aminothiazol-4-yl] -5-methylthiothiophene-2-carboxamidine; 4-[2-(2-bromo-4-methylphenyl)aminothiazol-4-yl]-5-methylthiothiophene-2-carboxamidine; 4-[2-(2,3-dichlorophenyl)aminothiazol-4-yl]-5-methylthiothiophene-2-carboxamidine; 4-[2-(3,4,5-trimethoxyphenyl)aminothiazol-4-yl]-5-methylthiothiophene-2-carboxamidine; 4-[2-(2-piperidinylethyl)aminothiazol-4yl]-5-methylthiothiophene-2-carboxamidine; 4-[2-(4-methylphenyl)aminothiazol-4-yl]-5-methylthiothiophene-2-carboxamidine; 4-(4-phenyloxazol-2-yl)-5-methylthiothiophene-2-carboxamidine; 4-[2-(diphenylmethyl)aminothiazol-4-yl]-5-methylthiothiophene-2-carboxamidine; 4-[2-(3-phenylpropyl)aminothiazol-4-yl]-5-methylthiothiophene-2-carboxamidine; or a solvate, hydrate or pharmaceutically acceptable salt thereof.

31. Use, in the manufacture of a medicament for the inhibition of a protease selected from the group consisting of leukocyte neutrophil elastase, chymotrypsin, trypsin, pancreatic elastase, cathepsin G, thrombin, urokinase, factor Xa, plasmin, thermolysin, C-1 esterase, C-3 convertase, acrosin, thrombin, kallikreins, and pepsin, of a compound according to claim 4 or claim 14.

32. Use according to claim 31 wherein said protease is trypsin, chymotrypsin, plasmin or urokinase.

33. Use, in the manufacture of a medicament for the treatment of adult respiratory distress syndrome, wound healing, gout, rheumatoid arthritis, reperfusion damage, atherosclerosis, restenosis, neoplasia, metastasis, emphysema, Alzheimer's disease, pancreatitis, benign prostatic hypertrophy, prostatic carcinoma, psoriasis or Parkinson's disease; of a compound according to claim 4 or claim 14.

34. A pharmaceutical composition comprising a compound according to claim 4 or claim 14, or a pharmaceutically acceptable ester, salt or ether thereof, and a pharmaceutically acceptable carrier.

35. A pharmaceutical composition according to claim 34, wherein said compound is present in an amount between 0.01 and 100 milligrams.

36. A pharmaceutical composition according to claim 35, suitable for parenteral, oral, subcutaneous, intravenous, intramuscular, intraperitoneal, transdermal, buccal or ocular administration.

37. A pharmaceutical composition suitable for oral administration according to claim 36, wherein said compound is present in an amount between 25 milligrams and 100 milligrams.

38. A pharmaceutical composition suitable for parenteral administration according to claim 34, wherein said compound is present in an amount between 0.5 milligrams and 10 milligrams.

39. A process for forming a compound of Formula I as defined in Claim 1 said process comprising:
(a) adding a Lewis acid to a suspension of anhydrous ammonium chloride in an aprotic solvent stirred under an inert atmosphere at a temperature near 0°C to form a mixture;
(b) allowing said mixture to warm to room temperature with stirring and thereafter stirring said mixture until substantially all of the solid has dissolved;
(c) adding to said mixture a compound of formula V 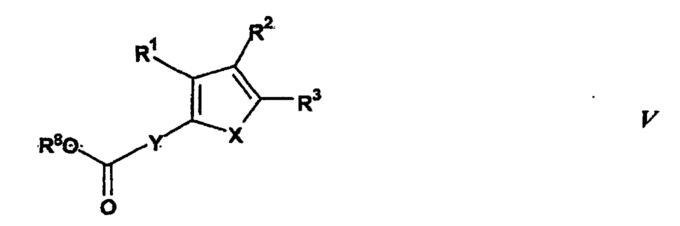 wherein R¹-R³, X and Y are as defined in claim 1; and R⁸ is selected from alkyl and aryl;
(d) heating said mixture at reflux for a predetermined period of time and thereafter allowing said mixture to cool to room temperature.

40. A process according to claim 39, wherein said Lewis acid is trimethylaluminum or triethylaluminum.

41. A process according to claim 39, wherein said aprotic solvent is selected from toluene, benzene, xylene, or mesitylene.

42. A process according to claim 39, wherein said inert atmosphere is created by performing said process under a gas selected from nitrogen or argon.

43. A compound of Formula III, 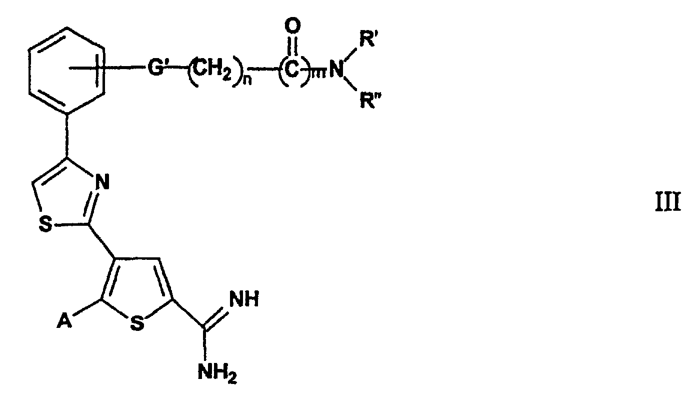 or a salt thereof, wherein
A is methylthio or methyl;
G¹ is ―O―, ―S―, ―NH―, or a covalent bond;
n is an integer from 1-10;
m is an integer from 0-1; and
R' and R" are independently selected from hydrogen, alkyl, aryl or aralkyl, or R' and R" taken together with the N atom to which they are attached form a 3-8 membered heterocyclic ring optionally containing an additional O, N, or S atom
and wherein, unless otherwise stated, alkyl represents a straight or branched chain alkyl radical having up to 12 carbon atoms; aryl represents a monocyclic or bicyclic aromatic group containing from 6 to 14 carbons in the ring portion; and aralkyl represent C₁₋₆ groups having an aryl substituent.

44. A compound according to claim 43, wherein said 3-8 membered heterocyclic ring contains an additional N atom, said additional N atom optionally substituted by hydrogen, C₁₋₄alkyl, C₆₋₁₀aryl, C₆₋₁₀ar(C₁₋₄)alkyl, C₁₋₆alkoxy, alkoxycarbonyl or benzyloxycarbonyl.

45. A compound according to claim 43, wherein said 3-8 membered heterocyclic ring is piperazinyl, pyrrolidinyl, piperidinyl or morpholinyl, which is further optionally substituted by 1-4 substituents selected from halogen, hydroxy, amino, monoalkylamino, dialkylamino, formylamino, acylamino, aminoacyl, monoalkylaminocarbonyl, dialkylaminocarbonyl, thiocarbonylamino, thioacylamino, aminothiocarbonyl, alkoxy, aryloxy, aminocarbonyloxy, monoalkylaminocarbonyloxy, dialkylaminocarbonyloxy, monoarylaminocarbonyloxy, diarylaminocarbonyloxy, monoarakylaminocarbonyloxy, diaralkylaminocarbonyloxy, alkylsulfonyl, arylsulfonyl, aralkylsulfonyl, alkylsulfonylamino, arylsulfonylamino, arakylsulfonylamino, alkoxycarbonylamino, aralkoxycarbonylamino, aryloxycarbonylamino, mono alkylaminothiocarbonyl, dialkylaminothiocarbonyl, aralkoxy, carboxy, carboxyalkyl, alkoxycarbonyl, alkoxycarbonylalkyl, nitro, cyano, trifluoromethyl, alkylthio and arylthio.

46. A compound of Formula IV 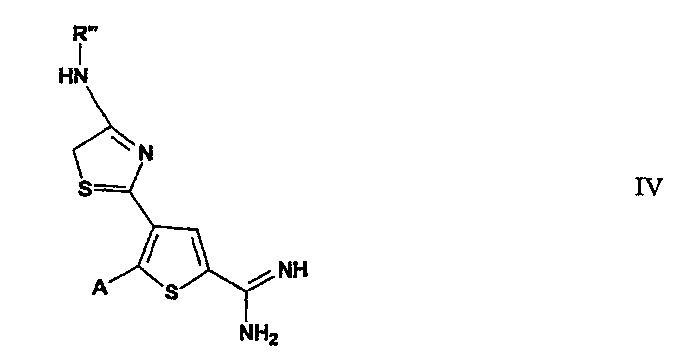 wherein
A is methylthio or methyl; and
R"' is hydrogen, C₆₋₁₄aryl, C₁₋₆alkyl, alkoxy (C₆₋₁₄)aryl, amino(C₆₋₁₄)aryl, monoalkylamino(C₆₋₁₄)aryl, dialkylamino(C₆₋₁₄)aryl, C₆₋₁₀ar(C₁₋₆)alkyl, C₁₋₆alk(C₆₋₁₄)aryl, amino(C₁₋₆)alkyl, monoalkylamino (C₁₋₆)alkyl, dialkylamino (C₁₋₆)alkyl, hydroxy(C₆₋₁₄)aryl, or hydroxy(C₁₋₆)alkyl, any of which is further optionally substituted by 1-4 non-hydrogen substituents selected from halogen, hydroxy, amino, monoalkylamino, dialkylamino, formylamino, acylamino, aminoacyl, mono- or di- alkylaminocarbonyl, thiocarbonylamino, thioacylamino, aminothiocarbonyl, alkoxy, aryloxy, aminocarbonyloxy, mono- or di-alkylaminocarbonyloxy, mono- or diarylaminocarbonyloxy, mono- or diaralkylaminocarbonyloxy, alkylsulfonyl, arylsulfonyl, aralkylsulfonyl, alkylsulfonylamino, arylsulfonylamino, aralkylsulfonylamino, alkoxycarbonylamino, aralkoxycarbonylamino, aryloxycarbonylamino, mono- or di- alkylaminothiocarbonyl, aralkoxy, carboxy, carboxyalkyl, alkoxycarbonyl, alkoxycarbonylalkyl, nitro, cyano, trifluoromethyl, alkylthio and arylthio
wherein, unless otherwise stated, alkyl represents a straight or branched chain alkyl radical having up to 12 carbon atoms; alkylthio represents a straight or branched chain alkyl radical bonded to a sulfur atom; alkoxy represents a straight or branched chain alkoxy radical of up to 20 carbon atoms bonded to an oxygen atom; aralkyl and arylalkyl represent C₁₋₆ groups having an aryl substituent; acyl represents the group -C(O)R^{g} where R^{g} is alkyl, alkenyl, alkynyl, aryl, or aralkyl; thioacyl represents the group -C(S)R^{g} where R^{g} is alkyl, alkenyl, alkynyl, aryl, or aralkyl; monoalkylamino represents an amino group substituted with one alkyl group having up to 6 carbon atoms; dialkylamino represents an amino group substituted with two alkyl groups having up to 6 carbon atoms; alkenyl represents a straight or branched chain alkenyl radical of 2 to 20 carbon atoms; and alkynyl represents a straight or branched chain alkynyl radical of 2 to 20 carbon atoms.

47. A compound of claim 46 which is one of: 4-{2-[(3-methoxyphenyl)amino](1,3-thiazol-4-yl)}-5-methylthiothiophene-2-carboxamidine, 4-{2-[(4-methoxyphenyl)amino](1,3-thiazol-4-yl)}-5-methylthiothiophene-2-carboxamidine, 4-(2-{[4-(dimethylamino)phenyl]amino}(1,3-thiazol-4-yl))-5-methylthiothiophene-2-carboxamidine, 4-{2-[(4-chloro-2-methylphenyl)amino](1,3-thiazol-4-yl)} -5-methylthiothiophene-2-carboxamidine, 4-{2-[(diphenylmethyl)amino](1,3-thiazol-4-yl)}-5-methylthiothiophene-2-carboxamidine, 5-methylthio-4-{2-[(3-phenylpropyl)amino](1,3-thiazol-4-yl)}thiophene-2-carboxamidine, 5-methylthio-4-{2-[(2,4,5-trimethylphenyl)amino](1,3-thiazol-4-yl)}thiophene-2-carboxamidine, 4-{2-[(2-fluorophenyl)amino](1,3-thiazol-4-yl)}-5-methylthiothiophene-2-carboxamidine, 4- {2-[(3-chloro-2-methylphenyl)amino](1,3-thiazol-4-yl)}-5-methylthiothiophene2-carboxamidine, 4-(2-{[2-(methylethyl)phenyl]amino}(1,3-thiazol-4-yl))-5-methylthiothiophene-2-carboxamidine, 5-methylthio-4-(2-{[4-(phenylmethoxy)phenyl]amino}(1,3-thiazol-4-yl))thiophene-2-carboxamidine, 4-{2-[(2-bromophenyl)amino](1,3-thiazol-4-yl)}-5-methylthiothiophene-2-carboxamidine, 4-{2-[(2,6-dichlorophenyl)amino](1,3-thiazol-4-yl)}-5-methylthiothiophene-2-carboxamidine, 4-{2-[(2-bromo-4-methylphenyl)amino](1,3-thiazol-4-yl)} -5-methylthiothiophene-2-carboxamidine, 5-methylthio-4-{2-[(2-morpholin-4-ylethyl)amino](1,3-thiazol-4-yl)}thiophene-2-carboxamidine, 4-{2-[(2,3-dichlorophenyl)amino](1,3-thiazol-4-yl)}-5-methylthiothiophene-2-carboxamidine, 5-methylthio-4-{2-[(3,4,5-trimethoxyphenyl)amino](1,3-thiazol-4-yl)}thiophene-2-carboxamidine, 5-methylthio-4-{2-[(2-piperidylethyl)amino](1,3-thiazol-4-yl)}thiophene-2-carboxamidine, 4-(2-{[(4-methylphenyl)methyl]amino}(1,3-thiazol-4-yl))-5-methylthiothiophene-2-carboxamidine, 4-(2-{[4-(4-chlorophenoxy)phenyl]amino}(1,3-thiazol-4-yl))-5-methylthiothiophene-2-carboxamidine 4-(2-{[4-phenoxyphenyl]amino}(1,3-thiazol-4-yl))-5-methylthiothiophene-2-carboxamidine, 5-methylthio-4-(2-{[4-(phenylamino)phenyl]amino}(1,3-thiazol-4-yl))thiophene-2-carboxamidine, 5-methylthio-4-(2- { [4-benzylphenyl]amino}(1,3-thiazol-4-yl))thiophene-2-carboxamidine, 5-methylthio-4-(2-{[4-(piperidylsulfonyl)phenyl]amino}(1,3-thiazol-4-yl))thiophene-2-carboxamidine 5-methylthio-4-[2-(3-quinolylamino)( 1,3-thiazol-4-yl)]thiophene-2-carboxamidine, 5-methylthio-4-[2-(2-naphthylamino)(1,3-thiazol-4-yl)]thiophene-2-carboxamidine, 4-[2-(2H-benzo[3,4-d] 1,3-dioxolan-5-ylamino)(1,3-thiazol-4-yl)]-5-methylthiothiophene-2-carboxamidine, 4-{2-[(7-bromofluoren-2-yl)amino](1,3-thiazol-4-yl)}-5-methylthiothiophene-2-carboxamidine, 4-{2-[(4-cyclohexylphenyl)amino](1,3-thiazol-4-yl)}-5-methylthiothiophene-2-carboxamidine, 5-methylthio-4-(2-{[4-(phenyldiazenyl)phenyl]amino}(1,3-thiazol-4-yl))thiophene-2-carboxamidine, 5-methylthio 4-(2-{[3-(hydroxymethyl)phenyl]amino}(1,3-thiazol-4-yl))-thiophene-2-carboxamidine, 4-[2-({3-[(3-methylpiperidyl)methyl]phenyl}amino)(1,3-thiazol-4-yl)]-5-methylthiothiophene-2-carboxamidine, 4-{2-[(3-hydroxyphenyl)amino)(1,3-thiazol-4-yl)}-5-methylthiothiophene-2-carboxamidine, 4-(2-{[4-(carbamoylmethoxy)phenyl]amino}(1,3-thiazol-4-yl))-5-methylthiothiophene-2-carboxamidine, 5-methyl-4-{2-[(3,4,5-trimethoxyphenyl)amino](1,3-thiazol-4-yl)}thiophene-2-carboxamidine, 5-methyl-4-{2-[(4-phenoxyphenyl)amino](1,3-thiazol-4-yl)}thiophene-2-carboxamidine, 5-methyl-4-[2-(phenylamino)(1,3-thiazol-4-yl)]thiophene-2-carboxamidine, 4-(4-isoxazol-5-yl(1,3-thiazol-2-yl))-5-methylthiothiophene-2-carboxamidine.

## Patentansprüche

1. Verwendung einer Verbindung der Formel I zur Herstellung eines Medikaments zur Behandlung einer Krankheit, die ausgewählt ist aus benigner Prostatavergrößerung, Prostatakrebs, Tumormetastase, Restenose und Psoriasis: 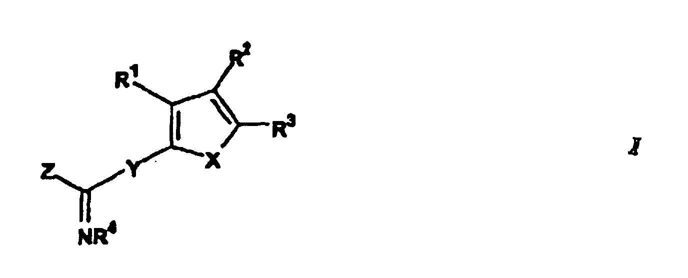 oder deren Solvat, Hydrat oder pharmazeutisch akzeptablem Salz, worin:
X Schwefel ist;
Y eine kovalente Bindung, CH₂ oder NH ist;
Z NR⁵R⁶, Wasserstoff oder Alkyl ist, vorausgesetzt, dass Y NH ist, jedesmal wenn Z Wasserstoff oder Allyl ist;
R¹ Wasserstoff, Amino, Hydroxy, Halogen, Cyan, C₁₋₄ Alkyl, -CH₂R ist, wobei R Hydroxyamino oder C₁₋₃ Alkoxy ist;
R² und R³ unabhängig voneinander sind:
1. Wasserstoff;
2. Halogen;
3. Hydroxy;
4. Nitro;
5. Cyan;
6. Amino, Monoalkylamino, Dialkylamino, Monoarylamino, Diarylamino, Monoalkylmonoarylamino, Monoaralkylamino, Diaralkylamino, Alkarylamino, Alkoxycarbonylamino, Aralkoxycarbonylamino, Aryloxycarbonylamino, Alkylsulfonylamino, Aralkylsulfonylamino, Arylsulfonylamino, Formylamino, Acylamino, H(S)CNH- oder Thioacylamino;
7. Aminocarbonyl, Monoalkylaminocarbonyl, Dialkylaminocarbonyl, Acyl, Arylaminocarbonyl oder Aminoacyl;
8. Aminothiocarbonyl, Monoalkylaminothiocarbonyl, Dialkylaminothiocarbonyl, Thioacyl der Aminothioacyl;
9. Aminocatbonylamino, Monoalkylaminocarbonylamino, Dialkylaminocarbonylamino, Monoarylaminocarbonylamino, Diarylaminocarbonylamino, Monoaralkylaminocarbonylamino oder Diaralkylaminocarbonylamino,
10. Aminocarbonyloxy, Monoalkylaminocarbonyloxy, Dialkylaminocarbonyloxy, Monoarylaminocarbonyloxy, Diarylaminocarbonyloxy, Monoaralkylaminocarbonyloxy oder Diaralkylaminocarbonyloxy,
11. Aminosulfonyl, Monoalkylaminosulfonyl, Dialkylaminosulfonyl, Monoarylaminosulfonyl, Diarylaminosulfonyl oder Monoaralkylaminosulfonyl oder Diaralkylaminosulfonyl,
12. Alkoxy oder Alkylthio, wobei der Alkylteil der Alkoxy- oder Alkylthio-Gruppe wahlweise substituiert sein kann;
13. Aralkoxy, Aryloxy, Aralkylthio oder Arylthio, wobei der Arylteil der Aralkoxy-, Aryloxy-, Aralkylthio- oder Arylthio-Gruppe wahlweise substituiert sein kann;
14. Alkylsulfonyl, wobei der Alkylteil wahlweise substituiert sein kann;
15. Aralkylsulfonyl oder Arylsulfonyl, wobei der Arylteil jeder Gruppe wahlweise substituiert sein kann;
16. Alkenyl oder Alkinyl;
17. wahlweise substituiertes Aryl;
18. wahlweise substituiertes Alkyl;
19. wahlweise substituiertes Aralkyl;
20. wahlweise substituierter Heterocyclus; oder
21. wahlweise substituiertes Cycloalkyl; und
R⁴, R⁵ und R⁶ unabhängig Wasserstoff, C₁₋₄ Alkyl, Aryl, Hydroxyalkyl, Aminoalkyl, Monoalkylamino(C₂₋₁₀)alkyl, Dialkylamino(C₂₋₁₀)alkyl, Carboxyalkyl, Cyan, Amino, Alkoxy oder Hydroxy sind, und worin, wenn nicht anders angegeben, Alkyl ein gerader oder verzweigter Alkylkettenrest mit bis zu 12 Kohlenstoffatomen darstellt; Alkenyl einen geraden oder verzweigten Alkenylkettenrest mit 2 bis 20 Kohlenstoffatornen darstellt; Alkinyl einen geraden oder verzweigten Alkinylkettenrest mit 2 bis 20 Kohlenstoffatomen darstellt; Alkylthio einen geraden oder verzweigten Alkylkettenrest darstellt, der mit einem Schwefelatom verbunden ist; Alkoxy einen geraden oder verzweigten Alkoxykettenrest mit bis zu 20 Kohlenstoffatomen darstellt, der mit einem Sauerstoffatom verbunden ist; Cycloalkyl eine cyclische Alkylgruppe darstellt, die 3 bis 9 Kohlenstoffatome enthält; Aryl eine monocyclische oder bicyclische aromatische Gruppe darstellt, die 6 bis 14 Kohlenstoffe in dem Ringteil enthält; Aralkyl und Arylalkyl C₁₋₆-Gruppen mit einem Aryl-Substituenten darstellen; Heterocyclus ein gesättigtes oder ganz oder teilweise ungesättigtes 3 bis 7 gliedriges monocyclisches oder 7 bis 10 gliedriges bicyclisches Ringsystem darstellt, das aus Kohlenstoffatomen und aus einem bis vier Heteroatomen besteht, die unabhängig ausgewählt sind aus der Gruppe, bestehend aus O, N und S, und einschließlich irgendeiner bicyclischen Gruppe, in der irgendeiner der vorstehend definierten hetreocyclischen Ringe mit einem Benzol-Ring kondensiert ist, und worin der heterocyclische Ring an einem Kohlenstoff- oder an einem Stickstoffatom substituiert sein kann; Heteroatom O, S oder darstellt, und worin, wenn Heteroatom Stickstoff darstellt, es eine NR^{y}R^{z}-Hälfte bilden kann, worin R^{y} und R^{z} unabhängig ausgewählt sind aus Wasserstoff oder C₁₋₈ Alkyl oder mit dem Stickstoff zusammen einen gesättigten oder ungesättigten 5-, 6- oder 7-gliedrigen Ring bilden; Heteroaryl Gruppen darstellt, die 5 bis 14 Ringatome aufweisen, wobei 6, 10 oder 14 π-Elektronen in einer cyclischen Gruppierung aufgeteilt sind, und die Kohlenstoffatome und 1, 2 oder 3 Sauerstoff-, Stickstoff- oder Schwefelheteroatome enthalten; Acyl die Gruppe -C(O)R^{g} darstellt, in der R^{g} Alkyl, Alkenyl, Alkinyl, Aryl oder Aralkyl ist; Thioacyl die Gruppe -C(S)R^{g} darstellt, in der R^{g} Alkyl, Alkenyl, Alkinyl, Aryl oder Aralkyl ist; Monoalkylamin eine Aminogruppe darstellt, die mit einer Alkylgruppe mit bis zu 6 Kohlenstoffatomen substitoiert ist; Dialkylamin eine Aminogruppe darstellt, die mit zwei Alkylgruppen mit bis zu 6 Kohlenstoffatomen substituiert ist; und worin wahlweise substituiertes Alkyl eine Alky)gruppe darstellt, die mit 1 bis 4 Gruppen substituiert ist, die unabhängig ausgewählt sind aus Halogen, Hydroxy, Thiol, Amino, Monoalkylamino, Dialkylamino, Formylamino. Aminoiminomethyl, Acylamino, Aminoacyl, Mono- oder Di-Alkylaminocarbonyl, Thiocarbonylamino, Thioacylamino, Amimothiocarbonyl, Alkoxy, Aryloxy, Aminocarbonyloxy, Mono- oder Di-Alkylaminocarbonyloxy, Mono- oder Di-Arylsulfonyl, Aralkylsulfonyl, Alkylsulfonylamino, Arylsulfonylamino, Aralkylsulfonylamino, Mono- oder Di-Alkylaminothiocarbonyl, Aralkoxy, Carboxy, Carboxyalkyl, Alkoxycarbonyl, Alkoxycarbonylalkyl, Nitro, Cyan, Trifluormethyl, Alkylthio und Arylthio; wahlweise substituiertes Aryl oder Heterocyclus eine Aryl- oder eine Heterocyclusgruppe darstellt, die 1 bis 4 Gruppen aufweist, die unabhängig ausgewähli sind aus Chlor, Hydroxy, Amino, Mono(C₁₋₄)alkylamino, Di(C₁₋₄)alkylamino, Formylamino, C₂₋₆ Acylamino, Aminocarbonyl, C₂₋₈ Aminoacyl, C₃₋₇ Cycloalkyl, C₁₋₆ Alkyl, C₁₋₆ Alkoxy; C₆₋₁₄ Aryloxy, Carboxy, Carboxy(C₁₋₆)alkyl, C₂₋₈ Alkoxycarbonyl, Nitro, Cyan, Trifluormethyl, C₁₋₆ Alkylthio, C₆₋₁₄ Arylthio, C₆₋₁₄ Aryl, substituiertes Phenyl, Tetrazolyl, Thienyl, wahlweise substituiert mit ein, zwei oder drei Chlor, Hydroxy, C₁₋₄ Alkyl, C₁₋₄ Alkoxy, Amino oder Carboxy), 3,4-ethylendioxy, 3,4-Ethylendioxy, 3,4-Propylendioxy, C₁₋₆ Alkylsulfonylamino, C₇₋₁₅ Aralkylsulfonylamino, C₁₋₆ Arylsulfonylamino, Mono- oder Di-(C₁₋₆)Alkylaminocarbonyloxy, Mono- oder Di-(C₆₋₁₀)Arylaminocarbonyloxy, Mono- oder Di-(C₇. ₅)Aralkylcarbonyloxy, C₁₋₆ Alkoxycarbonylamino, C₇₋₁₅ Aralkoxycarbonylamino, C₆₋₁₀ Aryloxycarbonylamino, C₂₋₆ Thioacylamino, Aminothiocarbonyl und C₂₋₈ Aminothioacyl.

2. Verwendung gemäß Anspruch 1, bei der die Verbindung zur Verabreichung in einer Menge zwischen 0,01 und 50 Milligramm pro Kilogramm pro Tag liegt.

3. Verwendung gemäß Anspruch 1, bei der die Verbindung zur Verabreichung in einer Menge zwischen 0,1 und 20 Milligramm pro Kilogramm pro Tag liegt.

4. Verpindung der Formel I gemäß Anspruch 1 und bei der X, Y, R¹, R², R³, R⁴, R⁵ und R⁶ wie darin definiert sind, vorausgesetzt, dass wenigstens einer von R² oder R³ ausgewählt ist aus der Gruppe, bestehend aus:
(a) einer wahlweise substituierten Alkylgruppe;
(b) Alkoxy, Aryloxy, Alkylthio oder Arylthio, welche wahlweise substituiert sind;
(c) wahlweise substituiertem C₆₋₁₄ Aryl, oder wahlweise substituiertes Aralkyl, mit der Ausnahme, dass R³ nicht Nitrophenyl oder Aminophenyl ist, wenn R¹ und R² beide Wasserstoff oder Methyl sind;
(d) wahlweise substituiertem Heterocyclus; und
(e) wahlweise substituiertem Cycloalkyl;
vorausgesetzt, dass die Verbindung nicht 5-(1,1-Dimethylethyl)-N-hydroxy-2-thiophenethanimidamid oder 5-Carbamimidoyl-3,4-dimethyl-thiophen-2-carbonsäureamid ist.

5. Verbindung gemäß Anspruch 4, bei der R² oder R³ Alkyl, Cycloalkyl, Alkoxy, Alkylthio oder Alkylsulfonyl ist, und der Alkylteil des Alkyl, Cycloalkyl, Alkyloxy, Alkylthio oder Alkylsulfonyl wahlweise mit 1 bis 4 Substituenten substituiert ist, die ausgewählt sind aus der Gruppe, bestehend aus Halogen, Hydroxy, Thiol, Amino, Monoalkylamino, Dialkylamino, Formylamino, Acylamino, Aminoacyl, Monoalkylaminocarbonyl, Dialkylaminocarbonyl, Thiocarbonylamino, Thioacylamino, Aminothiocarbonyl, Alkoxy, Aryloxy, Aminocarbonyloxy, Monoalkylaminocarbonyloxy, Dialkylaminocarbonyloxy, Monoarylaminocarbonyloxy, Diarylaminocarbonyloxy, Monoaralkylaminocarbonyloxy, Diaralkylaminocarbonyloxy, Alkylsulfonyl, Arylsulfonyl, Aralkylsulfonyl, Alkylsulfonylamino, Arylsulfonylamino, Aralkylsulfonylamino, Alkoxycarbonylamino, Aralkoxycarbonylamino, Aryloxycarbonylamino, Monoalkylaminothiocarbonyl, Dialkylaminothiocarbonyl, Aralkoxy, Carboxy, Carboxyalkyl, Alkoxycarbonyl, Alkoxycarbonylalkyl, Nitro, Cyan, Trifluormethyl, Alkylthio und Arylthio.

6. Verbindung gemäß Anspruch 4, bei der R³ wahlweise substituiertes Alkyl oder Alkylthio ist.

7. Verbindung gemäß Anspruch 5, bei der 1 bis 4 Substituenten ausgewählt sind aus der Gruppe, bestehend aus Chlor, Hydroxy, Amino, Mono(C₁₋₄)alkylamino, Di(C₁₋₄)alkylamino, Formylamino, C₂₋₆ Acylamino, Aminocarbonyl, C₂₋₈ Aminoacyl, C₂₋₆ Thioacylamino, Aminothiocarbonyl, C₂₋₈ Aminothioacyl, C₁₋₆ Alkoxy, C₆₋₁₄ Aryloxy, Carboxy, Carboxy(C₁₋₆)alkyl, C₂₋₈ Alkoxycarbonyl, Nitro, Cyan, Trifluormethyl, C₁₋₆ Alkylthio, C₆₋₁₄ Arylthio, C₁₋₆ Aralkylsulfonylamino, C₁₋₆ Arylsulfonylamino, Monoalkylaminocarbonyloxy, Dialkylaminocarbonyloxy, Mono(C₆₋₁₀)arylaminocarbonyloxy, Di(C₆₋₁₀)arylaminocarbonyloxy, Monoaralkylcarbonyloxy, Diaralkylcarbonyloxy, C₁₋₆ Alkoxycarbonylamino, C₇₋₁₅ Aralkoxycarbonylamino und C₆-C₁₀ Aryloxycarbonylamino.

8. Verbindung gemäß Anspruch 4, bei der wenigstens einer von R² oder R³ Aryl, Aralkoxy, Arylthio, Aralkyl, Aryloxy, Aralkylthio, Aralkylsulfonyl, Arylsulfonyl, Heterocyclus oder Heterocycloalkyl ist, der wahlweise mit 1 bis 4 Substituenten substituiert ist, die ausgewählt sind aus der Gruppe, bestehend aus Halogen, Hydroxy, Thiol, Amino, Monoalkylamino, Dialkylamino, Formylamino, Acylamino, Aminoacyl, Monoalkylaminocarbonyl, Dialkylaminocarbonyl, Thiocarbonylamino, Thioacylamino, Aminothiocarbonyl, Alkoxy, Aryloxy, Aminocarbonyloxy, Monoalkylaminocarbonyloxy, Dialkylaminocarbonyloxy, Monoarylaminocarbonyloxy, Diarylaminocarbonyloxy, Monoaralkylaminocarbonyloxy, Diaralkylaminocarbonyloxy, Alkylsulfonyl, Arylsulfonyl, Aralkylsulfonyl, Alkylsulfonylamino, Arylsulfonylamino, Aralkylsulfonylamino, Alkoxycarbonylamino, Aralkoxycarbonylamino, Aryloxycarbonylamino, Monoalkylaminothiocarbonyl, Dialkylaminothiocarbonyl, Aralkoxy, Carboxy, Carboxyalkyl, Alkoxycarbonyl, Alkoxycarbonylalkyl, Nitro, Cyan, Trifluormethyl, Alkylthio und Arylthio.

9. Verbindung gemäß Anspruch 8, bei der die 1 bis 4 Substituenten ausgewählt sind aus der Gruppe, bestehend aus Chlor, Hydroxy, Amino, Mono(C₁₋₄)alkylamino, Di(C₁₋₄)alkylamino, Formylamino, C₂₋₆ Acylamino, Aminocarbonyl, C₂₋₈ Aminoacyl, C₃₋₇ Cycloalkyl, C₁₋₆ Alkyl, C₁₋₆ Alkoxy, C₆₋₁₄ Aryloxy, Carboxy, Carboxy(C₁₋₆)alkyl, C₂₋₈ Alkoxycarbonyl, Nitro, Cyan, Trifluormethyl, C₁₋₆ Alkylthio, C₆₋₁₄ Arylthio, C₆₋₁₄ Aryl, Tetrazolyl, Thienyl, 3,4-Methylendioxy, 3,4-Ethylendioxy, 3,4-Propylendioxy, C₁₋₆ Alkylsulfonylamino, C₁₋₆ Aralkylsulfonylamino, C₁₋₆ Arylsulfonylamino, Mono- oder Dialkylaminocarbonyloxy, Mono- oder Di-C₆₋₁₀ Arylaminocarbonyloxy, Mono- oder Diaralkylcarbonyloxy, C₁₋₆ Alkoxycarbonylamino, C₇₋₁₅ Aralkoxycarbonylamino, C₆₋₁₀ Aryloxycarbonylamino, C₂₋₆ Thioacylamino, Aminothiocarbonyl und C₂₋₈ Aminothioacyl.

10. Verbindung gemäß Anspruch 4, bei der
X Schwefel ist;
Y eine kovalente Bindung oder -NH- ist;
R¹ Wasserstoff, Amino, Hydroxy oder Halogen ist;
einer von R² oder R³ Wasserstoff, C₁₋₆ Alkylthio, C₁₋₆ Alkyl oder C₁₋₆ Alkoxy ist und der andere von R² oder R³ Aminoacyl, Acylamino, Aminosulfonyl, Sulfonylamino, Aminocarbonylamino, Alkoxycarbonylamino, wahlweise substituiertes Oxazolyl, wahlweise substituiertes Isoxazolyl, wahlweise substituiertes Benzothienyl, wahlweise substituiertes Furanyl, wahlweise substituiertes Pyrazolyl oder wahlweise substituiertes Pyridyl ist.

11. Verbindung gemäß Anspruch 10, bei der R⁴, R⁵ und R⁶ Wasserstoff sind.

12. Verbindung gemäß Anspruch 4, bei der
X Schwefel ist;
Y eine kovalente Bindung oder -NH- ist;
Z NR⁵R⁶ ist;
R¹ Wasserstoff, Amino, Hydroxy oder Halogen ist;
einer von R² und R³ Wasserstoff, C₁₋₆ Alkylthio, C₁₋₆ Alkyl oder C₁₋₆ Alkoxy ist, und das andere von R² and R³ 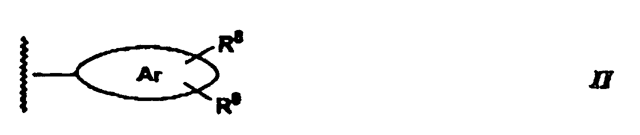 ist, worin
Ar Phenyl, Thiazolyl, Thiazolinyl, Oxazolyl, Isothiazolyl, Isoxazolyl, Furanyl, Imidazonlyl, Pyridyl, Pyrimidinyl, Pyrazinyl, Thienyl, Tetrazolyl, Pyrrolyl, Pyrazolyl, Oxadiazolyl, Oxazolinyl, Isoxazolinyl, Imidazolinyl, Triazolyl, Pyrrolinyl, Benzothiazolyl, Benzothienyl, Benzimidazolyl, 1,3-Oxazolidin-2-onyl und Imidazolin-2-onyl ist;
R⁸ und R⁹ unabhängig ausgewählt sind aus der Gruppe, bestehend aus Wasserstoff, Halogen, Amino, Mono(C₁₋₄)alkylamino, Arylamino, Mono(C₆₋₁₄)Arylamino, Di(C₆₋₁₄)arylamino, Mono(C₆₋₁₄)ar(C₁₋₆)alkylamino, Di(C₆₋₁₄)ar(C₁₋₆)alkylamino, Di(C₁₋₄)alkylamino, Formylamino, C₂₋₆Acylamino, Aminocarbonyl, C₂₋₈ Aminoacyl, C₂₋₆ Thioacylamino, Aminothiocarbonyl, C₂₋₈ Aminothioacyl, C₁₋₆ Alkyl, C₃₋₆ Cycloalkyl, C₁₋₆ Alkoxy, Carboxy, Carboxy-(C₁₋₆)alkyl, C₂₋₈ Alkoxycarbonyl, Nitro, Cyan, Trifluormethyl, Tetrazolyl, Thienyl, C₆₋₁₄ Aryloxy, C₁₋₆ Alkylthio, C₆₋₁₄ Arylthio, C₆₋₁₄ Aryl und C₆₋₁₄ Ar(C₁₋₆)alkyl, wobei die Arylteile von irgendeiner der Gruppen wahlweise mit 1 bis 3 Substituenten substituiert sein kann, die unathängig ausgewählt sind aus der Gruppe, bestehend aus Halogen, Hydroxy, Amino, Mono(C₁₋₄)alkylamino, Di(C₁₋₄)alkylamino, Formylamino, C₁₋₄ Acylamino, C₁₋₄ Aminoacyl, Mono(C₁₋₄)alkylaminocarbonyl, Di(C₁₋₄)alkylaminocarbonyl, Thiocarbonylamino, C₁₋₄ Thioacylamino, Aminothiocarbonyl, C₁₋₄ Alkoxy, C₆₋₁₀ Aryloxy, Aminocarbonyloxy, Mono(C₁₋₄)-alkylaminocarbonyloxy, Di(C₁₋₄)alkylaminocarbonyloxy, Mono(C₆₋₁₀)arylaminocarbonyloxy, Di(C₆₋₁₀)arylaminocarbonyloxy, Mono(C₄₋₁₂)aralkylaminocarbonyloxy, Di(C₄₋₁₂)aralkylaminocarbonyloxy, C₁₋₄ Alkylsulfonyl, C₆₋₁₀ Arylsulfonyl, (C₇₋₁₂) Aralkylsulfonyl, C₁₋₄ Alkylsulfonylamino, C₆₋₁₀ Arylsulfonylamino, (C₇₋₁₂) Aralkylsulfonylamino, C₁₋₄ Alkoxycarbonylamino, C₇₋₁₂ Aralkoxycarbonylamino, C₆₋₁₀ Aryloxycarbonylamino, Mono(C₁₋₄)alkylaminothiocarbonyl, Di(C₁₋₄)alkylaminothiocarbonyl, C₇₋₁₂ Aralkoxy, Carboxy, Carboxy(C₁₋₄)alkyl, C₁₋₄ Alkoxycarbonyl, C₁₋₄ Alkoxycarbonylalkyl, Nitro, Cyan, Trifluormethyl, C₁₋₄ Alkylthio, C₆₋₁₀ Arylthio, 3,4-Methylendioxy, 3,4-Ethylendioxy und 3,4-Propylendioxy; und
R⁴, R⁵, R⁶ unabhängig Wasserstoff, C₁₋₄ Alkyl, Amino, C₁₋₄ Alkoxy oder Hydroxy sind.

13. Verbindung gemäß Anspruch 12, bei der
X Schwefel ist;
Y eine kovalente Bindung ist;
Z R⁵R⁶ ist;
R¹ Wasserstoff ist;
R² 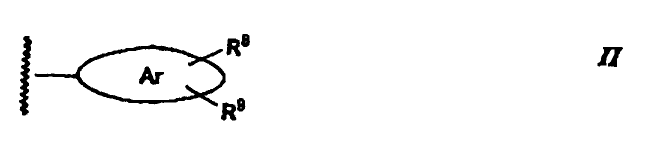 ist, worin
Ar Phenyl, Thiazolyl, Oxazolyl, Pyridyl oder Imidazolyl ist;
R⁸ und R⁹ unabhängig ausgewählt sind aus der Gruppe, bestehend aus Wasserstoff and C₆₋₁₀ Aryl das wahlweise mit 1 bis 3 Substituenten substituiert ist, die unabhängig ausgewählt sind aus der Gruppe, bestehend aus Chlor, Hydroxy, C₁₋₄ Alkyl, C₃₋₆ Cycloalkyl, C₁₋₄ Alkoxy, Amino, Carboxy, Phenyl, Naphthyl, Biphenyl, Hydroxyphenyl, Methoxyphenyl, Chlorphenyl, Dichlorphenyl, Aminophenyl, Carboxyphenyl, Nitrophenyl, 3,4-Ethylendioxy, 3,4-Methylendioxy und 3,4-Propylendioxy;
R³ Methylthio oder Methyl ist; und
R⁴, R⁵, R⁶ Wasserstoff sind.

14. Verbindung gemäß Anspruch 4, bei der
X Schwefel ist;
Y ne direkte kovalente Bindung ist;
Z NR⁵R⁶ ist;
R¹ Wasserstoff ist;
R² Alkyl, Ar(alkyl), Alkylsulfonyl, -SO₂-Alkyl, Amido, Amidino oder 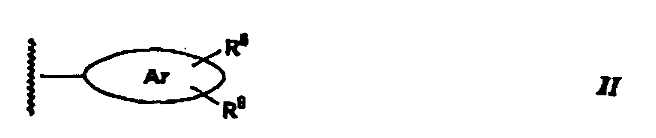 ist, worin
Ar eine aromatische oder heteroaromatische Gruppe ist, die ausgewählt ist aus der Gruppe, bestehend aus Phenyl, Thiazolyl, Oxazolyl, Imidazolyl und Pyridyl;
R⁸ und R⁹ unabhängig ausgewählt sind aus der Gruppe, bestehend aus Wasserstoff, Carboxy, Phenyl, Naphthyl, Alkyl, Pyridyl, Oxazolyl, Furanyl, Cycloalkyl und Amino, welche wahlweise mit 1 bis 3 Substituenten substituiert sein können, die unabhängig ausgewählt sind aus der Gruppe, bestehend aus Halogen, Alkyl, Haloalkyl, Alkaryl, Heteroaryl, Phenyl, Naphthyl, Alkoxy, Aryloxy, Hydroxy, Amino, Nitro, Thiophenyl, Benzothiophenyl, Fluorenyl, 3,4-Ethylendioxy, 3,4-Methylendioxy, 3,4-Propylendioxy, Arylsulfonamido, Alkylsulfonamido und Aryloxy, wobei jeder der 1 bis 3 Substituenten weiterhin wahlweise mit einer oder mehreren Gruppen substituiert sein kann, die ausgewählt sind aus Alkoxy, Haloalkyl, Halogen, Alkyl, Amino, Acetyl, Hydroxy, Dialkylamino, Dialkylaminoacyl, Monoalkylaminoacyl, -SO₂-Heteroaryl, -SO₂-Aryl oder Aryl;
R³ -SO₂-Alkyl, Trifluormethyl, S(O)-Alkyl, Wasserstoff, Alkoxy, Alkylthio, Alkyl, Aralkylthio; und
R⁴, R⁵, R⁶ Wasserstoff sind.

15. Verbindung gemäß Anspruch 14, bei der Ar Thiazolyl ist und wenigstens einer von R⁷ und R⁸ Phenyl ist.

16. Verbindung gemäß Anspruch 14 oder 15, bei der das Thiazolyl Thiazol-2-yl ist.

17. Verbindung gemäß Anspruch 13, bei der R² eine 4-Phenylthiazol-2-yl Gruppe ist, bei der die Phenylgruppe weiterhin wahlweise substituiert ist.

18. Verbindung gemäß Anspruch 14 oder 15, bei der das Thiazolyl Thiazol-4-yl ist.

19. Verbindung gemäß Anspruch 18, bei der R² eine 2-Aminothiazol-4-yl-Gruppe ist.

20. Verbindung gemäß Anspruch 14, bei der das Oxazolyl Oxazol-2-yl ist.

21. Verbindung gemäß Anspruch 14, bei der das Oxazolyl Oxazol-4-yl ist.

22. Verbindung gemäß Anspruch 14, bei der R³ Methylthio ist.

23. Verbindung mit der Formel I, wie in Anspruch 1 definiert, bei der
X Schwefel ist;
Y eine kovalente Bindung ist;
Z NR⁵R⁶ ist;
R¹ Wasserstoff ist;
R² 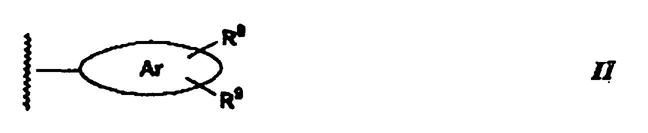 ist, worin
Ar Phenyl, Thiazolyl oder Oxazolyl ist;
R⁸ und R⁹ unabhängig ausgewählt sind aus der Gruppe, bestehend aus Wasserstoff and C₆₋₁₀ Aryl, das wahlweise mit 1 bis 3 Substituenten substituiert ist, die unabhängig ausgewählt sind aus der Gruppe, bestehend aus Chlor, Hydroxy, C₁₋₄ Alkyl, C₁₋₄ Alkoxy, Phenyl, 3,4-Ethylendioxy, 3,4-Methylendioxy und 3,4-Propylendioxy;
R³ Methylthio ist; und
R⁴, R⁵, R⁶ Wasserstoff sind.

24. Verbindung mit der Formel I, wie in Anspruch 1 definiert, bei der
X Schwefel ist;
Y eine kovalente Bindung ist;
R¹ Wasserstoff ist;
R² 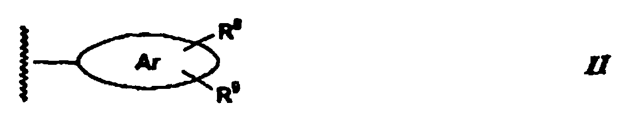 ist, worin
Ar Thiazolyl ist;
R⁸ und R⁹ unabhängig ausgewählt sind aus der Gruppe, bestehend aus Wasserstoff und C₆₋₁₀ Aryl, das mit einer Sulfonamidgruppe substituiert ist;
R³ Methylthio ist; und
R⁴, R⁵, R⁶ Wasserstoff sind.

25. Verbindung gemäß Anspruch 24, bei der die Sulfonamidgruppe ein C₆₋₁₀ Arylsulfonamid, Alkylsulfonamid, Alkoxysulfonamid oder Heterosul fonamid ist.

26. Verbindung gemäß Anspruch 25, bei der die Sulfonamidgruppe ausgewählt ist aus der Gruppe, bestehend aus 4-Methylphenylsulfonamid, Methylsulfonamid, Phenylsulfonamid, Trifluormethylsulfonamid, 4-Fluorphenylsulfonamid, 4-Chlorphenylsulfonamid, 3-Chlorphenylsulfonamid, 4-Methoxysulfonamid, 2,4-Difluorphenylsulfonamid, 2-(Thiophen)-sulfonamid, 2-(5-Chlorthiophen)sulfonamid, Butylsulfonamid und Isopropylsulfonamid.

27. Verbindung mit der Formel I, wie in Anspruch 1 definiert, bei der
X Schwefel ist;
Y eine kovalente Bindung ist;
Z NR⁵R⁶ ist;
R¹ Wasserstoff ist;
R2 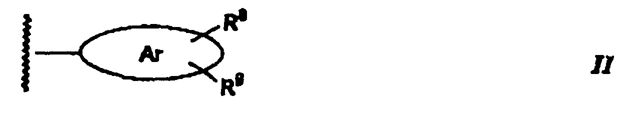 ist, worin
Ar Thiazolyl ist;
R⁸ und R⁹ unabhängig ausgewählt sind aus der Gruppe, bestehend aus Wasserstoff und C₆₋₁₀ Aryl, das mit einer Gruppe substituiert ist, die ausgewählt ist aus -OCH₂C(O)-Alkoxy, -OCH₂C(O)-Amino, -OCH₂C(O)-NH-Alkyl oder -OCH₂C(O)-N(Alkyl)₂;
R³ ethylthio ist; und
R⁴, R⁵, R⁶ Wasserstoff sind.

28. Verbindung gemäß Anspruch 14, bei der
Ar Thiazolyl ist; und
einer von R⁸ und R⁹ Wasserstoff ist und der andere von R⁸ und R⁹ Amino ist, wobei die Aminogruppe wahlweise substituiert ist mit Halogen, Hydroxy, Amino, Monoalkylamino, Dialkylamino, Formylamino, Acylamino, Aminoacyl, Manoalkylaminocarbonyl, Dialkylaminocarbonyl, Thiocarbonylamino, Thioacylamino, Aminothiocarbonyl, Alkoxy, Aryloxy, Aminocarbonyloxy, Monoalkylaminocarbonyloxy, Dialkylaminocarbonyloxy, Monoarylaminocarbonyloxy, Diarylaminocarbonyloxy, Monoaralkylaminocarbonyloxy, Diaralkylaminocarbonyloxy, Alkylsulfonyl, Arylsulfonyl, Ararlkylsulfonyl, Alkylsulfonylamino, Arylsulfonylamino, Aralkylsulfonylamino, Alkoxycarbonylamino, Aralkoxycarbonylamino, Aryloxycarbonylamino, Monoalkylaminothiocarbonyl, Dialkylaminothiocarbonyl, Aralkoxy, Carboxy, Carboxyalkyl, Alkoxycarbonyl, Alkoxycarbonylalkyl, Nitro, Cyan, Trifluormethyl, Alkylthio and Arylthio.

29. Verbindung gemäß Anspruch 4, die eine ist von: 4-[4-(4-Chlorphenyl)thiazol-2-yl]-5-methylthiothiophen-2-carboxamidin; 4-Phenyl-5-methylthiothiophen-2-carboxamidin, 4-[4-(2,4-Dichlorphenyl)thiazol-2-yl]-5-methylthiothiophen-2-carboxamidin; 4-(4-Methylthiazol-2-yl)-5-methylthiothiophen-2-carboxamidin; 4-[4-(3-Methoxyphenyl)thiazol-2-yl]-5-methylthiothiophen-2-carboxamidin; 4-[4-(3-Hydroxyphenyl)thiazol-2-yl]-5-methylthiothiophen-2-carboxamidin; 4-(4-Phenylthiazol-2-yl)-5-methylthiothiophen-2-carboxamidin; 4-[4-(4-Nitrophenyl)thiazol-2-yl]-5-methylthiothiophen-2-carboxamidin; 4-[4-(3,4-Ethylendioxyphenyl)thiazol-2-yl]-5-methylthiothiophen-2-carboxamidin; 4-[4-(3,4-Propylendioxyphenyl)thiazol-2-yl]-5-methylthiothiophen-2-carboxamidin; 4-[4-(4-(Naphth-2-yl)thiazol-2-yl]-5-methylthiothiophen-2-carboxamidin; und 4-Isopropylsulfonyl-5-methylthiothiophen-2-carboxamidin; oder deren Hydrat, Solvat oder pharmazeutisch akzeptables Salz.

30. Verbindung gemäß Anspzuch 14, die eine ist von: 4-Phenyl-5-methylthiothiophen-2-carboxamidin; 4-[4-(4-Chlorphenyl)thiazol-2-yl]-5-methylthiothiophen-2-carboxamidin; 4-[4-(4-Phenylphenyl)thiazol-2-yl]-5-methylthiothiophen-2-carboxamidin; 4-[4-(3-Methoxyphenyl)-thiazol-2-yl]-5-methylthiothiophen-2-carboxamidin; 4-[4-(3-Hydroxyphenyl)thiazol-2-yl]-5-me ylthiothiophen-2-carboxamidin; 4-(4-Phenyl)thiazol-2-yl)-5-methylthiothiophen-2-carboxamidin; 4-[4-(4-Nitrophenyl)thiazol-2-yl]-5-methylthiothiophen-2-carboxamidin; 4-[4-(3,4-Ethylendioxyphenyl)thiazol-2-yl]-5-methylthiothiophen-2-carboxamidin; 4-[4-(4-Methoxyphenyl)thiazol-2-yl]-5-methylthiothiophen-2-carboxamidin; 4-[4-(3,4-Propylendioxyphenyl)thiazol-2-yl]-5-methylthiothiophen-2-carboxamidin; 4-Isopropylsulfonyl-5-methylthiothiophen-2-carboxamidin; 4-(4-Methylthiazol-2-yl)-5-methylthiothiophen-2-carboxamidin; 4-[4-(2,4-Dichlorphenyl)thiazol-2-yl]-5-methylthiothiophen-2-carboxamidin; 4-(2-Naphthylthiazol-2-yl)-5-methylthiothiophen-2-car boxamidin; 4-[4-(4-Chlor-3-methylphenyl)thiazol-2-yl]-5-methylthiothiophen-2-carboxamidin; 4-(5-Methyl-4-phenyl)thiazol-2-yl)-5-methylthiothiophen-2-carboxamidin; 4-[4-(4-Chlor-3-nitrophenyl)thiazol-2-yl]-5-methylthiothiophen-2-carboxamidin; 4-(5-Phenyloxazol-2-yl)-5-methylthiothiophen-2-carboxamidin; -[4-(3-Fluor-5-trifluormethylphenyl)-5-methylthiazol-2-yl]-5-methylthiothiophen-2-carboxamidin; 4-[4-(3,5-Bis(trifluormethyl)phenyl)-5-methyl-thiazol-2-yl]-5-methylthiothiophen-2-carboxamidin; 4-[4-(3-Fluor-5-trifluormethylphenyl)thiazol-2-yl]-5-methylthiothiophen-2-carboxamidin; 4-[4-(3-Bromphenyl)thiazol-2-yl]-5-methylthiothiophen-2-carboxamidin; 4-[4-(3,4-Methylendioxyphenyl)thiazol-2-yl]-5-methylthiothiophen-2-carboxamidin; 4-[4-( -Methylphenyl)thiazol-2-yl]-5-methylthiothiophen-2-carboxamidin; 4-[4-(3,5-Bis(trifluormethyl)phenyl)thiazol-2-yl]-5-methylthiothiophen-2-carboxamidin; 4-[4-(2-Methoxyphenyl)thiazol-2-yl]-5-methylthiothiophen-2-carboxamidin; 4-(4-Phenylimidazol-2-yl)-5-methylthiothiophen-2-carboxamidin; 4-[4-(2,4-Dimethoxyphenyl)thiazol-2-yl]-5-methylthiothiophen-2-carboxamidin; 4-(4-Benzylthiazol-2-yl)-5-methylthiothiophen-2-carboxamidin; 4-[4-( ,4-Dichlorphenyl)thiazol-2-yl]-5-methylthiothiophen-2-carboxamidin; 4-[4-(3-Methylphenyl)thiazol-2-yl]-5-methylthiothiophen-2-carboxamidin; 4-[4-(3,5-Dimethoxyphenyl)-thiazol-2-yl]-5-methylthiothiophen-2-carboxamidin; 4-[4-(2-Methylphenyl)thiazol-2-yl]-5-methylthiothiophen-2-carboxamidin; 4-[4-(2,5-Dimethoxyphenyl)thiazol-2-yl]-5-methylthiothiophen-2-carboxamidin; 4-(4,5-Diphenyl)thiazol-2-yl-5-methylthiothiophen-Z-carboxamidin; 4-(2-Phenyl)thiazol-4-yl-5-methylthiothiophen-2-carboxamidin; 4-[4-(2-Chlor-3-pyridyl)thiazol-2-yl]-5-methylthiothiophen-2-carboxamidin; 4-[4-(Phenoxymethyl)thiazol-2-yl]-5-methylthiothiophen-2-carboxamidin; 4-(4-Cyclohexylthiazol-2-yl)-5-methylthiothiophen-2-carboxamidin; 4-[4-(4-Chlorphenyl)thiazol-2-yl]-5-methylthiothiophen-2-carboxamidin; 4-[4-(2-Hydroxyphenyl)thiazol-2-yl]-5-methylthiothiophen-2-carboxamidin; 4-[4-(3-Trifluormethoxyphenyl)thiazol-2-yl]-5-methylthiothiophen-2-carboxamidin; 4-[4-(2-Chlor-4-pyridyl)thiazol-2-yl]-5-methylthiothiophen-2-carboxamidin; 4-(5-Phenyl-2-pyridyl)-5-methylthiothiophen-2-carboxamidin; 4-[2-(2-Chlorphenylamino)thiazol-4-yl]-5-methylthiothiophen-2-carboxamidin; 4-[2-(3-Methoxyphenylamino)thiazol-4-yl]-5-methylthiothiophen-2-carboxamidin; 4-[2-(Phenylamino)thiazol-4-yl]-5-methylthiothiophen-2-carboxamidin; 4-[2-(2,5-Dimethoxyphenylamino)thiazol-4-yl]-5-methylthiothiophen-2-carboxamidin; 4-(2-Aminothiazol-4-yl)-5-methylthiothiophen-2-carboxamidin; 4-[2-(4-Chlor-2-methylphenylamino)-thiazol-4-yl]-5-methylthiothiophen-2-carboxamidin; 4-[2-(4-Dimethylaminophenylamino)-thiazol-4-yl]-5-methylthiothiophen-2-carboxamidin; 4-[2-(4-Methoxyphenylamino)thiazol-4-yl]-5-methylthiothiophen-2-carboxamidin; 4-[4-(4-Hydroxy-3-methoxyphenyl)thiazol-2-yl]-5-methylthiothiophen-2-carboxamidin; 4-[4-(3-Hydroxy-4-methoxyphenyl)thiazol-2-yl]-5-metylthiothiophen-2-carboxamidin; 4-[2-(2-Fluorphenylamino)thiazol-4-yl]-5-methylthiothiophen-2-carboxamidin; 4-[2-(2,4,5-Trimethylphenyl)aminothiazol-4-yl]-5-methylthiothiophen-2-carboxamidin; 4-[2-(3-Chlor-2-methylphenyl)aminothiazol-4-yl]-5-methylthiothiophen-2-carboxamidin; 4-[2-(2-Isopropylphenyl)aminothiazol-4-yl]-5-methylthiothiophen-2-carboxamidin; 4-[2-(4-Benzyloxyphenyl)aminothiazol-4-yl]-5-methylthiothiophen-2-carboxamidin; 4-[2-(2-Bromphenyl)aminothiazol-4-yl]-5-methylthiothiophen-2-carboxamidin; 4-[2-(2,5 Dichlorphenyl)aminothiazol-4-yl]-5-methylthiothiophen-2-carboxamidin; 4-[2-(2-Brom-4-methylphenyl)aminothiazol-4-yl]-5-methylthiothiophen-2-carboxamidin; 4-[2-(2,3-Dichlorphenyl)aminothiazol-4-yl]-5-methylthiothiophen-2-carboxamidin; 4-[2-(3,4,5-Trimethoxyphenyl)aminothiazol-4-yl]-5-methylthiothiophen-2-carboxamidin; 4-[2-(2-Piperidinylethyl)aminothiazol-4-yl]-5-methylthiothiophen-2-carboxamidin; 4-[2-(4-Methylphenyl)-aminothiazol-4-yl]-5-methylthiothiophen-2-carboxamidin; 4-(4-Phenyloxazol-2-yl)-5-methylthiothiophen-2-carboxamidin; 4-[2-(Diphenylmethyl)aminothiazol-4-yl]-5-methylthiothiophen-2-carboxamidin; 4-[2-(3-Phenylpropyl)aminothiazol-4-yl]-5-methylthiothiophen-2-carboxamidin oder deren Solvat, Hydrat oder pharmiazeutische akzeptables Salz.

31. Verwendung einer Verbindung gemäß Anspruch 4 oder Anspruch 14 zur Herstellung eines Medikaments für die Hemmung einer Protease, die ausgewählt ist aus der Gruppe, bestehend aus neutrophiler Leukozyt-Elastase, Chymotrypsin, Trypsin, Pankreaselastase, Kathepsin G, Thrombin, Urokinase, Faktor Xa, Plasmin, Thermolysin, C-1 Esterase, C-3 Convertase, Acrosin, Thrombin, Kallikrein und Pepsin.

32. Verwendung gemäß Anspruch 31, bei der die Protease Trypsin, Chymotrypsin, Plasmin oder Urokinase ist.

33. Verwendung einer Verbindung gemäß Anspruch 4 oder Anspruch 14 zur Herstellung eines Medikaments zur Behandlung von Schocklunge, Wundheilung, Gicht, rheumatischer Arthritis, Reperfusionsschaden, Atherosklerose, Restenose, Neoplasie, Metastase, Emphysem, Alzheimer -Krankheit, Pankreatitis, beaigner Prostaiav ergrößerung, Prostatakrebs, Psoriasis oder Parkonson -Krankheit.

34. Pharmazeutische Zusammensetzung, die eine Verbindung gemäß Anspruch **4** oder Anspruch 14 oder deren pharmazeutisch akzeptablen Ester, Salz oder Ether und einen pharmazeutisch akzeptablen Täger enthält.

35. Pharmazeutische Zusammensetzung gemäß Anspiuch 34, bei der die Verbindung in einer Menge zwischen 0,01 und 100 Milligramm vorhanden ist.

36. Pharmazeutische Zusammensetzung gemäß Anspnich 35, die für parenterale, orale, subkutane, intravenöse, intramuskuläre, intraperitoneale, transdermale, bukkale oder okulare Verabreichung geeignet ist.

37. Pharmazeutische Zusammensetzung, die für orale Verabreichung gemäß Anspruch 36 geeignet ist, bei der die Verbindung in einer Menge zwischen 25 Milligramm und 100 Milligramm vorhanden ist.

38. Pharmazeutische Zusammensetzung, die für parenterale Verabreichung gemäß Anspruch 34 geeignet ist, bei der die Verbindung in einer Menge zwischen 0,5 Milligramm und 10 Milligramm vorhanden ist.

39. Verfahren zur Herstellung einer Verbindung gemäß Formel I, wie in Anspruch definiert, wobei das Verfahren umfasst:
(a) Zusetzen einer Lewis-Säure zu einer Suspension aus wasserfreiem Ammoniumchlorid in einem aprotischen Lösungsmittel, das unter einer inerten Atmosphäre bei einer Tempertaur nahe bei 0°C gerührt wird, um eine Mischung zu bilden;
(b) Ermöglichen, dass die Mischung sich unter Rühren auf Raumtemperatur erwärmt, und danach Rühren der Mischung bis im Wesentlichen der gesamte Feststoff gelöst ist;
(c) Zugeben einer Verbindung gemäß Formel V zu der Mischung 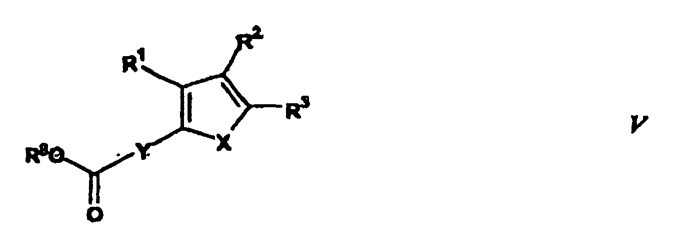 worin R¹-R³, X und Y wie in Anspruch 1 definiert sind; und
R⁸ aus Alkyl und Aryl ausgewählt ist;
(d) Erwärmen der Mischung am Rückfluß für eine vorbestimmte Zeitdauer und danach Ermöglichen, dass diese Mischung auf Raumtemperatur abkühlt.

40. Verfahren gemäß Anspruch 39, bei dem die Lewis Säure Trimethylaluminium oder Triethylaluminium ist.

41. Verfahren gemäß Anspruch 39, bei dem das aprotische Lösungsmittel ausgewählt ist aus Toluol, Benzol, Xylol oder Mesitylen.

42. Verfahren gemäß Anspruch 39, bei dem die inerte Atmosphäre durch Durchführen des Verfahrens unter einem Gas gebildet wird, das ausgewählt ist aus Stickstoff oder Argon.

43. Verbindung gemäß Formel III, 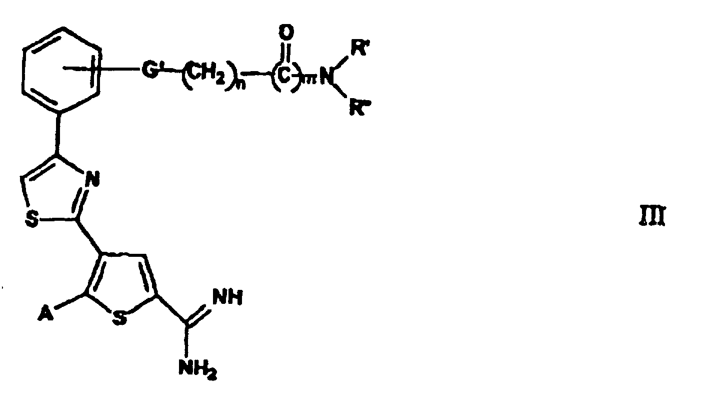 oder dessen Salz, worin
A Methylthio oder Methyl ist;
G¹ -O-, -S-, -NH- oder eine kovalente Bindung ist;
n eine ganze Zahl von 1 - 10 ist;
m eine ganze Zahl von 0 - 1 ist; und
R' und R" unabhängig ausgewählt sind aus Wasserstoff, Alkyl, Aryl oder Aralkyl oder R' und R" mit dem N-Atom, mit dem sie verbunden sind, zusammen genommen einen 3 - 8-gliedrigen heterocyclischen Ring bilden, der wahlweise ein zusätzliches O-, N-, oder S-Atom enthält
und worin, wenn nicht anders angegeben, Alkyl ein gerades oder verzweigtes Alkylkettenradikal mit bis zu 12 Kohlenstoffatomen darstellt; Aryl eine monocyclische oder bicyclische aromatische Gruppe darstellt, die 6 bis 14 Kohlenstoffe in dem Ringteil enthält; und Aralkyl C₁₋₆-Gruppen mit einem Aryl-Substituenten darstellt.

44. Verbindung gemäß Anspruch 43, bei der 3-8-gliedrige heterocyclische Ring ein zusätzliches N-Atom enthält, wobei das zusätzliche N-Atom wahlweise mit Wasserstoff, C₁₋₄ Alkyl, C₆₋₁₀ Aryl, C₆₋₁₀Ar(C₁₋₄)alkyl, C₁₋₆ Alkoxy, Alkoxycarbonyl oder Benzyloxycarbonyl substituiert ist.

45. Verbindung gemäß Anspruch 43, bei der der 3-8-ghedrige heterocyclische Ring Piperazinyl, Pyrrolidinyl, Piperidinyl oder Morpholinyl ist, welches weiterhin wahlweise mit 1 - 4 Substituenten substituiert ist, die ausgewählt sind aus Halogen, Hydroxy, Amino, Monoalkylamino, Dialkylamino, Formylamino, Acylamino, Aminoacyl, Monoalkylaminocarbonyl, Dialkylaminocarbonyl, Thiocarbonylamino, Thioacylamino, Aminothiocarbonyl, Alkoxy, Aryloxy, Aminocarbonyloxy, Monoalkylaminocarbonyloxy, Dialkylaminocarbonyloxy, Monoarylaminocarbonyloxy, Diarylaminocarbonyloxy, Monoaralkylaminocarbonyloxy, Diaralkylaminocarbonyloxy, Alkylsulfonyl, Arylsulfonyl, Ararlkylsulfonyl, Alkylsulfonylamino, Arylsulfonylamino, Aralkylsulfonylamino, Alkoxycarbonylamino, Aralkoxycarbonylamino, Aryloxycarbonylamino, Monoalkylaminothiocarbonyl, Dialkylaminothiocarbonyl, Aralkoxy, Carboxy, Carboxyalkyl, Alkoxycarbonyl. Alkoxycarbonylalkyl, Nitro, Cyan, Trifluormethyl, Alkylthio und Arylthio.

46. Verbindung gemäß Formel IV 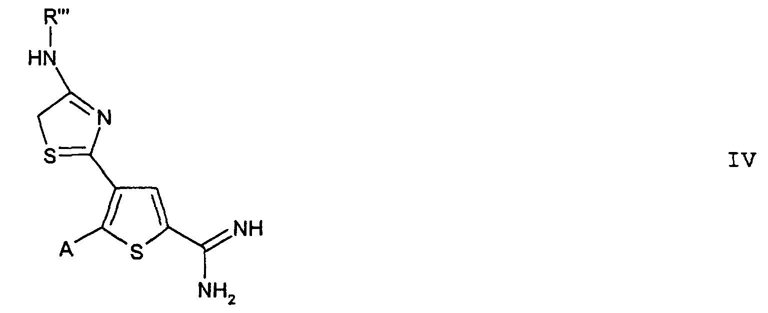 worin
A Methylthio oder Methyl ist; und
R" Wasserstoff, C₆₋₁₄ Aryl, C₁₋₆ Alkyl, Alkoxy(C₆₋₁₄)aryl, Amino(C₆₋₁₄)aryl, Monoalkylamino(C₆₋₁₄)aryl, Dialkylamino(C₆₋₁₄)aryl, C₆₋₁₀Ar(C₁₋₆)alkyl, C₁₋₆ Alk(C₆₋₁₄)aryl, Amino(C₁₋₆)alkyl, Monoalkylamino, (C₁₋₆)Alkyl, Dialkylamino(C₁₋₆)alkyl, Hydroxy(C₆₋₁₄)aryl oder Hydroxy(C₁₋₆)alkyl ist, welches weiterhin wahlweise mit 1-4 Substituenten, die nicht Wasserstoff sind, substituiert ist, ausgewählt aus Halogen, Hydroxy, Amino, Monoalkylamino, Dialkylamino, Formylamino, Acylamino, Aminoacyl, Mono- oder Di-Alkylaminocarbonyl, Thiocarbonylamino, Thioacylamino, Aminothiocarbonyl, Alkoxy, Aryloxy, Aminocarbonyloxy, Mono- oder Di-Alkylaminocarbonyloxy, Mono- oder Di-Arylaminocarbonyloxy, Mono- oder Di-Aralkylaminocarbonyloxy, Alkylsulfonyl, Arylsulfonyl, Aralkylsulfonyl, Alkylsulfonylamino, Arylsulfonylamino, Aralkylsulfonylamino, Alkoxycarbonylamino, Aralkoxycarbonylamino, Aryloxycarbonylamino, Mono- oder Di-Alkylaminothiocarbonyl, Aralkoxy, Carboxy, Carboxyalkyl, Alkoxycarbonyl, Alkoxycarbonylalkyl, Nitro, Cyan, Trifluormethyl, Alkylthio und Arylthio, wobei, wenn nicht anders angegeben, Alkyl ein gerades oder verzweigtes Alkylkettenradikal mit bis zu 12 Kohlenstoffatomen darstellt; Alkylthio ein gerades oder verzweigtes Alkylkettenradikal darstellt, das mit einem Schwefelatom verbunden ist; Alkoxy ein gerades oder verzweigtes Alkoxykettenradikal mit bis zu 20 Kohlenstoffatomen darstellt, das mit einem Sauerstoffatom verbunden ist; Aralkyl und Arylalkyl C₁₋₆-Gruppen mit einem Aryl-Substituenten darstellen; Acyl die Gruppe -C(O)R⁸ darstellt, in der R^{g} Alkyl, Alkenyl, Alkinyl, Aryl oder Aralkyl ist; Thioacyl die Gruppe -C(S)R^{g} darstellt, in der R⁸ Alkyl, Alkenyl, Alkinyl, Aryl oder Aralkyl ist; Monoalkylamino eine Aminogruppe darstellt, die mit einer Alkylgruppe mit bis zu 6 Kohlenstoffatomen substituiert ist; Dialkylamino eine Aminogruppe darstellt, die mit zwei Alkylgruppen mit bis zu 6 Kohlenstoffatomen substituiert ist; Alkenyl ein gerades oder verzweigtes Alkenylkettenradikal mit 2 bis 20 Kohlenstoffatomen darstellt; Alkinyl ein gerades oder verzweigtes Alkinylkettenradikal mit 2 bi 20 Kohlenstoffatomen darstellt.

47. Verbindung gemäß Anspruch 46, die eine ist von: 4-{2-[(3-Methoxyphenyl)amino](1,3-azol-4-yl)}-5-methylthiothiophen-2-carboxamidin, 4-{2-[(4-Methoxyphenyl)amino](1,3-thiazol-4-yl)}-5-methylthiothiophen-2-carboxamidin, 4-(2-{[4-Dimethylamino)phenyl]-amino}(1,3-thiazol-4-yl))-5-methylthiothiophen-2-carboxamidin, 4-{2-[(4-Chlor-2-methylphenyl)amino](1,3-thiazol-4-yl)}-5-methylthiothiophen-2-carboxamidin, 4-{2-[(Diphenylmethyl)amino](1,3-thiazol-4-yl)}-5-methylthiothiophen-2-carboxamidin, 5-Methyithio-4-{2-[(3-Phenylpropyl)amino](1,3-thiazol-4-yl)}thiophen-2-carboxamidin, 5-Methylthio-4-{2-[(2,4,5-Trimethylphenyl)amino](1,3-thiazol-4-yl))thiophen-2-carboxamidin, 4-{2-[(2-Fluorphenyl)amino](1,3-thiazol-4-yl)}-5-methylthiothiophen-2-carboxamidin, 4- {2-[(3-Chlor-2-methylphenyl)amino](1,3-thiazol-4-yl)}-5-methylthiothiophen-2-carboxamidin, 4-(2-{[2-(Methylethyl)phenyl]amino}(1,3-thiazol-4-yl))-5-methylthiothiophen-2-carboxamidin, 5-Methylthio-4-(2-{[4-(Phenylmethoxy)phenyl]amino}(1,3-thiazol-4-yl))thiophen-2-carboxamidin, 4-{2-[(2-Bromphenyl)amino](1,3-thiazol-4-yl)}-5-methylthiothiophen-2-carboxamidin, 4-{2-[(2,6-Dichlorphenyl)amino](1,3-thiazol-4-yl)}-5-methylthiothiophen-2-carboxamidin, 4-{2-[(2-Brom-4-methylphenyl)amino](1,3-thiazol-4-yl)}-5-methylthiothiophen-2-carboxamidin, 5-Methylthio-4-{2-[(2-morpholin-4-ylethyl)amino](1,3-thiazol-4-yl)}thiophen-2-carboxamidin, 4-{2-[(2,3-Dichlorphenyl)amino)(1,3-thiazol-4-yl)}-5-methylthiothiophen-2-carboxamidin, 5-Methylthio-4-{2-[(3,4,5-trimethoxyphenyl)amino](1,3-thiazol-4-yl)}thiophen-2-carboxamidin, 5-Methylthio-4-{2-[(2-piperidylethyl)amino](1,3-thiazol-4-yl)}thiophen-2-carboxamidin, 4-(2-{[(4-Methylphenyl)methyl]amino}(1,3-thiazol-4-yl)}-5-methylthiothiophen-2-carboxamidin, 4-(2-{[4-(4-Chlorphenoxy)phenyl]amino}(1,3-thiazol-4-yl))-5-methylthiothiophen-2-carboxamidin, 4-(2-{[4-Phenoxyphenyl]amino}(1,3-thiazol-4-yl))-5-methylthiothiophen-2-carboxamidin, 5-Methylthio-4-(2-{[4-(phenylamino)phenyl]amino}-(1,3-thiazol-4-yl))thiophen-2-carboxamidin, 5-Methylthio-4-(2-{(4-benzylphenyl]amino}-(1,3-thiazol-4-yl))thiophen-2-carboxamidin, 5-Methylthio-4-(2-{[4-(piperidylsulfonyl)phenyl]amino}(1,3-thiazol-4-yl))thiophen-2-carboxamidin, 5-Methylthio-4-[2-(3-chinolylamino)(1,3-thiazol-4-yl)]thiophen-2-carboxamidin, 5-Methylthio-4-[2-(2-naphthylamino)(1,3-thiazol-4-yl)]thiophen-2-carboxamidin, 4-[2-(2H-Benzo[3,4-d]1,3-dioxolan-5-ylamino)(1,3-thiazol-4-yl)]-5-methylthiothiophen-2-carboxamidin, 4-{2-[(7-Brom-fluoren-2-yl)amino]-(1,3-thiazol-4-yl)}-5-methylthiothiophen-2-carboxamidin, 4-{2-[(4-Cyclohexylphenyl)amino (1,3-thiazol-4-yl)}-5-methylthiothiophen-2-carboxamidin, 5-Methylthio-4-(2-{[4-(phenyldiazenyl)phenyl]amino}(1,3-thiazol-4-yl))thiophen-2-carboxamidin, 5-Methylthio-4-(2-{(3-(hydroxymethyl)phenyl]amino}(1,3-thiazol-4-yl)}thiophen-2-carboxamidin, 4-[2-({3-[(Methylpiperidyl)methyl]phenyl}amino)(1,3-thiazol-4-yl)]-5-methylthiothiophen-2-carboxamidin, 4-{2-[(3-Hydroxyphenyl)amino](1,3-thiazol-4-yl)}-5-methylthiothiophen-2-carboxamidin, 4-(2-{[(4-(Carbamoylmethoxy)phenyl]amino}(1,3-thiazol-4-yl))-5-methylthiothiophen-2-carboxamidin, 5-Methyl-4-{2-[(3,4,5-trimethoxyphenyl)amino](1,3-thiazol-4-yl)}thiophen-2-carboxamidin, 5-Methyl-4-{2-[(4-phenoxyphenyl)amino](1,3-thiazol-4-yl)}thiophen-2-carboxamidin, 5-Methyl-4-[2-(phenylamino)(1,3-thiazol-4-yl)thiophen-2-carboxamidin, 4-(4-Isoxazol-5-yl(1,3-thiazot-4-yl))-5-methylthiothiophen-2-carboxamidin.

## Revendications

1. Utilisation, dans la fabrication d'un médicament pour traiter une maladie choisie parmi l'hypertrophie prostatique bénigne, le carcinome prostatique, la métastase tumorale, la resténose et le psoriasis, d'un composé de formule I : 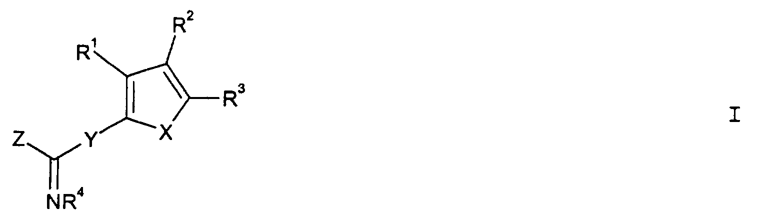 ou d'un solvate, hydrate ou sel acceptable en pharmacie d'un tel composé, formule dans laquelle :
X est le soufre ;
Y est une liaison covalente, CH₂ ou NH ;
Z est NR⁵R⁶, hydrogène ou alkyle, sous réserve que Y est NH quand Z est hydrogène ou alkyle ;
R¹ est hydrogène, halogène, amino, hydroxy, cyano, alkyle en C₁ à C₄, -CH₂R où R est hydroxyamino, ou alcoxy en C₁ à C₃ ;
R² et R³ sont indépendamment :
1. hydrogène ;
2. halogène ;
3. hydroxy ;
4. nitro ;
5. cyano ;
6. amino, monoalkylamino, dialkylamino, monoarylamino, diarylamino, monoalkylmonoarylamino, monoaralkylamino, diaralkylamino, alcarylamino, alcoxycarbonylamino, aralcoxycarbonylamino, aryloxycarbonylamino, alkylsulfonylamino, aralkylsulfonylamino, arylsulfonylamino, formylamino, acylamino, H(S)CNH-, ou thioacylamino ;
7. aminocarbonyle, monoalkylaminocarbonyle, dialkylaminocarbonyle, acyle, arylaminocarbonyle, ou aminoacyle ;
8. aminothiocarbonyle, monoalkylaminothiocarbonyle, dialkylaminothiocarbonyle, thioacyle, ou aminothioacyle ;
9. aminocarbonylamino, monoalkylaminocarbonylamino, dialkylaminocarbonylamino, monoarylaminocarbonylamino, diarylaminocarbonylamino, monoaralkylaminocarbonylamino, ou diaralkylaminocarbonylamino ;
10. aminocarbonyloxy, monoalkylaminocarbonyloxy, dialkylaminocarbonyloxy, monoarylaminocarbonyloxy, diarylaminocarbonyloxy, monoaralkylaminocarbonyloxy, ou diaralkylaminocarbonyloxy ;
11. aminosulfonyle, monoalkylaminosulfonyle, dialkylaminosulfonyle, monoarylaminosulfonyle, diarylaminosulfonyle, ou monoaralkylaminosulfonyle, ou diaralkylaminosulfonyle ;
12. alcoxy ou alkylthio, où ladite partie alkyle dudit groupe alcoxy ou alkylthio peut être éventuellement substituée ;
13. aralcoxy, aryloxy, aralkylthio, ou arylthio, où la partie aryle dudit groupe aralcoxy, aryloxy, aralkylthio ou arylthio peut être éventuellement substituée ;
14. alkylsulfonyle, où la partie alkyle peut être éventuellement substituée ;
15. aralkylsulfonyle, ou arylsulfonyle, où la partie aryle de chaque groupe peut être éventuellement substituée ;
16. alcényle ou alcynyle ;
17. aryle éventuellement substitué ;
18. alkyle éventuellement substitué ;
19. aralkyle éventuellement substitué ;
20. hétérocycle éventuellement substitué ; ou
21. cycloalkyle éventuellement substitué ; et
R⁴, R⁵ et R⁶ sont indépendamment hydrogène ou alkyle en C₁ à C₄, aryle, hydroxyalkyle, aminoalkyle, monoalkylamino-alkyle en C₂ à C₁₀, dialkylamino-alkyle en C₂ à C₁₀, carboxyalkyle, cyano, amino, alcoxy, ou hydroxy ; et dans laquelle, sauf mention contraire, alkyle représente un groupe alkyle à chaîne droite ou ramifiée ayant jusqu'à 12 atomes de carbone ; alcényle représente un groupe alcényle à chaîne droite ou ramifiée ayant 2 à 20 atomes de carbones ; alcènyle représente un groupe alcènyle à chaîne droite ou ramifiée ayant 2 à 20 atomes de carbone ; alcynyle représente un groupe alcynyle à chaîne droite ou ramifiée ayant de 2 à 20 atomes de carbone ; alkylthio représente un groupe alkyle à chaîne droite ou ramifiée lié à un atome de soufre ; alcoxy représente un groupe alcoxy à chaîne droite ou ramifiée ayant jusqu'à 20 atomes de carbone, lié à un atome d'oxygène ; cycloalkyle représente un groupe alkyle cyclique contenant de 3 à 9 atomes de carbone ; aryle représente un groupe aromatique monocyclique ou bicyclique contenant de 6 à 14 carbones dans la partie cyclique ; aralkyle et arylalkyle représentent des groupes en C₁ à C₆ ayant un substituant aryle ; hétérocycle représente un monocycle à 3 à 7 chaînons ou un système bicyclique à 7 à 10 chaînons, saturé ou complètement ou partiellement insaturé, qui est constitué d'atomes de carbone et de 1 à 4 hétéroatomes indépendamment choisis dans l'ensemble constitué par O, N et S, et comprenant tout groupe bicyclique dans lequel l'un quelconque des hétérocycles définis ci-dessus est. condensé à un cycle benzène et où l'hétérocycle peut être substitué sur un atome de carbone ou d'azote ; hétéroatome représente O, S ou N, et lorsque l'hétéroatome est l'azot il peut former un fragment NR^{y}R^{z} où R^{y} et R^{z} sont indépendamment choisis parmi hydrogène et alkyle en C₁ à C₈, ou bien, conjointement avec l'azote, forment un cycle saturé ou insaturé à 5, 6 ou 7 chaînons ; hétéroaryle représente des groupes ayant de 5 à 14 atomes sur le cycle, 6, 10 ou 14 électrons π partagés en agencement cyclique, et contenant des atomes de carbone et 1, 2 ou 3 hétéroatomes oxygène, azote ou soufre ; acyle représente le groupe -C(O)R^{g} où R^{g} est alkyle, alcényle, alcynyle, aryle ou aralkyle ; thioacyle représente le groupe -C(S)R^{g} ou R^{g} est alkyle, alcènyle, alcynyle, aryle ou aralkyle ; monoalkylamine représente un groupe amino substitué par un seul groupe alkyle ayant jusqu'à 6 atomes de carbone ; dialkylamine représente un groupe amino substitué par deux groupes alkyle ayant jusqu'à 6 atomes de carbone ; et où "alkyle éventuellement substitué" représente un groupe alkyle substitué par 1 à 4 groupes indépendamment choisis parmi halogène, hydroxy, thiol, amino, monoalkylamino, dialkylamino, formylamino, aminoiminométhyle, acylamino, aminoacyle, mono- ou di- alkylaminocarbonyle, thiocarbonylamino, thioacylamino, aminothiocarbonyle, alkyloxy, aryloxy, aminocarbonyloxy, mono- ou di-alkylaminocarbonyloxy, mono- ou di- arylsulfonyle, aralkylsulfonyle, alkylsulfonylamino, arylsulfonylamino, aralkylsulfonylamino, mono- ou dialkylaminothiocarbonyle, aralcoxy, carboxy, carboxyalkyle, alcoxycarbonyle, alcoxycarbonylalkyle, nitro, cyano, trifluorométhyle, alkylthio et arylthio ; "aryle ou hétérocycle éventuellement substitué" représente un groupe aryle ou hétérocyclique ayant de 1 à 4 groupes indépendamment choisis parmi chloro, hydroxy, amino, mono(alkyl en C₁ à C₄) amino, di(alkyl en C₁ à C₄) amino, formylamino, acylamino en C₂ à C₆, aminocarbonyle, aminoacyle en C₂ à C₈, cycloalkyle en C₃ à C₇, alkyle en C₁ à C₆, alcoxy en C₁ à C₆, aryloxy en C₆ à C₁₄, carboxy, carboxy-alkyle en C₁ à C₆, alcoxycarbonyle en C₂ à C₈, nitro, cyano, trifluorométhyle, alkylthio en C₁ à C₆, arylthio en C₆ à C₁₄, aryle en C₆ à C₁₄, phényle substitué, tétrazolyle, thiényle éventuellement substitué par un, deux ou trois groupes choisis parmi chloro, hydroxy, alkyle en C₁ à C₄, alcoxy en C₁ à C₄, amino ou carboxy, 3,4-méthylènedioxy, 3,4-éthylènedioxy, 3,4-propylènedioxy, alkylsulfonylamino en C₁ à C₆, aralkylsulfonylamino en C₇ à C₁₅, arylsulfonylamino en C₁ à C₆, mono- ou di-(alkyl en C₁ à C₆)aminocarbonyloxy, mono- ou di-(aryl en C₆ à C₁₀)aminocarbonyloxy, mono- ou di-(aralkyl en C₇ à C₁₅)carbonyloxy, (alcoxy en C₁ à C₆)carbonylamino, (aralcoxy en C₇ à C₁₅)carbonylamino, (aryloxy en C₆ à C₁₀)carbonylamino, thioacylamino en C₂ à C₆, aminothiocarbonyle et aminothioacyle en C₂ à C₈.

2. Utilisation selon la revendication 1, dans laquelle ledit composé est destiné à une administration en une quantité comprise entre 0,01 et 50 milligrammes par kilogramme et par jour.

3. Utilisation selon la revendication 1, dans laquelle ledit composé est destiné à une administration en une quantité comprise entre 0,1 et 20 milligrammes par kilogramme et par jour.

4. Composé de formule I telle que définie dans la revendication 1, dans lequel X, Y, R¹, R², R³, R⁴, R⁵ et R⁶ sont tels que définis ici, sous réserve qu'au moins l'un de R² et R³ est choisi dans l'ensembl constitué par :
(a) un groupe alkyle éventuellement substitué ;
(b) alcoxy, aryloxy, alkylthio ou arylthio, l'un quelconque d'entre eux étant éventuellement substitué ;
(c) aryle en C₆ à C₁₄ éventuellement substitué, ou aralkyle éventuellement substitué, sauf que R³ n'est ni nitrophényle ni aminophényle, quand R¹ et R² sont tous deux hydrogène ou méthyle ;
(d) hétérocycle éventuellement substitué ; et
(e) cycloalkyle éventuellement substitué ;
sous réserve que le composé n'est ni le 5-(1,1-diméthyléthyl)-N-hydroxy-2-thiophènéthanimidamide ni l'amid d'acide 5-carbamimidoyl-3,4-diméthylthiophène-2-carboxylique.

5. Composé selon la revendication 4, dans lequel R² ou R³ est alkyle, cycloalkyle, alcoxy, alkylthio ou alkylsulfonyle, et la partie alkyle dudit alkyle, cycloalkyle, alcoxy, alkylthio ou alkylsulfonyle est éventuellement substituée par 1 à 4 substituants choisis dans l'ensemble constitué par halogène, hydroxy, thiol, amino, monoalkylamino, dialkylamino, formylamino, acylamino, aminoacyle, monoalkylaminocarbonyle, dialkylaminocarbonyle, thiocarbonylamino, thioacylamino, aminothiocarbonyle, alcoxy, aryloxy, aminocarbonyloxy, monoalkylaminocarbonyloxy, dialkylaminocarbonyloxy, monoarylaminocarbonyloxy, diarylaminocarbonyloxy, monoaralkylaminocarbonyloxy, diaralkylaminocarbonyloxy, alkylsulfonyle, arylsulfonyle, aralkylsulfonyle, alkylsulfonylamino, arylsulfonylamino, aralkylsulfonylamino, alcoxycarbonylamino, aralcoxycarbonylamino, aryloxycarbonylamino, monoalkylaminothiocarbonyle, dialkylaminothiocarbonyle, aralcoxy, carboxy, carboxyalkyle, alcoxycarbonyle, alcoxycarbonylalkyle, nitro, cyano, trifluorométhyle, alkylthio et arylthio.

6. Composé selon la revendication 4, dans lequel R³ est alkyle ou alkylthio, éventuellement substitué.

7. Composé selon la revendication 5, dans lequel lesdits 1 à 4 substituants sont choisis dans l'ensemble constitué par chloro, hydroxy, amino, mono(alkyl en C₁ à C₄) amino, di(alkyl en C₁ à C₄)amino, formylamino, acylamino en C₂' à C₆, aminocarbonyle, aminoacyle en C₂ à C₈, thioacylamino en C₂ à C₆, aminothiocarbonyle, aminothioacyle en C₂ à C₈, alcoxy en C₁ à C₆, aryloxy en C₆ à C₁₄, carboxy, carboxy-alkyle en C₁ à C₆, alcoxycarbonyle en C₂ à C₈, nitro, cyano, trifluorométhyle, alkylthio en C₁ à C₆, arylthio en C₆ à C₁₄, aralkylsulfonylamino en C₁ à C₆, arylsulfonylamino en C₁ à C₆, monoalkylaminocarbonyloxy, dialkylaminocarbonyloxy, mono(aryl en C₆ à C₁₀)aminocarbonyloxy, di(aryl en C₆ à C₁₀)aminocarbonyloxy, monoaralkylcarbonyloxy, diaralkylcarbonyloxy, (alcoxy en C₁ à C₆)carbonylamino, (aralcoxy en C₇ à C₁₅)carbonylamino et (aryloxy en C₆ à C₁₀)carbonylamino.

8. Composé selon la revendication 4, dans lequel au moins l'un de R² et R³ est alkyle, aralcoxy, arylthio, aralkyle, aryloxy, aralkylthio, aralkylsulfonyle, arylsulfonyle, hétérocycle ou hétérocycloalkyle éventuellement substitué par 1 à 4 substituants choisis dans l'ensemble constitué par halogène, hydroxy, thiol, amino, monoalkylamino, dialkylamino, formylamino, acylamino, aminoacyle, monoalkylaminocarbonyle, dialkylaminocarbonyle, thiocarbonylamino, thioacylamino, aminothiocarbonyle, alcoxy, aryloxy, aminocarbonyloxy, monoalkylaminocarbonyloxy, dialkylaminocarbonyloxy, monoarylaminocarbonyloxy, diarylaminocarbonyloxy, monoaralkylaminocarbonyloxy, diaralkylaminocarbonyloxy, alkylsulfonyle, arylsulfonyle, aralkylsulfonyle, alkylsulfonylamino, arylsulfonylamino, aralkylsulfonylamino, alcoxycarbonylamino, aralcoxycarbonylamino, aryloxycarbonylamino, monoalkylaminothiocarbonyle, dialkylaminothiocarbonyle, aralcoxy, carboxy, carboxyalkyle, alcoxycarbonyle, alcoxycarbonylalkyle, nitro, cyano, trifluorométhyle, alkylthio et arylthio.

9. Composé selon la revendication 8, dans lequel lesdits 1 à 4 substituants sont choisis dans l'ensembl constitué par chloro, hydroxy, amino, mono(alkyl en C₁ à C₄) amino, di(alkyl en C₁ à C₄) amino, formylamino, acylamino en C₂ à C₆, aminocarbonyle, aminoacyle en C₂ à C₈, cycloalkyle en C₃ à C₇, alkyle en C₁ à C₆, alcoxy en C₁ à C₆, aryloxy en C₆ à C₁₄, carboxy, carboxy-alkyle en C₁ à C₆, alcoxycarbonyle en C₂ à C₈, nitro, cyano, trifluorométhyle, alkylthio en C₁ à C₆, arylthio en C₆ à C₁₄, aryle en C₆ à C₁₄, tétrazolyle, thiényle, 3,4-méthylènedioxy, 3,4-éthylènedioxy, 3,4-propylènedioxy, alkylsulfonylamino en C₁ à C₆, aralkylsulfonylamino en C₁ à C₆, arylsulfonylamino en C₁ à C₆, mono- ou dialkylaminocarbonyloxy, mono- ou di(aryl en C₆ à C₁₀)aminocarbonyloxy, mono- ou di-aralkylcarbonyloxy, (alcoxy en C₁ à C₆)carbonyl amino, (aralcoxy en C₇ à C₁₅) carbonylamino, (aryloxy en C₆ à C₁₀) carbonylamino, thioacylamino en C₂ à C₆, aminothiocarbonyle, et aminothioacyle en C₂ à C₈.

10. Composé selon la revendication 4, dans lequel :
X est soufre ;
Y est une liaison covalente ou -NH- ;
R¹ est hydrogène, halogène, amino ou hydroxy ;
l'un de R² et R³ est hydrogène, alkylthio en C₁ à C₆, alkyle en C₁ à C₆, ou alcoxy en C₁ à C₆, et l'autre de R² et R³ est aminoacyle, acylamino, aminosulfonyle, sulfonylamino, aminocarbonylamino, alcoxycarbonylamino, oxazolyle éventuellement substitué, isoxazolyle éventuellement substitué, benzothiényle éventuellement substitué, furanyle éventuellement substitué, pyrazolyle éventuellement substitué ou pyridyle éventuellement substitué ;

11. Composé selon la revendication 10, dans lequel R⁴, R⁵ et R⁶ sont hydrogène.

12. Composé selon la revendication 4, dans lequel :
X est soufre ;
Y est une liaison covalente ou -NH- ;
Z est NR⁵R⁶ ;
R¹ est hydrogène, halogène, amino ou hydroxy ;
l'un de R² et R³ est hydrogène, alkylthio en C₁ à C₆, alkyle en C₁ à C₆ ou alcoxy en C₁ à C₆, et l'autre de R² et R³ est : 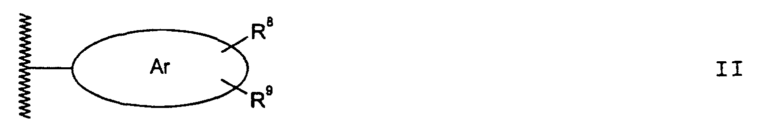 où :
Ar est phényle, thiazolyle, thiazolinyle, oxazolyle, isothiazolyle, isoxazolyle, furanyle, imidazolyle, pyridyle, pyrimidinyle, pyrazinyle, thiényle, tétrazolyle, pyrrolyle, pyrazolyle, oxadiazolyle, oxazolinyle, isoxazolinyle, imidazolinyle, triazolyle, pyrrolinyle, benzothiazolyle, benzothiényle, benzimidazolyle, 1,3-oxazolidin-2-onyle, et imidazolin-2-onyle ;
R⁸ et R⁹ sont indépendamment choisis dans l'ensemble constitué par hydrogène, halogène, amino, monoalkylamino en C₁ à C₄, arylamino, mono(aryl en C₆ à C₁₄) amino, di(aryl en C₆ à C₁₄) amino, mono(aryl en C₆ à C₁₄) (alkyl en C₁ à C₆) amino, di(aryl en C₆à C₁₄) (alkyl en C₁ à C₆)amino, di(alkyl en C₁ à C₄)amino, formylamino, acylamino en C₂ à C₆, aminocarbonyle, aminoacyle en C₂ à C₈, thioacylamino en C₂ à C₆, aminothiocarbonyle, aminothioacyle en C₂ à C₈, alkyle en C₁ à C₆, cycloalkyle en C₃ à C₆, alcoxy en C₁ à C₆, carboxy, carboxy-alkyle en C₁ à C₆, alcoxycarbonyle en C₂ à C₈, nitro, cyano, trifluorométhyle, tétrazolyle, thiényle, aryloxy en C₆ à C₁₄, alkylthio en C₁ à C₆, arylthio en C₆ à C₁₄, aryle en C₆ à C₁₄, et (aryl en C₆ à C₁₄)alkyle en C₁ à C₆, où les parties aryle de l'un quelconque desdits groupes peuvent être éventuellement substituées par 1 à 3 substituants indépendamment choisis dans l'ensemble constitué par halogène, hydroxy, amino, mono(alkyl en C₁ à C₄) amino, di(alkyl en C₁ à C₄)amino, formylamino, acylamino en C₁ à C₄, aminoacyle en C₁ à C₄, mono(alkyl en C₁ à C₄) aminocarbonyle, di(alkyl en C₁ à C₄)aminocarbonyle, thiocarbonylamino, thioacylamino en C₁ à C₄, aminothiocarbonyle, alcoxy en C₁ à C₄, aryloxy en C₆ à C₁₀, aminocarbonyloxy, mono(alkyl en C₁ à C₄)aminocarbonyloxy, di(alkyl en C₁ à C₄)aminocarbonyloxy, mono(aryl en C₆ à C₁₀)aminocarbonyloxy, di(aryl en C₆ à C₁₀)aminocarbonyloxy, mono(aralkyl en C₄ à C₁₂)aminocarbonyloxy, di(aralkyl en C₄ à C₁₂)aminocarbonyloxy, alkylsulfonyle en C₁ à C₄, arylsulfonyle en C₆ à C₁₀, aralkylsulfonyle en C₇ à C₁₂, alkylsulfonylamino en C₁ à C₄, arylsulfonylamino en C₆ à C₁₀, (aralkyl en C₇ à C₁₂) sulfonylamino, (alcoxy en C₁ à C₄) carbonylamino, (aralcoxy en C₇ à C₁₂) carbonylamino, (aryloxy en C₆ à C₁₀)carbonylamino, mono(alkyl en C₁ à C₄)aminothiocarbonyle, di(alkyl en C₁ à C₄)aminothiocarbonyle, aralcoxy en C₇ à C₁₂, carboxy, carboxy-alkyle en C₁ à C₄, (alcoxy en C₁ à C₄)carbonyle, (alcoxy en C₁ à C₄)carbonylalkyle, nitro, cyano, trifluorométhyle, alkylthio en C₁ à C₄, arylthio en C₆ à C₁₀, 3,4-méthylènedioxy, 3,4-éthylènedioxy, et 3,4-propylènedioxy ; et
R⁴, R⁵ et R⁶ sont indépendamment hydrogène, alkyle en C₁ à C₄, amino, alcoxy en C₁ à C₄ ou hydroxy.

13. Composé selon la revendication 12, dans lequel :
X est soufre ;
Y est une liaison covalente ;
Z est NR⁵R⁶ ;
R¹ est hydrogène ;
R² est : 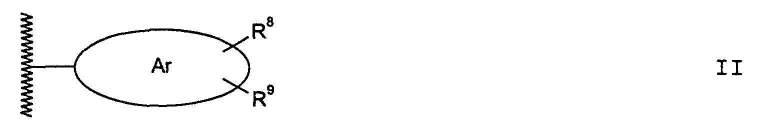
où :
Ar est phényle, thiazolyle, oxazolyle, pyridyle ou imidazolyle ;
R⁸ et R⁹ sont indépendamment choisis dans l'ensemble constitué par hydrogène, aryle en C₆ à C₁₀ éventuellement substitué par 1 à 3 substituants indépendamment choisis dans l'ensembl constitué par chloro, hydroxy, alkyle en C₁ à C₄, cycloalkyle en C₃ à C₆, alcoxy en C₁ à C₄, amino, carboxy, phényle, naphtyle, biphényle, hydroxyphényle, méthoxyphényle, chlorophényle, dichlorophényle, aminophényle, carboxyphényle, nitrophényle, 3,4-éthylènedioxy, 3,4-méthylènedioxy, et 3,4-propylènedioxy ; R³ est méthylthio ou méthyle ; et
R⁴, R⁵ et R⁶ sont hydrogène.

14. Composé selon la revendication 4, dans lequel
X est soufre ;
Y est une liaison covalente directe ;
Z est NR⁵R⁶ ;
R¹ est hydrogène ;
R² est alkyle, ar(alkyle), alkylsulfonyle, -SO₂-alkyle, amido, amidino, ou 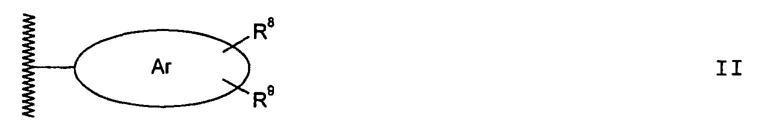 où :
Ar est un groupe aromatique ou hétéroaromatique choisi dans l'ensembl constitué par phényle, thiazolyle, oxazolyle, imidazolyle et pyridyle ;
R⁸ et R⁹ sont indépendamment choisis dans l'ensemble constitué par hydrogène, carboxy, phényle, naphtyle, alkyle, pyridyle, oxazolyle, furanyle, cycloalkyle et amino, l'un quelconque d'entre eux pouvant être éventuellement substitué par 1 à 3 substituants indépendamment choisis dans l'ensembl constitué par halogène, alkyle, halogénoalkyle, alkaryle, hétéroaryle, phényle, naphtyle, alcoxy, aryloxy, hydroxy, amino, nitro, thiophényle, benzothiophényle, fluorényle, 3,4-éthylènedioxy, 3,4-méthylènedioxy, 3,4-propylènedioxy, arylsulfonamido, alkylsulfonamido et aryloxy, chacun desdits 1 à 3 substituants pouvant être en outre éventuellement substitué par un ou plusieurs groupes choisis parmi halogène, alcoxy, halogénoalkyle, alkyle, amino, acétyle, hydroxy, dialkylamino, dialkylaminoacyle, monoalkylaminoacyle, -SO₂-hétéroaryle, -SO₂-aryle, et aryle ;
R³ est hydrogène, -SO₂-alkyle, trifluorométhyle, S(O)-alkyle, alcoxy, alkylthio, alkyle, aralkylthio ; et
R⁴, R⁵ et R⁶ sont hydrogène.

15. Composé selon la revendication 14, dans lequel Ar est thiazolyle et au moins l'un de R⁷ et R⁸ est phényle.

16. Composé selon la revendication 14 ou 15, dans lequel ledit thiazolyle est thiazol-2-yle.

17. Composé selon la revendication 13, dans lequel R² est un groupe 4-phénylthiazol-2-yle, où ledit phényle est en outre éventuellement substitué.

18. Composé selon la revendication 14 ou 15, dans lequel ledit thiazolyle est thiazol-4-yle.

19. Composé selon la revendication 18, dans lequel R² est un groupe 2-aminothiazol-4-yle.

20. Composé selon la revendication 14, dans lequel ledit oxazolyle est oxazol-2-yle.

21. Composé selon la revendication 14, dans lequel ledit oxazolyle est oxazol-4-yle.

22. Composé selon la revendication 14, dans lequel R³ est méthylthio.

23. Composé de formule I telle que définie dans la revendication 1, dans lequel :
X est soufre ;
Y est une liaison covalente ;
Z est NR⁵R⁶ ;
R¹ est hydrogène ;
R² est : 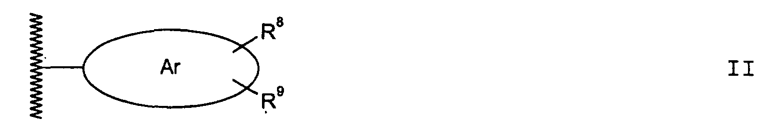
où :
Ar est phényle, thiazolyle, ou oxazolyle ;
R⁸ et R⁹ sont indépendamment choisis dans l'ensemble constitué par hydrogène, aryle en C₆ à C₁₀ éventuellement substitué par 1 à 3 substituants indépendamment choisis dans l'ensemble constitué par chloro, hydroxy, alkyle en C₁ à C₄, alcoxy en C₁ à C₄, phényle, 3,4-éthylènedioxy, 3,4-méthylènedioxy, et 3,4-propylènedioxy ;
R³ est méthylthio ; et
R⁴, R⁵ et R⁶ sont hydrogène.

24. Composé de formule I telle que définie dans la revendication 1, dans lequel :
X est soufre ;
Y est une liaison covalente ;
R¹ est hydrogène ;
R² est : 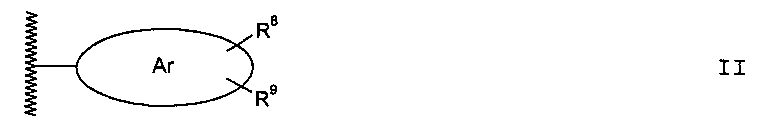
où :
Ar est thiazolyle ;
R⁸ et R⁹ sont indépendamment choisis dans l'ensemble constitué par hydrogène, aryle en C₆ à C₁₀ substitué par un groupe sulfonamide ;
R³ est méthylthio ; et
R⁴, R⁵ et R⁶ sont hydrogène.

25. Composé selon la revendication 24, dans lequel ledit groupe sulfonamide est arylsulfonamide, alkylsulfonamide, alcoxysulfonamide ou hétéroarylsulfonamide, en C₆ à C₁₀.

26. Composé selon la revendication 25, dans lequel ledit groupe sulfonamide est choisi dans l'ensemble constitué par le 4-méthylphénlsulfonamide, le méthylsulfonamide, le phénylsulfonamide, le trifluorométhylsulfonamide, le 4-fluorophénylsulfonamide, le 4-chlorophénylsulfonamide, le 3-chlorophénylsulfonamide, le 4-méthoxysulfonamide, le 2,4-difluorophénylsulfonamide, le 2-(thiophène)sulfonamide, le 2-(5-chlorothiophène)sulfonamide, le butylsulfonamide, et l'isopropylsulfonamide.

27. Composé de formule I telle que définie dans la revendication 1, dans lequel :
X est soufre ;
Y est une liaison covalente ;
Z est NR⁵R⁶ ;
R¹ est hydrogène ;
R² est : 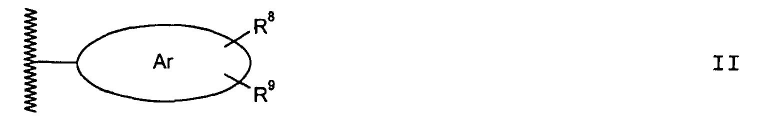
où :
Ar est thiazolyle ;
R⁸ et R⁹ sont indépendamment choisis dans l'ensemble constitué par hydrogène, aryle en C₆ à C₁₀ substitué par un groupe choisi parmi -OCH₂C(O)-alcoxy, -OCH₂C(O)amino, -OCH₂C(O)-NH-alkyle et -OCH₂C(O)-N-(alkyle)₂ ;
R³ est méthylthio ; et
R⁴, R⁵ et R⁶ sont hydrogène.

28. Composé selon la revendication 14, dans lequel :
Ar est thiazolyle ; et
l'un de R⁸ et R⁹ est hydrogène et l'aut de R⁸ et R⁹ est amino, ledit groupe amino étant éventuellement substitué par halogène, hydroxy, amino, monoalkylamino, dialkylamino, formylamino, acylamino, aminoacyle, monoalkylaminocarbonyle, dialkylaminocarbonyle, thiocarbonylamino, thioacylamino, aminothiocarbonyle, alcoxy, aryloxy, aminocarbonyloxy, monoalkylaminocarbonyloxy, dialkylaminocarbonyloxy, monoarylaminocarbonyloxy, diarylaminocarbonyloxy, monoaralkylaminocarbonyloxy, diaralkylaminocarbonyloxy, alkylsulfonyle, arylsulfonyle, aralkylsulfonyle, alkylsulfonylamino, arylsulfonylamino, aralkylsulfonylamino, alcoxycarbonylamino, aralcoxycarbonylamino, aryloxycarbonylamino, monoalkylaminothiocarbonyle, dialkylaminothiocarbonyle, aralcoxy, carboxy, carboxyalkyle, alcoxycarbonyle, alcoxycarbonylalkyle, nitro, cyano, trifluorométhyle, alkylthio ou arylthio.

29. Composé selon la revendication 4, qui est l'un des suivants : 4-[4-(4-chlorophényl)thiazol-2-yl]-5-méthylthiothiophène-2-carboxamidine ; 4-phényl-5-méthylthiothiophène-2-carboxamidine ; 4-[4-(2,4-dichlorophényl)thiazol-2-yl]-5-méthylthiothiophène-2-carboxamidine ; 4-(4-méthylthiazol-2-yl)-5-méthylthiothiophène-2-carboxamidine ; 4-[4-(3-méthoxyphényl)thiazol-2-yl]-5-méthylthiothiophène-2-carboxamidine ; 4-[4-(3-hydroxyphényl)thiazol-2-yl]-5-méthylthiothiophène-2-carboxamidine ; 4-(4-phénylthiazol-2-yl)-5-méthylthiothiophène-2-carboxamidine ; 4-[4-(4-nitrophényl)thiazol-2-yl]-5-méthylthiothiophène-2-carboxamidine ; 4-[4-(3,4-éthylènedioxyphényl)thiazol-2-yl]-5-méthylthiothiophène-2-carboxamidine ; 4-[4-(3,4-propylènedioxyphényl)thiazol-2-yl]-5-méthylthiothiophène-2-carboxamidine ; 4-[4-(4-(napht-2-yl)thiazol-2-yl]-5-méthylthiothiophène-2-carboxamidine ; et 4-isopropylsulfonyl-5-méthylthiothiophène-2-carboxamidine ; ou un hydrate, solvate ou sel acceptable en pharmacie d'un tel composé.

30. Composé selon la revendication 14, qui est l'un des suivants : 4-phényl-5-méthylthiothiophène-2-carboxamidine ; 4-[4-(4-chlorophényl)thiazol-2-yl]-5-méthylthiothiophène-2-carboxamidine ; 4-[4-(4-phénylphényl)thiazol-2-yl]-5-méthylthiothiophène-2-carboxamidine ; 4-[4-(3-méthoxyphényl)thiazol-2-yl]-5-méthylthiothiophène-2-carboxamidine ; 4-[4-(3-hydroxyphényl)thiazol-2-yl]-5-méthylthiothiophène-2-carboxamidine ; 4-(4-phénylthiazol-2-yl)-5-méthylthiothiophène-2-carboxamidine ; 4- [4- (4-nitrophényl)thiazol-2-yl]-5-méthylthiothiophène-2-carboxamidine ; 4-[4-(3,4-éthylènedioxyphényl)thiazol-2-yl]-5-méthylthiothiophène-2-carboxamidine ; 4-[4-(4-méthoxyphényl)thiazol-2-yl]-5-méthylthiothiophène-2-carboxamidine ; 4-[4-(3,4-propylènedioxyphényl)thiazol-2-yl]-5-méthylthiothiophène-2-carboxamidine ; 4-isopropylsulfonyl-5-méthylthiothiophène-2-carboxamidine ; 4-(4-méthylthiazol-2-yl)-5-méthylthiothiophène-2-carboxamidine ; 4-[4-(2,4-dichlorophényl)thiazol-2-yl]-5-méthylthiothiophène-2-carboxamidine ; 4-(2-naphtylthiazol-2-yl)-5-méthylthiothiophène-2-carboxamidine ; 4-[4-(4-chloro-3-méthylphényl)thiazol-2-yl]-5-méthylthiothiophène-2-carboxamidine ; 4-(5-méthyl-4-phénylthiazol-2-yl)-5-méthylthiothiophène-2-carboxamidine ; 4-[4-(4-chlore-3-nitrophényl)thiazol-2-yl]-5-méthylthiothiophène-2-carboxamidine ; 4-(5-phényloxazol-2-yl)-5-méthylthiothiophène-2-carboxamidine ; 4-[4-(3-fluoro-5-trifluorométhylphényl)-5-méthylthiazol-2-yl]-5-méthylthiothiophène-2-carboxamidine ; 4-[4-(3,5-bis(trifluorométhyl)phényl)-5-méthylthiazol-2-yl]-5-méthylthiothiophène-2-carboxamidine ; 4-[4-(3-fluoro-5-trifluorométhylphényl)thiazol-2-yl]-5-méthylthiothiophène-2-carboxamidine ; 4-[4-(3-bromophényl)thiazol-2-yl]-5-méthylthiothiophène-2-carboxamidine ; 4-[4-(3,4-méthylènedioxyphényl)thiazol-2-yl]-5-méthylthiothiophène-2-carboxamidine ; 4-[4-(4-méthylphényl)thiazol-2-yl]-5-méthylthiothiophène-2-carboxamidine ; 4-[4-(3,5-bis(trifluorométhyl)phényl)thiazol-2-yl]-5-méthylthiothiophène-2-carboxamidine ; 4- [4- (2-méthoxyphényl)thiazol-2-yl]-5-méthylthiothiophène-2-carboxamidine ; 4-(4-phénylimidazol-2-yl)-5-méthylthiothiophène-2-carboxamidine ; 4-[4-(2,4-diméthoxyphényl)thiazol-2-yl]-5-méthylthiothiophène-2-carboxamidine ; 4-(4-benzylthiazol-2-yl)-5-méthylthiothiophène-2-carboxamidine ; 4-[4-(3,4-dichlorophényl)thiazol-2-yl]-5-méthylthiothiophène-2-carboxamidine ; 4-[4-(3-méthylphényl)thiazol-2-yl]-5-méthylthiothiophène-2-carboxamidine ; 4-[4-(3,5-diméthoxyphényl)thiazol-2-yl]-5-méthylthiothiophène-2-carboxamidine ; 4-[4-(2-méthylphényl)thiazol-2-yl]-5-méthylthiothiophène-2-carboxamidine ; 4-[4-(2,5-diméthoxyphényl)thiazol-2-yl]-5-méthylthiothiophène-2-carboxamidine ; 4-(4,5-diphényl)thiazol-2-yl-5-méthylthiothiophène-2-carboxamidine ; 4-(2-phényl)thiazol-4-yl-5-méthylthiothiophène-2-carboxamidine ; 4- [4- (2-chloro-3-pyridyl)thiazol-2-yl]-5-méthylthiothiophène-2-carboxamidine ; 4-[4-(phénoxyméthyl)thiazol-2-yl]-5-méthylthiothiophène-2-carboxamidine ; 4-(4-cyclohexylthiazol-2-yl)-5-méthylthiothiophène-2-carboxamidine ; 4-[4-(4-chlorophényl)thiazol-2-yl]-5-méthylthiothiophène-2-carboxamidine ; 4-[4-(2-hydroxyphényl)thiazol-2-yl]-5-méthylthiothiophène-2-carboxamidine ; 4-[4-(3-trifluorométhoxyphényl)thiazol-2-yl]-5-méthylthiothiophène-2-carboxamidine ; 4-[4-(2-chloro-4-pyridyl)thiazol-2-yl]-5-méthylthiothiophène-2-carboxamidine ; 4-(5-phényl-2-pyridyl)-5-méthylthiothiophène-2-carboxamidine ; 4-[2-(2-chlorophénylamino)thiazol-4-yl]-5-méthylthiothiophène-2-carboxamidine ; 4-[2-(3-méthoxyphénylamino)thiazol-4-yl]-5-méthylthiothiophène-2-carboxamidine ; 4-[2-(phénylamino)thiazol-4-yl]-5-méthylthiothiophène-2-carboxamidine ; 4-[2-(2,5-diméthoxyphénylamino)thiazol-4-yl]-5-méthylthiothiophène-2-carboxamidine ; 4-(2-aminothiazol-4-yl)-5-méthylthiothiophène-2-carboxamidine ; 4-[2-(4-chlore-2-méthylphénylamino)thiazol-4-yl]-5-méthylthiothiophène-2-carboxamidine ; 4-[2-(4-diméthylaminophénylamino)thiazol-4-yl]-5-méthylthiothiophène-2-carboxamidine ; 4-[2-(4-méthoxyphénylamino)thiazol-4-yl]-5-méthylthiothiophène-2-carboxamidine ; 4-[4-(4-hydroxy-3-méthoxyphényl)thiazol-2-yl)-5-méthylthiothiophène-2-carboxamidine ; 4-[4-(3-hydroxy-4-méthoxyphényl)thiazol-2-yl]-5-méthylthiothiophène-2-carboxamidine ; 4-[2-(2-fluorophénylamino)thiazol-4-yl]-5-méthylthiothiophène-2-carboxamidine ; 4-[2-(2,4,5-triméthylphényl)aminothiazol-4-yl]-5-méthylthiothiophène-2-carboxamidine ; 4-[2-(3-chloro-2-méthylphényl)aminothiazol-4-yl]-5-méthylthiothiophène-2-carboxamidine ; 4-[2-(2-isopropylphényl)aminothiazol-4-yl]-5-méthylthiothiophène-2-carboxamidine ; 4-[2-(4-benzyloxyphényl)aminothiazol-4-yl]-5-méthylthiothiophène-2-carboxamidine ; 4-[2-(2-bromophényl)aminothiazol-4-yl]-5-méthylthiothiophène-2-carboxamidine ; 4-[2-(2,5-dichlorophényl)aminothiazol-4-yl]-5-méthylthiothiophène-2-carboxamidine ; 4-[2-(2-bromo-4-méthylphényl)aminothiazol-4-yl]-5-méthylthiothiophène-2-carboxamidine ; 4-[2-(2,3-dichlorophényl)aminothiazol-4-yl]-5-méthylthiothiophène-2-carboxamidine ; 4-[2-(3,4,5-triméthoxyphényl)aminothiazol-4-yl]-5-méthylthiothiophène-2-carboxamidine ; 4-[2-(2-pipéridinyléthyl)aminothiazol-4-yl]-5-méthylthiothiophène-2-carboxamidine ; 4-[2-(4-méthylphényl)aminothiazol-4-yl]-5-méthylthiothiophène-2-carboxamidine ; 4-(4-phényloxazol-2-yl)-5-méthylthiothiophène-2-carboxamidine ; 4-[2-(diphénylméthyl)aminothiazol-4-yl]-5-méthylthiothiophène-2-carboxamidine ; 4-[2-(3-phénylpropyl)aminothiazol-4-yl]-5-méthylthiothiophène-2-carboxamidine ; ou un solvate, hydrate ou sel acceptable en pharmacie d'un tel composé.

31. Utilisation, dans la fabrication d'un médicament pour l'inhibitio d'une protéase choisie dans l'ensembl constituée par l'élastase de neutrophile leucocytaire, la chymotrypsine, la trypsine, l'élastas pancréatique, la cathepsine G, la thrombine, l'urokinase, le facteur Xa, la plasmine, la thermolysine, l'estérase C-1, la convertase C-3, l'acrosine, la thrombine, les kallikréines, et la pepsine, d'un composé selon la revendication 4 ou la revendication 14.

32. Utilisation selon la revendication 31, dans laquelle ladite protéase est la trypsine, la chymotrypsine, la plasmine ou l'urokinase.

33. Utilisation, dans la fabrication d'un médicament pour le traitement du syndrome de détresse respiratoire chez l'adulte, de la cicatrisation d'une plaie, de la goutte, de la polyarthrite rhumatoïde, d'un dommage par reperfusion, de l'athérosclérose, d'une resténose, d'une néoplasie, de métastases, de l'emphysème, de la maladie d'Alzheimer, d'une pancréatite, d'une hypertrophie prostatique bénigne, d'un carcinome prostatique, du psoriasis ou de la maladie de Parkinson, d'un composé selon la revendication 4 ou la revendication 14.

34. Composition pharmaceutique comprenant un composé selon la revendication 4 ou la revendication 14, ou un ester, sel ou éther acceptable en pharmacie d'un tel composé, et un véhicule acceptable en pharmacie.

35. Composition pharmaceutique selon la revendication 34, dans laquelle ledit composé est présent en une quantité comprise entre 0,01 et 100 milligrammes.

36. Composition pharmaceutique selon la revendication 35, convenant pour une administration par voie parentérale, orale, sous-cutanée, intraveineuse, intramusculaire, intrapéritonéale, transdermique, buccale ou oculaire.

37. Composition pharmaceutique convenant à une administration par voie orale selon la revendication 36, dans laquelle ledit composé est présent en une quantité comprise entre 25 milligrammes et 100 milligrammes.

38. Composition pharmaceutique convenant à une administration par voie parentérale selon la revendication 34, dans laquelle ledit composé est présent en une quantité comprise entre 0,5 milligramme et 10 milligrammes.

39. Procédé pour former un composé de formule I telle que définie dans la revendication 1, ledit procédé comprenant les étapes consistant à :
(a) ajouter un acide de Lewis à une suspension de chlorure d'ammonium anhydre dans un solvant aprotique sous agitation dans une atmosphère inerte à une température proche de 0°C pour former un mélange ;
(b) laisser ledit mélange revenir à la température ambiante sous agitation et ensuite agiter ledit mélange jusqu'à ce que pratiquement tout le solide soit dissous ;
(c) ajouter audit un mélange un composé de formule V : 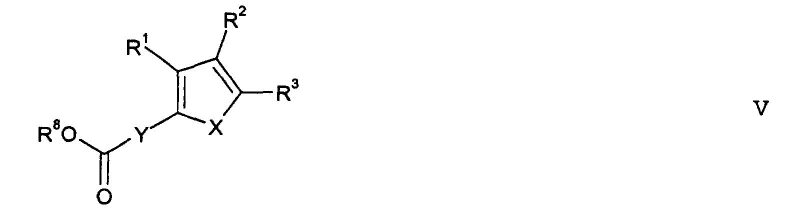 dans laquelle R¹-R³, X et Y sont tels que définis dans la revendication 1 ; et
R⁸ est choisi parmi alkyle et aryle ;
(d) chauffer ledit mélange au reflux pendant une période de temps prédéterminée et ensuite laisser ledit mélange revenir à la température ambiante.

40. Procédé selon la revendication 39, dans lequel ledit acide de Lewis est le triméthyl-aluminium ou le triéthyl-aluminium.

41. Procédé selon la revendication 39, dans lequel ledit solvant aprotique est choisi parmi le toluène, le benzène, le xylène et le mésitylène.

42. Procédé selon la revendication 39, dans lequel ladite atmosphère inerte est créée par mise en oeuvre dudit procédé dans un gaz choisi parmi l'azo et l'argon.

43. Composé de formule III : 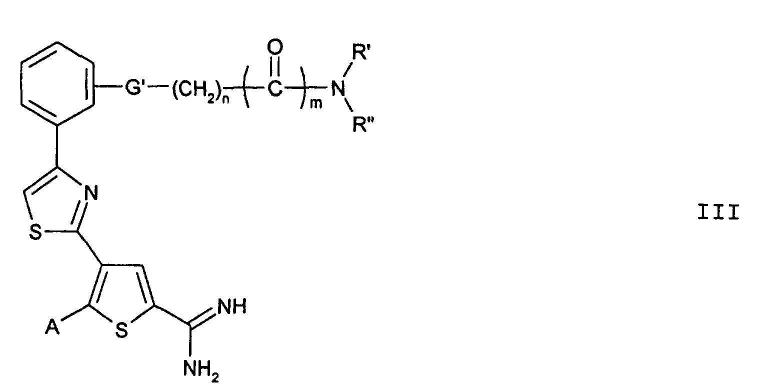 ou un sel d'un tel composé, formule dans laquelle :
A est méthylthio ou méthyle ;
G¹ est -O-, -S-, -NH- ou une liaison covalente ;
n est un entier de 1 à 10 ;
m est un entier de 0 à 1 ; et
R' et R" sont indépendamment choisis parmi hydrogène, alkyle, aryle et aralkyle, ou bien R' et R", pris ensemble avec l'atome N auquel ils sont rattachés, forment un hétérocycle à 3 à 8 chaînons contenant éventuellement un atome O, N ou S additionnel,
et dans laquelle, sauf mention contraire, alkyle représente un groupe alkyle à chaîne droite ou ramifiée ayant jusqu'à 12 atomes de carbone ; aryle représente un groupe aromatique monocyclique ou bicyclique contenant de 6 à 14 carbones dans la partie cyclique ; et aralkyle représente des groupes en C₁ à C₆ ayant un substituant aryle.

44. Composé selon la revendication 43, dans lequel ledit hétérocycle à 3 à 8 chaînons contient un atome N additionnel, ledit atome N additionnel étant éventuellement substitué par hydrogène, alkyle en C₁ à C₄, aryle en C₆ à C₁₀, (aryl en C₆ en C₁₀)alkyle en C₁-C₄, alcoxy en C₁ à C₆, alcoxycarbonyle ou benzyloxycarbonyle.

45. Composé selon la revendication 43, dans lequel ledit hétérocycle à 3 à 8 chaînons est pipérazinyle, pyrrolidinyle, pipéridinyle ou morpholinyle, qui est en outre éventuellement substitué par 1 à 4 substituants choisis parmi halogène, hydroxy, amino, monoalkylamino, dialkylamino, formylamino, acylamino, aminoacyle, monoalkylaminocarbonyle, dialkylaminocarbonyle, thiocarbonylamino, thioacylamino, aminothiocarbonyle, alcoxy, aryloxy, aminocarbonyloxy, monoalkylaminocarbonyloxy, dialkylaminocarbonyloxy, monoarylaminocarbonyloxy, diarylaminocarbonyloxy, monoaralkylaminocarbonyloxy, diaralkylaminocarbonyloxy, alkylsulfonyle, arylsulfonyle, aralkylsulfonyle, alkylsulfonylamino, arylsulfonylamino, aralkylsulfonylamino, alcoxycarbonylamino, aralcoxycarbonylamino, aryloxycarbonylamino, monoalkylaminothiocarbonyle, dialkylaminothiocarbonyle, aralcoxy, carboxy, carboxyalkyle, alcoxycarbonyle, alcoxycarbonylalkyle, nitro, cyano, trifluorométhyle, alkylthio et arylthio.

46. Composé de formule IV : 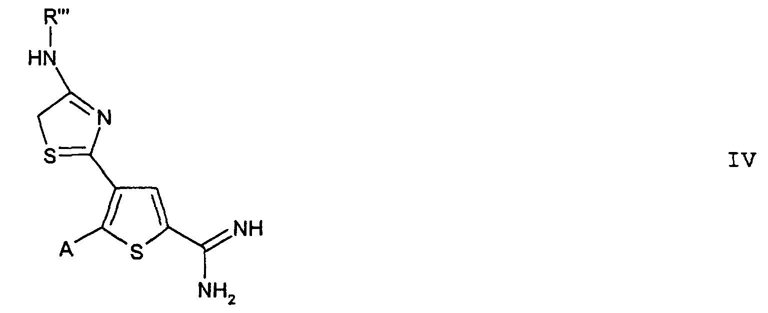 dans laquelle :
A est méthylthio ou méthyle ; et
R"' est hydrogène, aryle en C₆ à C₁₄, alkyle en C₁ à C₆, alcoxy-aryle en C₆ à C₁₄, aminoaryle en C₆ à C₁₄, monoalkylamino-aryle en C₆ à C₁₄, dialkylamino-aryle en C₆ à C₁₄, (aryl en C₆ à C₁₀) alkyle en C₁ à C₆, (alkyl en C₁ à C₆) -aryle en C₆ à C₁₄, aminoalkyle en C₁ à C₆, monoalkylamino-alkyle en C₁ à C₆, dialkylamino-alkyle en C₁ à C₆, hydroxyaryle en C₆ à C₁₄, ou hydroxyalkyle en C₁ à C₆, l'un quelconque d'entre eux étant en outre éventuellement substitué par 1 à 4 substituants non hydrogène choisis parmi halogène, hydroxy, amino, monoalkylamino, dialkylamino, formylamino, acylamino, aminoacyle, mono- ou di-alkylaminocarbonyle, thiocarbonylamino, thioacylamino, aminothiocarbonyle, alcoxy, aryloxy, aminocarbonyloxy, mono- ou di-alkylaminocarbonyloxy, mono- ou diarylaminocarbonyloxy, mono- ou di-aralkylaminocarbonyloxy, alkylsulfonyle, arylsulfonyle, aralkylsulfonyle, alkylsulfonylamino, arylsulfonylamino, aralkylsulfonylamino, alcoxycarbonylamino, aralcoxycarbonylamino, aryloxycarbonylamino, mono- ou dialkylaminothiocarbonyle, aralcoxy, carboxy, carboxyalkyle, alcoxycarbonyle, alcoxycarbonylalkyle, nitro, cyano, trifluorométhyle, alkylthio et arylthio,
où, sauf mention contraire, alkyle représente un groupe alkyle à chaîne droite ou ramifiée ayant jusqu'à 12 atomes de carbone ; alkylthio représente un groupe alkyle à chaîne droite ou ramifiée lié à un atome de soufre ; alcoxy représente un groupe alcoxy à chaîne droite ou ramifiée ayant jusqu'à 20 atomes de carbone, lié à un atome d'oxygène ; aralkyle et arylalkyle représentent des groupes en C₁ à C₆ ayant un substituant aryle ; acyle représente le groupe -C(O)R^{g} où R^{g} est alkyle, alcényle, alcynyle, aryle ou aralkyle ; thioacyle représente le groupe -C(S)=R^{g} où R^{g} est alkyle, alcényle, alcynyle, aryle ou aralkyle ; monoalkylamino représente un groupe amino substitué par un seul groupe alkyle ayant jusqu'à 6 atomes de carbone ; dialkylamino représente un groupe amino substitué par deux groupes alkyle ayant jusqu'à 6 atomes de carbone ; alcényle représente un groupe alcényle à chaîne droite ou ramifiée ayant de 2 à 20 atomes de carbone ; et alcynyle représente un groupe alcynyle à chaîne droite ou ramifiée ayant de 2 à 20 atomes de carbone.

47. Composé selon la revendication 46, qui est l'un des suivants : 4-{2-[(3-méthoxyphényl)amino] (1,3-thiazol-4-yl)}-5-méthylthiothiophène-2-carboxamidine, 4-{2-[(4-méthoxyphényl)amino](1,3-thiazol-4-yl)}-5-méthylthiothiophène-2-carboxamidine, 4-(2-{[4-(diméthylamino)phényl]amino}(1,3-thiazol-4-yl))-5-méthylthiothiophène-2-carboxamidine, 4-{2-[(4-chloro-2-méthylphényl)amino](1,3-thiazol-4-yl)}-5-méthylthiothiophène-2-carboxamidine, 4-{2-[(diphénylméthyl)amino](1,3-thiazol-4-yl)}-5-méthylthiothiophène-2-carboxamidine, 5-méthylthio-4-{2-[(3-phénylpropyl)amino](1,3-thiazol-4-yl)}thiophène-2-carboxamidine, 5-méthylthio-4-{2-[(2,4,5-triméthylphényl)amino](1,3-thiazol-4-yl)}thiophène-2-carboxamidine, 4-{2-[(2-fluorophényl)amino](1,3-thiazol-4-yl)}-5-méthylthiothiophène-2-carboxamidine, 4-{2-[(3-chloro-2-méthylphényl)amino](1,3-thiazol-4-yl)}-5-méthylthiothiophène-2-carboxamidine, 4-(2-{[2-(méthyléthyl)phényl]amino}(1,3-thiazol-4-yl))-5-méthylthiothiophène-2-carboxamidine, 5-méthylthio-4-(2-{[4-(phénylméthoxy)phényl]amino}(1,3-thiazol-4-yl))thiophène-2-carboxamidine, 4-{2-[(2-bromophényl)amino](1,3-thiazol-4-yl)}-5-méthylthiothiophène-2-carboxamidine, 4-{2-[(2,6-dichlorophényl)amino](1,3-thiazol-4-yl)}-5-méthylthiothiophène-2-carboxamidine, 4-{2-[{2-bromo-4-méthylphényl)amino](1,3-thiazol-4-yl)}-5-méthylthiothiophène-2-carboxamidine, 5-méthylthio-4-{2-[(2-morpholin-4-yléthyl)amino](1,3-thiazol-4-yl)}thiophène-2-carboxamidine, 4-{2-[(2,3-dichlorophényl)amino](1,3-thiazol-4-yl)}-5-méthylthiothiophène-2-carboxamidine, 5-méthylthio-4-{2-[(3,4,5-triméthoxyphényl)amino](1,3-thiazol-4-yl)}thiophène-2-carboxamidine, 5-méthylthio-4-{2-[(2-pipéridyléthyl)amino](1,3-thiazol-4-yl)}thiophène-2-carboxamidine, 4-(2-{[(4-méthylphényl)méthyl]amino}(1,3-thiazol-4-yl))-5-méthylthiothiophène-2-carboxamidine, 4-(2-{[4-(4-chlorophénoxy)phényl]amino}(1,3-thiazol-4-yl)) - 5-méthylthiothiophène-2-carboxamidine, 4-(2-{[4-phénoxyphényl]amino}(1,3-thiazol-4-yl))-5-méthylthiothiophène-2-carboxamidine, 5-méthylthio-4-(2-{[4-(phénylamino)phényl]amino(1,3-thiazol-4-yl))thiophène-2-carboxamidine, 5-méthylthio-4-(2-{[4-benzylphényl]amino}(1,3-thiazol-4-yl))thiophène-2-carboxamidine, 5-méthylthio-4-(2-{[4-(pipéridylsulfonyl)phényl]amino}(1,3-thiazol-4-yl))thiophène-2-carboxamidine, 5-méthylthio-4-[2-(3-quinolylamino)(1,3-thiazol-4-yl)]thiophène-2-carboxamidine, 5-méthylthio-4-[2-(2-naphtylamino)(1,3-thiazol-4-yl)]thiophène-2-carboxamidine, 4-[2-(2H-benzo[3,4-d]1,3-dioxolan-5-ylamino)(1,3-thiazol-4-yl)]-5-méthylthiothiophène-2-carboxamidine, 4-{2-[(7-bromofluorén-2-yl)amino](1,3-thiazol-4-yl)}-5-méthylthiothiophène-2-carboxamidine, 4-{2-[(4-cyclohexylphényl)amino](1,3-thiazol-4-yl)}-5-méthylthiothiophène-2-carboxamidine, 5-méthylthio-4-(2-{[4-(phényldiazényl)phényl]amino}(1,3-thiazol-4-yl))thiophène-2-carboxamidine, 5-méthylthio-4-(2-{[3-(hydroxyméthyl)phényl]amino}(1,3-thiazol-4-yl))thiophène-2-carboxamidine, 4-[2-({3-[(3-méthylpipéridyl)méthyl)phényl}amino)(1,3-thiazol-4-yl)-5-méthylthiothiophène-2-carboxamidine, 4-{2-[(3-hydroxyphényl)amino](1,3-thiazol-4-yl)}-5-méthylthiothiophène-2-carboxamidine, 4-(2-{[4-(carbamoylméthoxy)phényl]amino}(1,3-thiazol-4-yl))-5-méthylthiothiophène-2-carboxamidine, 5-méthyl-4-{2-[(3,4,5-triméthoxyphényl)amino] (1,3-thiazol-4-yl)}thiophène-2-carboxamidine, 5-méthyl-4-{2-[(4-phénoxyphényl)amino] (1,3-thiazol-4-yl)}thiophène-2-carboxamidine, 5-méthyl-4-[2-(phénylamino)(1,3-thiazol-4-yl)]thiophène-2-carboxamidine, 4-(4-isoxazol-5-yl(1,3-thiazol-2-yl))-5-méthylthiothiophène-2-carboxamidine.
